# EUROPEAN PATENT APPLICATION

(11) **EP 4 538 263 A1**
(43) Date of publication of application: **16.04.2025**
(21) Application number: 23807086.6
(22) Date of filing: 22.05.2023
(51) Int. Cl.: C07D 401/06, C07D 401/14, C07D 417/14, C07D 413/14, C07D 491/107, C07D 471/04, C07D 405/14, A61K 31/4725, A61K 31/5377, A61K 31/496, A61K 31/475, A61K 31/506, A61K 31/501, A61P 3/10, A61P 1/10, A61P 1/14, A61P 1/16

(54) **NOVEL TETRAHYDROISOQUINOLINE COMPOUND, PREPARATION METHOD THEREFOR, PHARMACEUTICAL COMPOSITION CONTAINING SAID COMPOUND, AND USE THEREOF**

(30) Priority: 20.05.2022 CN 202210557284
(71) Applicant: Shanghai Institute of Materia Medica, Chinese Academy of Sciences, Shanghai 201203 (CN)
(72) Inventor: LIU, Hong, Shanghai 201203 (CN); YANG, Dehua, Shanghai 201203 (CN); WANG, Jiang, Shanghai 201203 (CN); CHEN, Yan, Shanghai 201203 (CN); LI, Chenghao, Shanghai 201203 (CN); CAI, Xiaoqing, Shanghai 201203 (CN); ZHANG, Rui, Shanghai 201203 (CN); WANG, Mingwei, Shanghai 201203 (CN); CHEN, Kaixian, Shanghai 201203 (CN); SHANG, Aolong, Shanghai 201203 (CN); JIANG, Hualiang, deceased (CN)
(74) Representative: Mewburn Ellis LLP
(86) International application number: PCT/CN2023/095615
(87) International publication number: WO 2023/222137

(57) **Abstract**

Provided are a novel tetrahydroisoquinoline compound, a preparation method therefor, a pharmaceutical composition containing said compound, and a use thereof. Specifically, provided are a nitrogen-containing heterocyclic compound as represented by general formula (I), a pharmaceutically acceptable salt, an enantiomer, a diastereoisomer, or a racemate thereof. The compound may be used for preparing a pharmaceutical composition for treating a disease or condition related to the activity or expression levels of relaxin family receptor 4.

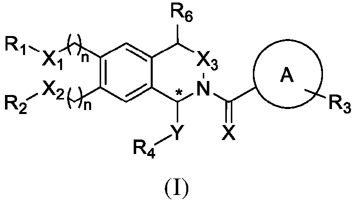

## Description

### Technical field

The present invention relates to novel nitrogen-containing heterocyclic compounds, preparation method therefor, pharmaceutical composition containing said compound, and use thereof, belonging to the field of pharmaceutical technology. Related to a novel nitrogen-containing heterocyclic compound of formula (I), a pharmaceutically acceptable salt, a isomer, a solvate, a metabolite, a metabolic precursor, a pharmaceutical composition containing them, and a use of the compound in the prevention and treatment of constipation, anorexia, diabetes, nonalcoholic steatohepatitis and other disease or disorder related to RXFP4 activation.

### Background

The human relaxin (R)/insulin (INS) superfamily includes INS, insulin-like growth factor (IGF) 1, IGF2, R1, R2, INSL3, INSL4, INSL5, INSL6, and INSL7 (R3). Relaxin and related hormone peptides have multiple functions and are involved in various physiological and pathological processes, including reproduction, neural signal transduction, wound healing, collagen metabolism, and tumor development. Insulin like peptide5(INSL5) is a member of the relaxin/insulin superfamily, consisting of a signal peptide, A chain, B chain, and a connecting C chain. The A chain and B chain are connected by two disulfide bonds, and there is also one disulfide bond inside the A chain. After the hydrolysis and removal of the C-peptide chain under the action of enzymes, INSL5 can be activated to exert its biological activity. The Northern blot detection results showed that INSL5 was expressed in the human uterus and digestive tract, with the highest expression in the rectum and also in the ascending and descending colon; quantitative reverse transcription polymerase chain reaction (RT-PCR) detection revealed that human INSL5 is present in various peripheral tissues, especially in the colon, rectum, uterus, and also in the human brain, mainly in the pituitary gland.

The relaxin family peptides generate further biological signal transduction by binding to G protein coupled receptors. At present, four types of relaxin receptors have been discovered, which are named RXFP 1-4. In 2003, Liu first discovered RXFP4 in the human genome database, originally named GPR100, and initially described as a bradykinin receptor. In 2005, a study found that GPR100 is a receptor for INSL5, so it was renamed RXFP4, also known as G protein coupled receptor 142 (GPCR142). INSL5 is a specific agonist of RXFP4, human RXFP4 is composed of 374 amino acids and encoded by an independent exon, it belongs to the A-class neuropeptide like G protein coupled receptor (rhodopsin like receptor) and is composed of a short N-terminus in the extracellular domain, 7 α - helix transmembrane domains, and an intracellular C-terminus. After INSL5 binds to RXFP4, the conformation of RXFP4 coupled Gi protein will change, and the α subunit is activated, inducing Gi α - guanosine diphosphate (GDP) to exchange with guanosine triphosphate (GTP) to generate Gi α - GTP; Subsequently, Gi α - GTP separates from the β and γ subunits and moves to adjacent adenylate cyclase (AC), inhibiting AC and thus suppressing the production of intracellular cyclic adenosine monophosphate (cAMP), resulting in a decrease in cAMP concentration in tissues expressing RXFP4. Northern blot analysis showed that human RXFP4 is expressed in the heart, skeletal muscle, kidneys, liver, and placenta, with the highest expression in the pancreas; RT-PCR analysis revealed that human RXFP4 is expressed in the heart, skeletal muscle, kidneys, liver, and placenta, as well as in the colon, thyroid, salivary glands, prostate, thymus, testes, and brain of humans; Western blot analysis revealed that human RXFP4 is expressed in the hypothalamus, pituitary gland, testes, epididymis, ovaries, uterus, pancreas, and liver; Immunohistochemistry detection revealed that human RXFP4 is expressed in pancreatic islet B cells, as well as in the pituitary gland, testes, and ovaries. RXFP4 is only expressed in some species, rat and dog RXFP4 being pseudogenes, and mouse RXFP4 being less conserved than human RXFP4. Further research on monkey, cow, and pig RXFP4 confirms that these species have high homology with human RXFP4, and their in vitro expression is similar to that of human receptors.

Appetite regulation is one of the main concerns in the current medical field. Gastrointestinal hormones have multiple physiological functions outside the intestine, playing a key role in food intake behavior, appetite changes, and nutrient metabolism processes. INSL5 is a gastrointestinal hormone secreted by intestinal L cells and is the second discovered appetite stimulating hormone after ghrelin. INSL5 and RXFP4 are mainly expressed and distributed in the colon. Therefore, compared with the receptor of ghrelin, which is mainly distributed in the pituitary gland, INSL5 and its analogues do not need to pass through the blood-brain barrier and can directly exert physiological functions by affecting the enteric nervous system. This also makes its receptor RXFP4 a potential therapeutic target.

Constipation refers to the condition where the stool is too hard or too dry, making it difficult to pass or defecate smoothly. Constipation usually has many causes, including slow movement of the colon with feces, irritable bowel syndrome, or bone cavity diseases. Related underlying diseases include hypothyroidism, diarrhea, Parkinson's disease, colon cancer, diverticulitis, inflammatory bowel disease, etc. Recent studies have shown that RXFP4 agonists can promote intestinal peristalsis and significantly reduce defecation time in constipated mouse models, suggesting that the discovery of RXFP4 agonists may provide a new safe and effective drug treatment for constipation.

In summary, RXFP4 target is closely related to important physiological functions of the human body such as appetite regulation and intestinal peristalsis. The design and discovery of related agonists can provide strong support for future research on the INSL5-RXFP4 system and may also provide a new class of drugs for treating metabolic related diseases.

### Summary

One purpose of the present invention is to provide a nitrogen-containing heterocyclic compound of formula (I), or a pharmaceutically acceptable salt, a enantiomer, a diastereomer, or a racemate thereof.

Another purpose of the present invention is to provide a method for preparing the compound of formula (I).

Another purpose of the present invention is to provide a pharmaceutical composition comprising a therapeutic effective amount of one or more compounds of formula (I) or their pharmaceutically acceptable salts.

Another purpose of the present invention is to provide a use of the compound of formula (I) in the preparation of drugs for treating constipation, anorexia, diabetes, nonalcoholic steatohepatitis and other glycolipid metabolic diseases.

The compounds of the present invention can be used to activate relaxin family peptide receptor 4 (RXFP4).

The first aspect of the present invention provided a nitrogen-containing heterocyclic compound of formula (I), or a pharmaceutically acceptable salt, a enantiomer, a diastereomer, or a racemate thereof: 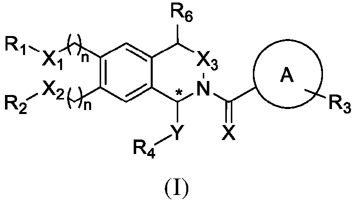 wherein,
the chiral carbon atom C* is independently a *S-configuration,* a *R-configuration,* a racemate, or a combination thereof;
n=0, 1, or 2;
X₁ and X₂ are independently selected from the group consisting of: O and NH;
X₃ is a chemical bond, CHR₆ or (CHR₆) 2;
R₁ and R₂ are independently selected from the group consisting of: hydrogen, deuterium, tritium, halogen, hydroxyl, carboxyl, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted C6-C10 aryl, substituted or unsubstituted 5-7 membered heterocyclyl, substituted or unsubstituted C1-C6 alkylphenyl, substituted or unsubstituted C1-C6 alkyl 5-7 membered heteroaryl, substituted or unsubstituted C3-C12 cycloalkyl, substituted or unsubstituted C2-C10 acyl, substituted or unsubstituted C2-C10 ester group, amino, substituted or unsubstituted C1-C6 alkylamine, substituted or unsubstituted C1-C6 amido, -SOR₅, -SOR₅, -OCOR₅, C(=NH)NH₂, Fmoc, Alloc;
and when n=0, R₁ and R₂ are not methyl at the same time; when n=1, R₁ and R₂ are not hydrogen at the same time;
or R₁ and R₂together with the adjacent (CH₂) ₙO and C=C form a substituted or unsubstituted 5-7 membered heterocyclyl, wherein the heterocycle is a fully saturated heterocycle, a partially unsaturated heterocycle, or an aromatic heterocycle;
X is selected from the group consisting of: O, S, CHR₆, C₂H₄ (i.e., forming a cyclopropyl at the X substitution position) and NR₆; wherein R₆ is selected from the group consisting of: H, CN, C1-C6 alkyl and C1-C6 alkoxy;
Y is a linking group selected from the group consisting of: a chemical bond, C1-C6 straight or branched alkyl, -CH₂NH-, C2-C6 straight or branched alkenyl, -CH₂O-, -CH₂S-, -CONH-, -NHCO-, -COO-, 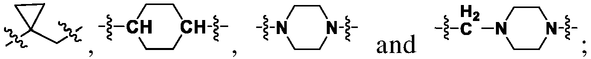
ring A is a substituted or unsubstituted group selected from the group consisting of: a 5-12 membered nitrogen-containing heterocycle (including monocyclic, fused polycyclic, bridged or spirocyclic groups, wherein the connecting site preferably located on the nitrogen atom), a C6-C12 aryl (preferably C6-C10 aryl), and a 5-12 membered heteroaryl (preferably a 5-7 membered heteroaryl); wherein the heterocyclyl or heteroaryl includes heteroatoms selected from the group consisting of N,NH,S,O andS(O)₂ as the ring skeleton;
R₄ is selected from the following groups that are unsubstituted or substituted with 1-3 substituents: C3-C7 alkyl, 5-12 membered heterocyclyl,C6-C12 aryl, 5-12 membered heteroaryl (preferably 5-7 membered heteroaryl, or benzo 5-7 membered heteroaryl); wherein, each of the heterocycle or heteroaryl contains 1-3 heteroatoms selected from oxygen, sulfur, and nitrogen; each substituent is independently selected from halogen, C1 - C6 straight or branched alkyl, C2-C6 straight or branched alkenyl, C2-C6 straight or branched alkynyl, C1-C6 straight or branched alkoxy,C1-C6 straight or branched alkylcarbonyloxy, cyano, nitro, hydroxyl, amino, hydroxymethyl, trifluoromethyl, trifluoromethoxy, carboxyl, thiol, C1-C4 acyl, amido, sulfonyl, aminosulfonyl, C1-C4 alkyl substituted sulfonyl, or two substituents located on adjacent ring atoms together with the attached carbon atom form a 5-7 membered ring;
R₃ and R₅ are each independently selected from the group consisting of: hydrogen, deuterium, tritium, halogen, unsubstituted or 1-3 halogens substituted C1-C6 alkyl, unsubstituted or 1-3 halogens substituted C3-C6 cycloalkyl, unsubstituted or 1-3 halogens substituted C6-C10 aryl, unsubstituted or 1-3 halogens substituted C1-C3 alkyl-C6-C10 aryl, and unsubstituted or 1-3 halogens substituted 5-7 membered heteroaryl;
unless otherwise specified, the term "substituted" refers to one or more hydrogen atoms on the group are substituted by substituent selected from the group consisting of: C1-C10 alkyl, C1-C10 alkoxy, C3-C10 cycloalkyl, C1-C10 alkoxy, 5-12 membered heterocyclyl, halogen, hydroxyl, carboxyl (-COOH), C1-C10 aldehyde, C2-C10 acyl, C2-C10 ester group, cyano, amino, and phenyl; wherein the phenyl includes an unsubstituted phenyl or a substituted phenyl group with 1-3 substituents, wherein the substituents are selected from the group consisting of halogen, C1-C10 alkyl, cyano, hydroxyl, nitro, C3-C10 alkyl, C1-C10 alkoxy and amino;
and the compound is not a structure selected from the group consisting of: 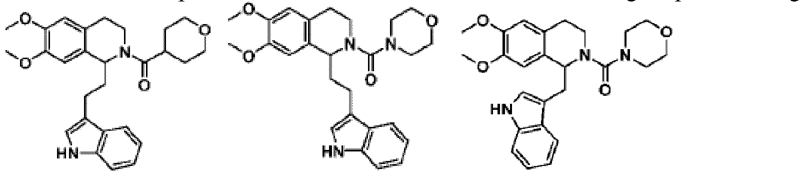 and

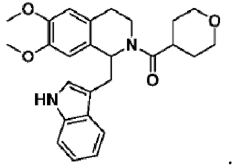

In another preferred embodiment, the compound has a structure as shown in the following formula: 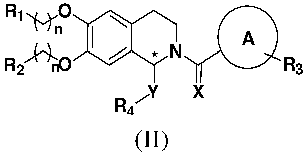 wherein,
The chiral carbon atom C* is independently a *S*-configuration, a *R*-configuration, racemate, or a combination thereof;
n=0, 1, or 2;
R₁ and R₂ are independently selected from the group consisting of: hydrogen, deuterium, tritium, halogen, hydroxyl, carboxyl, substituted or unsubstituted C1-C6 alkyl, substituted or unsubstituted C1-C6 alkoxy, substituted or unsubstituted C6-C10 aryl, substituted or unsubstituted 5-7 membered heterocycle, substituted or unsubstituted C1-C6 alkylphenyl, substituted or unsubstituted C1-C6 alkyl 5-7 membered heteroaromatic, substituted or unsubstituted C3-C12 cycloalkyl, substituted or unsubstituted C2-C10 acyl, substituted or unsubstituted C2-C10 ester group, amino, substituted or unsubstituted C1-C6 alkylamine, substituted or unsubstituted C1-C6 amido, - OR₅, - OSOR₅ and - OCOR₅;
and when n=0, R₁ and R₂ are not methyl at the same time; when n=1, R₁ and R₂ are not hydrogen at the same time;
or R₁ and R₂ together with the adjacent (CH₂) ₙO and C=C form a substituted or unsubstituted 5-7 membered heterocycle, wherein the heterocycle is a fully saturated heterocycle, a partially unsaturated heterocycle, or an aromatic heterocycle;
X is O or S;
Y is a linking group selected from the group consisting of chemical bond, C1-C6 straight or branched alkyl, - CH₂NH -, C2-C6 straight or branched alkenyl, -CH₂O-, -CH₂S-, -CONH-, -NHCO-, -COO- 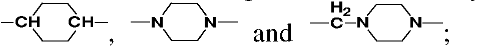
ring A is a substituted or unsubstituted group selected from the group consisting of: 5-12 membered nitrogen-containing heterocyclyl (with the connecting site preferably located on the nitrogen atom), C6-C12 aryl (preferably C6-C10 aryl), 5-12 membered heteroaryl (preferably 5-7 membered heteroaryl); wherein the heterocyclyl or heteroaryl includes heteroatoms selected from the group consisting of N, NH, S, O and S(O)₂ as the ring skeleton;
R₄ is selected from the following groups that are unsubstituted or substituted with 1-3 substituents: C3-C7 cycloalkyl, 5-12 heterocyclyl, C6-C12 aryl, 5-12 heteroaryl (preferably 5-7 heteroaryl, or benzo5-7 heteroaryl); wherein each of the heterocyclyl or heteroaryl contains 1-3 heteroatoms selected from oxygen, sulfur, and nitrogen; the substituents are independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, trifluoromethoxy, carboxyl, thiol, C1-C4 acyl, amido, sulfonyl, aminosulfonyl, C1-C4 alkyl substituted sulfonyl, or two substituents located on adjacent ring atoms together with the attached carbon atoms form a 5-7 membered ring;
R₃ and R₅ are independently selected from the group consisting of: hydrogen, deuterium, tritium, halogen, unsubstituted or 1-3 halogens substituted C1-C6 alkyl, or unsubstituted or 1-3 halogens substituted C3-C6 cycloalkyl, unsubstituted or 1-3 halogens substituted C6-C10 aryl, unsubstituted or 1-3 halogens substituted C1-C3 alkyl-C6-C10 aryl, unsubstituted or 1-3 halogens substituted 5-7 membered heteroaryl.

In another preferred embodiment, ring A is selected from the group consisting of: aziridinyl, azetidinyl, pyrrolidinyl, piperidinyl, azepanyl, morpholinyl, piperazinyl, homopiperazinyl, thiomorpholinyl, thiomorpholinyl in which the cyclic sulfur is substituted by sulfoxide or sulfone, imidazolidinyl, pyrazinyl, hexahydropyrimidinyl and 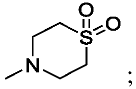 ring A can be optionally substituted by 1-2 groups selected from hydrogen, C1-C3 straight or branched alkyl, halogen, hydroxyl, and C1-C4 alkoxycarbonyl.

In another preferred embodiment, ring A is selected from the group consisting of: 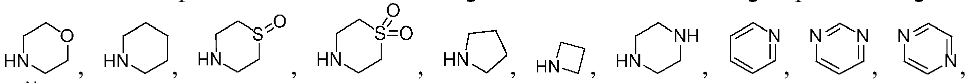 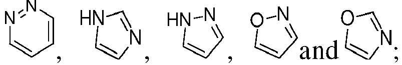 and ring A can be optionally substituted with 1-2 groups selected from hydrogen, C1-C3 straight or branched alkyl, halogen, hydroxyl, and C1-C4 alkoxycarbonyl.

In another preferred embodiment, R₁ and R₂ are independently selected from the group consisting of hydrogen, deuterium, tritium, halogen, hydroxyl, carboxyl, phenyl, substituted or unsubstituted C1-C6 alkyl, substituted or unsubstituted C1-C6 alkoxy, substituted or unsubstituted 5-7 membered heterocycle, substituted or unsubstituted C1-C6 alkyl 5-7 membered heteroaryl, substituted or unsubstituted C3-C8 cycloalkyl, substituted or unsubstituted C2-C10 acyl, substituted or unsubstituted C2-C10 ester group, amino, substituted or unsubstituted C1-C6 alkylamine, substituted or unsubstituted C1-C6 amido, - SOR₅, - SOR₅ and - OCOR₅.

In another preferred embodiment, X is O;
Y is selected from the group consisting of: -CH₂-, -CH₂-CH₂-, -CH₂-CH₂-CH₂-, - CH₂NH-, -CH₂O- and -CH₂S-.

In another preferred embodiment, R₄ is selected from the following groups that are unsubstituted or substituted with 1-3 substituents: C6-C10 aryl, 5-7 membered heteroaryl, or benzo 5-7 membered heteroaryl; preferably, the heterocycle and heteroaromatic ring moiety in the group is selected from the groups formed from indole, benzodioxole, isoxazole, pyridine, pyrazole, dihydroimidazopyridine, imidazopyridine, benzothiophene, dihydrobenzodioxolane, quinolone, pyrrole, benzofuran, indazole, benzimidazole, quinoline and 1,3-dioxoindoleline.

In another preferred embodiment, hydrogen, deuterium, tritium, halogen, unsubstituted or 1-3 halogens substituted C1-C6 alkyl, or unsubstituted or 1-3 halogens substituted C3-C6 cycloalkyl.

In another preferred embodiment, the chiral carbon atom C* is S-configuration.

The second aspect of the present invention provided a method for preparing the compound of formula (I) as described in the first aspect of the present invention, comprising the steps of:
(1) Reacting the compound of formula II and the compound of formula I_{c} in an inert solvent in the presence of a condensing agent to obtain the compound of formula I_{d}; Preferably, the condensing agent is EDCI (1-ethyl-(3-dimethylaminopropyl) carbodiimide hydrochloride); 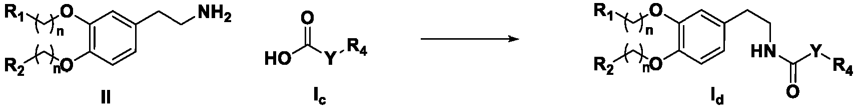
(2) In an inert solvent, the compound of formula I_{d} udgergoes a Bischler= Napieralski cyclization reactionto obtain the compound of formula Iₑ; Preferably, the cyclization reaction uses phosphorus oxychloride as the Lewis acid; 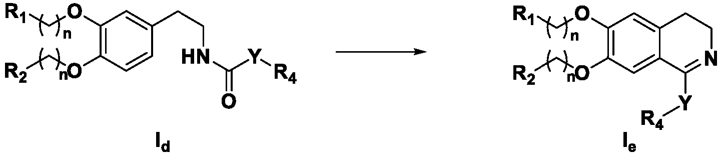
(3) In an inert solvent, the compound of formula Iₑ udgergoes a reduction reaction to obtain the compound of formula I_{f}; Preferably, the reduction reaction uses borohydride as a reducing agent or Noyori catalyst as an asymmetric reduction catalyst; 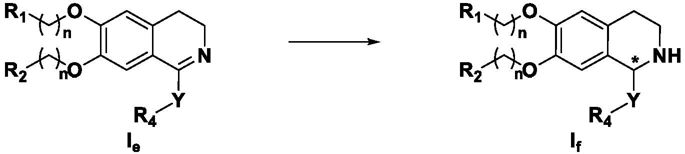
(4) In an inert solvent, the compound of formula I_{f} and 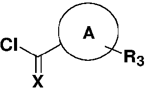 udgergoes a condensation reaction to obtain the compound of formula (I); 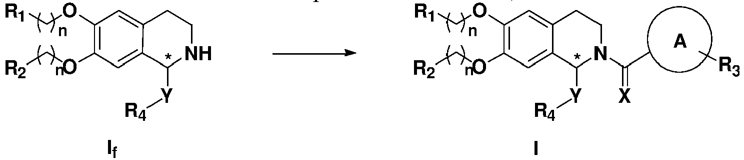
wherein the definitions of the group in each of the above formula are as described in the first aspect of the present invention.

The third aspect of the present invention provides a pharmaceutical composition comprising: a therapeutic effective amount of a compound of formula (I) as described in the first aspect of the present invention, or a pharmaceutically acceptable salt thereof, and a pharmaceutically acceptable carrier.

The fourth aspect of the present invention provides a use of a compound of formula (I) as described in the first aspect of the present invention in the preparation of a pharmaceutical composition for the treatment of diseases or disorders associated with the activity or expression level of relaxin family peptide receptor 4; Preferably, the compound is used in the preparation of a pharmaceutical composition for treating diseases or disorders selected from the group consisting of constipation, anorexia, or glucolipid metabolic diseases.

In another preferred embodiment, the compound is used in the preparation a pharmaceutical composition for treating diseases or disorders caused by low activity or expression level of relaxin family peptide receptor 4.

In another preferred embodiment, the diseases related to glucose and lipid metabolism are selected from the group consisting of: diabetes and nonalcoholic steatohepatitis.

It should be understood that within the scope of the present invention, the above-mentioned technical features of the present invention and the specific technical features described in the following examples can be combined with each other to form new or preferred technical solutions. Due to space limitations, it will not repeated here.

### Figures

Figure 1. The promoting effect of different doses of compound on intestinal peristalsis in constipation model mice. A. Fecal moisture content; B. Fecal weight; C. Number of fecal particles; D. Ball exclusion time;
Figure 2: Pathological changes in colon tissue of constipation model miceimproved by compounds. A. Thickness of colonic muscle layer; B. Thickness of colonic mucosal layer; C. The number of Libeikun's glands; D. Number of goblet cells;
Figure 3. The regulatory effect of compounds on the expression levels of AQP3, TRPV1, and CGRP in mouse colon;
Figure 4. Effects of compounds on serum levels of 5-HT, NO, and VIP in mice.

### Examples

After long-term and in-depth research, the inventor has prepared a class of compounds of formula I that can activate relaxin family peptide receptor 4 (RXFP4). And compared with the type 4 relaxin family receptor (RXFP4) in the prior art, the compound has higher inhibitory activity and selectivity. Based on the abovediscovery, the inventor has completed the present invention.

### term

As used herein, unless otherwise specified, the term "substituted" refers to one or more hydrogen atoms on thegroup are substituted by substituent selected from the group consisting of C₁-C₁₀ alkyl, C₃-C₁₀ cycloalkyl, C₁-C₁₀ alkoxy, halogen, hydroxyl, carboxyl (-COOH), C₁-C₁₀ aldehyde, C₂-C₁₀ acyl, C₂₋C₁₀ ester group, amino, and phenyl; whereinthe phenyl includes an unsubstituted phenyl or a substituted phenyl with 1-3 substituents, wherein the substituents are selected from the group consisting of halogen, C₁-C₁₀ alkyl, cyano, hydroxyl, nitro, C₃-C₁₀ cycloalkyl, C₁-C₁₀ alkoxy, and amino.

Unless otherwise specified, each chiral carbon atom in all compounds of the present invention can optionally be *R* or *S*-configuration, or a mixture of *R* and *S*-configuration.

The term "C1-C6 alkyl" refers to straight or branched alkyl with 1 to 6 carbon atoms, such as methyl, ethyl, propyl, isopropyl, butyl, isobutyl, sec butyl, tert butyl, or similar groups.

The term "3-8-membered heterocycle" refers to a group formed by the loss of one hydrogen atom from a 3-8-membered saturated ring containing 1-3 heteroatoms selected from the group consisting of: N, S and O; For example, pyrrolidinyl, piperidinyl, piperazinyl, morpholinyl, or similar groups.

The term "6-10 membered aryl" refers to a group formed by the loss of one hydrogen atom from a 6-10 membered aryl; For example, phenyl, naphthyl, or similar groups.

The term "5-10 membered heteroaryl" refers to a group formed by the loss of one hydrogen atom from a 5-8 membered aromatic group containing 1-3 heteroatoms selected from the group consisting of: N, S and O, wherein the cyclic system of each heteroaryl can be monocyclic or polycyclic; For example, , pyrrolyl, pyridyl, thienyl, furanyl, imidazolyl, pyrimidinyl, benzothienyl, indolyl, imidazopyridinyl, quinolinyl or similar groups.

The term "C1-C6 alkoxy" refers to straight or branched chain alkoxy with 1-6 carbon atoms, such as methoxy, ethoxy, propoxy, isopropoxy, butoxy, isobutoxy, sec butoxy, tert butoxy, or similar groups.

The term "C2-C6 ester group" refers to an R-O-C (=O) - group with 2-6 carbon atoms, such as - COOCH₃, - COOC₂H₅, - COOC₃H₇, - COOC₄H₉, or similar groups.

The term 'C2-C6 alkenyl' refers to a group formed by the loss of one or two hydrogen atoms from an alkene with 2-6 carbon atoms. The alkene can be a monoalkene, diene, or trialkene, such as - CH=CH₂, - C₂H₄=CH₂, - CH=C₂H₄, or similar groups.

The term 'halogen' refers to F, Cl, Br, and I.

Unless otherwise specified, the structural formulas described in the present invention are intended to include all isomeric forms (such as enantiomers, diastereomers, and geometric isomers (or conformational isomers): for example, containing *R, S* configurations ofasymmetric centers, (Z), (E) isomers of double bonds, and (Z), (E) conformational isomers. Therefore, individual stereoisomers or mixtures of enantiomers, diastereomers, or geometric isomers (or conformational isomers) of the compounds of the present invention are within the scope of the present invention.

The term 'tautomer' refers to structural isomers with different energies that can exceed a low energy barrier and thus transform into each other. For example, proton tautomers (i.e. proton transfer) involve interconversion through proton transfer, such as 1H indazole and 2H indazole, 1H benzo [d] imidazole and 3H benzo [d] imidazole, and valence tautomers involve interconversion through recombination of some bonding electrons.

As used herein, the form 'C1-C6' indicates that the group can have 1 to 6 carbon atoms, such as 1, 2, 3, 4, or 5 carbon atoms.

### Tetrahydroisoquinoline compound of formula (I)

The present invention provides a tetrahydroisoquinoline compound represented by formula (I), comprising enantiomers, diastereomers, racemates, and mixtures thereof, or pharmaceutically acceptable salts thereof, 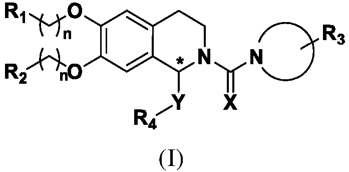 wherein, the definitions of each group are as described above.

In another preferred embodiment, n, X, Y, R₁, R₂, R₃, and R₄ are each independently the corresponding group of the specific compounds in the example. Specifically, the tetrahydroisoquinoline compound described in the present invention are preferably selected from the compounds shown in Table A below:

| number | structure | name |
|---|---|---|
| **A1** | 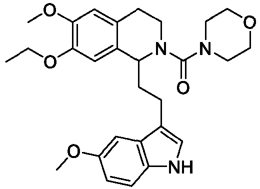 | (7-ethoxy-6-methoxy-1-(2-(5-methoxy-1H-indol-3-yl)ethyl)-3,4-dihydroisoquinolin-2(1H)-yl) (morpholino)methanone |
| **(S)-A1** | 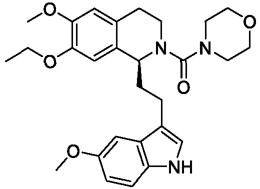 | (S)-(7-ethoxy-6-methoxy-1-(2-(5-methoxy-1H-indol-3-yl)ethyl)-3,4-dihydroisoquinolin-2(1H)-yl) (morpholino)methanone |
| **(R)-A1** | 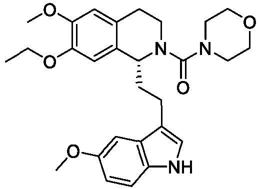 | (R)-(7-ethoxy-6-methoxy-1-(2-(5-methoxy-1H-indol-3-yl)ethyl)-3,4-dihydroisoquinolin-2(1H)-yl) (morpholino)methanone |
| **A2** | 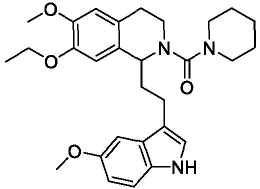 | (7-ethoxy-6-methoxy-1-(2-(5-methoxy-1H-indol-3-yl)ethyl)-3,4-dihydroisoquinolin-2(1H)-yl) (piperidin-1-yl)methanone |
| **A3** | 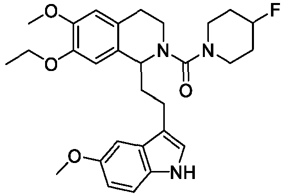 | (4-fluoropiperidin-1-yl)(7-ethoxy-6-methoxy-1-(2-(5-methoxy-1H-indol-3-yl)ethyl)-3,4-dihydroisoquinolin-2(1H)-yl)methanone |
| **A4** | 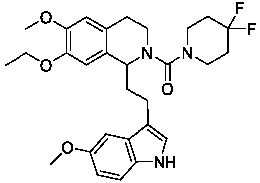 | (4,4-difluoropiperidin-1-yl)(7-ethoxy-6-methoxy-1-(2-(5-methoxy-1H-indol-3-yl)ethyl)-3,4-dihydroisoquinolin-2(1H)-yl)methanone |
| **A5** | 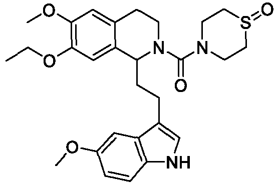 | (7-ethoxy-6-methoxy-1-(2-(5-methoxy-1H-indol-3-yl)ethyl)-3,4-dihydroisoquinolin-2(1H)-yl)(1-oxothiomorpholinyl)methanone |
| **A6** | 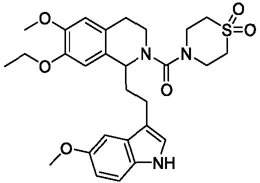 | (1,1-dioxothiomorpholino)(7-ethoxy-6-methoxy-1-(2-(5-methoxy-1H-indol-3-yl)ethyl)-3,4-dihydroisoquinolin-2(1H)-yl) methylmethanone |
| **A7** | 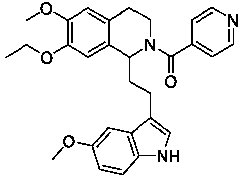 | (7-ethoxy-6-methoxy-1-(2-(5-methoxy-1H-indol-3-yl)ethyl)-3,4-dihydroisoquinolin-2(1H)-yl) (pyridin-4-yl)methanone |
| **A8** | 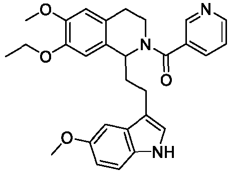 | (7-ethoxy-6-methoxy-1-(2-(5-methoxy-1H-indol-3-yl)ethyl)-3,4-dihydroisoquinolin-2(1H)-yl) (pyridin-3-yl)methanone |
| **A9** | 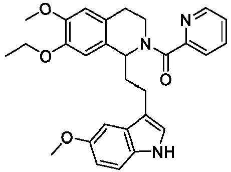 | (7-ethoxy-6-methoxy-1-(2-(5-methoxy-1H-indol-3-yl)ethyl)-3,4-dihydroisoquinolin-2(1H)-yl) (pyridin-2-yl)methanone |
| **A10** | 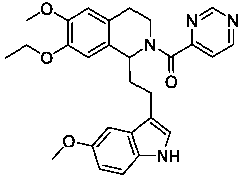 | (7-ethoxy-6-methoxy-1-(2-(5-methoxy-1H-indol-3-yl)ethyl)-3,4-dihydroisoquinolin-2(1H)-yl) (pyrimidin-4-yl)methanone |
| **A11** | 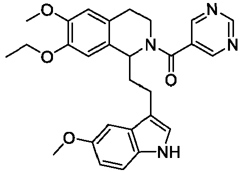 | (7-ethoxy-6-methoxy-1-(2-(5-methoxy-1H-indol-3-yl)ethyl)-3,4-dihydroisoquinolin-2(1H)-yl) (pyrimidin-5-yl)methanone |
| **A12** | 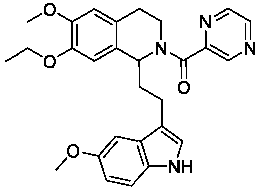 | (7-ethoxy-6-methoxy-1-(2-(5-methoxy-1H-indol-3-yl)ethyl)-3,4-dihydroisoquinolin-2(1H)-yl) (pyrazin-2-yl)methanone |
| **A13** | 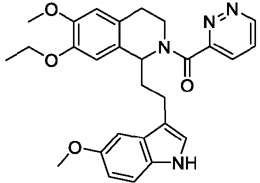 | (7-ethoxy-6-methoxy-1-(2-(5-methoxy-1H-indol-3-yl)ethyl)-3,4-dihydroisoquinolin-2(1H)-yl) (pyridazin-3-yl)methanone |
| **A14** | 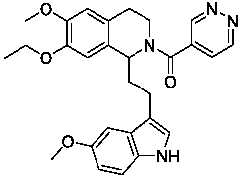 | (7-ethoxy-6-methoxy-1-(2-(5-methoxy-1H-indol-3-yl)ethyl)-3,4-dihydroisoquinolin-2(1H)-yl) (pyridazin-4-yl)methanone |
| **A15** | 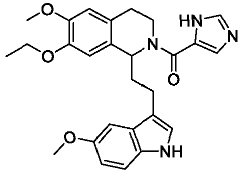 | (7-ethoxy-6-methoxy-1-(2-(5-methoxy-1H-indol-3-yl)ethyl)-3,4-dihydroisoquinolin-2(1H)-yl)(1H-imidazol-5-yl)methanone |
| **A16** | 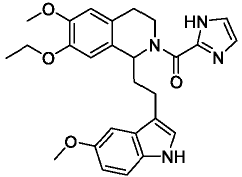 | (7-ethoxy-6-methoxy-1-(2-(5-methoxy-1H-indol-3-yl)ethyl)-3,4-dihydroisoquinolin-2(1H)-yl)(1H-imidazol-2-yl)methanone |
| **A17** | 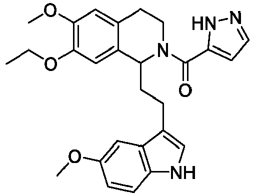 | (7-ethoxy-6-methoxy-1-(2-(5-methoxy-1H-indol-3-yl)ethyl)-3,4-dihydroisoquinolin-2(1H)-yl)(1H-pyrazol-5-yl)methanone |
| **A18** | 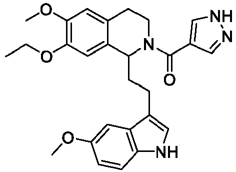 | (7-ethoxy-6-methoxy-1-(2-(5-methoxy-1H-indol-3-yl)ethyl)-3,4-dihydroisoquinolin-2(1H)-yl)(1H-pyrazol-4-yl)methanone |
| **A19** | 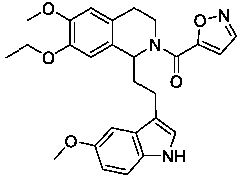 | (7-ethoxy-6-methoxy-1-(2-(5-methoxy-1H-indol-3-yl)ethyl)-3,4-dihydroisoquinolin-2(1H)-yl) (isoxazole-5-yl)methanone |
| **A20** | 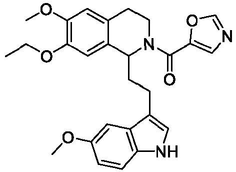 | (7-ethoxy-6-methoxy-1-(2-(5-methoxy-1H-indol-3-yl)ethyl)-3,4-dihydroisoquinolin-2(1H)-yl) (oxazol-2-yl)methanone |
| **A21** | 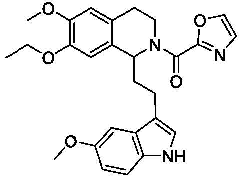 | (7-ethoxy-6-methoxy-1-(2-(5-methoxy-1H-indol-3-yl)ethyl)-3,4-dihydroisoquinolin-2(1H)-yl) (oxazol-2-yl)methanone |
| **A22** | 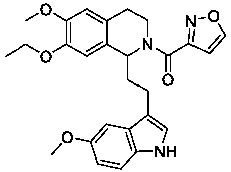 | (7-ethoxy-6-methoxy-1-(2-(5-methoxy-1H-indol-3-yl)ethyl)-3,4-dihydroisoquinolin-2(1H)-yl) (isoxazol-3-yl)methanone |
| **A23** | 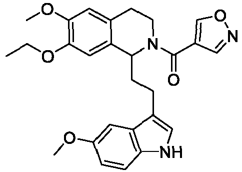 | (7-ethoxy-6-methoxy-1-(2-(5-methoxy-1H-indol-3-yl)ethyl)-3,4-dihydroisoquinolin-2(1H)-yl) (isoxazol-4-yl)methanone |
| **A24** | 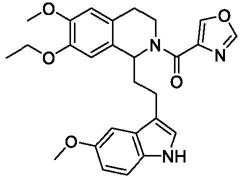 | (7-ethoxy-6-methoxy-1-(2-(5-methoxy-1H-indol-3-yl)ethyl)-3,4-dihydroisoquinolin-2(1H)-yl) (oxazol-4-yl)methanone |
| **A25** | 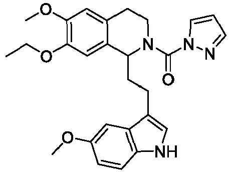 | (7-ethoxy-6-methoxy-1-(2-(5-methoxy-1H-indol-3-yl)ethyl)-3,4-dihydroisoquinolin-2(1H)-yl)(1H-pyrazol-1-yl)methanone |
| **A26** | 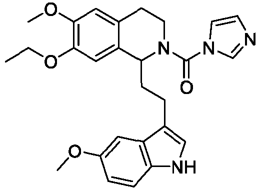 | (7-ethoxy-6-methoxy-1-(2-(5-methoxy-1H-indol-3-yl)ethyl)-3,4-dihydroisoquinolin-2(1H)-yl)(1H-imidazol-1-yl)methanone |
| **A27** | 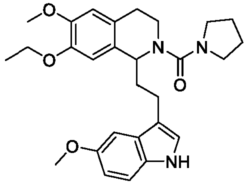 | (7-ethoxy-6-methoxy-1-(2-(5-methoxy-1H-indol-3-yl)ethyl)-3,4-dihydroisoquinolin-2(1H)-yl) (pyrrolidin-1-yl)methanone |
| **A28** | 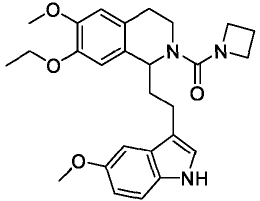 | Nitrogenheterocyclicbut-1-yl(7-ethoxy-6-methoxy-1-(2-(5-methoxy-1H-indol-3-yl)ethyl)-3,4-dihydroisoquinolin-2(1H)-yl)methanone |
| **A29** | 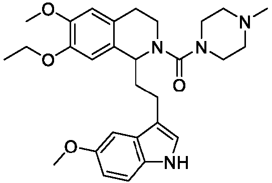 | (7-ethoxy-6-methoxy-1-(2-(5-methoxy-1H-indol-3-yl)ethyl)-3,4-dihydroisoquinolin-2(1H)-yl)(4-methylpiperazin-1-yl)methanone |
| **A30** | 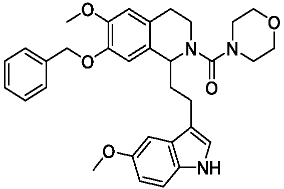 | (7-(benzyloxy)-6-methoxy-1-(2-(5-methoxy-1H-indol-3-yl)ethyl)-3,4-dihydroisoquinolin-2(1H)-yl) (morpholino)methanone |
| **A31** | 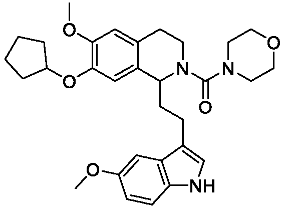 | (7-(Cyclopentaxy)-6-methoxy-1-(2-(5-methoxy-1H-indol-3-yl) ethyl)-3,4-dihydroisoquinolin-2 (1H)-yl)(morpholino)methanone |
| **A32** | 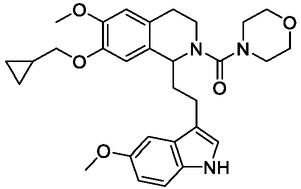 | (7-(Cyclopropylmethoxy)-6-methoxy-1-(2-(5-methoxy-1H-indol-3-yl)ethyl)-3,4-dihydroisoquinolin-2(1H)-yl) (morpholino)methanone |
| **A33** | 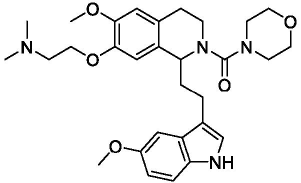 | (7-(2-(dimethylamino)ethoxy)-6-methoxy-1-(2-(5-methoxy-1H-indol-3-yl)ethyl)-3,4-dihydroisoquinolin-2(1H)-yl) (morpholino)methanone |
| **A34** | 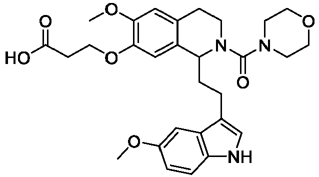 | 3-((6-methoxy-1-(2-(5-methoxy-1H-indol-3-yl)ethyl)-2-(morpholin-4-carbonyl)-1,2,3,4-tetrahydroisoquinolin-7-yl)oxy) propionicacid |
| **A35** | 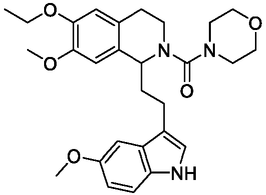 | (6-Ethoxy-7-methoxy-1-(2-(5-methoxy-1H-indol-3-yl)ethyl)-3,4-dihydroisoquinolin-2(1H)-yl) (morpholino)methanone |
| **A36** | 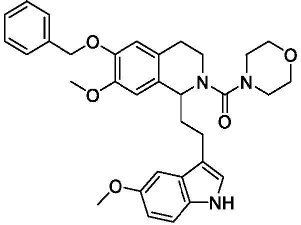 | (6-(benzyloxy)-7-methoxy-1-(2-(5-methoxy-1H-indol-3-yl)ethyl)-3,4-dihydroisoquinolin-2(1H)-yl) (morpholino)methanone |
| **A37** | 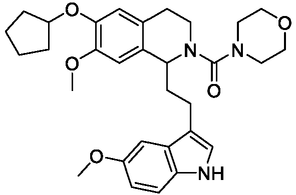 | (6-(Cyclopentaxy)-7-Methoxy-1-(2-(5-methoxy-1H-indol-3-yl) ethyl)-3,4-Dihydroisoquinolin-2 (1H)-yl)(morpholino)methanone |
| **A38** | 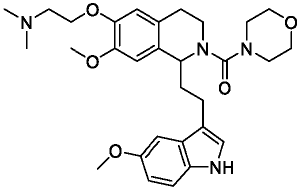 | (6-(2-(dimethylamino)ethoxy)-7-methoxy-1-(2-(5-methoxy-1H-indol-3-yl)ethyl)-3,4-dihydroisoquinolin-2(1H)-yl) (morpholino)methanone |
| **A39** | 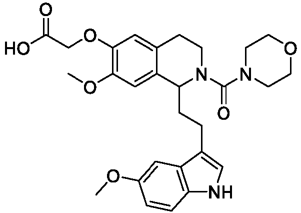 | 2-((7-Methoxy-1-(2-(5-methoxy-1H-indol-3-yl)ethyl)-2-(morpholin-4-carbonyl)-1,2,3,4-tetrahydroisoquinolin-6-yl)oxy) aceticacid |
| **A40** | 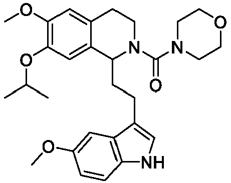 | (7-isopropoxy-6-methoxy-1-(2-(5-methoxy-1H-indol-3-yl)ethyl)-3,4-dihydroisoquinolin-2(1H)-yl) (morpholino)methanone |
| **A41** | 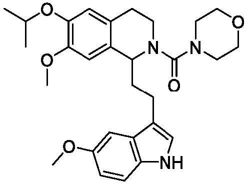 | (6-isopropoxy-7-methoxy-1-(2-(5-methoxy-1H-indol-3-yl)ethyl)-3,4-dihydroisoquinolin-2(1H)-yl) (morpholino)methanone |
| **A42** | 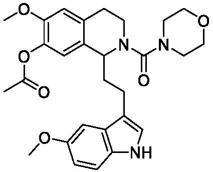 | 6-methoxy-1-(2-(5-methoxy-1H-indol-3-yl)ethyl)-2-(morpholine-4-carbonyl)-1,2,3,4-tetrahydroisoquinoline-7-ylacetate |
| **A43** | 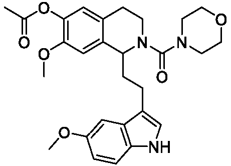 | 7-Methoxy-1-(2-(5-methoxy-1H-indol-3-yl)ethyl)-2-(morpholine-4-carbonyl)-1,2,3,4-tetrahydroisoquinoline-6-ylacetate |
| **A44** | 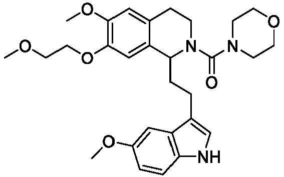 | (6-methoxy-1-(2-(5-methoxy-1H-indol-3-yl)ethyl)-7-(2-methoxyethoxy)-3,4-dihydroisoquinolin-2(1H)-yl) (morpholino)methanone |
| **A45** | 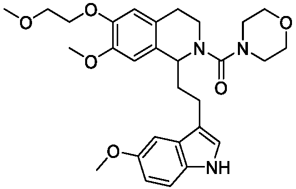 | (7-Methoxy-1-(2-(5-methoxy-1H-indol-3-yl)ethyl)-6-(2-methoxyethoxy)-3,4-dihydroisoquinolin-2(1H)-yl) (morpholino)methanone |
| **A46** | 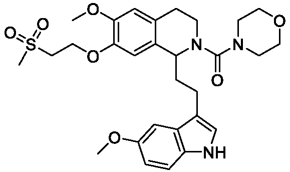 | (6-methoxy-1-(2-(5-methoxy-1H-indol-3-yl)ethyl)-7-(2-(methylsulfonyl)ethoxy)-3,4-dihydroisoquinolin-2(1H)-yl) (morpholino)methanone |
| **A47** | 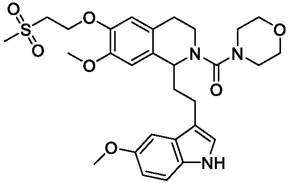 | (7-Methoxy-1-(2-(5-methoxy-1H-indol-3-yl)ethyl)-6-(2-(methylsulfonyl)ethoxy)-3,4-dihydroisoquinolin-2(1H)-yl) (morpholino)methanone |
| **A48** | 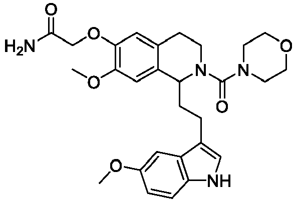 | 2-((7-Methoxy-1-(2-(5-methoxy-1H-indol-3-yl)ethyl)-2-(morpholin-4-carbonyl)-1,2,3,4-tetrahydroisoquinolin-6-yl)oxy) acetamide |
| **A49** | 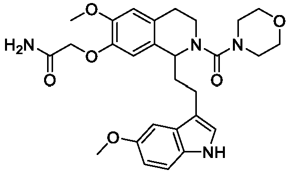 | 2-((6-methoxy-1-(2-(5-methoxy-1H-indol-3-yl)ethyl)-2-(morpholin-4-carbonyl)-1,2,3,4-tetrahydroisoquinolin-7-yl)oxy) acetamide |
| **A50** | 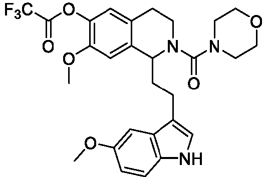 | 7-Methoxy-1-(2-(5-methoxy-1H-indol-3-yl)ethyl)-2-(morpholin-4-carbonyl)-1,2,3,4-tetrahydroisoquinolin-6-yl2,2,2-trifluoroacetate |
| **A51** | 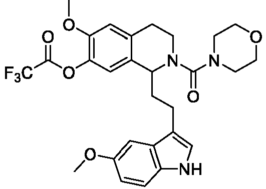 | 6-methoxy-1-(2-(5-methoxy-1H-indol-3-yl)ethyl)-2-(morpholin-4-carbonyl)-1,2,3,4-tetrahydroisoquinolin-7-yl2,2,2-trifluoroacetate |
| **A52** | 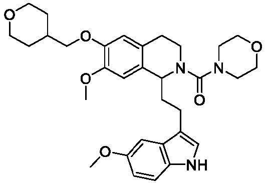 | (7-Methoxy-1-(2-(5-methoxy-1H-indol-3-yl)ethyl)-6-(tetrahydro-2H-pyran-4-yl)methoxy)-3,4-dihydroisoquinolin-2(1H)-yl) (morpholino)methanone |
| **A53** | 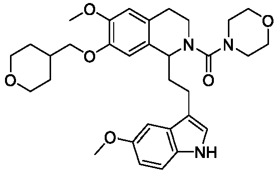 | (6-methoxy-1-(2-(5-methoxy-1H-indol-3-yl)ethyl)-7-(tetrahydro-2H-pyran-4-yl)methoxy)-3,4-dihydroisoquinolin-2(1H)-yl) (morpholino)methanone |
| **A54** | 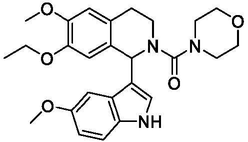 | (7-ethoxy-6-methoxy-1-(5-methoxy-1H-indol-3-yl)-3,4-dihydroisoquinolin-2(1H)-yl) (morpholino)methanone |
| **A55** | 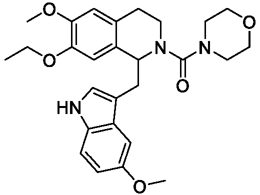 | (7-ethoxy-6-methoxy-1-((5-methoxy-1H-indol-3-yl)methyl)-3,4-dihydroisoquinolin-2(1H)-yl) (morpholino)methanone |
| **A56** | 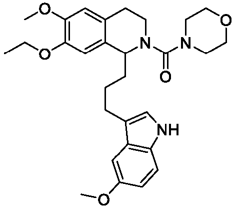 | (7-ethoxy-6-methoxy-1-(3-(5-methoxy-1H-indol-3-yl)propyl)-3,4-dihydroisoquinolin-2(1H)-yl) (morpholino)methanone |
| **A57** | 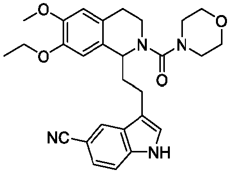 | 3-(2-(7-ethoxy-6-methoxy-2-(morpholine-4-carbonyl)-1,2,3,4-tetrahydroisoquinolin-1-yl)ethyl)-1H-indole-5-carbonitrile |
| **A58** | 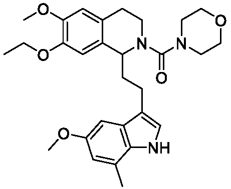 | (7-ethoxy-6-methoxy-1-(2-(5-methoxy-7-methyl-1H-indol-3-yl) ethyl)-3,4-dihydroisoquinolin-2 (1H)-yl)(morpholino)methanone |
| **A59** | 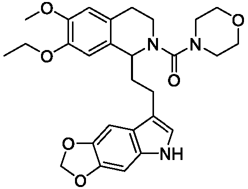 | (1-(2-(5H-[1,3]dioxolane[4,5-f] indole-7-yl)ethyl)-7-ethoxy-6-methoxy-3,4-dihydroisoquinolin-2 (1H)-yl)(morpholino)methanone |
| **A60** | 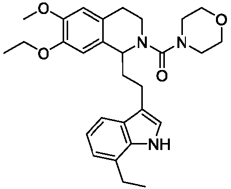 | (7-Ethoxy-1-(2-(7-ethyl-1H-indol-3-yl)ethyl)-6-methoxy-3,4-dihydroisoquinolin-2(1H)-yl) (morpholino)methanone |
| **A61** | 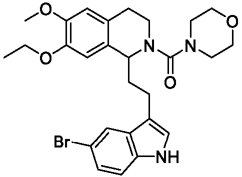 | (1-(2-(5-bromo-1H-indol-3-yl) ethyl)-7-ethoxy-6-methoxy-3,4-dihydroisoquinolin-2(1H)-yl) (morpholino)methanone |
| **A62** | 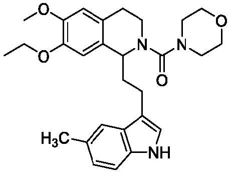 | (7-ethoxy-6-methoxy-1-(2-(5-methyl-1H-indol-3-yl)ethyl)-3,4-dihydroisoquinolin-2(1H)-yl) (morpholino)methanone |
| **A63** | 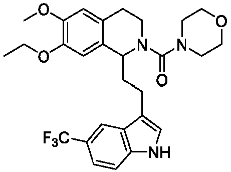 | (7-ethoxy-6-methoxy-1-(2-(5-(trifluoromethyl)-1H-indol-3-yl) ethyl)-3,4-dihydroisoquinolin-2 (1H)-yl)(morpholino)methanone |
| **A64** | 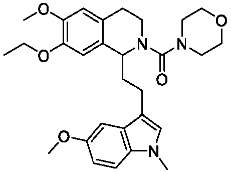 | (7-ethoxy-6-methoxy-1-(2-(5-methoxy-1-methyl-1H-indol-3-yl) ethyl)-3,4-dihydroisoquinolin-2 (1H)-yl)(morpholino)methanone |
| **A65** | 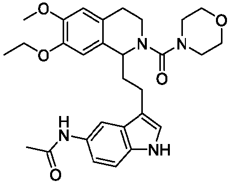 | N-(3-(2-(7-ethoxy-6-methoxy-2-(morpholine-4-carbonyl)-1,2,3,4-tetrahydroisoquinolin-1-yl)ethyl)-1H-indole-5-yl)acetamide |
| **A66** | 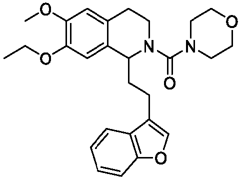 | (1-(2-(benzofuran-3-yl)ethyl)-7-ethoxy-6-methoxy-3,4-dihydroisoquinolin-2(1H)-yl) (morpholino)methanone |
| **A67** | 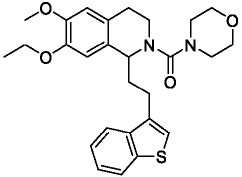 | (1-(2-(benzo[b]thiophen-3-yl) ethyl)-7-ethoxy-6-methoxy-3,4-dihydroisoquinolin-2(1H)-yl) (morpholino)methanone |
| **A68** | 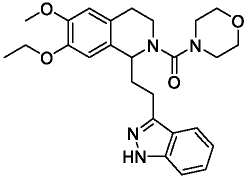 | (1-(2-(1H-indazol-3-yl)ethyl)-7-ethoxy-6-methoxy-3,4-dihydroisoquinolin-2(1H)-yl) (morpholino)methanone |
| **A69** | 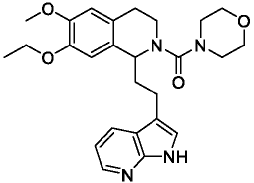 | (1-(2-(1H-pyrrolo[2,3-b]pyridin-3-yl)ethyl)-7-ethoxy-6-methoxy-3,4-dihydroisoquinolin-2(1H)-yl) (morpholino)methanone |
| **A70** | 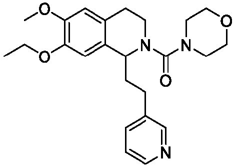 | (7-ethoxy-6-methoxy-1-(2-(pyridin-3-yl)ethyl)-3,4-dihydroisoquinolin-2(1H)-yl) (morpholino)methanone |
| **A71** | 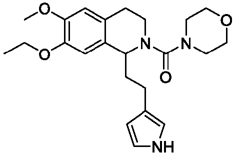 | (1-(2-(1H-pyrrole-3-yl)ethyl)-7-ethoxy-6-methoxy-3,4-dihydroisoquinolin-2(1H)-yl) (morpholino)methanone |
| **A72** | 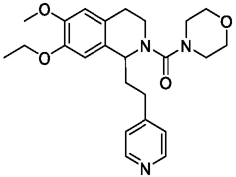 | (7-ethoxy-6-methoxy-1-(2-(pyridin-4-yl)ethyl)-3,4-dihydroisoquinolin-2(1H)-yl) (morpholino)methanone |
| **A73** | 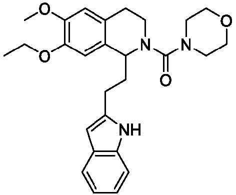 | (1-(2-(1H-indol-2-yl)ethyl)-7-ethoxy-6-methoxy-3,4-dihydroisoquinolin-2(1H)-yl) (morpholino)methanone |
| **A74** | 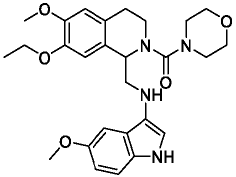 | (7-ethoxy-6-methoxy-1-((5-methoxy-1H-indol-3-yl)amino) methyl)-3,4-dihydroisoquinolin-2 (1H)-yl)(morpholino)methanone |
| **A75** | 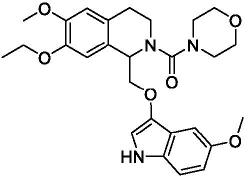 | (7-ethoxy-6-methoxy-1-((5-methoxy-1H-indol-3-yl)oxy) methyl)-3,4-dihydroisoquinolin-2 (1H)-yl)(morpholino)methanone |
| **A76** | 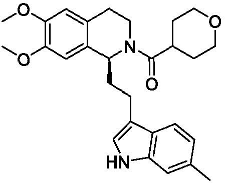 | (S)-(6,7-dimethoxy-1-(2-(6-methyl-1H-indol-3-yl)ethyl)-3,4-dihydroisoquinolin-2(1H)-yl) (tetrahydro-2H-pyran-4-yl)methanone |
| **A77** | 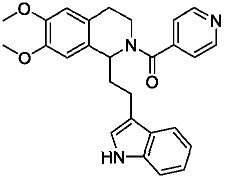 | (1-(2-(1H-indol-3-yl)ethyl)-6,7-dimethoxy-3,4-dihydroisoquinolin-2 (1H)-yl)(pyridin-4-yl)methanone |
| **A80** | 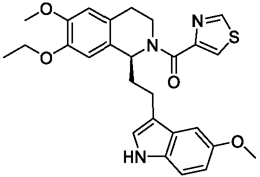 | (S)-(7-ethoxy-6-methoxy-1-(2-(5-methoxy-1H-indol-3-yl)ethyl)-3,4-dihydroisoquinolin-2(1H)-yl) (thiazol-4-yl)methanone |
| **A83** | 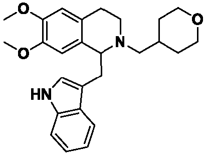 | 1-(1H-indol-3-yl)methyl)-6,7-dimethoxy-2-((tetrahydro-2H-pyran-4-yl)methyl)-1,2,3,4-tetrahydroisoquinoline |
| **A84** | 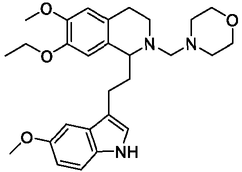 | 4-((7-ethoxy-6-methoxy-1-(2-(5-methoxy-1H-indol-3-yl)ethyl)-3,4-dihydroisoquinolin-2(1H)-yl) methyl)morpholine |
| **A85** | 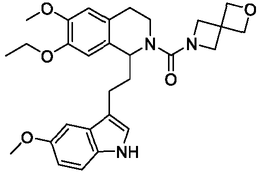 | (7-ethoxy-6-methoxy-1-(2-(5-methoxy-1H-indol-3-yl)ethyl)-3,4-dihydroisoquinolin-2(1H)-yl)(2-oxo-6-azaspiro[3.3]heptan-6-yl)methanone |
| **A86** | 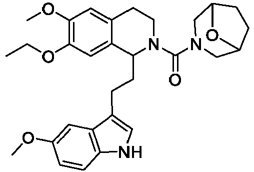 | (8-oxo-3-azabicyclo[3.2.1]oct-3-yl) (7-ethoxy-6-methoxy-1-(2-(5-methoxy-1H-indol-3-yl)ethyl)-3,4-dihydroisoquinolin-2(1H)-yl)methanone |
| **A87** | 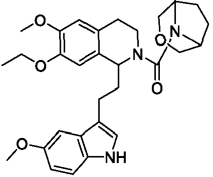 | (3-Oxo-8-azabicyclo[3.2.1]octan-8-yl)(7-ethoxy-6-methoxy-1-(2-(5-methoxy-1H-indol-3-yl)ethyl)-3,4-dihydroisoquinolin-2(1H)-yl)methanone |
| **A88** | 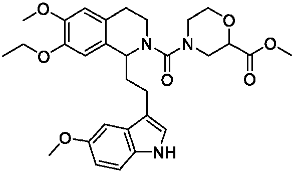 | Methyl4-(7-ethoxy-6-methoxy-1-(2-(5-methoxy-1H-indol-3-yl)ethyl)-1,2,3,4-tetrahydroisoquinoline-2-carbonyl)morpholine-2-carboxylate |
| **A89** | 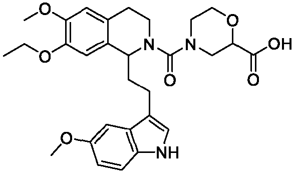 | 4-(7-ethoxy-6-methoxy-1-(2-(5-methoxy-1H-indol-3-yl)ethyl)-1,2,3,4-tetrahydroisoquinoline-2-carbonyl)morpholine-2-carboxylic acid |
| **A90** | 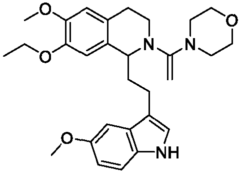 | 4-(1-(7-ethoxy-6-methoxy-1-(2-(5-methoxy-1H-indol-3-yl)ethyl)-3,4-dihydroisoquinolin-2(1H)-yl) ethenyl)morpholine |
| **A96** | 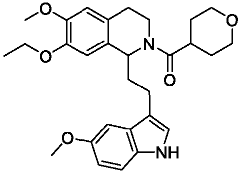 | (7-ethoxy-6-methoxy-1-(2-(5-methoxy-1H-indol-3-yl)ethyl)-3,4-dihydroisoquinolin-2(1H)-yl) (tetrahydro-2H-pyran-4-yl)methanone |
| **A97** | 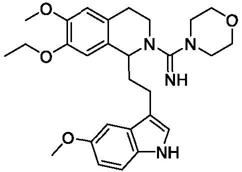 | (7-ethoxy-6-methoxy-1-(2-(5-methoxy-1H-indol-3-yl)ethyl)-3,4-dihydroisoquinolin-2(1H)-yl) (morpholinyl)carboxamide |
| **A98** | 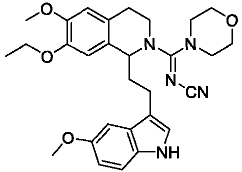 | (E)N-((7-ethoxy-6-methoxy-1-(2-(5-methoxy-1H-indol-3-yl)ethyl)-3,4-dihydroisoquinolin-2(1H)-yl) (morpholinyl)methylene) cyanamide |
| **A99** | 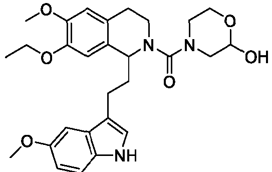 | (7-ethoxy-6-methoxy-1-(2-(5-methoxy-1H-indol-3-yl)ethyl)-3,4-dihydroisoquinolin-2(1H)-yl)(2-hydroxymorpholino)methanone |
| **A100** | 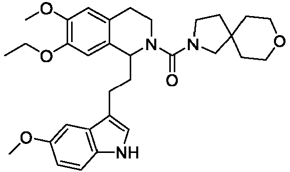 | (7-ethoxy-6-methoxy-1-(2-(5-methoxy-1H-indol-3-yl)ethyl)-3,4-dihydroisoquinolin-2(1H)-yl)(8-oxa-2-azaspiro[4.5]dec-2-yl)methanone |
| **A101** | 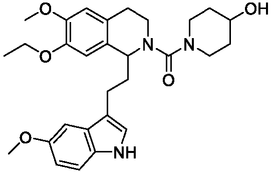 | (7-ethoxy-6-methoxy-1-(2-(5-methoxy-1H-indol-3-yl)ethyl)-3,4-dihydroisoquinolin-2(1H)-yl)(4-hydroxypiperidin-1-yl)methanone |
| **A102** | 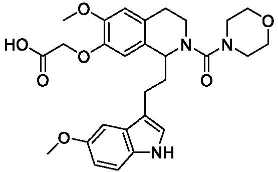 | 2-((6-methoxy-1-(2-(5-methoxy-1H-indol-3-yl)ethyl)-2-(morpholin-4-carbonyl)-1,2,3,4-tetrahydroisoquinolin-7-yl)oxy) aceticacid |
| **A103** | 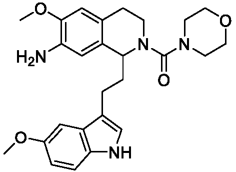 | (7-Amino-6-methoxy-1-(2-(5-methoxy-1H-indol-3-yl)ethyl)-3,4-dihydroisoquinolin-2(1H)-yl) (morpholinyl)methanone |
| **A104** | 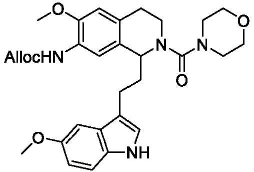 | Allyl(6-methoxy-1-(2-(5-methoxy-1H-indol-3-yl)ethyl)-2-(morpholin-4-carbonyl)-1,2,3,4-tetrahydroisoquinolin-7-yl) carbamate |
| **A105** | 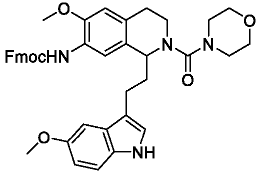 | (9H-fluoren-9-yl)methyl(6-methoxy-1-(2-(5-methoxy-1H-indol-3-yl)ethyl)-2-(morpholine-4-carbonyl)-1,2,3,4-tetrahydroisoquinolin-7-yl) carbamate |
| **A106** | 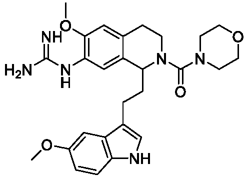 | 1-(6-methoxy-1-(2-(5-methoxy-1H-indol-3-yl)ethyl)-2-(morpholin-4-carbonyl)-1,2,3,4-tetrahydroisoquinolin-7-yl) guanidine |
| **A107** | 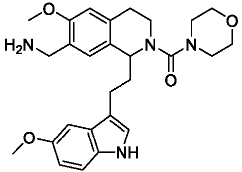 | (7-(Aminomethyl)-6-methoxy-1-(2-(5-methoxy-1H-indol-3-yl) ethyl)-3,4-dihydroisoquinolin-2 (1H)-yl)(morpholinyl)methanone |
| **A108** | 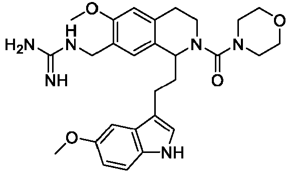 | 1-((6-methoxy-1-(2-(5-methoxy-1H-indol-3-yl)ethyl)-2-(morpholin-4-carbonyl)-1,2,3,4-tetrahydroisoquinolin-7-yl)methyl) guanidine |
| **A109** | 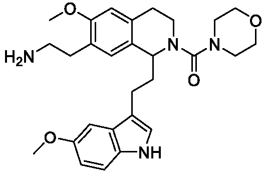 | (7-(2-aminoethyl)-6-methoxy-1-(2-(5-methoxy-1H-indol-3-yl) ethyl)-3,4-dihydroisoquinolin-2 (1H)-yl)(morpholinyl)methanone |
| **A110** | 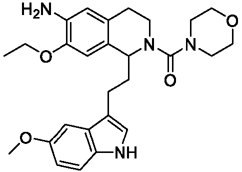 | (6-Amino-7-ethoxy-1-(2-(5-methoxy-1H-indol-3-yl)ethyl)-3,4-dihydroisoquinolin-2(1H)-yl) (morpholinyl)methanone |
| **A111** | 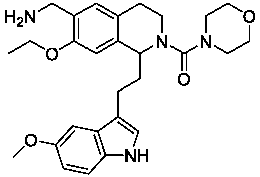 | (6-(Aminomethyl)-7-ethoxy-1-(2-(5-methoxy-1H-indol-3-yl)ethyl)-3,4-dihydroisoquinolin-2(1H)-yl) (morpholinyl)methanone |
| **A112** | 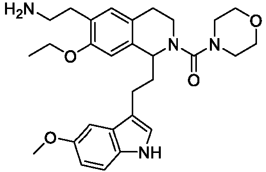 | (6-(2-aminoethyl)-7-ethoxy-1-(2-(5-methoxy-1H-indol-3-yl)ethyl)-3,4-dihydroisoquinolin-2(1H)-yl) (morpholinyl)methanone |
| **A113** | 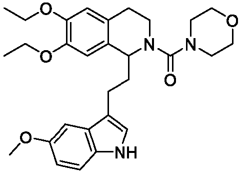 | (6,7-diethoxy-1-(2-(5-methoxy-1H-indol-3-yl)ethyl)-3,4-dihydroisoquinolin-2(1H)-yl) (morpholinyl)methanone |
| **A114** | 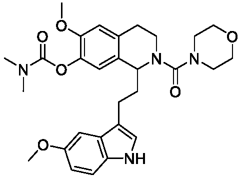 | 6-methoxy-1-(2-(5-methoxy-1H-indol-3-yl)ethyl)-2-(morpholin-4-carbonyl)-1,2,3,4-tetrahydroisoquinoline-7-yldimethylaminoformate |
| **A115** | 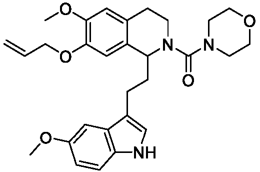 | (7-(Allyloxy)-6-methoxy-1-(2-(5-methoxy-1H-indol-3-yl)ethyl)-3,4-dihydroisoquinolin-2(1H)-yl) (morpholinyl)methanone |
| **A116** | 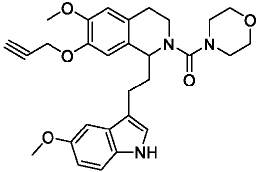 | (6-methoxy-1-(2-(5-methoxy-1H-indol-3-yl)ethyl)-7-(prop-2-yn-1-yloxy)-3,4-dihydroisoquinolin-2 (1H)-yl)(morpholinyl)methanone |
| **A117** | 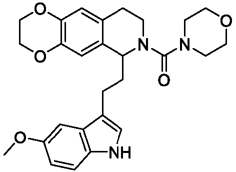 | (6-(2-(5-methoxy-1H-indol-3-yl) ethyl)-2,3,8,9-tetrahydro-[1,4] dioxy[2,3-g]isoquinolin-7(6H)-yl)(morpholinyl)methanone |
| **A118** | 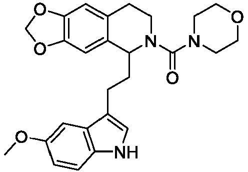 | (5-(2-(5-methoxy-1H-indol-3-yl) ethyl)-7,8-dihydro-[1,3]dioxolane [4,5-g]isoquinolin-6(5H)-yl) (morpholinyl)methanone |
| **A119** | 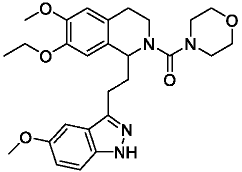 | (7-ethoxy-6-methoxy-1-(2-(5-methoxy-1H-indazol-3-yl)ethyl)-3,4-dihydroisoquinolin-2(1H)-yl) (morpholinyl)methanone |
| **A120** | 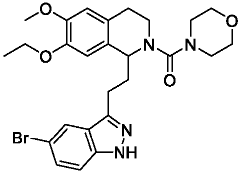 | (1-(2-(5-bromo-1H-indazol-3-yl) ethyl)-7-ethoxy-6-methoxy-3,4-dihydroisoquinolin-2(1H)-yl) (morpholinyl)methanone |
| **A121** | 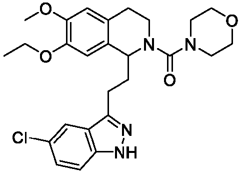 | (1-(2-(5-Chloro-1H-indazol-3-yl) ethyl)-7-ethoxy-6-methoxy-3,4-dihydroisoquinolin-2(1H)-yl) (morpholinyl)methanone |
| **A122** | 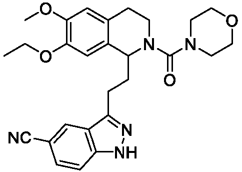 | 3-(2-(7-ethoxy-6-methoxy-2-(morpholine-4-carbonyl)-1,2,3,4-tetrahydroisoquinolin-1-yl)ethyl)-1H-indazole-5-nitrile |
| **A123** | 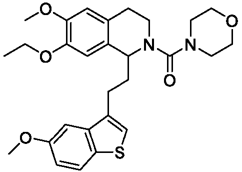 | (7-ethoxy-6-methoxy-1-(2-(5-methoxybenzo[b]thiophen-3-yl) ethyl)-3,4-dihydroisoquinolin-2 (1H)-yl)(morpholinyl)methanone |
| **A124** | 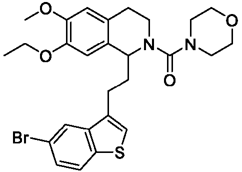 | (1-(2-(5-bromobenzo[b]thiophen-3-yl)ethyl)-7-ethoxy-6-methoxy-3,4-dihydroisoquinolin-2(1H)-yl) (morpholinyl)methanone |
| **A125** | 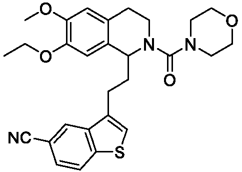 | 3-(2-(7-ethoxy-6-methoxy-2-(morpholin-4-carbonyl)-1,2,3,4-tetrahydroisoquinolin-1-yl)ethyl) benzo[b]thiophene-5-nitrile |
| **A126** | 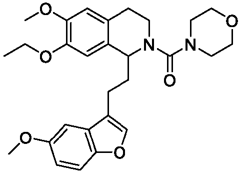 | (7-ethoxy-6-methoxy-1-(2-(5-methoxybenzofuran-3-yl)ethyl)-3,4-dihydroisoquinolin-2(1H)-yl) (morpholinyl)methanone |
| **A127** | 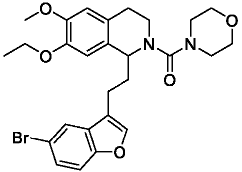 | (1-(2-(5-bromobenzofuran-3-yl) ethyl)-7-ethoxy-6-methoxy-3,4-dihydroisoquinolin-2(1H)-yl) (morpholinyl)methanone |
| **A128** | 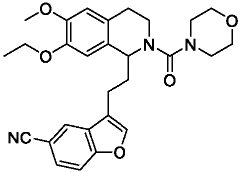 | 3-(2-(7-ethoxy-6-methoxy-2-(morpholin-4-carbonyl)-1,2,3,4-tetrahydroisoquinolin-1-yl)ethyl) benzofuran-5-carbonitrile |
| **A129** | 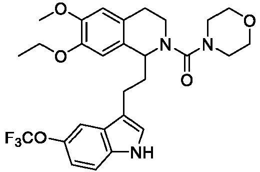 | (7-ethoxy-6-methoxy-1-(2-(5-(trifluoromethoxy)-1H-indol-3-yl) ethyl)-3,4-dihydroisoquinolin-2 (1H)-yl)(morpholinyl)methanone |
| **A130** | 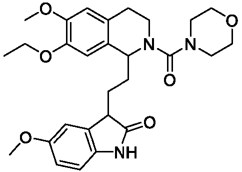 | 3-(2-(7-ethoxy-6-methoxy-2-(morpholin-4-carbonyl)-1,2,3,4-tetrahydroisoquinolin-1-yl)ethyl)-5-methoxyindol-2-one |
| **A131** | 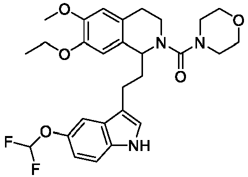 | (1-(2-(5-(difluoromethoxy)-1H-indol-3-yl)ethyl)-7-ethoxy-6-methoxy-3,4-dihydroisoquinolin-2 (1H)-yl)(morpholinyl)methanone |
| **A132** | 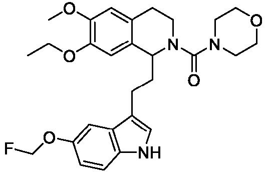 | (7-Ethoxy-1-(2-(5-(fluoromethoxy)-1H-indol-3-yl) ethyl)-6-methoxy-3,4-dihydroisoquinolin-2(1H)-yl) (morpholinyl)methanone |
| **A133** | 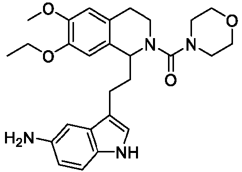 | (1-(2-(5-amino-1H-indol-3-yl) ethyl)-7-ethoxy-6-methoxy-3,4-dihydroisoquinolin-2(1H)-yl) (morpholinyl)methanone |
| **A134** | 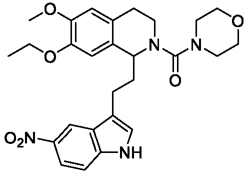 | (7-ethoxy-6-methoxy-1-(2-(5-nitro-1H-indol-3-yl)ethyl)-3,4-dihydroisoquinolin-2(1H)-yl) (morpholinyl)methanone |
| **A135** | 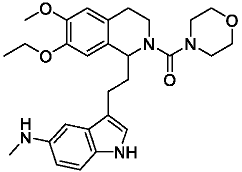 | (7-ethoxy-6-methoxy-1-(2-(5-(methylamino)-1H-indol-3-yl) ethyl)-3,4-dihydroisoquinolin-2 (1H)-yl)(morpholinyl)methanone |
| **A136** | 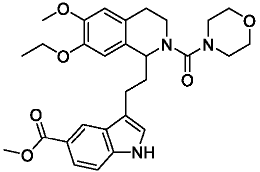 | Methyl3-(2-(7-ethoxy-6-methoxy-2-(morpholine-4-carbonyl)-1,2,3,4-tetrahydroisoquinolin-1-yl)ethyl)-1H-indole-5-carboxylate |
| **A137** | 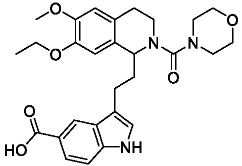 | 3-(2-(7-ethoxy-6-methoxy-2-(morpholin-4-carbonyl)-1,2,3,4-tetrahydroisoquinolin-1-yl)ethyl)-1H-indole-5-carboxylicacid |
| **A138** | 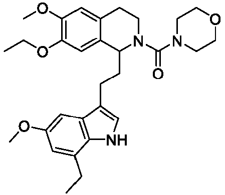 | (7-Ethoxy-1-(2-(7-ethyl-5-methoxy-1H-indol-3-yl)ethyl)-6-methoxy-3,4-dihydroisoquinolin-2 (1H)-yl)(morpholinyl)methanone |
| **A139** | 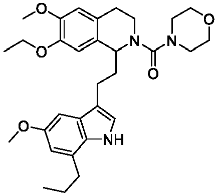 | (7-ethoxy-6-methoxy-1-(2-(5-methoxy-7-propy-1H-indol-3-yl) ethyl)-3,4-dihydroisoquinolin-2 (1H)-yl)(morpholinyl)methanone |
| **A140** | 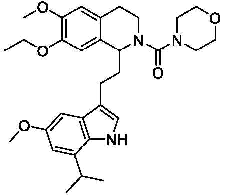 | (7-Ethoxy-1-(2-(7-isopropyl-5-methoxy-1H-indol-3-yl)ethyl)-6-methoxy-3,4-dihydroisoquinolin-2 (1H)-yl)(morpholinyl)methanone |
| **A141** | 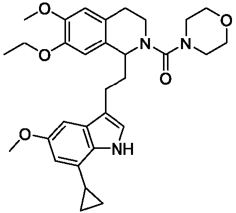 | (1-(2-(7-cyclopropyl-5-methoxy-1H-indol-3-yl)ethyl)-7-ethoxy-6-methoxy-3,4-dihydroisoquinolin-2 (1H)-yl)(morpholinyl)methanone |
| **A142** | 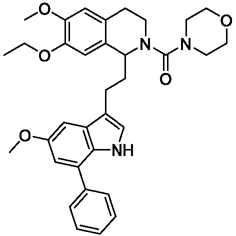 | (7-ethoxy-6-methoxy-1-(2-(5-methoxy-7-phenyl-1H-indol-3-yl) ethyl)-3,4-dihydroisoquinolin-2 (1H)-yl)(morpholinyl)methanone |
| **A143** | 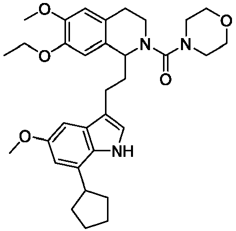 | (1-(2-(7-Cyclopentyl-5-methoxy-1H-indol-3-yl)ethyl)-7-ethoxy-6-methoxy-3,4-dihydroisoquinolin-2 (1H)-yl)(morpholinyl)methanone |
| **A144** | 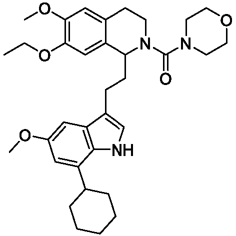 | (1-(2-(7-cyclohexyl-5-methoxy-1H-indol-3-yl)ethyl)-7-ethoxy-6-methoxy-3,4-dihydroisoquinolin-2 (1H)-yl)(morpholinyl)methanone |
| **A145** | 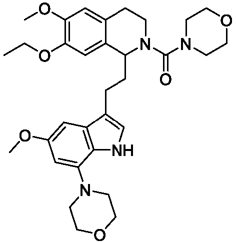 | (7-ethoxy-6-methoxy-1-(2-(5-methoxy-7morpholino-1H-indol-3-yl)ethyl)-3,4-dihydroisoquinolin-2 (1H)-yl)(morpholinyl)methanone |
| **A146** | 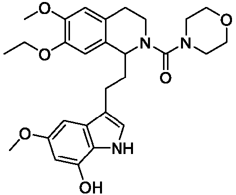 | (7-ethoxy-1-(2-(7-hydroxy-5-methoxy-1H-indol-3-yl)ethyl)-6-methoxy-3,4-dihydroisoquinolin-2 (1H)-yl)(morpholinyl)methanone |
| **A147** | 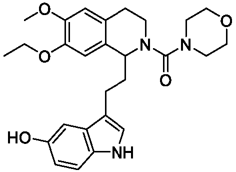 | (7-Ethoxy-1-(2-(5-hydroxy-1H-indol-3-yl)ethyl)-6-methoxy-3,4-dihydroisoquinolin-2(1H)-yl) (morpholinyl)methanone |
| **A148** | 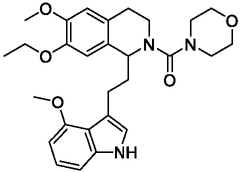 | (7-ethoxy-6-methoxy-1-(2-(4-methoxy-1H-indol-3-yl)ethyl)-3,4-dihydroisoquinolin-2(1H)-yl) (morpholinyl)methanone |
| **A149** | 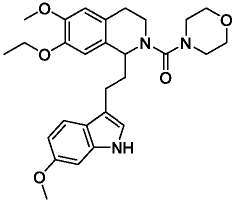 | (7-ethoxy-6-methoxy-1-(2-(6-methoxy-1H-indol-3-yl)ethyl)-3,4-dihydroisoquinolin-2(1H)-yl) (morpholinyl)methanone |
| **A150** | 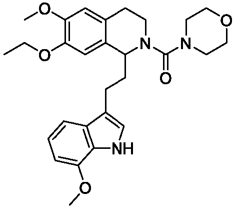 | (7-ethoxy-6-methoxy-1-(2-(7-methoxy-1H-indol-3-yl)ethyl)-3,4-dihydroisoquinolin-2(1H)-yl) (morpholinyl)methanone |
| **A151** | 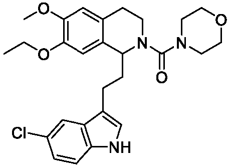 | (1-(2-(5-Chloro-1H-indol-3-yl) ethyl)-7-ethoxy-6-methoxy-3,4-dihydroisoquinolin-2(1H)-yl) (morpholinyl)methanone |
| **A152** | 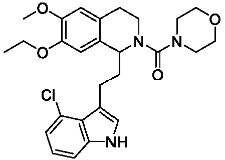 | (1-(2-(4-chloro-1H-indol-3-yl) ethyl)-7-ethoxy-6-methoxy-3,4-dihydroisoquinolin-2(1H)-yl) (morpholinyl)methanone |
| **A153** | 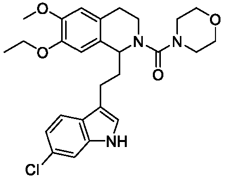 | (1-(2-(6-Chloro-1H-indol-3-yl) ethyl)-7-ethoxy-6-methoxy-3,4-dihydroisoquinolin-2(1H)-yl) (morpholinyl)methanone |
| **A154** | 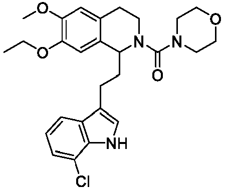 | (1-(2-(7-chloro-1H-indol-3-yl) ethyl)-7-ethoxy-6-methoxy-3,4-dihydroisoquinolin-2(1H)-yl) (morpholinyl)methanone |
| **A155** | 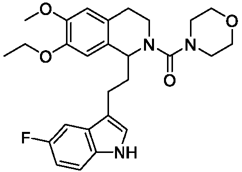 | (7-ethoxy-1-(2-(5-fluoro-1H-indol-3-yl)ethyl)-6-methoxy-3,4-dihydroisoquinolin-2(1H)-yl) (morpholinyl)methanone |
| **A156** | 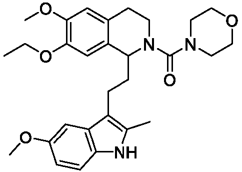 | (7-ethoxy-6-methoxy-1-(2-(5-methoxy-2-methyl-1H-indol-3-yl) ethyl)-3,4-dihydroisoquinolin-2 (1H)-yl)(morpholinyl)methanone |
| **A157** | 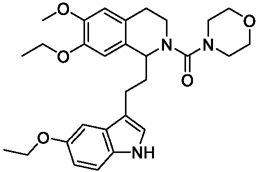 | (7-ethoxy-1-(2-(5-ethoxy-1H-indol-3-yl)ethyl)-6-methoxy-3,4-dihydroisoquinolin-2(1H)-yl) (morpholinyl)methanone |
| **A158** | 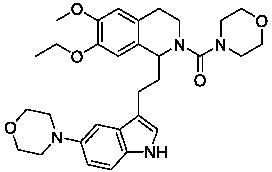 | (7-ethoxy-6-methoxy-1-(2-(5-morpholino-1H-indol-3-yl)ethyl)-3,4-dihydroisoquinolin-2(1H)-yl) (morpholinyl)methanone |
| **A159** | 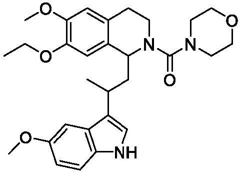 | (7-ethoxy-6-methoxy-1-(2-(5-methoxy-1H-indol-3-yl)propyl)-3,4-dihydroisoquinolin-2(1H)-yl) (morpholinyl)methanone |
| **A160** | 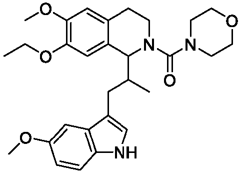 | (7-ethoxy-6-methoxy-1-(1-(5-methoxy-1H-indol-3-yl)propan-2-yl)-3,4-dihydroisoquinolin-2(1H)-yl)(morpholinyl)methanone |
| **A161** | 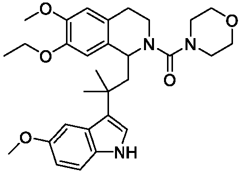 | (7-ethoxy-6-methoxy-1-(2-(5-methoxy-1H-indol-3-yl)-2-methylpropyl)-3,4-dihydroisoquinolin-2(1H)-yl) (morpholinyl)methanone |
| **A162** | 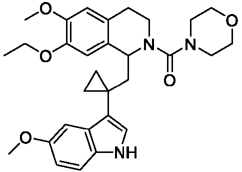 | (7-ethoxy-6-methoxy-1-((1-(5-methoxy-1H-indol-3-yl) cyclopropyl)methyl)-3,4-dihydroisoquinolin-2(1H)-yl) (morpholinyl)methanone |
| **A163** | 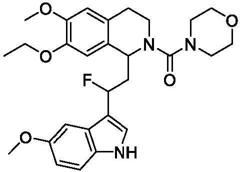 | (7-ethoxy-1-(2-fluoro-2-(5-methoxy-1H-indol-3-yl)ethyl)-6-methoxy-3,4-dihydroisoquinolin-2 (1H)-yl)(morpholinyl)methanone |
| **A164** | 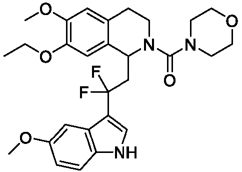 | (1-(2,2-difluoro-2-(5-methoxy-1H-indol-3-yl)ethyl)-7-ethoxy-6-methoxy-3,4-dihydroisoquinolin-2 (1H)-yl)(morpholinyl)methanone |
| **A165** | 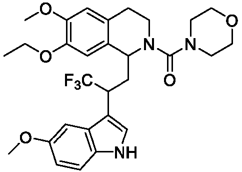 | (7-ethoxy-6-methoxy-1-(3,3,3-trifluoro-2-(5-methoxy-1H-indol-3-yl)propyl)-3,4-dihydroisoquinolin-2(1H)-yl) (morpholinyl)methanone |
| **A166** | 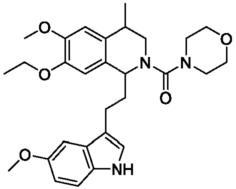 | (7-ethoxy-6-methoxy-1-(2-(5-methoxy-1H-indol-3-yl)ethyl)-4-methyl-3,4-dihydroisoquinolin-2 (1H)-yl)(morpholinyl)methanone |
| **A167** | 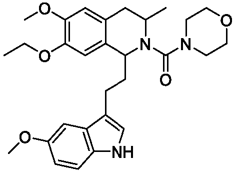 | (7-ethoxy-6-methoxy-1-(2-(5-methoxy-1H-indol-3-yl)ethyl)-3-methyl-3,4-dihydroisoquinolin-2 (1H)-yl)(morpholinyl)methanone |
| **A168** | 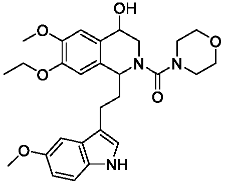 | (7-ethoxy-4-hydroxy-6-methoxy-1-(2-(5-methoxy-1H-indol-3-yl) ethyl)-3,4-dihydroisoquinolin-2 (1H)-yl)(morpholinyl)methanone |
| **A169** | 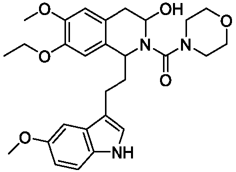 | (7-ethoxy-3-hydroxy-6-methoxy-1-(2-(5-methoxy-1H-indol-3-yl) ethyl)-3,4-dihydroisoquinolin-2 (1H)-yl)(morpholinyl)methanone |
| **A170** | 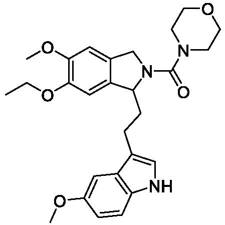 | (6-Ethoxy-5-methoxy-1-(2-(5-methoxy-1H-indol-3-yl)ethyl) isoindol-2-yl) (morpholinyl)methanone |
| **A171** | 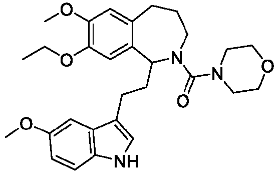 | (8-ethoxy-7-methoxy-1-(2-(5-methoxy-1H-indol-3-yl)ethyl)-1,3,4,5-tetrahydro-2H-benzo[c] aza-2-yl)(morpholinyl)methanone |

### Preparation of formula (I) compound

The present invention also provided a method for synthesizing the compound of formula I, specifically, the compound of formula I is prepared by the following process: 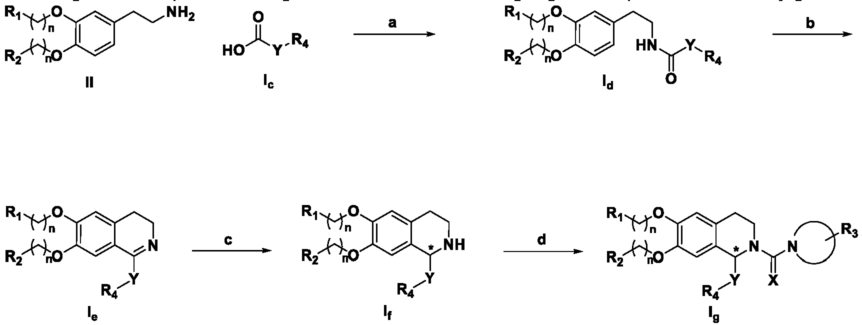
Step a: Dissolve I_{c} in a solvent and undergo a condensation reaction with II under the assistance of a condensing agent to obtain compound I_{d}; wherein the solvent is dichloromethane;
Step b: Dissolve d I_{d} in a solvent, add excess phosphorus oxychloride, reflux and stir to obtain compound Ie; the solvent is anhydrous acetonitrile;
Step c: Dissolve Iₑ in a solvent, add excess sodium borohydride, stir until the reaction is complete, and spin dry the solvent to obtain compound I_{f}, the solvent is methanol; Alternatively, Noyori catalyst can be added and stirred until the reaction is complete, the solvent is a mixture of water and methanol.
Step d: Dissolve I_{f} in a solvent and react with the corresponding raw materials to obtain compound Ig; the solvent is dichloromethane;
X, Y, R₁, R₂, R₃ and R₄ are as defined above.

### Pharmaceutical composition containing compound of formula (I)

The present invention also relates to a pharmaceutical composition comprising a therapeutic effective amount of one or more selected from the group consisting of nitrogen-containing heterocyclic compoundof formula (I), a pharmaceutical salt, a prodrug and a hydrate and a solvate thereof, and optionally, a pharmaceutically acceptable carrier, which can be used for treating autoimmune related diseases such as psoriasis. The pharmaceutical composition can be prepared in various forms according to different administration routes.

One or more of the aldehyde compound represented by formula (I) of the invention, a pharmaceutical salt, a prodrug , a hydrate and a solvate thereof, or the pharmaceutical composition containing therapeutic effective amount of one or more selected from the tetrahydroisoquinoline compound represented by formula (I), a pharmaceutical salt, a prodrug, a hydrate and solvate thereof can be used as phosphodiesterase 4 (PDE4) inhibitors for the treatment of constipation, anorexia, diabetes, nonalcoholic steatohepatitis and other glycolipid metabolic diseases.

The preparation of the pharmaceutical salt of the compound of the present invention can be carried out by directly reacting the free base of the compound with inorganic or organic acids to form a salt. Inorganic or organic acids can be selected from hydrochloric acid, sulfuric acid, phosphoric acid, nitric acid, hydrofluoric acid, hydrobromic acid, formic acid, acetic acid, picric acid, citric acid, maleic acid, methanesulfonic acid, trifluoromethanesulfonic acid, ethanesulfonic acid, and p-toluenesulfonic acid.

Due to the excellent inhibitory activity of the compounds of the present invention against the relaxin family receptor 4 (RXFP4), the compounds of the present invention and their various crystal forms, pharmaceutically acceptable inorganic or organic salts, hydrates or solvates, as well as pharmaceutical compositions containing the compounds of the present invention as the main active ingredient, can be used for the treatment and alleviation of diseases associated with the relaxin family receptor 4 (RXFP4). According to the prior art, the compound of the invention can be used to treat the following diseases: constipation, anorexia, diabetes, nonalcoholic steatohepatitis and other glycolipid metabolic diseases.

The pharmaceutical composition of the present invention comprises a safe and effective amount of the compound of the present invention or its pharmaceutically acceptable salt, as well as a pharmaceutically acceptable excipient or carrier. The term 'safe and effective dose' refers to the amount of a compound that is sufficient to significantly improve the condition without causing serious side effects. Generally, pharmaceutical compositions contain 1-2000 mg of the compound of the present invention per dose, and more preferably 5-200 mg of the compound of the present invention per dose. Preferably, the 'one dose' is a capsule or tablet.

"Pharmaceutically acceptable carrier" refers to one or more compatible solid or liquid fillers or gel substances, which are suitable for human use and must have sufficient purity and low toxicity. "Compatible" here refers to the ability of each component in the composition to blend with the compounds of the present invention and with each other without significantly reducing the efficacy of the compounds. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween ^{®}), wetting agents (such as sodium dodecyl sulfate), coloring agents, flavouring agent, stabilizers, antioxidants, preservatives, pyrogen-free water, etc.

The administration mode of the compound or pharmaceutical combination of the present invention is not particularly limited, and representative administration modes include (but are not limited to) oral, intratumoral, rectal, parenteral (intravenous, intramuscular or subcutaneous), and local administration.

Solid dosage forms for oral administration include capsules, tablets, pills, powders, and granules. In these solid dosage forms, the active compound is mixed with at least one conventional inert excipient (or carrier), such as sodium citrate or dicalcium phosphate, or with the following components: (a) fillers or compatibilizers, such as starch, lactose, sucrose, glucose, mannitol, and silica; (b) Adhesives, such as hydroxymethylcellulose, alginate, gelatin, polyvinylpyrrolidone, sucrose, and arabic gum; (c) Moisturizing agents, such as glycerin; (d) Disinfectants, such as agar, calcium carbonate, potato starch or cassava starch, alginic acid, certain complex silicates, and sodium carbonate; (e) Slow solvents, such as paraffin wax; (f) Absorption accelerators, such as quaternary amine compounds; (g) Wetting agents, such as cetyl alcohol and glycerol monostearate; (h) Adsorbents, such as kaolin; And (i) lubricants, such as talc, calcium stearate, magnesium stearate, solid polyethylene glycol, sodium dodecyl sulfate, or mixtures thereof. Capsules, tablets, and pills may also contain buffering agents.

Solid dosage forms such as tablets, sugar pills, capsules, pills, and granules can be prepared using coating and shell materials, such as casings and other materials known in the art. They may contain opaque agents, and the release of active compounds or compounds in this combination can be delayed in a certain part of the digestive tract. Examples of usable embedding components are polymeric substances and wax based substances. When necessary, the active compound can also form microcapsules with one or more of the above-mentioned excipients.

Liquid dosage forms for oral administration include pharmaceutically acceptable lotion, solutions, suspensions, syrups or tinctures. In addition to active compound, liquid dosage form may include inert diluents commonly used in this field, such as water or other solvents, solubilizers, and emulsifiers, such as ethanol, isopropanol, ethyl carbonate, ethyl acetate, propylene glycol, 1,3-butanediol, dimethylformamide, as well as oils, particularly cottonseed oil, peanut oil, corn germ oil, olive oil, castor oil, and sesame oil, or mixtures of these substances.

In addition to these inert diluents, the composition may also contain adjuvant such as wetting agents, emulsifiers and suspensions, sweeteners, flavor enhancers, and flavorings.

In addition to active compounds, a suspension may contain suspending agent such as ethoxylated isooctadecanol, polyoxyethylene sorbitol and dehydrated sorbitol esters, microcrystalline cellulose, methanol aluminum and agar, or mixtures of these substances.

Compositions for parenteral injection may include physiologically acceptable sterile aqueous or anhydrous solutions, dispersions, suspensions or lotion, and sterile powders for re dissolution into sterile injectable solutions or dispersions. Suitable aqueous and nonaqueous carriers, diluents, solvents, or excipients include water, ethanol, polyols, and their suitable mixtures.

The dosage forms of the compound of the present invention for local administration include ointments, powders, patches, sprays, and inhalants. The active ingredient is mixed with physiologically acceptable carriers and any preservatives, buffering agents, or necessary propellants under sterile conditions.

The compound of the present invention can be administered alone or in combination with other pharmaceutically acceptable compound.

The compound according to the present invention as described above can be used clinically in mammals, including humans and animals, and can be administered through oral, nasal, dermal, pulmonary, or gastrointestinal routes, with oral administration being more preferred. The recommended daily dose is 0.01-200 mg/kg body weight, taken in one dose, or 0.01-100 mg/kg body weight taken in divided doses. Regardless of the method of administration, the optimal dosage for an individual should be determined based on the specific treatment. Usually, it starts with a small dose and gradually increases until the most suitable dose is found. Of course, the specific dosage should also consider factors such as the route of administration and the patient's health condition, which are within the skill of skilled physicians.

The present invention will be further explained in conjunction with specific examples. It should be understood that these examples are only used to illustrate the present invention and not to limit the scope of the present invention. The experimental methods without specific conditions specified in the following examples are usually carried out under conventional conditions or conditions recommended by the manufacturer. Unless otherwise specified, percentages and portions are calculated by weight.

In the following examples, further examples will be provided to illustrate the present invention. These embodiments are only used to illustrate the present invention, but do not limit the present invention in any way. All parameters and other explanations in the implementation examples are based on quality, unless otherwise specified.

The analysis data of the samples were determined by the following instruments: nuclear magnetic resonance was measured by GEMINI-300, Bruker AMX-400, or INVOA-600 nuclear magnetic resonance instruments, TMS (tetramethylsilane) was used as the internal standard, chemical shift unit was ppm, and coupling constant unit was Hz; The mass spectrometry was determined by Finnigan MAT-711, MAT-95, LCQ-DECA mass spectrometers, and IonSpec 4.7 Tesla mass spectrometer.

Column chromatography used silica gel 200-300 mesh (produced by Qingdao Marine Chemical Plant); The TLC silica gel plate is a HSGF-254 thin layer chromatography prefabricated plate produced by Yantai Chemical Plant; The boiling range of petroleum ether is 60-90 °C; ultraviolet lamp and iodine cylinder was used for developing. Unless otherwise specified, the conventional reagents and drugs used in the following examples were purchased from Sinopharm Group. The reagents and solvents used in the experiment are handled according to the specific reaction conditions.

### Example A1: Synthesis of Compound A1

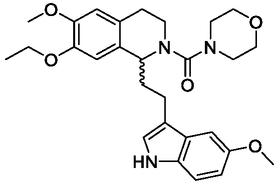

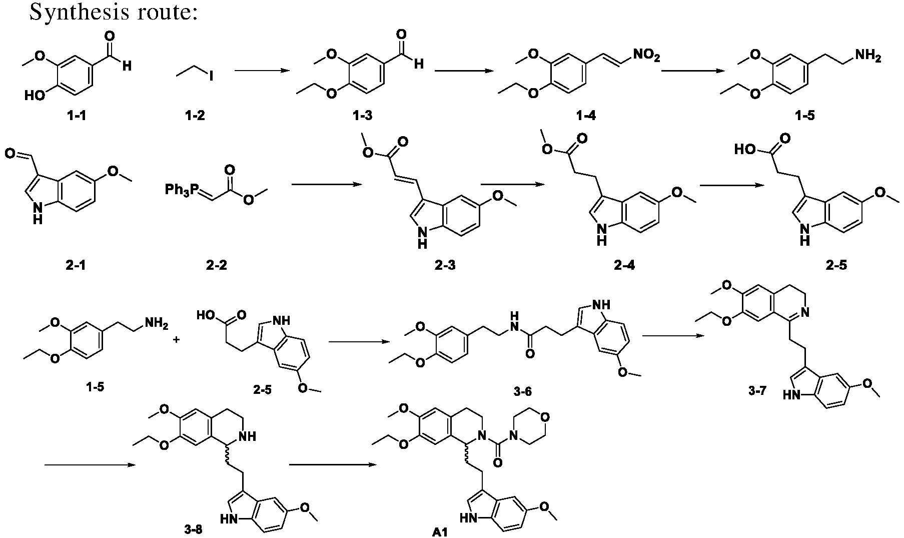

### Synthesis of compound 1-3:

To a solution of 1-1 in acetone was added potassium carbonate, and 1-2 was added dropwise, then refluxed and stirred overnight under argon protection. The reaction solution was then filtered, evaporated to dryness, diluted with dichloromethane, added water and stirred for 10 minutes, and static layered, the organic layer was evaporate to drynessto obtain yellow solids 1-3, which was usedto the next step without purification.

### Synthesis of compound 1-4:

To a solution of 1-3 in nitromethane was added ammonium acetate and refluxed for 2 hours, then evaporated the solvent, ice water was added and stirred for 2 hours, stand, filtered, the filter cake was washed with ethyl acetate to obtain a yellow solid with a yield of 90%.¹H NMR (500 MHz, CDCl₃) δ 7.95 (s, 2H), 7.41 (dd, *J =* 8.5, 2.0 Hz, 1H), 7.21 (d, *J* = 1.9 Hz, 1H), 6.99 (d, *J =* 8.4 Hz, 1H), 4.09 (q, *J =* 7.0 Hz, 2H), 3.87 (s, 2H), 1.43 (t, *J* = 6.9 Hz, 3H). ESI-MS *m*/*z* 224.2 [M+H]⁺.

### Synthesis of compound 1-5:

lithium aluminum tetrahydroxide was added in batches to tetrahydrofuran under an ice bath and the argon gas protection, a solution of 1-4 tetrahydrofuran was added dropwise while stirring. After the addition was complete, the mixture was stirred at room temperature for 2 hours. The reaction was then slowly quenched by adding water under an ice bath, filtered, the filter cake was washed with ethyl acetate and methanol, and evaporated to dryness to obtain a slightly yellow transparent oil, which was used to the next step without purification.

### Synthesis of compound 2-3:

Compounds 2-1 and 2-2 were dissolved in toluene and refluxed for 18 hours, then cooled, evaporated the solvent, and subjected to a column chromatography (dichloromethane: methanol=100:1) to obtain a white solid with a yield of 85%. ¹H NMR (500 MHz, CDCl₃) δ 9.49 (d, *J =* 2.5 Hz, 1H), 8.10 (d, *J =* 15.9 Hz, 1H), 7.83 (d, *J =* 2.7 Hz, 1H), 7.47 (d, *J =* 2.7 Hz, 1H), 7.08 (d, *J =* 8.4 Hz, 1H), 6.80 (dd, *J* = 8.4, 2.7 Hz, 1H), 6.39 (d, *J =* 15.9 Hz, 1H), 3.86 (s, 3H), 3.75 (s, 3H). ESI-MS *m*/*z* 232.2 [M+H]⁺.

### Synthesis of compounds 2-4:

To a solution of compound 2-3 in methanol was added palladium on carbon, and the reaction was carried out at 60°C for 6 hours. Then filtered to remove the palladium on carbon, and spun-dried the solvent to obtain a white solid, which was used to the next reaction without purification.

### Synthesis of compound 2-5:

To a solution of compound 2-4 in methanol was added 1 M sodium hydroxide aqueous solution. The reaction was carried out at 50° C for 2 hours. Then most of the methanol was removed by rotary evaporation, and most of the impurities was washed away with dichloromethane, the aqueous phase was adjusted to pH 1-2 with 1M hydrochloric acid, and extractd with ethyl acetate. The ethyl acetate layers were combined, dried with anhydrous sodium sulfate, and spun-dried the solvent to obtain a white solid, which was directly used to the next reaction.

### Synthesis of compound 3-6:

To a solution of 2-5 in dichloromethane was add EDCI, HOBT and TEA, and stirred for 30 minutes, then a solution of 1-5 in dichloromethanewas added slowly, stirred overnight, diluted with dichloromethane, washed with saturated sodium bicarbonate, saturated ammonium chloride and saturated sodium chloride in sequence, dried with anhydrous sodium sulfate, evaporated, and purified by column chromatography with petroleum ether/ethyl acetate=1:1 to obtain 1.2 g of yellow white solid with a yield of 92%. ¹H NMR (500 MHz, CDCl₃) δ 9.48 (t, *J =* 2.3 Hz, 1H), 7.22 (dd, *J =* 8.4, 2.0 Hz, 1H), 7.04 (dd, *J =* 12.4, 2.6 Hz, 2H), 6.87 - 6.81 (m, 1H), 6.80 - 6.70 (m, 2H), 6.66 (dt, *J* = 9.1, 0.9 Hz, 2H), 4.08 (q, *J =* 7.0 Hz, 2H), 3.84 (d, *J =* 7.9 Hz, 6H), 3.42 (td, *J =* 5.6, 4.4 Hz, 2H), 3.06 (t, *J* = 7.8 Hz, 2H), 2.74 (tt, *J =* 5.6, 1.0 Hz, 2H), 2.65 (t, *J =* 7.8 Hz, 2H), 1.43 (t, *J* = 7.0 Hz, 3H). ESI-MS *m*/*z* 397.2 [M+H]⁺.

### Synthesis of compound 3-7:

To a solution of compound 3-6 1g in 100 mL anhydrous acetonitrile was added phosphorus oxychloride, and stirred under reflux and argon protection. After TLC monitored the completion of the reaction, it was evaporated under reduced pressure, adjusted to alkalescence with ice saturated sodium bicarbonate, extracted with dichloromethane, dried with anhydrous sodium sulfate, and evaporated to obtain an orange oil, which was directly used to the next reaction step without purification.

### Synthesis of compound 3-8:

To a solution of compound 3-7 in methanol was added sodium borohydride in batches under an ice bath, and stirred at room temperature for 4 hours, then the reaction was quenched with saturated ammonium chloride solution, extracted with dichloromethane, washed with saturated sodium bicarbonate, dried the organic layer with anhydrous sodium sulfate, concentrated, and subjected to column chromatography (dichloromethane: methanol 20:1) to obtain a yellow solid with a two-step yield of 70%.¹H NMR (500 MHz, CDCl₃) δ 9.63 (t, *J* = 2.4 Hz, 1H), 7.22 (dd, *J =* 8.4, 2.0 Hz, 1H), 7.07 (d, *J =* 2.5 Hz, 1H), 7.02 (d, *J =* 2.8 Hz, 1H), 6.94 (d, *J =* 1.1 Hz, 1H), 6.72 (dd, *J =* 8.4, 2.7 Hz, 1H), 6.61 (t, *J* = 1.0 Hz, 1H), 5.21 (td, *J =* 5.4, 1.0 Hz, 1H), 4.14 (dq, *J =* 10.1, 6.9 Hz, 1H), 4.11 - 4.01 (m, 2H), 3.83 (d, *J =* 1.8 Hz, 6H), 3.63 - 3.51 (m, 5H), 3.16 - 3.03 (m, 4H), 2.98 - 2.85 (m, 3H), 2.50 (dt, *J =* 14.7, 7.2 Hz, 1H), 2.40 (dtd, *J* = 12.6, 7.3, 5.3 Hz, 1H), 2.29 (dtd, *J* = 12.6, 7.2, 5.3 Hz, 1H), 1.42 (t, *J =* 7.0 Hz, 3H). ESI-MS *m*/*z* 494.2 [M+H]⁺.

### Synthesis of compound A1:

to a solution of 3-8 100 mg in dichloromethane was add morpholinyl chloride and triethylamine, and stirred at room temperature for 2 hours. After TLC monitored the completion of the reaction, saturated ammonium chloride was added and extracted three times with dichloromethane. The organic phases were combined and washed with saturated sodium chloride, dried with anhydrous sodium sulfate, evaporated the solvent, and subjected to column chromatography (petroleum ether: ethyl acetate 1:1) to obtain a white solid A1 with a yield of 90%. ¹H NMR (500 MHz, CDCl₃) δ 9.63 (t, *J =* 2.4 Hz, 1H), 7.22 (dd, *J =* 8.4, 2.0 Hz, 1H), 7.07 (d, *J* = 2.5 Hz, 1H), 7.02 (d, *J* = 2.8 Hz, 1H), 6.94 (d, *J* = 1.1 Hz, 1H), 6.72 (dd, *J* = 8.4, 2.7 Hz, 1H), 6.61 (t, *J =* 1.0 Hz, 1H), 5.21 (td, *J =* 5.4, 1.0 Hz, 1H), 4.14 (dq, *J =* 10.1, 6.9 Hz, 1H), 4.11 - 4.01 (m, 2H), 3.83 (d, *J =* 1.8 Hz, 6H), 3.63 - 3.51 (m, 5H), 3.16 - 3.03 (m, 4H), 2.98 - 2.85 (m, 3H), 2.50 (dt, *J =* 14.7, 7.2 Hz, 1H), 2.40 (dtd, *J* = 12.6, 7.3, 5.3 Hz, 1H), 2.29 (dtd, *J* = 12.6, 7.2, 5.3 Hz, 1H), 1.42 (t, *J =* 7.0 Hz, 3H). ESI-MS *m*/*z* 494.2 [M+H]⁺.

### Example (S) - A1: Synthesis of Compound (S) - A1

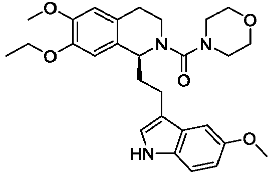

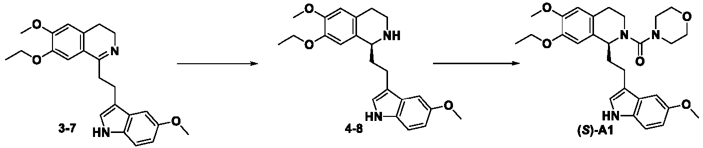

To a solution of 3-7 in a small amount of methanol was added deionized water, (R, *R*) - Noyori catalyst, silver hexafluoroantimonate, lanthanum trifluoromethanesulfonate and sodium formate dihydrate, and stirred at room temperature under argon protection overnight, then extracted with dichloromethane, washed with water. The organic layer was filtered with diatomaceous earth, concentrated, and subjected to column chromatography with dichloromethane/methanol=40:1 to obtain 4-8. Referring to the synthesis method of compound A1, compound (S) - A1 was obtained. ¹H NMR (500 MHz, CDCl₃) δ 9.63 (t, *J =* 2.4 Hz, 1H), 7.22 (dd, *J* = 8.4, 2.0 Hz, 1H), 7.07 (d, *J* = 2.5 Hz, 1H), 7.02 (d, *J* = 2.8 Hz, 1H), 6.94 (d, *J =* 1.1 Hz, 1H), 6.72 (dd, *J* = 8.4, 2.7 Hz, 1H), 6.61 (t, *J =* 1.0 Hz, 1H), 5.21 (td, *J* = 5.4, 1.0 Hz, 1H), 4.14 (dq, *J =* 10.1, 6.9 Hz, 1H), 4.11 - 4.01 (m, 2H), 3.83 (d, *J =* 1.8 Hz, 6H), 3.63 - 3.51 (m, 5H), 3.16 - 3.03 (m, 4H), 2.98 - 2.85 (m, 3H), 2.50 (dt, *J =* 14.7, 7.2 Hz, 1H), 2.40 (dtd, *J =* 12.6, 7.3, 5.3 Hz, 1H), 2.29 (dtd, *J =* 12.6, 7.2, 5.3 Hz, 1H), 1.42 (t, *J* = 7.0 Hz, 3H). ESI-MS *m*/*z* 494.2 [M+H]⁺.

### Example (R) - A1: Synthesis of Compound (R) - A1

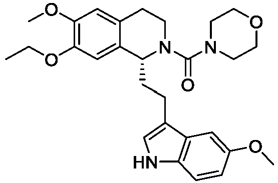

Refering to the synthesis method of compound (*S*) - A1, compound (*R*) - A1 was obtained by replacing the (*R, R*) *- Noyori* catalyst in the example with the (*S, S*) - Noyori catalyst. ¹H NMR (500 MHz, CDCl₃) δ 9.63 (t, *J =* 2.4 Hz, 1H), 7.22 (dd, *J =* 8.4, 2.0 Hz, 1H), 7.07 (d, *J =* 2.5 Hz, 1H), 7.02 (d, *J =* 2.8 Hz, 1H), 6.94 (d, *J =* 1.1 Hz, 1H), 6.72 (dd, *J =* 8.4, 2.7 Hz, 1H), 6.61 (t, *J* = 1.0 Hz, 1H), 5.21 (td, *J* = 5.4, 1.0 Hz, 1H), 4.14 (dq, *J =* 10.1, 6.9 Hz, 1H), 4.11 - 4.01 (m, 2H), 3.83 (d, *J =* 1.8 Hz, 6H), 3.63 - 3.51 (m, 5H), 3.16 - 3.03 (m, 4H), 2.98 - 2.85 (m, 3H), 2.50 (dt, *J =* 14.7, 7.2 Hz, 1H), 2.40 (dtd, *J =* 12.6, 7.3, 5.3 Hz, 1H), 2.29 (dtd, *J* = 12.6, 7.2, 5.3 Hz, 1H), 1.42 (t, *J* = 7.0 Hz, 3H). ESI-MS *m*/*z* 494.2 [M+H]⁺.

### Example A2: Synthesis of Compound A2

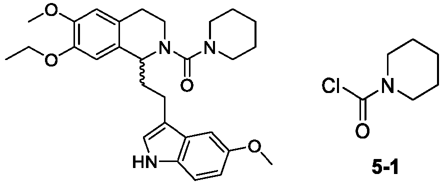

Refering to the synthesis method of compound A1, compound A2 was obtained by replacing the morpholinyl chloride in Example A1 with compound 5-1. ¹H NMR (500 MHz, CDCl₃) δ 9.63 (t, *J* = 2.4 Hz, 1H), 7.22 (dd, *J =* 8.5, 2.0 Hz, 1H), 7.07 (d, *J =* 2.5 Hz, 1H), 7.02 (d, *J* = 2.8 Hz, 1H), 6.93 (d, *J =* 1.0 Hz, 1H), 6.72 (dd, *J =* 8.4, 2.7 Hz, 1H), 6.61 (t, *J* = 0.9 Hz, 1H), 5.17 (td, *J =* 5.4, 1.0 Hz, 1H), 4.14 (dq, *J =* 10.1, 6.9 Hz, 1H), 4.11 - 3.99 (m, 2H), 3.83 (d, *J* = 4.2 Hz, 6H), 3.59 (ddd, *J* = 12.1, 6.3, 4.4 Hz, 1H), 3.56 - 3.50 (m, 4H), 2.98 - 2.85 (m, 3H), 2.83 (dt, *J =* 14.8, 7.3 Hz, 1H), 2.41 (dtd, *J* = 12.7, 7.3, 5.4 Hz, 1H), 2.28 (dtd, *J =* 12.6, 7.2, 5.3 Hz, 1H), 1.67 - 1.57 (m, 6H), 1.42 (t, *J* = 7.0 Hz, 3H). ESI-MS *m*/*z* 492.3 [M+H]⁺.

### Example A3: Synthesis of Compound A3

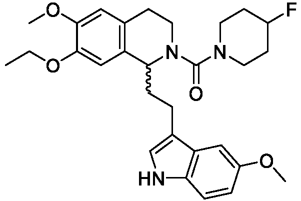

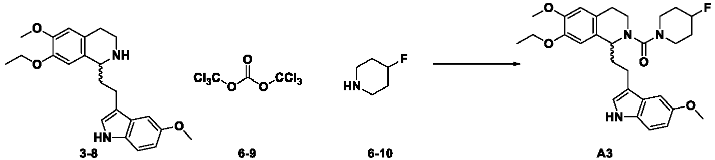

To a solution of compound 4-8 in dichloromethane was added saturated sodium bicarbonate, and a solution of triphosgene in dichloromethane was added to the two-phase system. After 1 hour of reaction at room temperature, the dichloromethane phase was separated. The aqueous phase was extracted twice with dichloromethane, dried with anhydrous sodium sulfate. The solution was transferred to a single necked bottle, added 6-3 and diisopropylethylamine, and reacted overnight at room temperature. After TLC monitored the completion of the reaction, saturated ammonium chloride was added and extracted three times with dichloromethane. The organic phases were combined, washed with saturated sodium chloride, dried with anhydrous sodium sulfate, evaporated the solvent, and subjected to column chromatography (petroleum ether: ethyl acetate 1:1) to obtain white solid A3 with a yield of 90%. ¹H NMR (500 MHz, CDCl₃) δ 9.63 (t, *J =* 2.4 Hz, 1H), 7.22 (dd, *J* = 8.4, 2.1 Hz, 1H), 7.07 (d, *J =* 2.5 Hz, 1H), 7.02 (d, *J =* 2.8 Hz, 1H), 6.76 - 6.69 (m, 2H), 6.61 (t, *J =* 1.0 Hz, 1H), 5.21 (td, *J =* 5.3, 1.0 Hz, 1H), 4.69 (p, *J =* 4.2 Hz, 1H), 4.60 (p, *J* = 4.2 Hz, 1H), 4.20 - 3.95 (m, 5H), 3.83 (d, *J =* 1.0 Hz, 5H), 3.60 (dddd, *J* = 13.2, 10.6, 7.3, 4.9 Hz, 3H), 3.00 - 2.78 (m, 4H), 2.39 (dtd, *J* = 12.7, 7.3, 5.3 Hz, 1H), 2.28 (dtd, *J =* 12.6, 7.2, 5.3 Hz, 1H), 2.06 - 1.79 (m, 5H), 1.42 (t, *J =* 6.9 Hz, 3H). ESI-MS *m*/*z* 510.0 [M+H]⁺.

### Example A4: Synthesis of Compound A4

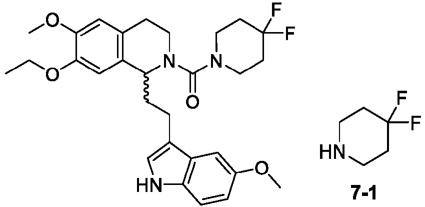

Refering to the synthesis method of compound A3, compound A4 was obtained by replacing 6-10 in Example A3 with compound 7-1. ¹H NMR (500 MHz, CDCl₃) δ 9.63 (t, *J =* 2.4 Hz, 1H), 7.23 (dd, *J =* 8.4, 1.9 Hz, 1H), 7.09 (d, *J =* 2.5 Hz, 1H), 7.05 (d, *J =* 1.7 Hz, 1H), 6.97 (d, *J =* 1.0 Hz, 1H), 6.75 (dd, *J =* 8.4, 2.0 Hz, 1H), 6.63 (t, *J =* 1.0 Hz, 1H), 5.18 (td, *J* = 5.2, 1.0 Hz, 1H), 4.20 - 4.02 (m, 5H), 3.83 (s, 5H), 3.68 (ddd, *J =* 12.1, 6.5, 4.2 Hz, 2H), 3.42 (ddd, *J* = 11.9, 6.5, 4.1 Hz, 1H), 3.02 (dddd, *J =* 14.5, 6.4, 4.2, 0.9 Hz, 1H), 2.90 (dddd, *J* = 14.6, 6.6, 4.1, 1.0 Hz, 1H), 2.68 - 2.58 (m, 1H), 2.55 - 2.40 (m, 2H), 2.40 - 2.29 (m, 1H), 2.32 - 2.21 (m, 2H), 2.14 (tddd, *J =* 21.0, 14.5, 6.5, 4.2 Hz, 2H), 1.42 (t, J = 7.0 Hz, 3H). ESI-MS *m*/*z* 528.3 [M+H]⁺.

### Example A5: Synthesis of Compound A5

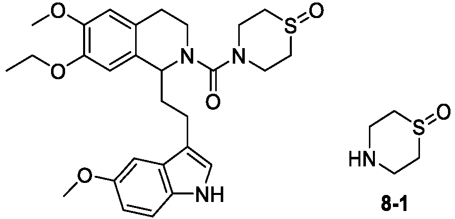

Refering to the synthesis method of compound A3, compound A5 was obtained by replacing 6-10 in Example A3 with compound 8-1. ¹H NMR (500 MHz, CDCl₃) δ 9.63 (t, *J* = 2.4 Hz, 1H), 7.23 (dd, *J* = 8.4, 1.9 Hz, 1H), 7.09 (d, *J =* 2.6 Hz, 1H), 7.05 - 6.97 (m, 2H), 6.74 (dd, *J =* 8.4, 1.8 Hz, 1H), 6.63 (t, *J =* 1.0 Hz, 1H), 5.11 (td, *J =* 5.3, 1.0 Hz, 1H), 4.15 (dq, *J =* 10.1, 6.9 Hz, 1H), 4.12 - 3.96 (m, 2H), 3.83 (d, *J =* 0.7 Hz, 6H), 3.66 (dt, *J =* 10.8, 4.9 Hz, 2H), 3.62 - 3.50 (m, 3H), 3.05 - 2.92 (m, 2H), 2.90 (dt, *J =* 14.6, 7.4 Hz, 1H), 2.80 (t, *J* = 4.9 Hz, 4H), 2.52 - 2.37 (m, 2H), 2.29 (dtd, *J =* 12.6, 7.4, 5.3 Hz, 1H), 1.42 (t, *J* = 7.0 Hz, 3H). ESI-MS *m*/*z* 526.1 [M+H]⁺.

### Example A6: Synthesis of Compound A6

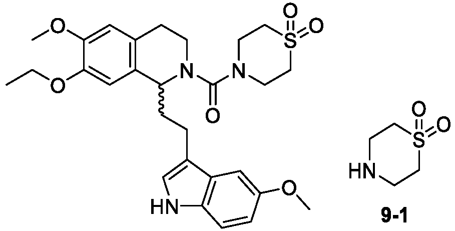

Refering to the synthesis method of compound A3, compound A6 was obtained by replacing 6-10 in Example A3 with compound 9-1. ¹H NMR (500 MHz, CDCl₃) δ 9.63 (t, *J =* 2.4 Hz, 1H), 7.22 (dd, *J =* 8.4, 2.1 Hz, 1H), 7.06 (dd, *J =* 13.2, 2.6 Hz, 2H), 6.85 (d, *J =* 1.1 Hz, 1H), 6.72 (dd, *J =* 8.4, 2.7 Hz, 1H), 6.63 (t, *J =* 1.0 Hz, 1H), 5.13 (td, *J =* 5.3, 0.9 Hz, 1H), 4.20 - 4.01 (m, 3H), 3.83 (s, 5H), 3.67 (dt, *J* = 10.8, 5.0 Hz, 2H), 3.61 (dt, *J =* 10.5, 5.1 Hz, 2H), 3.56 (ddd, *J =* 12.1, 6.4, 4.2 Hz, 1H), 3.26 (t, *J =* 5.1 Hz, 4H), 2.97 (dddd, *J* = 14.5, 6.4, 4.2, 0.9 Hz, 1H), 2.91 (dddd, *J =* 14.5, 6.3, 4.1, 0.9 Hz, 1H), 2.85 (dt, *J =* 14.7, 7.2 Hz, 1H), 2.68 - 2.58 (m, 1H), 2.38 (dtd, *J =* 12.7, 7.3, 5.4 Hz, 1H), 2.27 (dtd, *J =* 12.6, 7.3, 5.5 Hz, 1H), 1.42 (t, *J =* 7.0 Hz, 3H). ESI-MS *m*/*z* 542.2 [M+H]⁺.

### Example A7: Synthesis of Compound A7

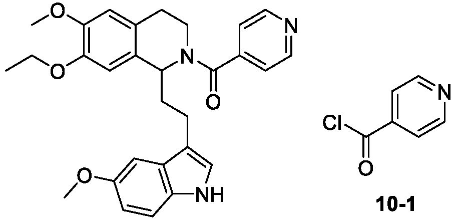

Refering to the synthesis method of compound A1, compound A7 was obtained by replacing the morpholinyl chloride in Example A1 with compound 10-1. ¹H NMR (500 MHz, DMSO-*d*₆) δ 8.69 - 8.64 (m, 2H), 7.77 - 7.72 (m, 2H), 7.26 (d, *J =* 7.6 Hz, 1H), 7.14 (d, *J =* 1.8 Hz, 1H), 7.09 (d, *J =* 8.4 Hz, 1H), 6.82 (dd, *J =* 7.5, 1.5 Hz, 1H), 6.74 (d, *J =* 0.6 Hz, 1H), 6.60 (t, *J =* 1.0 Hz, 1H), 4.93 - 4.86 (m, 1H), 4.06 (qd, *J =* 8.0, 1.3 Hz, 2H), 3.83 - 3.73 (m, 8H), 2.91 (tt, *J=* 7.1*,* 0.8 Hz, 2H), 2.78 - 2.64 (m, 2H), 2.36 - 2.19 (m, 2H), 1.44 - 1.37 (m, 3H). ESI-MS *m*/*z* 486.1 [M+H]⁺.

### Example A8: Synthesis of compound A8

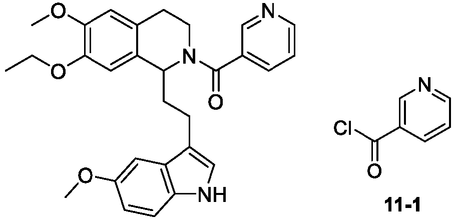

Refering to the synthesis method of compound A1, compound A8 was obtained by replacing the morpholinyl chloride in Example A1 with compound 11-1. ¹H NMR(500 MHz, DMSO-*d*₆) δ 9.01 (d, *J =* 1.5 Hz, 1H), 8.75 (dd, *J* = 7.5, 1.6 Hz, 1H), 8.15 (dt, *J =* 7.5, 1.5 Hz, 1H), 7.43 (t, *J =* 7.5 Hz, 1H), 7.26 (d, *J =* 7.6 Hz, 1H), 7.14 (d, *J =* 1.8 Hz, 1H), 7.09 (d, *J =* 8.4 Hz, 1H), 6.82 (dd, *J =* 7.5, 1.5 Hz, 1H), 6.74 (d, *J =* 0.6 Hz, 1H), 6.60 (t, *J =* 1.0 Hz, 1H), 4.93 - 4.86 (m, 1H), 4.06 (qd, *J =* 8.0, 1.3 Hz, 2H), 3.84 - 3.77 (m, 8H), 2.91 (tt, *J =* 7.1, 0.9 Hz, 2H), 2.78 - 2.64 (m, 2H), 2.36 - 2.19 (m, 2H), 1.44 - 1.37 (m, 3H). ESI-MS *m*/*z* 486.1 [M+H]⁺.

### Example A9: Synthesis of compound A9

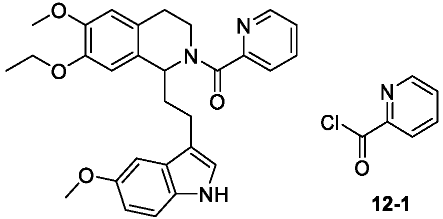

Refering to the synthesis method of compound A1, compound A9 was obtained by replacing the morpholinyl chloride in Example A1 with compound 12-1. ¹H NMR(500 MHz, DMSO-*d*₆) δ 8.64 (dd, *J* = 7.5, 1.5 Hz, 1H), 7.91 (td, *J* = 7.5, 1.6 Hz, 1H), 7.79 (dd, *J =* 7.4, 1.6 Hz, 1H), 7.57 (td, *J* = 7.5, 1.5 Hz, 1H), 7.26 (d, *J* = 7.6 Hz, 1H), 7.14 (d, *J* = 1.8 Hz, 1H), 7.09 (d, *J =* 8.4 Hz, 1H), 6.82 (dd, *J* = 7.5, 1.5 Hz, 1H), 6.74 (d, *J =* 0.6 Hz, 1H), 6.60 (t, *J* = 1.0 Hz, 1H), 4.98 - 4.92 (m, 1H), 4.06 (qd, *J =* 8.0, 1.3 Hz, 2H), 3.87 - 3.75 (m, 9H), 2.91 (tt, *J =* 7.1, 0.8 Hz, 2H), 2.78 - 2.64 (m, 2H), 2.36 - 2.19 (m, 2H), 1.44 - 1.37 (m, 3H). ESI-MS *m*/*z* 486.2 [M+H]⁺.

### Example A10: Synthesis of Compound A10

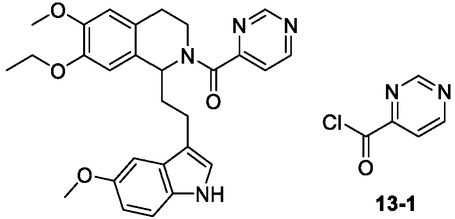

Refering to the synthesis method of compound A1, compound A10 was obtained by replacing the morpholinyl chloride in Example A1 with compound 13-1. ¹H NMR(500 MHz, DMSO-*d*₆) δ 9.43 (d, *J =* 1.5 Hz, 1H), 8.84 (d, *J =* 7.5 Hz, 1H), 7.72 (dd, *J* = 7.5, 1.6 Hz, 1H), 7.26 (d, *J =* 7.6 Hz, 1H), 7.14 (d, *J =* 1.8 Hz, 1H), 7.09 (d, *J =* 8.4 Hz, 1H), 6.82 (dd, *J =* 7.5, 1.5 Hz, 1H), 6.74 (d, *J* = 0.7 Hz, 1H), 6.60 (t, *J =* 1.0 Hz, 1H), 4.98 - 4.92 (m, 1H), 4.06 (qd, *J* = 8.0, 1.3 Hz, 2H), 3.84 - 3.75 (m, 8H), 2.91 (tt, *J =* 7.1, 0.8 Hz, 2H), 2.78 (dt, *J =* 12.3, 7.1 Hz, 1H), 2.70 (dt, *J =* 12.3, 7.0 Hz, 1H), 2.36 - 2.19 (m, 2H), 1.44 - 1.37 (m, 3H). ESI-MS *m*/*z* 487.2 [M+H]⁺.

### Example A11: Synthesis of compound A11

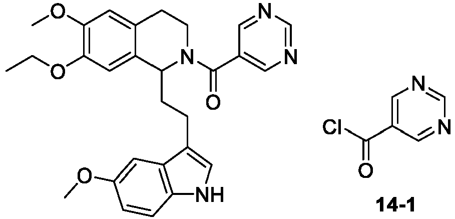

Refering to the synthesis method of compound A1, compound A11 was obtained by replacing the morpholinyl chloride in Example A1 with compound 14-1. ¹H NMR(500 MHz, DMSO-*d*₆) δ 9.21 (s, 2H), 9.07 (s, 1H), 7.26 (d, *J =* 7.6 Hz, 1H), 7.14 (d, *J =* 1.8 Hz, 1H), 7.09 (d, *J* = 8.4 Hz, 1H), 6.82 (dd, *J* = 7.5, 1.5 Hz, 1H), 6.74 (d, *J =* 0.7 Hz, 1H), 6.60 (t, *J* = 1.0 Hz, 1H), 4.92 - 4.85 (m, 1H), 4.06 (qd, *J* = 8.0, 1.3 Hz, 2H), 3.84 - 3.77 (m, 8H), 2.91 (tt, *J =* 7.2, 1.0 Hz, 2H), 2.78 - 2.64 (m, 2H), 2.35 - 2.18 (m, 2H), 1.44 - 1.37 (m, 3H). ESI-MS *m*/*z* 487.2 [M+H]⁺.

### Example A12: Synthesis of compound A12

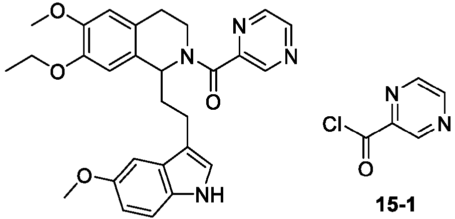

Refering to the synthesis method of compound A1, compound A12 was obtained by replacing the morpholinyl chloride in Example A1 with compound 15-1. ¹H NMR(500 MHz, DMSO-*d*₆) δ 9.22 (s, 1H), 8.82 (d, *J =* 1.8 Hz, 2H), 7.26 (d, *J =* 7.6 Hz, 1H), 7.14 (d, *J =* 1.8 Hz, 1H), 7.09 (d, *J =* 8.4 Hz, 1H), 6.82 (dd, *J* = 7.5, 1.5 Hz, 1H), 6.74 (d, *J =* 0.6 Hz, 1H), 6.60 (t, *J =* 1.0 Hz, 1H), 4.95 (td, *J =* 6.9, 0.6 Hz, 1H), 4.06 (qd, *J =* 8.0, 1.3 Hz, 2H), 3.89 - 3.77 (m, 8H), 2.91 (tt, *J =* 7.1, 0.9 Hz, 2H), 2.78 (dt, *J =* 12.3, 7.1 Hz, 1H), 2.70 (dt, *J =* 12.3, 7.1 Hz, 1H), 2.36 - 2.19 (m, 2H), 1.44 - 1.37 (m, 3H). ESI-MS *m*/*z* 487.2 [M+H]⁺.

### Example A13: Synthesis of compound A13

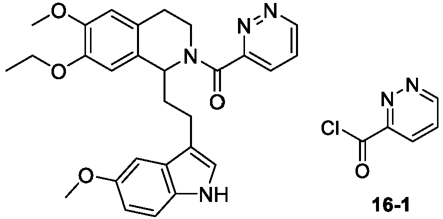

Refering to the synthesis method of compound A1, compound A13 was obtained by replacing the morpholinyl chloride in Example A1 with compound 16-1. ¹H NMR(500 MHz, DMSO-*d*₆) δ 8.82 (dd, *J =* 7.5, 1.5 Hz, 1H), 8.13 (dd, *J* = 7.5, 1.5 Hz, 1H), 7.95 (t, *J =* 7.4 Hz, 1H), 7.26 (d, *J =* 7.6 Hz, 1H), 7.14 (d, *J =* 1.8 Hz, 1H), 7.09 (d, *J =* 8.4 Hz, 1H), 6.82 (dd, *J* = 7.5, 1.5 Hz, 1H), 6.74 (d, *J =* 0.6 Hz, 1H), 6.60 (d, *J =* 1.0 Hz, 1H), 4.95 (td, *J =* 6.9, 0.7 Hz, 1H), 4.06 (qd, *J =* 8.0, 1.3 Hz, 2H), 3.89 - 3.77 (m, 8H), 2.91 (tt, *J* = 7.1, 0.9 Hz, 2H), 2.78 (dt, *J =* 12.3, 7.1 Hz, 1H), 2.70 (dt, *J =* 12.2, 7.1 Hz, 1H), 2.36 - 2.19 (m, 2H), 1.44 - 1.37 (m, 3H). ESI-MS *m*/*z* 487.2 [M+H]⁺.

### Example A14: Synthesis of compound A14

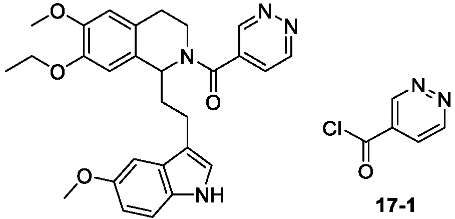

Refering to the synthesis method of compound A1, compound A14 was obtained by replacing the morpholinyl chloride in Example A1 with compound 17-1. ¹H NMR(500 MHz, DMSO-*d*₆) δ 9.46 (d, *J =* 1.5 Hz, 1H), 9.25 (d, *J =* 7.5 Hz, 1H), 7.97 (dd, *J* = 7.5, 1.6 Hz, 1H), 7.26 (d, *J* = 7.6 Hz, 1H), 7.14 (d, *J =* 1.8 Hz, 1H), 7.09 (d, *J =* 8.4 Hz, 1H), 6.82 (dd, *J =* 7.5, 1.5 Hz, 1H), 6.74 (d, *J =* 0.6 Hz, 1H), 6.60 (t, *J =* 1.0 Hz, 1H), 4.93 - 4.86 (m, 1H), 4.06 (qd, *J* = 8.0, 1.3 Hz, 2H), 3.84 - 3.71 (m, 9H), 2.91 (tt, *J* = 7.1, 0.8 Hz, 2H), 2.78 - 2.64 (m, 2H), 2.36 - 2.19 (m, 2H), 1.44 - 1.37 (m, 3H). ESI-MS *m*/*z* 487.2 [M+H]⁺.

### Example A15: Synthesis of compound A15

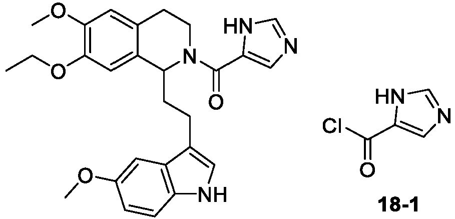

Refering to the synthesis method of compound A1, compound A15 was obtained by replacing the morpholinyl chloride in Example A1 with compound 18-1. ¹H NMR(500 MHz, DMSO-*d*₆) δ 8.19 (s, 1H), 8.15 (d, *J =* 8.2 Hz, 1H), 7.26 (d, *J =* 7.6 Hz, 1H), 7.14 (d, *J =* 1.4 Hz, 1H), 7.09 (d, *J* = 8.4 Hz, 1H), 6.82 (dd, *J* = 7.5, 1.5 Hz, 1H), 6.74 (d, *J* = 0.7 Hz, 1H), 6.60 (t, *J* = 1.0 Hz, 1H), 5.01 - 4.95 (m, 1H), 4.08 (qd, *J* = 8.0, 4.6 Hz, 2H), 3.87 - 3.77 (m, 7H), 2.96 (dt, *J* = 12.3, 7.1 Hz, 1H), 2.91 (tt, *J* = 7.1, 0.9 Hz, 2H), 2.70 (dt, *J =* 12.4, 7.1 Hz, 1H), 2.36 - 2.19 (m, 2H), 1.40 (t, *J =* 8.0 Hz, 3H). ESI-MS *m*/*z* 475.2 [M+H]⁺.

### Example A16: Synthesis of compound A16

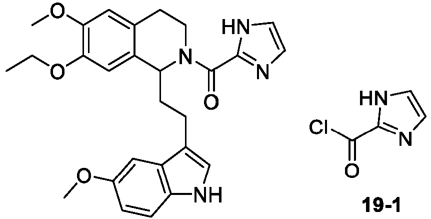

Refering to the synthesis method of compound A1, compound A16 was obtained by replacing the morpholinyl chloride in Example A1 with compound 19-1. ¹H NMR (500 MHz, CDCl₃) δ 9.52 (t, *J* = 2.4 Hz, 1H), 7.56 - 7.51 (m, 2H), 7.23 (dd, *J =* 8.4, 2.1 Hz, 1H), 7.09 (d, *J* = 2.6 Hz, 1H), 7.02 (d, *J* = 1.8 Hz, 1H), 6.95 (d, *J* = 0.9 Hz, 1H), 6.75 (dd, *J =* 8.4, 1.8 Hz, 1H), 6.64 (t, *J* = 1.0 Hz, 1H), 5.06 (td, *J* = 5.5, 1.1 Hz, 1H), 4.19 - 3.96 (m, 3H), 3.88 - 3.80 (m, 7H), 3.12 - 3.01 (m, 2H), 2.98 - 2.86 (m, 2H), 2.42 (dtd, *J =* 12.6, 7.3, 5.3 Hz, 1H), 2.29 (dtd, *J* = 12.6, 7.3, 5.4 Hz, 1H), 1.42 (t, *J* = 7.0 Hz, 3H). ESI-MS *m*/*z* 475.2 [M+H]⁺.

### Example A17: Synthesis of compound A17

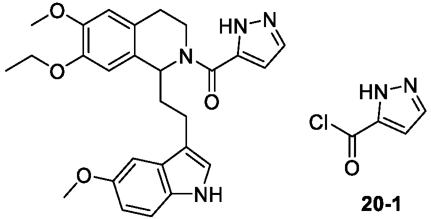

Refering to the synthesis method of compound A1, compound A17 was obtained by replacing the morpholinyl chloride in Example A1 with compound 20-1. ¹H NMR(500 MHz, DMSO-*d*₆) δ 8.34 (d, *J =* 7.5 Hz, 1H), 7.26 (d, *J* = 7.6 Hz, 1H), 7.14 (d, *J =* 1.4 Hz, 1H), 7.09 (d, *J* = 8.4 Hz, 1H), 6.96 (d, *J =* 7.5 Hz, 1H), 6.82 (dd, *J =* 7.5, 1.5 Hz, 1H), 6.74 (d, *J* = 0.6 Hz, 1H), 6.60 (t, *J =* 1.0 Hz, 1H), 4.97 (t, *J =* 6.9 Hz, 1H), 4.08 (qd, *J =* 8.0, 4.6 Hz, 2H), 3.87 - 3.77 (m, 7H), 2.96 (dt, *J =* 12.3, 7.1 Hz, 1H), 2.91 (tt, *J =* 7.1*,* 0.8 Hz, 2H), 2.70 (dt, *J =* 12.4, 7.1 Hz, 1H), 2.36 - 2.19 (m, 2H), 1.40 (t, *J =* 8.0 Hz, 3H). ESI-MS *m*/*z* 475.2 [M+H]⁺.

### Example A18: Synthesis of compound A18

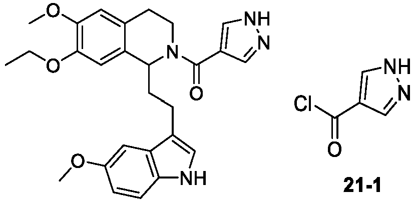

Refering to the synthesis method of compound A1, compound A18 was obtained by replacing the morpholinyl chloride in Example A1 with compound 21-1. ¹H NMR(500 MHz, DMSO-*d*₆) δ 8.18 (d, *J =* 1.4 Hz, 1H), 8.06 (dd, *J* = 5.9, 1.5 Hz, 1H), 7.26 (d, *J =* 7.6 Hz, 1H), 7.14 (d, *J =* 1.4 Hz, 1H), 7.09 (d, *J =* 8.4 Hz, 1H), 6.82 (dd, *J =* 7.5, 1.5 Hz, 1H), 6.74 (d, *J* = 0.6 Hz, 1H), 6.59 (d, *J =* 1.0 Hz, 1H), 4.92 - 4.85 (m, 1H), 4.08 (qd, *J =* 8.0, 4.6 Hz, 2H), 3.84 - 3.77 (m, 8H), 2.96 (dt, *J =* 12.3, 7.1 Hz, 1H), 2.91 (tt, *J =* 7.2*,* 0.9 Hz, 2H), 2.70 (dt, *J =* 12.3, 7.1 Hz, 1H), 2.34 - 2.17 (m, 2H), 1.40 (t, *J =* 8.0 Hz, 3H). ESI-MS *m*/*z* 475.2 [M+H]⁺.

### Example A19: Synthesis of compound A19

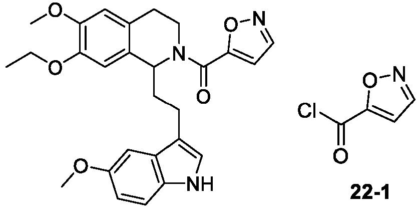

Refering to the synthesis method of compound A1, compound A19 was obtained by replacing the morpholinyl chloride in Example A1 with compound 22-1. ¹H NMR(500 MHz, DMSO-*d*₆) δ 8.34 (d, *J* = 7.5 Hz, 1H), 7.26 (d, *J* = 7.6 Hz, 1H), 7.14 (d, *J* = 1.4 Hz, 1H), 7.09 (d, *J* = 8.4 Hz, 1H), 7.00 (d, *J* = 7.5 Hz, 1H), 6.82 (dd, *J* = 7.5, 1.5 Hz, 1H), 6.74 (s, 1H), 6.59 (t, *J =* 0.9 Hz, 1H), 4.89 - 4.83 (m, 1H), 4.08 (qd, *J =* 8.0, 4.6 Hz, 2H), 3.80 (d, *J* = 13.7 Hz, 5H), 3.72 (td, *J* = 7.0, 1.2 Hz, 2H), 2.91 (tt, *J* = 7.0, 0.8 Hz, 2H), 2.74 (qt, *J =* 12.5, 7.1 Hz, 2H), 2.35 - 2.19 (m, 2H), 1.40 (t, *J =* 8.0 Hz, 3H). ESI-MS *m*/*z* 476.2 [M+H]⁺.

### Example A20: Synthesis of Compound A20

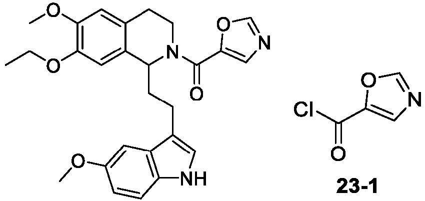

Refering to the synthesis method of compound A1, compound A20 was obtained by replacing the morpholinyl chloride in Example A1 with compound 23-1. ¹H NMR(500 MHz, DMSO-*d*₆) δ 8.29 (s, 1H), 8.05 (s, 1H), 7.26 (d, *J =* 7.6 Hz, 1H), 7.14 (d, *J =* 1.4 Hz, 1H), 7.09 (d, *J* = 8.4 Hz, 1H), 6.82 (dd, *J* = 7.5, 1.5 Hz, 1H), 6.74 (d, *J =* 0.7 Hz, 1H), 6.59 (t, *J* = 0.9 Hz, 1H), 4.89 - 4.83 (m, 1H), 4.08 (qd, *J* = 8.0, 4.6 Hz, 2H), 3.80 (d, *J* = 13.7 Hz, 5H), 3.72 (td, *J =* 7.0, 1.2 Hz, 2H), 2.91 (tt, *J =* 7.0, 0.8 Hz, 2H), 2.74 (qt, *J =* 12.5, 7.1 Hz, 2H), 2.35 - 2.19 (m, 2H), 1.40 (t, *J =* 8.0 Hz, 3H). ESI-MS *m*/*z* 476.2 [M+H]⁺.

### Example A21: Synthesis of Compound A21

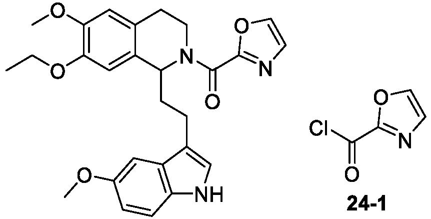

Refering to the synthesis method of compound A1, compound A22 was obtained by replacing the morpholinyl chloride in Example A1 with compound 24-1. ¹H NMR(500 MHz, DMSO-*d*₆) δ 8.07 (d, *J* = 7.5 Hz, 1H), 7.47 (d, *J* = 7.5 Hz, 1H), 7.26 (d, *J* = 7.6 Hz, 1H), 7.14 (d, *J =* 1.4 Hz, 1H), 7.09 (d, *J =* 8.4 Hz, 1H), 6.82 (dd, *J =* 7.5, 1.5 Hz, 1H), 6.74 (d, *J* = 0.6 Hz, 1H), 6.59 (t, *J* = 0.9 Hz, 1H), 4.94 - 4.87 (m, 1H), 4.08 (qd, *J =* 8.0, 4.6 Hz, 2H), 3.83 - 3.72 (m, 7H), 2.91 (tt, *J* = 7.1, 0.8 Hz, 2H), 2.74 (qt, *J* = 12.5, 7.1 Hz, 2H), 2.36 - 2.19 (m, 2H), 1.40 (t, *J =* 8.0 Hz, 3H). ESI-MS *m*/*z* 476.2 [M+H]⁺.

### Example A22: Synthesis of Compound A22

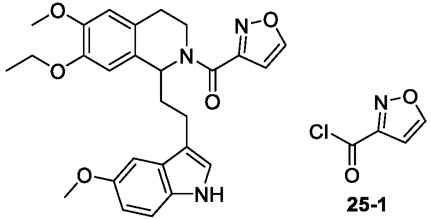

Refering to the synthesis method of compound A1, compound A22 was obtained by replacing the morpholinyl chloride in Example A1 with compound 25-1. ¹H NMR(500 MHz, DMSO-*d*₆) δ 8.93 (d, *J* = 7.5 Hz, 1H), 7.26 (d, *J* = 7.6 Hz, 1H), 7.14 (d, *J* = 1.4 Hz, 1H), 7.09 (d, *J* = 8.4 Hz, 1H), 6.85 - 6.76 (m, 2H), 6.74 (d, *J =* 0.6 Hz, 1H), 6.60 (t, *J =* 1.0 Hz, 1H), 4.95 (td, *J* = 6.9, 0.6 Hz, 1H), 4.08 (qd, *J* = 8.0, 4.6 Hz, 2H), 3.87 - 3.77 (m, 7H), 2.91 (tt, *J =* 7.1, 0.9 Hz, 2H), 2.78 (dt, *J =* 12.3, 7.1 Hz, 1H), 2.72 (dt, *J =* 12.2, 7.1 Hz, 1H), 2.35 - 2.19 (m, 2H), 1.40 (t, *J =* 8.0 Hz, 3H). ESI-MS *m*/*z* 476.2 [M+H]⁺.

### Example A23: Synthesis of Compound A23

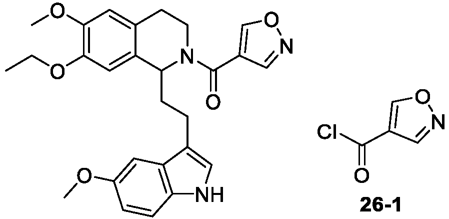

Refering to the synthesis method of compound A1, compound A23 was obtained by replacing the morpholinyl chloride in Example A1 with compound 26-1. ¹H NMR(500 MHz, DMSO-*d*₆) δ 9.02 (d, *J* = 1.4 Hz, 1H), 8.38 (d, *J* = 1.4 Hz, 1H), 7.26 (d, *J* = 7.6 Hz, 1H), 7.14 (d, *J =* 1.4 Hz, 1H), 7.09 (d, *J =* 8.4 Hz, 1H), 6.82 (dd, *J =* 7.5, 1.5 Hz, 1H), 6.74 (d, *J* = 0.6 Hz, 1H), 6.59 (d, *J =* 1.0 Hz, 1H), 4.92 - 4.85 (m, 1H), 4.08 (qd, *J =* 8.0, 4.6 Hz, 2H), 3.83 - 3.77 (m, 8H), 2.91 (tt, *J =* 7.2, 0.9 Hz, 2H), 2.79 - 2.66 (m, 2H), 2.34 - 2.17 (m, 2H), 1.40 (t, *J* = 8.0 Hz, 3H). ESI-MS *m*/*z* 476.2 [M+H]⁺.

### Example A24: Synthesis of Compound A24

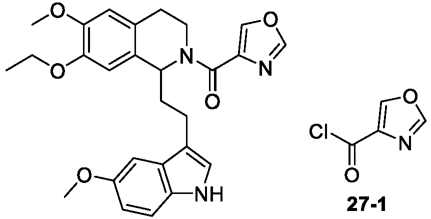

Refering to the synthesis method of compound A1, compound A24 was obtained by replacing the morpholinyl chloride in Example A1 with compound 27-1. ¹H NMR(500 MHz, DMSO-*d*₆) δ 8.25 (d, *J* = 1.4 Hz, 1H), 7.99 (d, *J* = 1.4 Hz, 1H), 7.26 (d, *J* = 7.6 Hz, 1H), 7.14 (d, *J =* 1.4 Hz, 1H), 7.09 (d, *J =* 8.4 Hz, 1H), 6.82 (dd, *J =* 7.5, 1.5 Hz, 1H), 6.74 (d, *J* = 0.7 Hz, 1H), 6.60 (t, *J =* 1.0 Hz, 1H), 4.95 (td, *J =* 6.9, 0.6 Hz, 1H), 4.08 (qd, *J =* 8.0, 4.6 Hz, 2H), 3.87 - 3.77 (m, 8H), 2.91 (tt, *J* = 7.1, 0.9 Hz, 2H), 2.83 - 2.67 (m, 2H), 2.35 - 2.19 (m, 2H), 1.40 (t, *J =* 8.0 Hz, 3H). ESI-MS *m*/*z* 476.2 [M+H]⁺.

### Example A25: Synthesis of Compound A25

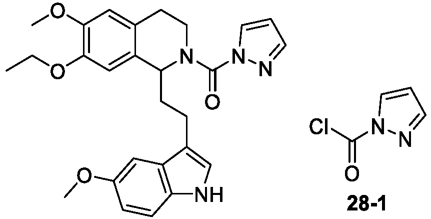

Refering to the synthesis method of compound A1, compound A25 was obtained by replacing the morpholinyl chloride in Example A1 with compound 28-1. ¹H NMR(500 MHz, DMSO-*d*₆) δ 8.47 (dd, *J =* 7.5, 1.6 Hz, 1H), 7.72 (dd, *J =* 7.5, 1.5 Hz, 1H), 7.26 (d, *J =* 7.6 Hz, 1H), 7.14 (d, *J =* 1.4 Hz, 1H), 7.09 (d, *J =* 8.4 Hz, 1H), 6.82 (dd, *J* = 7.5, 1.5 Hz, 1H), 6.77 - 6.71 (m, 2H), 6.60 (t, *J =* 1.0 Hz, 1H), 4.98 - 4.92 (m, 1H), 4.15 - 4.00 (m, 2H), 3.88 - 3.77 (m, 8H), 2.96 (dt, *J =* 12.3, 7.1 Hz, 1H), 2.91 (tt, *J =* 7.1, 0.9 Hz, 2H), 2.70 (dt, *J =* 12.4, 7.1 Hz, 1H), 2.35 - 2.18 (m, 2H), 1.40 (t, *J =* 8.0 Hz, 3H). ESI-MS *m*/*z* 475.2 [M+H]⁺.

### Example A26: Synthesis of Compound A26

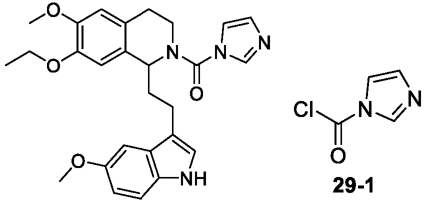

Refering to the synthesis method of compound A1, compound A26 was obtained by replacing the morpholinyl chloride in Example A1 with compound 29-1. ¹H NMR(500 MHz, DMSO-*d*₆) δ 8.27 (s, 1H), 7.28 - 7.23 (m, 2H), 7.22 (d, *J =* 7.5 Hz, 1H), 7.14 (d, *J =* 1.4 Hz, 1H), 7.09 (d, *J =* 8.4 Hz, 1H), 6.82 (dd, *J* = 7.5, 1.5 Hz, 1H), 6.74 (d, *J =* 0.6 Hz, 1H), 6.60 (t, *J* = 1.0 Hz, 1H), 4.93 - 4.87 (m, 1H), 4.08 (qd, *J =* 8.0, 4.6 Hz, 2H), 3.88 - 3.75 (m, 8H), 2.96 (dt, *J* = 12.3, 7.1 Hz, 1H), 2.91 (tt, *J* = 7.1, 1.1 Hz, 2H), 2.70 (dt, *J =* 12.3, 7.1 Hz, 1H), 2.33 - 2.17 (m, 2H), 1.40 (t, *J =* 8.0 Hz, 3H). ESI-MS *m*/*z* 475.2 [M+H]⁺.

### Example A27: Synthesis of Compound A27

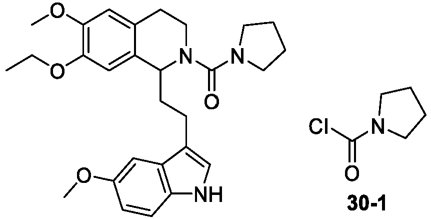

Refering to the synthesis method of compound A1, compound A27 was obtained by replacing the morpholinyl chloride in Example A1 with compound 30-1. ¹H NMR(500 MHz, DMSO-*d*₆) δ 7.26 (d, *J =* 7.6 Hz, 1H), 7.14 (d, *J* = 1.4 Hz, 1H), 7.09 (d, *J =* 8.4 Hz, 1H), 6.82 (dd, *J =* 7.5, 1.5 Hz, 1H), 6.75 (d, *J =* 0.6 Hz, 1H), 6.60 (t, *J =* 1.0 Hz, 1H), 4.87 (td, *J* = 7.0, 0.6 Hz, 1H), 4.07 (qd, *J* = 7.9, 4.4 Hz, 2H), 3.80 (d, *J =* 13.5 Hz, 7H), 3.56 (dddt, *J* = 7.5, 6.1, 2.9, 1.5 Hz, 4H), 3.47 (dt, *J =* 12.5, 7.1 Hz, 1H), 2.91 (tt, *J =* 7.1, 0.9 Hz, 2H), 2.78 - 2.64 (m, 2H), 2.37 - 2.19 (m, 2H), 1.98 - 1.86 (m, 4H), 1.40 (t, *J =* 8.0 Hz, 3H). ESI-MS *m*/*z* 478.3 [M+H]⁺.

### Example A28: Synthesis of Compound A28

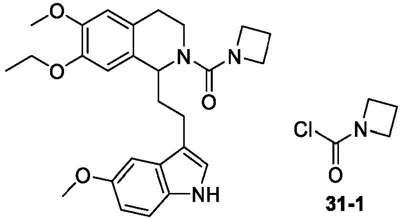

Refering to the synthesis method of compound A1, compound A28 was obtained by replacing the morpholinyl chloride in Example A1 with compound 31-1. ¹H NMR(500 MHz, DMSO-*d*₆) δ 7.26 (d, *J* = 7.6 Hz, 1H), 7.14 (d, *J* = 1.4 Hz, 1H), 7.09 (d, *J* = 8.4 Hz, 1H), 6.82 (dd, J= 7.5, 1.5 Hz, 1H), 6.75 (d, *J =* 0.6 Hz, 1H), 6.60 (t, *J =* 1.0 Hz, 1H), 4.90 - 4.84 (m, 1H), 4.08 (qd, *J =* 8.0, 2.9 Hz, 2H), 3.80 (d, *J =* 12.1 Hz, 7H), 3.57 (t, *J =* 7.1 Hz, 2H), 3.52 - 3.43 (m, 3H), 2.96 (dt, *J =* 12.3, 7.1 Hz, 1H), 2.91 (tt, *J =* 7.1, 0.9 Hz, 2H), 2.70 (dt, *J* = 12.4, 7.1 Hz, 1H), 2.37 - 2.19 (m, 2H), 1.93 (p, *J* = 7.1 Hz, 2H), 1.40 (t, *J* = 8.0 Hz, 3H). ESI-MS *m*/*z* 464.2 [M+H]⁺.

### Example A29: Synthesis of Compound A29

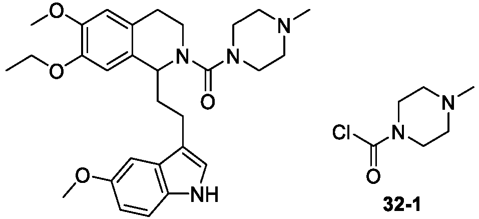

Refering to the synthesis method of compound A1, compound A29 was obtained by replacing the morpholinyl chloride in Example A1 with compound 32-1. ¹H NMR(500 MHz, DMSO-*d*₆) δ 7.26 (d, *J =* 7.6 Hz, 1H), 7.14 (d, *J* = 1.8 Hz, 1H), 7.09 (d, *J =* 8.4 Hz, 1H), 6.82 (dd, *J =* 7.4, 1.5 Hz, 1H), 6.74 (s, 1H), 6.60 (t, *J =* 0.9 Hz, 1H), 4.87 (td, *J =* 6.9, 0.6 Hz, 1H), 4.06 (qd, *J =* 8.1, 2.1 Hz, 2H), 3.83 - 3.74 (m, 7H), 3.52 - 3.42 (m, 5H), 2.91 (tt, *J =* 7.1, 0.9 Hz, 2H), 2.77 - 2.64 (m, 2H), 2.45 (t, *J =* 7.1 Hz, 4H), 2.28 (s, 3H), 2.36 - 2.18 (m, 2H), 1.44 - 1.37 (m, 3H). ESI-MS *m*/*z* 507.3 [M+H]⁺.

### Example A30: Synthesis of Compound A30

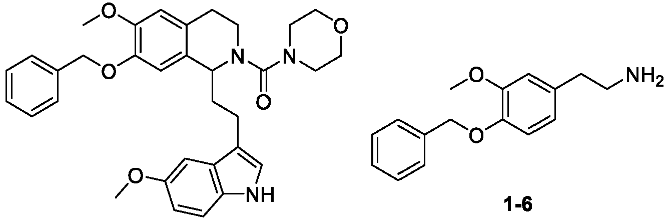

Refering to the synthesis method of compound A1, compound A30 was obtained by replacing 1-5 in Example A1 with compound 1-6. ¹H NMR(500 MHz, DMSO-*d₆*) δ 7.42 (ddt, J = 7.8, 1.9, 1.0 Hz, 2H), 7.36 (ddt, J = 7.5, 6.5, 1.0 Hz, 2H), 7.36 - 7.28 (m, 1H), 7.26 (d, *J* = 7.6 Hz, 1H), 7.14 (d, *J =* 1.4 Hz, 1H), 7.10 (d, *J =* 8.4 Hz, 1H), 6.82 (dd, *J =* 7.5, 1.6 Hz, 1H), 6.76 (d, *J =* 0.6 Hz, 1H), 6.60 (d, *J =* 1.0 Hz, 1H), 5.16 (dt, *J =* 12.5, 1.0 Hz, 1H), 5.08 (dt, *J =* 12.4, 1.1 Hz, 1H), 4.87 (td, *J* = 7.0, 0.6 Hz, 1H), 3.83 - 3.74 (m, 7H), 3.52 (t, *J =* 7.1 Hz, 4H), 3.47 (dt, *J =* 12.5, 7.1 Hz, 1H), 3.13 (dt, *J =* 12.5, 7.0 Hz, 2H), 2.96 (dt, *J =* 12.5, 7.2 Hz, 2H), 2.91 (tt, *J =* 7.1, 1.1 Hz, 2H), 2.77 - 2.65 (m, 2H), 2.36 - 2.18 (m, 2H). ESI-MS *m*/*z* 556.3 [M+H]⁺.

### Example A31: Synthesis of Compound A31

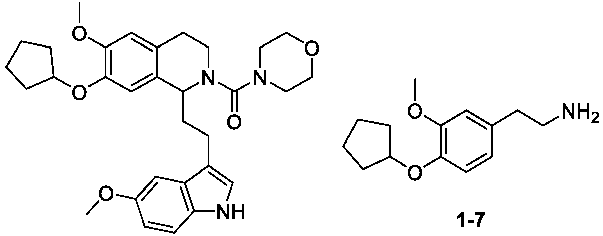

Refering to the synthesis method of compound A1, compound A31 was obtained by replacing 1-5 in Example A1 with compound 1-7. ¹H NMR(500 MHz, DMSO-*d₆*) δ 7.26 (d, *J* = 7.6 Hz, 1H), 7.15 (d, *J* = 1.8 Hz, 1H), 7.10 (d, *J* = 8.4 Hz, 1H), 6.85 - 6.79 (m, 2H), 6.59 (t, *J =* 1.0 Hz, 1H), 4.87 (t, *J =* 6.9 Hz, 1H), 4.84 - 4.75 (m, 1H), 3.79 (d, *J =* 4.4 Hz, 8H), 3.55 - 3.43 (m, 5H), 3.13 (dt, *J =* 12.5, 7.0 Hz, 2H), 3.00 - 2.86 (m, 4H), 2.77 - 2.65 (m, 2H), 2.36 - 2.18 (m, 2H), 1.76 - 1.66 (m, 1H), 1.70 - 1.63 (m, 2H), 1.66 - 1.56 (m, 1H), 1.57 (ddt, *J =* 4.2, 3.2, 1.6 Hz, 1H). ESI-MS *m*/*z* 534.3 [M+H]⁺.

### Example A32: Synthesis of Compound A32

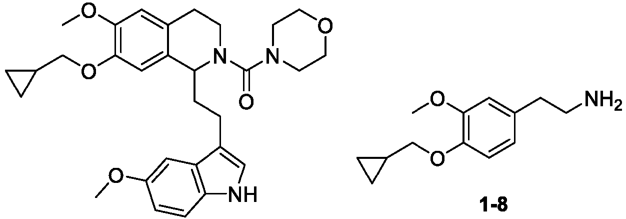

Refering to the synthesis method of compound A1, compound A32 was obtained by replacing 1-5 in Example A1 with compound 1-8. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 6.82 (dd, *J* = 7.5, 1.6 Hz, 1H), 6.75 (d, *J =* 0.6 Hz, 1H), 6.61 (t, *J =* 1.0 Hz, 1H), 4.87 (td, *J =* 7.0, 0.6 Hz, 1H), 3.94 (dd, *J =* 12.4, 7.0 Hz, 1H), 3.86 (dd, *J =* 12.4, 7.1 Hz, 1H), 3.82 - 3.74 (m, 7H), 3.53 (s, 1H), 3.53 - 3.43 (m, 4H), 3.07 - 3.01 (m, 1H), 3.01 (d, *J* = 7.1 Hz, 1H), 2.98 (d, *J =* 7.2 Hz, 1H), 2.98 - 2.91 (m, 1H), 2.91 (tt, *J* = 7.2, 0.9 Hz, 2H), 2.71 (td, *J =* 7.1, 0.9 Hz, 2H), 2.36 - 2.18 (m, 2H), 1.26 (hept, *J =* 7.0 Hz, 1H), 0.72 - 0.59 (m, 2H), 0.48 - 0.35 (m, 2H). ESI-MS *m*/*z* 520.3 [M+H]⁺.

### Example A33: Synthesis of Compound A33

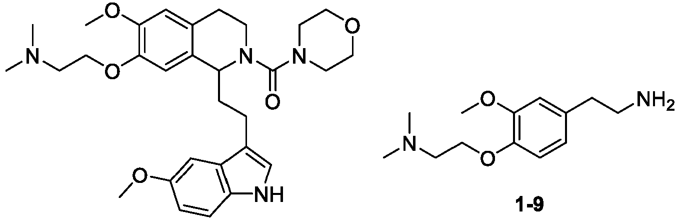

Refering to the synthesis method of compound A1, compound A33 was obtained by replacing 1-5 in Example A1 with compound 1-9. ¹H NMR(500 MHz, DMSO-*d₆*) δ 7.26 (d, *J =* 7.6 Hz, 1H), 7.15 (d, *J* = 1.8 Hz, 1H), 7.10 (d, *J =* 8.4 Hz, 1H), 6.82 (dd, *J* = 7.5, 1.6 Hz, 1H), 6.75 (d, *J =* 0.6 Hz, 1H), 6.59 (t, *J =* 1.0 Hz, 1H), 4.87 (td, *J =* 7.0, 0.6 Hz, 1H), 4.15 (dt, *J =* 12.5, 7.1 Hz, 1H), 4.09 (dt, *J =* 12.5, 7.1 Hz, 1H), 3.83 - 3.74 (m, 7H), 3.52 (t, *J =* 7.1 Hz, 4H), 3.47 (dt, *J =* 12.5, 7.1 Hz, 1H), 3.07 - 3.01 (m, 1H), 3.03 - 2.86 (m, 6H), 2.80 (dt, *J =* 12.4, 7.1 Hz, 1H), 2.76 - 2.65 (m, 2H), 2.35 (s, 5H), 2.36 - 2.25 (m, 1H), 2.27 - 2.18 (m, 1H). ESI-MS *m*/*z* 537.3 [M+H]⁺.

### Example A34: Synthesis of Compound A34

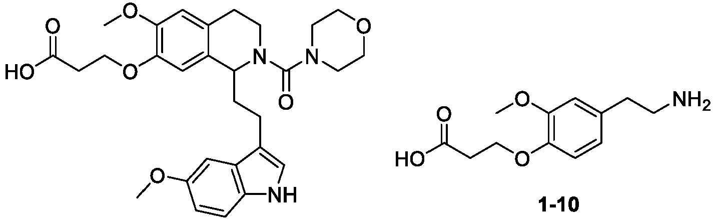

Refering to the synthesis method of compound A1, compound A34 was obtained by replacing 1-5 in Example A1 with compound 1-10. ¹H NMR(500 MHz, DMSO-*d₆*) δ 7.26 (d, *J* = 7.6 Hz, 1H), 7.15 (d, *J* = 1.8 Hz, 1H), 7.10 (d, *J* = 8.4 Hz, 1H), 6.82 (dd, *J* = 7.5, 1.6 Hz, 1H), 6.75 (d, *J* = 0.6 Hz, 1H), 6.60 (t, *J* = 1.0 Hz, 1H), 4.87 (td, *J =* 6.9, 0.6 Hz, 1H), 4.24 (t, *J* = 7.1 Hz, 2H), 3.83 - 3.74 (m, 7H), 3.52 (t, *J* = 7.1 Hz, 4H), 3.47 (dt, *J* = 12.5, 7.1 Hz, 1H), 3.07 - 3.01 (m, 1H), 3.01 (d, *J* = 7.1 Hz, 1H), 2.97 (d, *J =* 7.0 Hz, 1H), 2.97 - 2.91 (m, 1H), 2.91 (tt, *J =* 7.2, 0.9 Hz, 2H), 2.84 - 2.65 (m, 4H), 2.36 - 2.18 (m, 2H). ESI-MS *m*/*z* 538.2 [M+H]⁺.

### Example A35: Synthesis of Compound A35

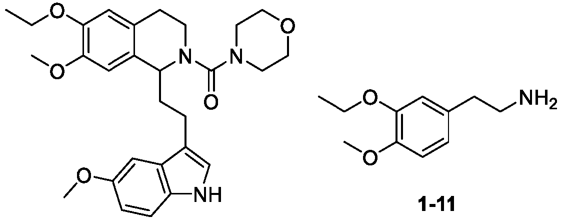

Refering to the synthesis method of compound A1, compound A35 was obtained by replacing 1-5 in Example A1 with compound 1-11. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 6.86 - 6.79 (m, 2H), 6.70 (t, *J =* 1.0 Hz, 1H), 4.87 (td, *J =* 7.0, 0.6 Hz, 1H), 4.06 (qd, *J =* 8.0, 2.2 Hz, 2H), 3.83 - 3.74 (m, 7H), 3.52 (t, *J =* 7.1 Hz, 4H), 3.47 (dt, *J =* 12.5, 7.1 Hz, 1H), 3.03 (dt, *J =* 12.5, 7.0 Hz, 2H), 2.98 (d, *J =* 7.2 Hz, 1H), 2.98 - 2.91 (m, 1H), 2.91 (ddd, *J =* 7.1, 6.6, 1.0 Hz, 2H), 2.78 - 2.64 (m, 2H), 2.36 - 2.18 (m, 2H), 1.40 (t, *J =* 8.0 Hz, 3H). ESI-MS *m*/*z* 494.3 [M+H]⁺.

### Example A36: Synthesis of Compound A36

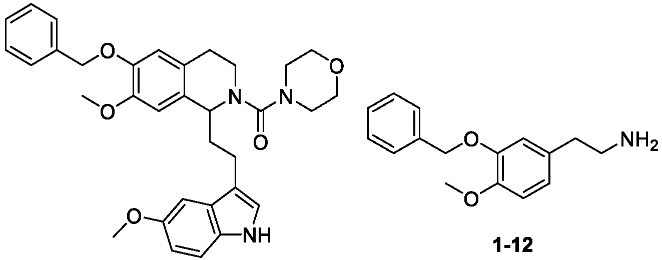

Refering to the synthesis method of compound A1, compound A36 was obtained by replacing 1-5 in Example A1 with compound 1-12. ¹H NMR(500 MHz, DMSO-*d₆*) δ 7.42 (ddt, *J* = 7.8, 1.9, 0.9 Hz, 2H), 7.36 (ddt, *J* = 7.5, 6.5, 1.0 Hz, 2H), 7.36 - 7.28 (m, 1H), 7.26 (d, *J =* 7.6 Hz, 1H), 7.14 (d, *J =* 1.4 Hz, 1H), 7.10 (d, *J =* 8.4 Hz, 1H), 6.87 (d, *J =* 0.7 Hz, 1H), 6.82 (dd, *J* = 7.5, 1.6 Hz, 1H), 6.70 (t, *J =* 1.1 Hz, 1H), 5.14 (dt, *J =* 12.5, 1.0 Hz, 1H), 5.09 (dt, *J* = 12.4, 1.0 Hz, 1H), 4.87 (td, *J* = 6.9, 0.6 Hz, 1H), 3.79 (d, *J* = 12.3 Hz, 7H), 3.52 (t, *J =* 7.1 Hz, 4H), 3.47 (dt, *J* = 12.5, 7.1 Hz, 1H), 3.13 (dt, *J* = 12.5, 7.0 Hz, 2H), 2.96 (dt, *J* = 12.5, 7.2 Hz, 2H), 2.91 (tt, *J* = 7.1, 1.1 Hz, 2H), 2.77 - 2.65 (m, 2H), 2.36 - 2.18 (m, 2H). ESI-MS *m*/*z* 556.3 [M+H]⁺.

### Example A37: Synthesis of Compound A37

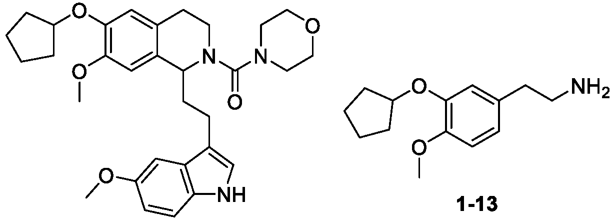

Refering to the synthesis method of compound A1, compound A37 was obtained by replacing 1-5 in Example A1 with compound 1-13. ¹H NMR(500 MHz, DMSO-*d₆*) δ 7.26 (d, *J* = 7.6 Hz, 1H), 7.15 (d, *J* = 1.8 Hz, 1H), 7.10 (d, *J* = 8.4 Hz, 1H), 6.87 (s, 1H), 6.82 (dd, *J* = 7.5, 1.6 Hz, 1H), 6.75 (t, *J =* 1.1 Hz, 1H), 4.87 (td, *J =* 7.0, 0.6 Hz, 1H), 4.84 - 4.75 (m, 1H), 3.83 - 3.74 (m, 7H), 3.52 (t, *J =* 7.1 Hz, 4H), 3.47 (dt, *J =* 12.5, 7.1 Hz, 1H), 3.13 (dt, *J =* 12.5, 7.0 Hz, 2H), 2.96 (dt, *J =* 12.5, 7.2 Hz, 2H), 2.91 (tt, *J* = 7.1, 1.1 Hz, 2H), 2.77 - 2.65 (m, 2H), 2.36 - 2.18 (m, 2H), 1.76 - 1.50 (m, 6H). ESI-MS *m*/*z* 534.3 [M+H]⁺.

### Example A38: Synthesis of Compound A38

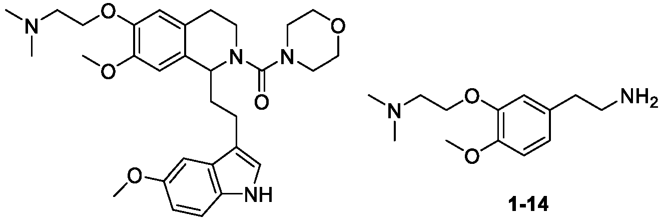

Refering to the synthesis method of compound A1, compound A38 was obtained by replacing 1-5 in Example A1 with compound 1-14. ¹H NMR(500 MHz, DMSO-*d₆*) δ 7.26 (d, *J =* 7.6 Hz, 1H), 7.15 (d, *J =* 1.8 Hz, 1H), 7.10 (d, *J =* 8.4 Hz, 1H), 6.87 (d, *J =* 0.7 Hz, 1H), 6.82 (dd, *J =* 7.5, 1.6 Hz, 1H), 6.69 (d, *J =* 1.0 Hz, 1H), 4.87 (td, *J =* 6.9, 0.6 Hz, 1H), 4.20 - 4.05 (m, 2H), 3.83 - 3.74 (m, 7H), 3.52 (t, *J =* 7.1 Hz, 4H), 3.47 (dt, *J =* 12.5, 7.1 Hz, 1H), 3.07 - 3.01 (m, 1H), 3.03 - 2.86 (m, 6H), 2.80 (dt, *J =* 12.4, 7.1 Hz, 1H), 2.76 - 2.65 (m, 2H), 2.35 (s, 5H), 2.36 - 2.25 (m, 1H), 2.27 - 2.18 (m, 1H). ESI-MS *m*/*z* 537.3 [M+H]⁺.

### Example A39: Synthesis of Compound A39

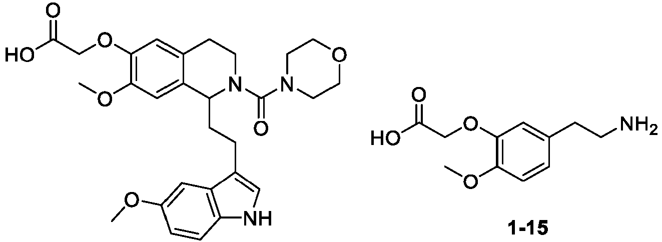

Refering to the synthesis method of compound A1, compound A39 was obtained by replacing 1-5 in Example A1 with compound 1-15. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 6.85 - 6.79 (m, 2H), 6.77 (t, *J* = 1.0 Hz, 1H), 4.87 (td, *J =* 6.9, 0.6 Hz, 1H), 4.68 - 4.58 (m, 2H), 3.83 - 3.74 (m, 7H), 3.52 (d, *J* = 7.2 Hz, 3H), 3.52 - 3.43 (m, 2H), 3.02 - 2.87 (m, 6H), 2.77 - 2.64 (m, 2H), 2.36 - 2.18 (m, 2H). ESI-MS *m*/*z* 524.2 [M+H]⁺.

### Example A40: Synthesis of Compound A40

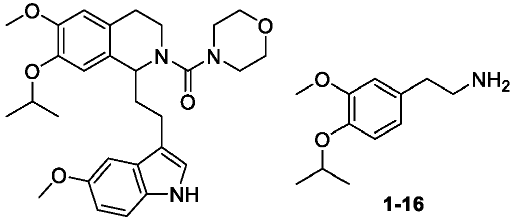

Refering to the synthesis method of compound A1, compound A40 was obtained by replacing1-5 in Example A1 with compound 1-16. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 6.84 - 6.79 (m, 2H), 6.59 (t, *J =* 1.0 Hz, 1H), 4.87 (t, *J =* 6.9 Hz, 1H), 4.59 (hept, *J =* 6.8 Hz, 1H), 3.83 - 3.74 (m, 7H), 3.54 - 3.43 (m, 5H), 3.02 - 2.94 (m, 2H), 2.97 - 2.87 (m, 4H), 2.77 - 2.69 (m, 1H), 2.71 - 2.64 (m, 1H), 2.36 - 2.18 (m, 2H), 1.35 (d, *J =* 6.9 Hz, 3H), 1.30 (d, *J =* 6.8 Hz, 3H). ESI-MS *m*/*z* 508.3 [M+H]⁺.

### Example A41: Synthesis of Compound A41

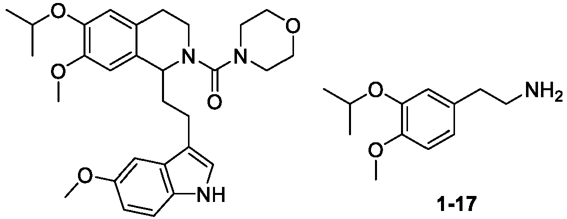

Refering to the synthesis method of compound A1, compound A41 was obtained by replacing 1-5 in Example A1 with compound 1-17. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 6.89 (d, *J =* 0.6 Hz, 1H), 6.82 (dd, *J* = 7.4, 1.5 Hz, 1H), 6.76 (t, *J =* 1.0 Hz, 1H), 4.87 (td, *J =* 6.9, 0.6 Hz, 1H), 4.59 (hept, *J =* 6.8 Hz, 1H), 3.83 - 3.74 (m, 7H), 3.54 - 3.43 (m, 5H), 3.02 - 2.94 (m, 2H), 2.97 - 2.87 (m, 4H), 2.77 - 2.69 (m, 1H), 2.71 - 2.64 (m, 1H), 2.36 - 2.18 (m, 2H), 1.32 (d, *J* = 6.8 Hz, 3H), 1.27 (d, *J =* 6.8 Hz, 3H). ESI-MS *m*/*z* 508.3 [M+H]⁺.

### Example A42: Synthesis of Compound A42

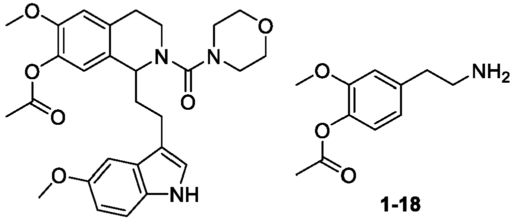

Refering to the synthesis method of compound A1, compound A42 was obtained by replacing 1-5 in Example A1 with compound 1-18. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 7.03 (d, *J =* 0.7 Hz, 1H), 6.82 (dd, *J* = 7.4, 1.5 Hz, 1H), 6.66 (t, *J =* 1.0 Hz, 1H), 4.87 (t, *J =* 6.9 Hz, 1H), 3.85 (s, 2H), 3.79 (dt, *J =* 12.5, 7.1 Hz, 1H), 3.78 (s, 3H), 3.51 (t, *J =* 7.0 Hz, 4H), 3.47 (dd, *J =* 12.4, 7.1 Hz, 1H), 3.02 - 2.94 (m, 2H), 2.97 - 2.86 (m, 4H), 2.77 - 2.69 (m, 1H), 2.71 - 2.64 (m, 1H), 2.36 - 2.18 (m, 2H), 2.25 (s, 3H). ESI-MS *m*/*z* 508.2 [M+H]⁺.

### Example A43: Synthesis of Compound A43

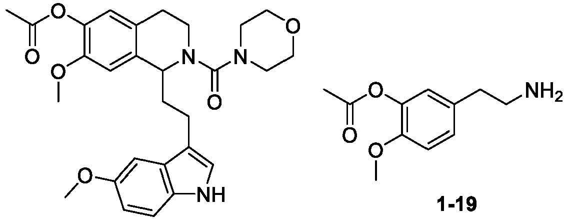

Refering to the synthesis method of compound A1, compound A43 was obtained by replacing 1-5 in Example A1 with compound 1-19. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 6.90 - 6.85 (m, 2H), 6.82 (dd, *J* = 7.4, 1.5 Hz, 1H), 4.87 (td, *J* = 6.9, 0.6 Hz, 1H), 3.82 (s, 2H), 3.78 (s, 3H), 3.76 (dt, *J =* 12.5, 7.2 Hz, 1H), 3.51 (t, *J =* 7.0 Hz, 4H), 3.47 (dd, *J =* 12.5, 7.1 Hz, 1H), 3.02 - 2.94 (m, 2H), 2.97 - 2.87 (m, 4H), 2.77 - 2.64 (m, 2H), 2.36 - 2.18 (m, 2H), 2.25 (s, 3H). ESI-MS *m*/*z* 508.2 [M+H]⁺.

### Example A44: Synthesis of Compound A44

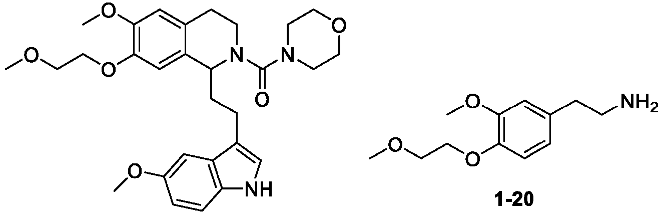

Refering to the synthesis method of compound A1, compound A44 was obtained by replacing 1-5 in Example A1 with compound 1-20. ¹H NMR(500 MHz, DMSO-*d₆*) δ 7.26 (d, *J =* 7.6 Hz, 1H), 7.14 (d, *J* = 1.8 Hz, 1H), 7.09 (d, *J =* 8.4 Hz, 1H), 6.82 (dd, *J =* 7.5, 1.6 Hz, 1H), 6.75 (d, *J =* 0.7 Hz, 1H), 6.61 (t, *J =* 1.0 Hz, 1H), 4.87 (td, *J =* 7.0, 0.6 Hz, 1H), 4.36 (dt, *J =* 12.5, 7.1 Hz, 1H), 4.24 (dt, *J =* 12.5, 7.1 Hz, 1H), 3.83 - 3.74 (m, 7H), 3.58 (td, *J =* 7.2, 4.0 Hz, 2H), 3.52 (d, *J =* 7.2 Hz, 3H), 3.52 - 3.43 (m, 2H), 3.37 (s, 2H), 3.02 - 2.94 (m, 2H), 2.97 - 2.86 (m, 4H), 2.77 - 2.64 (m, 2H), 2.36 - 2.18 (m, 2H). ESI-MS *m*/*z* 524.3 [M+H]⁺.

### Example A45: Synthesis of Compound A45

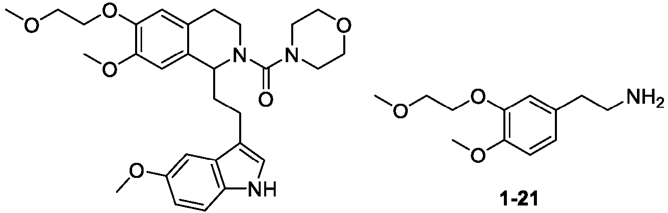

Refering to the synthesis method of compound A1, compound A45 was obtained by replacing 1-5 in Example A1 with compound 1-21. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 6.85 - 6.79 (m, 2H), 6.70 (t, *J* = 1.0 Hz, 1H), 4.87 (td, *J =* 6.9, 0.6 Hz, 1H), 4.36 (dt, *J =* 12.5, 7.0 Hz, 1H), 4.25 (dt, *J =* 12.5, 7.1 Hz, 1H), 3.83 - 3.74 (m, 7H), 3.58 (td, *J =* 7.2, 4.0 Hz, 2H), 3.52 (d, *J =* 7.1 Hz, 3H), 3.52 - 3.43 (m, 2H), 3.37 (s, 2H), 3.02 - 2.94 (m, 2H), 2.97 - 2.86 (m, 4H), 2.77 - 2.64 (m, 2H), 2.36 - 2.18 (m, 2H). ESI-MS *m*/*z* 524.3 [M+H]⁺.

### Example A46: Synthesis of Compound A46

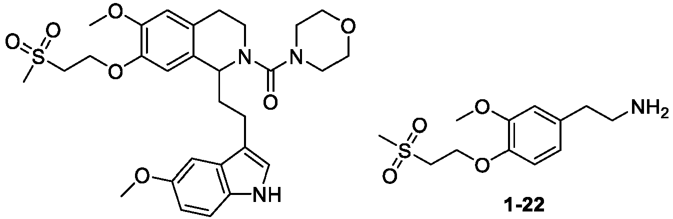

Refering to the synthesis method of compound A1, compound A46 was obtained by replacing 1-5 in Example A1 with compound 1-22. ¹H NMR(500 MHz, DMSO-*d₆*) δ 7.26 (d, *J* = 7.6 Hz, 1H), 7.14 (d, *J* = 1.4 Hz, 1H), 7.10 (d, *J* = 8.4 Hz, 1H), 6.82 (dd, *J* = 7.5, 1.5 Hz, 1H), 6.74 (d, *J =* 0.6 Hz, 1H), 6.61 (d, *J =* 1.0 Hz, 1H), 4.87 (td, *J =* 7.0, 0.6 Hz, 1H), 4.32 (td, *J =* 7.1, 2.8 Hz, 2H), 3.86 - 3.77 (m, 7H), 3.53 (d, *J =* 7.1 Hz, 3H), 3.50 (s, 1H), 3.52 - 3.43 (m, 2H), 3.45 - 3.38 (m, 1H), 3.07 - 3.01 (m, 1H), 3.04 - 2.91 (m, 3H), 2.96 (s, 3H), 2.91 (tt, *J =* 7.2, 0.9 Hz, 2H), 2.77 - 2.65 (m, 2H), 2.36 - 2.18 (m, 2H). ESI-MS *m*/*z* 572.2 [M+H]⁺.

### Example A47: Synthesis of Compound A47

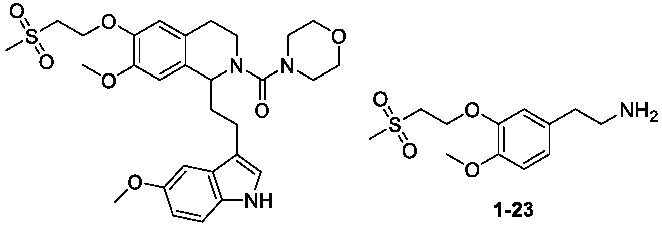

Refering to the synthesis method of compound A1, compound A47 was obtained by replacing 1-5 in Example A1 with compound 1-23. ¹H NMR(500 MHz, DMSO-*d₆*) δ 7.26 (d, *J =* 7.6 Hz, 1H), 7.14 (d, *J =* 1.4 Hz, 1H), 7.10 (d, *J =* 8.4 Hz, 1H), 6.86 - 6.79 (m, 2H), 6.73 (t, *J* = 1.0 Hz, 1H), 4.87 (td, *J =* 7.0, 0.6 Hz, 1H), 4.38 - 4.26 (m, 2H), 3.86 - 3.77 (m, 7H), 3.53 (d, *J* = 7.1 Hz, 3H), 3.50 (s, 1H), 3.52 - 3.43 (m, 2H), 3.45 - 3.38 (m, 1H), 3.07 - 3.01 (m, 1H), 3.01 (d, *J =* 7.2 Hz, 1H), 2.98 (d, *J =* 7.2 Hz, 1H), 2.96 (s, 3H), 2.98 - 2.91 (m, 1H), 2.94 - 2.86 (m, 2H), 2.77 - 2.65 (m, 2H), 2.36 - 2.18 (m, 2H). ESI-MS *m*/*z* 572.2 [M+H]⁺.

### Example A48: Synthesis of Compound A48

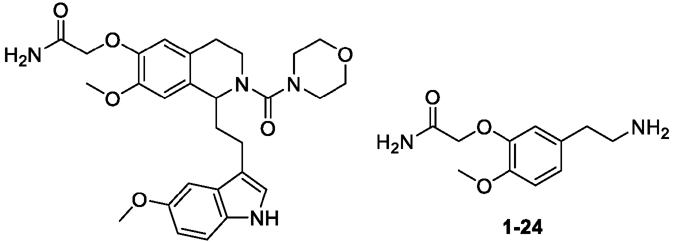

Refering to the synthesis method of compound A1, compound A48 was obtained by replacing 1-5 in Example A1 with compound 1-24. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 6.86 - 6.79 (m, 2H), 6.77 (t, *J* = 1.0 Hz, 1H), 6.73 (d, *J =* 7.7 Hz, 1H), 6.67 (d, *J =* 7.7 Hz, 1H), 4.87 (td, *J =* 6.9, 0.6 Hz, 1H), 4.61 - 4.52 (m, 2H), 3.83 - 3.74 (m, 7H), 3.53 (s, 1H), 3.52 (s, 2H), 3.52 - 3.43 (m, 2H), 3.02 - 2.87 (m, 6H), 2.77 - 2.69 (m, 1H), 2.71 - 2.64 (m, 1H), 2.36 - 2.18 (m, 2H). ESI-MS *m*/*z* 523.2 [M+H]⁺.

### Example A49: Synthesis of Compound A49

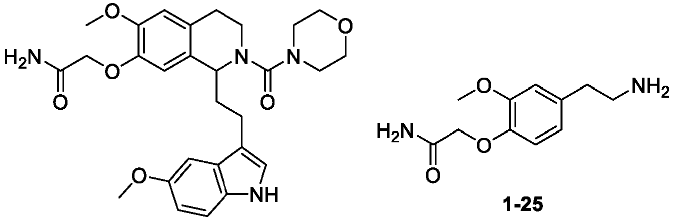

Refering to the synthesis method of compound A1, compound A49 was obtained by replacing 1-5 in Example A1 with compound 1-25. ¹H NMR(500 MHz, DMSO-*d₆*) δ 7.26 (d, *J =* 7.6 Hz, 1H), 7.14 (d, *J =* 1.8 Hz, 1H), 7.09 (d, *J =* 8.4 Hz, 1H), 6.85 - 6.77 (m, 2H), 6.73 (d, *J* = 7.7 Hz, 1H), 6.67 (d, *J =* 7.7 Hz, 1H), 6.62 (t, *J =* 1.0 Hz, 1H), 4.87 (t, *J =* 6.9 Hz, 1H), 4.64 - 4.53 (m, 2H), 3.83 - 3.74 (m, 7H), 3.52 (d, *J =* 7.2 Hz, 3H), 3.52 - 3.43 (m, 2H), 3.02 - 2.86 (m, 6H), 2.77 - 2.69 (m, 1H), 2.71 - 2.64 (m, 1H), 2.36 - 2.18 (m, 2H). ESI-MS *m*/*z* 523.2 [M+H]⁺.

### Example A50: Synthesis of Compound A50

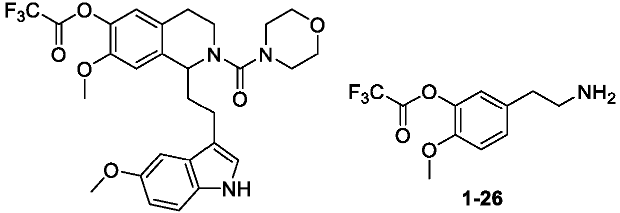

Refering to the synthesis method of compound A1, compound A50 was obtained by replacing 1-5 in Example A1 with compound 1-26. ¹H NMR(500 MHz, DMSO-*d₆*) δ 7.26 (d, *J =* 7.6 Hz, 1H), 7.14 (d, *J* = 1.4 Hz, 1H), 7.10 (d, *J* = 8.4 Hz, 1H), 6.93 (t, *J* = 1.0 Hz, 1H), 6.86 (d, *J =* 0.7 Hz, 1H), 6.82 (dd, *J =* 7.5, 1.6 Hz, 1H), 4.87 (td, *J =* 7.0, 0.6 Hz, 1H), 3.84 (s, 2H), 3.81 - 3.72 (m, 3H), 3.52 (t, *J =* 7.1 Hz, 4H), 3.47 (dt, *J =* 12.5, 7.1 Hz, 1H), 3.07 - 2.87 (m, 6H), 2.77 - 2.65 (m, 2H), 2.36 - 2.18 (m, 2H). ESI-MS *m*/*z* 562.2 [M+H]⁺.

### Example A51: Synthesis of Compound A51

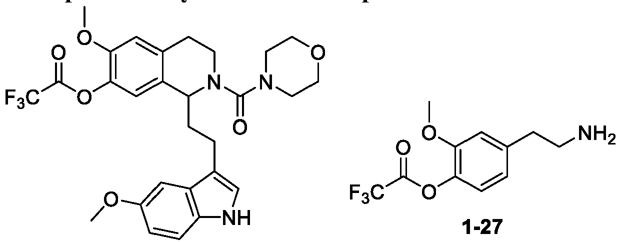

Refering to the synthesis method of compound A1, compound A51 was obtained by replacing 1-5 in Example A1 with compound 1-27. ¹H NMR(500 MHz, DMSO-*d₆*) δ 7.26 (d, *J =* 7.6 Hz, 1H), 7.14 (d, *J* = 1.4 Hz, 1H), 7.12 - 7.06 (m, 2H), 6.82 (dd, *J =* 7.5, 1.6 Hz, 1H), 6.67 (t, *J* = 1.0 Hz, 1H), 4.91 - 4.85 (m, 1H), 3.84 - 3.74 (m, 6H), 3.52 (t, *J =* 7.1 Hz, 4H), 3.47 (dt, *J =* 12.5, 7.1 Hz, 1H), 3.07 - 3.01 (m, 1H), 3.01 (d, *J* = 7.2 Hz, 1H), 2.98 (d, *J =* 7.2 Hz, 1H), 2.98 - 2.91 (m, 1H), 2.94 - 2.86 (m, 2H), 2.77 - 2.65 (m, 2H), 2.37 - 2.19 (m, 2H). ESI-MS *m*/*z* 562.2 [M+H]⁺.

### Example A52: Synthesis of Compound A52

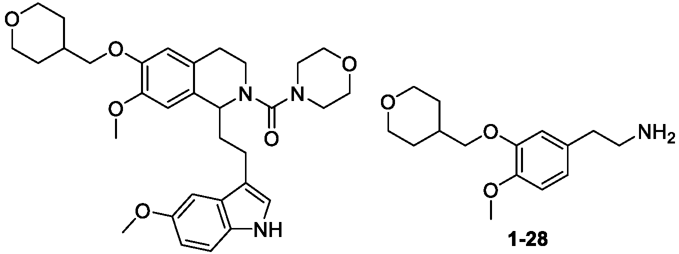

Refering to the synthesis method of compound A1, compound A52 was obtained by replacing 1-5 in Example A1 with compound 1-28. ¹H NMR(500 MHz, DMSO-*d₆*) δ 7.26 (d, *J =* 7.6 Hz, 1H), 7.14 (d, *J =* 1.4 Hz, 1H), 7.10 (d, *J =* 8.4 Hz, 1H), 6.87 - 6.79 (m, 2H), 6.70 (t, *J* = 1.0 Hz, 1H), 4.87 (td, *J =* 6.9, 0.6 Hz, 1H), 4.00 (dd, *J =* 12.3, 7.0 Hz, 1H), 3.87 (dd, *J =* 12.4, 7.0 Hz, 1H), 3.79 (d, *J =* 9.0 Hz, 7H), 3.73 (dt, *J =* 12.4, 7.1 Hz, 2H), 3.63 (dt, *J =* 12.4, 7.1 Hz, 2H), 3.52 (t, *J =* 7.1 Hz, 4H), 3.47 (dt, *J =* 12.5, 7.1 Hz, 1H), 3.07 - 2.86 (m, 6H), 2.77 - 2.65 (m, 2H), 2.36 - 2.18 (m, 2H), 2.11 (hept, *J =* 7.0 Hz, 1H), 1.83 - 1.68 (m, 4H). ESI-MS *m*/*z* 564.3 [M+H]⁺.

### Example A53: Synthesis of Compound A53

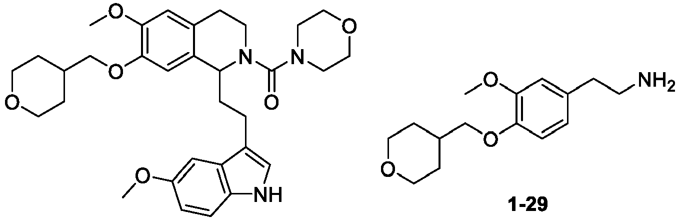

Refering to the synthesis method of compound A1, compound A53 was obtained by replacing 1-5 in Example A1 with compound 1-29. ¹H NMR(500 MHz, DMSO-*d₆*) δ 7.26 (d, *J* = 7.6 Hz, 1H), 7.14 (d, *J* = 1.4 Hz, 1H), 7.10 (d, *J* = 8.4 Hz, 1H), 6.82 (dd, *J* = 7.5, 1.5 Hz, 1H), 6.75 (d, *J =* 0.6 Hz, 1H), 6.61 (t, *J =* 1.0 Hz, 1H), 4.87 (td, *J =* 6.9, 0.6 Hz, 1H), 3.99 (dd, *J =* 12.4, 7.0 Hz, 1H), 3.87 (dd, *J =* 12.4, 7.0 Hz, 1H), 3.83 - 3.74 (m, 7H), 3.73 (dt, *J =* 12.4, 7.1 Hz, 2H), 3.63 (dt, *J =* 12.4, 7.1 Hz, 2H), 3.52 (t, *J =* 7.1 Hz, 4H), 3.47 (dt, *J =* 12.5, 7.1 Hz, 1H), 3.07 - 2.86 (m, 6H), 2.77 - 2.65 (m, 2H), 2.36 - 2.18 (m, 2H), 2.11 (hept, *J =* 7.0 Hz, 1H), 1.83 - 1.68 (m, 4H). ESI-MS *m*/*z* 564.3 [M+H]⁺.

### Example A54: Synthesis of Compound A54

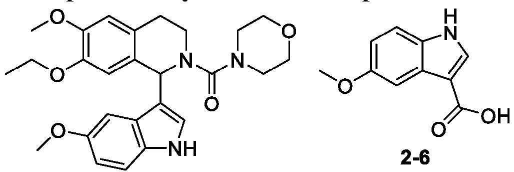

Refering to the synthesis method of compound A1, compound A54 was obtained by replacing 2-5 in Example A1 with compound 2-6. ¹H NMR(500 MHz, DMSO-*d₆*) δ 9.27 (d, *J* = 8.4 Hz, 1H), 7.47 - 7.41 (m, 2H), 7.28 (d, *J* = 7.3 Hz, 1H), 6.93 (d, *J* = 0.6 Hz, 1H), 6.81 (dd, *J =* 7.5, 1.5 Hz, 1H), 6.63 (t, *J =* 0.9 Hz, 1H), 6.18 (s, 1H), 4.08 (qd, *J =* 7.9, 2.5 Hz, 2H), 3.90 (dt, *J =* 12.4, 7.1 Hz, 1H), 3.82 (d, *J =* 4.2 Hz, 6H), 3.57 (q, *J =* 7.2 Hz, 5H), 3.12 (td, *J =* 7.1, 0.9 Hz, 4H), 2.91 (tt, *J =* 7.2, 0.8 Hz, 2H), 1.40 (t, *J =* 8.0 Hz, 3H). ESI-MS *m*/*z* 466.2 [M+H]⁺.

### Example A55: Synthesis of Compound A55

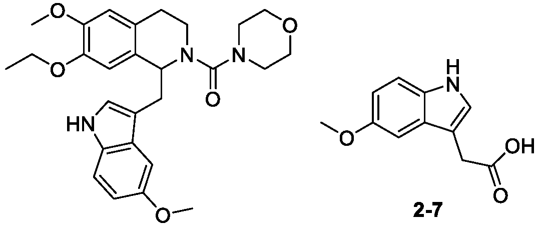

Refering to the synthesis method of compound A1, compound A55 was obtained by replacing 2-5 in Example A1 with compound 2-7. ¹H NMR(500 MHz, DMSO-*d₆*) δ 7.26 (d, *J* = 7.5 Hz, 1H), 7.18 (d, *J =* 8.4 Hz, 1H), 7.12 (d, *J* = 1.4 Hz, 1H), 6.81 (dd, *J* = 7.5, 1.5 Hz, 1H), 6.76 (d, *J =* 0.6 Hz, 1H), 6.60 (t, *J =* 1.0 Hz, 1H), 5.07 - 5.01 (m, 1H), 4.06 (qd, *J* = 8.0, 2.3 Hz, 2H), 3.83 - 3.74 (m, 6H), 3.61 - 3.49 (m, 4H), 3.51 - 3.44 (m, 1H), 3.13 (dt, *J =* 12.5, 7.1 Hz, 2H), 3.03 - 2.87 (m, 5H), 2.83 (dd, *J =* 12.4, 7.1 Hz, 1H), 1.40 (t, *J =* 8.0 Hz, 3H). ESI-MS *m*/*z* 480.2 [M+H]⁺.

### Example A56: Synthesis of Compound A56

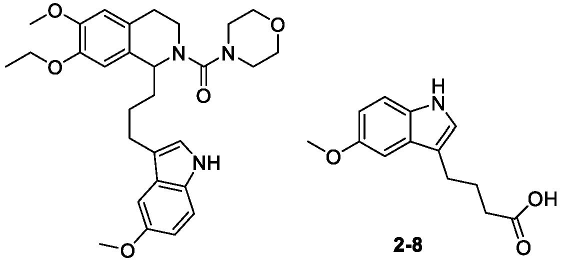

Refering to the synthesis method of compound A1, compound A56 was obtained by replacing 2-5 in Example A1 with compound 2-8. ¹H NMR(500 MHz, DMSO-*d₆*) δ 7.26 (d, *J = 7.6* Hz, 1H), 7.17 - 7.13 (m, 1H), 7.10 (d, *J* = 8.4 Hz, 1H), 6.82 (dd, *J* = 7.4, 1.5 Hz, 1H), 6.74 (d, *J* = 0.7 Hz, 1H), 6.59 (t, *J* = 1.0 Hz, 1H), 4.90 - 4.83 (m, 1H), 4.06 (qd, *J =* 8.1, 2.1 Hz, 2H), 3.83 - 3.74 (m, 7H), 3.54 - 3.43 (m, 5H), 3.02 - 2.94 (m, 2H), 2.97 - 2.86 (m, 4H), 2.72 - 2.63 (m, 2H), 2.06 - 1.88 (m, 3H), 1.92 - 1.79 (m, 1H), 1.44 - 1.37 (m, 3H). ESI-MS *m*/*z* 508.3 [M+H]⁺.

### Example A57: Synthesis of Compound A57

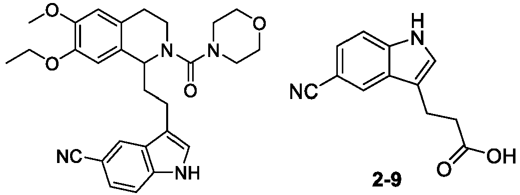

Refering to the synthesis method of compound A1, compound A57 was obtained by replacing 2-5 in Example A1 with compound 2-9. ¹H NMR(500 MHz, DMSO-*d₆*) δ 7.86 (t, *J* = 1.0 Hz, 1H), 7.47 - 7.40 (m, 2H), 7.17 (d, *J* = 8.4 Hz, 1H), 6.74 (d, *J* = 0.6 Hz, 1H), 6.59 (t, *J* = 1.0 Hz, 1H), 4.87 (t, *J =* 6.9 Hz, 1H), 4.06 (qd, *J =* 8.0, 1.3 Hz, 2H), 3.83 - 3.74 (m, 3H), 3.52 (t, *J =* 7.1 Hz, 4H), 3.47 (dt, *J =* 12.5, 7.1 Hz, 1H), 3.07 - 2.86 (m, 6H), 2.78 (dt, *J =* 12.3, 7.1 Hz, 1H), 2.70 (dt, *J =* 12.3, 7.0 Hz, 1H), 2.37 - 2.19 (m, 2H), 1.44 - 1.37 (m, 3H). ESI-MS *m*/*z* 489.2 [M+H]⁺.

### Example A58: Synthesis of Compound A58

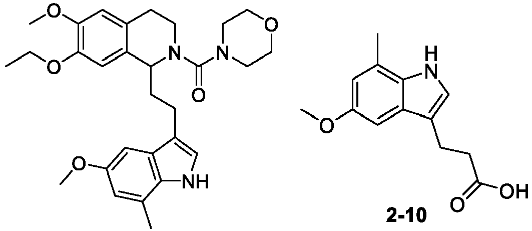

Refering to the synthesis method of compound A1, compound A58 was obtained by replacing 2-5 in Example A1 with compound 2-10. ¹H NMR(500 MHz, DMSO-*d₆*) δ 7.10 - 7.03 (m, 2H), 6.81 - 6.77 (m, 1H), 6.74 (d, *J =* 0.7 Hz, 1H), 6.60 (t, *J =* 0.9 Hz, 1H), 4.87 (td, *J =* 6.9, 0.6 Hz, 1H), 4.06 (qd, *J =* 8.1, 2.1 Hz, 2H), 3.81 (s, 3H), 3.82 - 3.74 (m, 1H), 3.58 (s, 2H), 3.53 (s, 1H), 3.53 - 3.43 (m, 4H), 3.02 - 2.94 (m, 2H), 2.97 - 2.86 (m, 4H), 2.77 - 2.64 (m, 2H), 2.33 (s, 3H), 2.36 - 2.18 (m, 2H), 1.44 - 1.37 (m, 3H). ESI-MS *m*/*z* 508.3 [M+H]⁺.

### Example A59: Synthesis of Compound A59

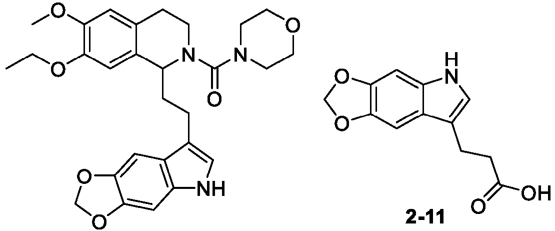

Refering to the synthesis method of compound A1, compound A59 was obtained by replacing 2-5 in Example A1 with compound 2-11. ¹H NMR(500 MHz, DMSO-*d₆*) δ 7.29 (s, 1H), 7.07 (d, *J =* 8.4 Hz, 1H), 6.81 (s, 1H), 6.74 (d, *J =* 0.7 Hz, 1H), 6.60 (t, *J =* 0.9 Hz, 1H), 6.03 (d, *J =* 3.7 Hz, 2H), 4.87 (td, *J =* 6.9, 0.6 Hz, 1H), 4.06 (qd, *J =* 8.1, 2.1 Hz, 2H), 3.83 - 3.74 (m, 3H), 3.53 (s, 1H), 3.53 - 3.43 (m, 4H), 3.02 - 2.94 (m, 2H), 2.97 - 2.86 (m, 4H), 2.77 - 2.64 (m, 2H), 2.36 - 2.18 (m, 2H), 1.44 - 1.37 (m, 3H). ESI-MS *m*/*z* 508.2 [M+H]⁺.

### Example A60: Synthesis of Compound A60

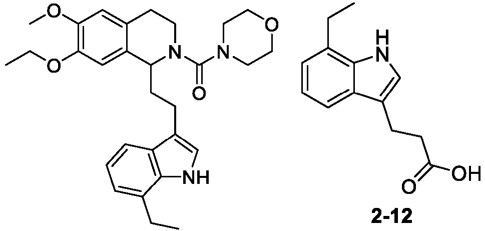

Refering to the synthesis method of compound A1, compound A60 was obtained by replacing 2-5 in Example A1 with compound 2-12. ¹H NMR(500 MHz, DMSO-*d₆*) δ 7.48 (dd, *J* = 7.5, 1.6 Hz, 1H), 7.20 (t, *J =* 7.5 Hz, 1H), 7.10 (d, *J =* 8.4 Hz, 1H), 7.03 (dd, *J =* 7.5, 1.5 Hz, 1H), 6.74 (d, *J =* 0.6 Hz, 1H), 6.59 (t, *J =* 1.0 Hz, 1H), 4.87 (t, *J =* 6.9 Hz, 1H), 4.06 (qd, *J =* 8.0, 1.3 Hz, 2H), 3.83 - 3.74 (m, 3H), 3.52 (t, *J =* 7.1 Hz, 4H), 3.47 (dt, *J =* 12.5, 7.1 Hz, 1H), 3.07 - 2.86 (m, 6H), 2.78 - 2.64 (m, 2H), 2.61 (qd, *J =* 8.0, 1.5 Hz, 2H), 2.37 - 2.19 (m, 2H), 1.44 - 1.37 (m, 3H), 1.23 - 1.17 (m, 3H). ESI-MS *m*/*z* 492.3 [M+H]⁺.

### Example A61: Synthesis of Compound A61

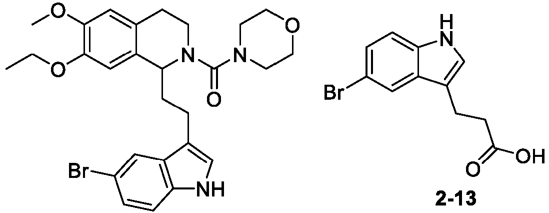

Refering to the synthesis method of compound A1, compound A61 was obtained by replacing 2-5 in Example A1 with compound 2-13. ¹H NMR(500 MHz, DMSO-*d₆*) δ 7.74 - 7.70 (m, 1H), 7.36 - 7.28 (m, 2H), 7.15 (d, *J =* 8.4 Hz, 1H), 6.75 (d, *J =* 0.6 Hz, 1H), 6.60 (t, *J* = 1.0 Hz, 1H), 4.87 (t, *J =* 6.9 Hz, 1H), 4.06 (qd, *J =* 8.0, 2.2 Hz, 2H), 3.83 - 3.74 (m, 3H), 3.59 - 3.43 (m, 5H), 3.13 (dt, *J* = 12.5, 7.0 Hz, 2H), 2.96 (dt, *J* = 12.5, 7.2 Hz, 2H), 2.91 (tt, *J* = 7.1, 0.9 Hz, 2H), 2.77 - 2.64 (m, 2H), 2.36 - 2.19 (m, 2H), 1.40 (t, *J =* 8.0 Hz, 3H). ESI-MS *m*/*z* 542.2 [M+H]⁺.

### Example A62: Synthesis of Compound A62

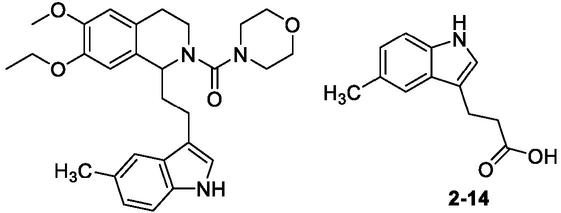

Refering to the synthesis method of compound A1, compound A62 was obtained by replacing 2-5 in Example A1 with compound 2-14. ¹H NMR(500 MHz, DMSO-*d₆*) δ 7.49 - 7.45 (m, 1H), 7.39 (d, *J* = 7.5 Hz, 1H), 7.13 - 7.07 (m, 2H), 6.74 (d, *J =* 0.6 Hz, 1H), 6.60 (t, *J* = 1.1 Hz, 1H), 4.87 (t, *J =* 6.9 Hz, 1H), 4.07 (qd, *J* = 7.9, 4.4 Hz, 2H), 3.83 - 3.74 (m, 3H), 3.61 - 3.53 (m, 2H), 3.56 - 3.48 (m, 2H), 3.47 (dt, *J =* 12.5, 6.5 Hz, 1H), 3.13 (dt, *J =* 12.5, 7.0 Hz, 2H), 3.00 - 2.86 (m, 4H), 2.78 - 2.64 (m, 2H), 2.41 (s, 2H), 2.37 - 2.19 (m, 2H), 1.40 (t, *J =* 8.0 Hz, 3H). ESI-MS *m*/*z* 478.3 [M+H]⁺.

### Example A63: Synthesis of Compound A63

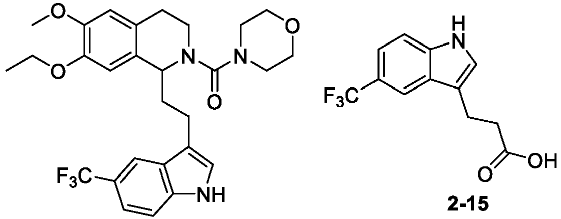

Refering to the synthesis method of compound A1, compound A63 was obtained by replacing 2-5 in Example A1 with compound 2-15. ¹H NMR(500 MHz, DMSO-*d₆*) δ 7.91 (d, *J =* 1.5 Hz, 1H), 7.57 (dd, *J =* 7.5, 1.6 Hz, 1H), 7.32 (d, *J* = 7.5 Hz, 1H), 7.12 (d, *J =* 8.4 Hz, 1H), 6.74 (s, 1H), 6.60 (d, *J* = 1.0 Hz, 1H), 4.87 (t, *J* = 6.9 Hz, 1H), 4.14 - 4.00 (m, 2H), 3.81 (s, 2H), 3.79 (dt, *J =* 12.5, 7.1 Hz, 1H), 3.52 (d, *J =* 7.2 Hz, 3H), 3.52 - 3.43 (m, 2H), 3.02 - 2.86 (m, 6H), 2.78 - 2.65 (m, 2H), 2.37 - 2.19 (m, 2H), 1.40 (t, *J =* 8.0 Hz, 3H). ESI-MS *m*/*z* 532.2 [M+H]⁺.

### Example A64: Synthesis of Compound A64

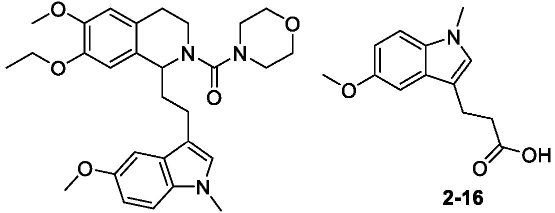

Refering to the synthesis method of compound A1, compound A64 was obtained by replacing 2-5 in Example A1 with compound 2-16. ¹H NMR(500 MHz, DMSO-*d₆*) δ 7.37 (d, *J* = 7.5 Hz, 1H), 7.08 (d, *J* = 1.7 Hz, 1H), 6.94 (s, 1H), 6.83 (dd, *J* = 7.5, 1.5 Hz, 1H), 6.74 (d, *J =* 0.6 Hz, 1H), 6.60 (t, *J =* 0.9 Hz, 1H), 4.87 (t, *J =* 6.9 Hz, 1H), 4.06 (qd, *J =* 8.1, 2.1 Hz, 2H), 3.82 (d, *J* = 3.1 Hz, 6H), 3.82 - 3.75 (m, 1H), 3.73 (s, 3H), 3.54 - 3.43 (m, 5H), 3.02 - 2.94 (m, 2H), 2.97 - 2.87 (m, 4H), 2.79 (t, *J =* 7.0 Hz, 2H), 2.26 - 2.08 (m, 2H), 1.44 - 1.37 (m, 3H). ESI-MS *m*/*z* 508.3 [M+H]⁺.

### Example A65: Synthesis of Compound A65

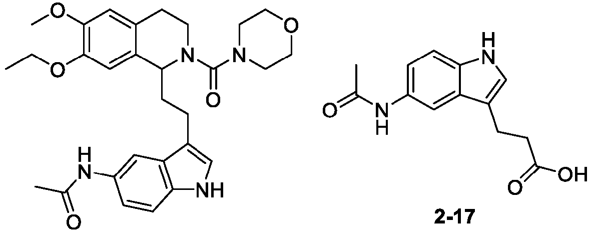

Refering to the synthesis method of compound A1, compound A65 was obtained by replacing 2-5 in Example A1 with compound 2-17. ¹H NMR(500 MHz, DMSO-*d₆*) δ 9.72 (s, 1H), 7.78 (d, *J =* 1.4 Hz, 1H), 7.49 (dd, *J =* 7.5, 1.5 Hz, 1H), 7.37 (d, *J =* 7.5 Hz, 1H), 7.15 (d, *J* = 8.4 Hz, 1H), 6.74 (d, *J =* 0.6 Hz, 1H), 6.60 (t, *J =* 0.9 Hz, 1H), 4.87 (t, *J =* 6.9 Hz, 1H), 4.14 - 4.00 (m, 2H), 3.81 (s, 2H), 3.79 (dt, *J =* 12.5, 7.1 Hz, 1H), 3.52 (t, *J =* 7.1 Hz, 4H), 3.47 (dd, *J =* 12.4, 7.1 Hz, 1H), 3.02 - 2.94 (m, 2H), 2.97 - 2.86 (m, 4H), 2.77 - 2.64 (m, 2H), 2.37 - 2.19 (m, 2H), 2.15 (s, 2H), 1.40 (t, *J =* 8.0 Hz, 3H). ESI-MS *m*/*z* 521.3 [M+H]⁺.

### Example A66: Synthesis of Compound A66

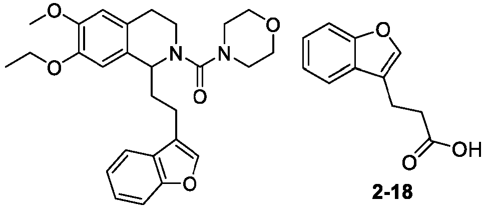

Refering to the synthesis method of compound A1, compound A66 was obtained by replacing 2-5 in Example A1 with compound 2-18. ¹H NMR(500 MHz, DMSO-*d₆*) δ 7.69 - 7.63 (m, 2H), 7.51 (dd, *J =* 7.5, 1.6 Hz, 1H), 7.31 (td, *J =* 7.5, 1.5 Hz, 1H), 7.23 (td, *J =* 7.5, 1.5 Hz, 1H), 6.74 (d, *J =* 0.6 Hz, 1H), 6.60 (t, *J =* 1.0 Hz, 1H), 4.91 - 4.84 (m, 1H), 4.08 (qd, *J =* 8.0, 2.9 Hz, 2H), 3.83 - 3.74 (m, 3H), 3.57 (t, *J =* 7.1 Hz, 4H), 3.47 (dt, *J =* 12.5, 7.1 Hz, 1H), 3.17 - 3.08 (m, 4H), 2.91 (tt, *J =* 7.1, 0.9 Hz, 2H), 2.84 - 2.72 (m, 2H), 2.21 - 2.03 (m, 2H), 1.40 (t, *J =* 8.0 Hz, 3H). ESI-MS *m*/*z* 465.2 [M+H]⁺.

### Example A67: Synthesis of Compound A67

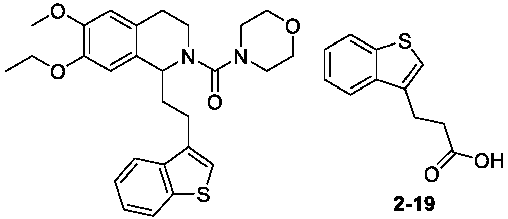

Refering to the synthesis method of compound A1, compound A67 was obtained by replacing 2-5 in Example A1 with compound 2-19. ¹H NMR(500 MHz, DMSO-*d₆*) δ 7.83 - 7.76 (m, 1H), 7.76 - 7.69 (m, 1H), 7.45 - 7.36 (m, 2H), 7.31 (s, 1H), 6.75 (d, *J =* 0.6 Hz, 1H), 6.60 (t, *J =* 1.0 Hz, 1H), 4.87 (t, *J =* 6.9 Hz, 1H), 4.06 (qd, *J =* 8.0, 1.1 Hz, 2H), 3.83 - 3.74 (m, 3H), 3.56 (td, *J =* 7.0, 2.7 Hz, 4H), 3.47 (dt, *J =* 12.5, 7.1 Hz, 1H), 3.13 (dt, *J =* 12.5, 7.0 Hz, 2H), 2.96 (dt, *J =* 12.5, 7.1 Hz, 2H), 2.91 (tt, *J =* 7.2, 0.9 Hz, 2H), 2.84 (td, *J* = 7.1, 3.1 Hz, 2H), 2.26 - 2.07 (m, 2H), 1.40 (t, *J =* 8.0 Hz, 3H). ESI-MS *m*/*z* 481.2 [M+H]⁺.

### Example A68: Synthesis of Compound A68

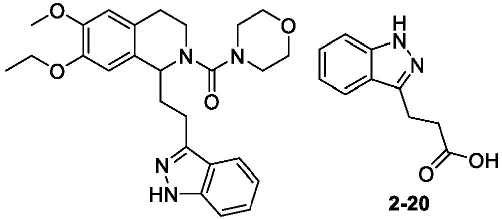

Refering to the synthesis method of compound A1, compound A68 was obtained by replacing 2-5 in Example A1 with compound 2-20. ¹H NMR(500 MHz, DMSO-*d₆*) δ 7.91 (dd, *J* = 7.5, 1.6 Hz, 1H), 7.56 (dd, *J* = 7.6, 1.5 Hz, 1H), 7.41 (td, *J =* 7.5, 1.5 Hz, 1H), 7.26 (td, *J =* 7.5, 1.5 Hz, 1H), 6.75 (s, 1H), 6.60 (t, *J =* 1.0 Hz, 1H), 4.96 - 4.89 (m, 1H), 4.15 - 4.00 (m, 2H), 3.83 - 3.73 (m, 3H), 3.57 (t, *J =* 7.1 Hz, 4H), 3.47 (dt, *J =* 12.5, 7.1 Hz, 1H), 3.22 (dt, *J =* 12.4, 7.1 Hz, 1H), 3.20 - 3.11 (m, 4H), 3.11 (d, *J =* 1.1 Hz, 1H), 2.91 (tt, *J =* 7.1, 1.1 Hz, 2H), 2.48 - 2.31 (m, 2H), 1.40 (t, *J =* 8.0 Hz, 3H). ESI-MS *m*/*z* 465.2 [M+H]⁺.

### Example A69: Synthesis of Compound A69

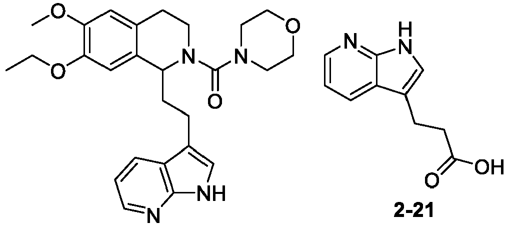

Refering to the synthesis method of compound A1, compound A69 was obtained by replacing 2-5 in Example A1 with compound 2-21. ¹H NMR(500 MHz, DMSO-*d₆*) δ 9.94 (d, *J =* 8.4 Hz, 1H), 8.62 (dd, *J =* 7.5, 1.6 Hz, 1H), 8.38 (dd, *J =* 7.5, 1.6 Hz, 1H), 7.39 (t, *J* = 7.5 Hz, 1H), 6.94 (d, *J =* 8.6 Hz, 1H), 6.74 (d, *J =* 0.6 Hz, 1H), 6.60 (t, *J =* 1.0 Hz, 1H), 4.87 (t, *J =* 6.9 Hz, 1H), 4.08 (qd, *J* = 8.0, 2.9 Hz, 2H), 3.83 - 3.74 (m, 3H), 3.57 (t, *J* = 7.1 Hz, 4H), 3.47 (dt, *J* = 12.5, 7.1 Hz, 1H), 3.17 - 3.08 (m, 4H), 2.99 (dt, *J* = 12.3, 7.1 Hz, 1H), 2.91 (tt, *J =* 7.1, 0.9 Hz, 2H), 2.78 (dt, *J =* 12.4, 7.1 Hz, 1H), 2.36 - 2.19 (m, 2H), 1.40 (t, *J =* 8.0 Hz, 3H). ESI-MS *m*/*z* 465.2 [M+H]⁺.

### Example A70: Synthesis of Compound A70

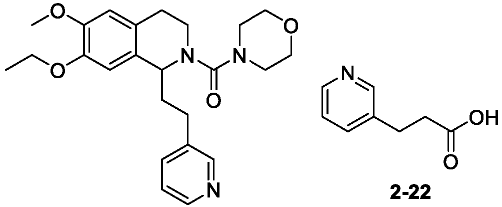

Refering to the synthesis method of compound A1, compound A70 was obtained by replacing 2-5 in Example A1 with compound 2-22. ¹H NMR(500 MHz, DMSO-*d₆*) δ 8.43 (dd, *J* = 7.4, 1.6 Hz, 1H), 8.39 (d, *J* = 1.5 Hz, 1H), 7.50 (dt, *J* = 7.5, 1.5 Hz, 1H), 7.26 (t, *J* = 7.5 Hz, 1H), 6.74 (d, *J =* 0.6 Hz, 1H), 6.60 (t, *J =* 1.0 Hz, 1H), 4.91 - 4.84 (m, 1H), 4.17 - 4.02 (m, 2H), 3.85 (dt, *J =* 12.3, 7.1 Hz, 1H), 3.83 (s, 3H), 3.78 (dt, *J =* 12.5, 7.0 Hz, 1H), 3.58 (t, *J* = 7.1 Hz, 4H), 3.13 (t, *J* = 7.1 Hz, 4H), 2.90 (td, *J* = 7.1, 1.0 Hz, 2H), 2.82 (td, *J* = 7.1, 1.4 Hz, 2H), 2.32 - 2.15 (m, 2H), 1.40 (t, *J =* 8.0 Hz, 3H). ESI-MS *m*/*z* 426.2 [M+H]⁺.

### Example A71: Synthesis of Compound A71

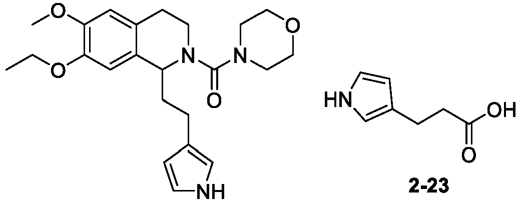

Refering to the synthesis method of compound A1, compound A71 was obtained by replacing 2-5 in Example A1 with compound 2-23. ¹H NMR(500 MHz, DMSO-*d₆*) δ 6.94 (dd, *J* = 7.3, 1.6 Hz, 1H), 6.81 - 6.72 (m, 3H), 6.60 (t, *J =* 1.0 Hz, 1H), 4.91 - 4.84 (m, 1H), 4.17 - 4.02 (m, 2H), 3.84 (dt, *J =* 12.5, 7.1 Hz, 1H), 3.82 (s, 3H), 3.75 (dt, *J =* 12.5, 7.1 Hz, 1H), 3.58 (t, *J* = 7.1 Hz, 4H), 3.22 - 3.08 (m, 4H), 2.95 - 2.87 (m, 3H), 2.89 - 2.82 (m, 1H), 2.34 - 2.17 (m, 2H), 1.40 (t, *J =* 8.0 Hz, 3H). ESI-MS *m*/*z* 414.2 [M+H]⁺.

### Example A72: Synthesis of Compound A72

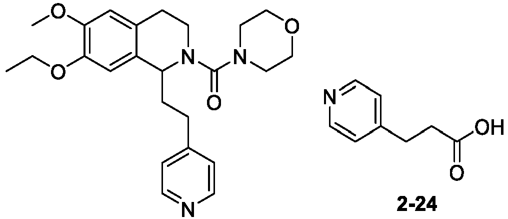

Refering to the synthesis method of compound A1, compound A72 was obtained by replacing 2-5 in Example A1 with compound 2-24. ¹H NMR(500 MHz, DMSO-*d₆*) δ 8.51 - 8.46 (m, 2H), 7.21 - 7.16 (m, 2H), 6.74 (d, *J =* 0.6 Hz, 1H), 6.59 (t, *J =* 1.0 Hz, 1H), 4.91 - 4.84 (m, 1H), 4.17 - 4.02 (m, 2H), 3.85 (dt, *J =* 12.3, 7.1 Hz, 1H), 3.83 (s, 3H), 3.78 (dt, *J=* 12.5, 7.0 Hz, 1H), 3.58 (t, *J =* 7.1 Hz, 4H), 3.13 (t, *J* = 7.1 Hz, 4H), 2.95 - 2.86 (m, 3H), 2.79 (dt, *J =* 12.5, 7.1 Hz, 1H), 2.24 - 2.07 (m, 2H), 1.40 (t, *J =* 8.0 Hz, 3H). ESI-MS *m*/*z* 426.2 [M+H]⁺.

### Example A73: Synthesis of Compound A73

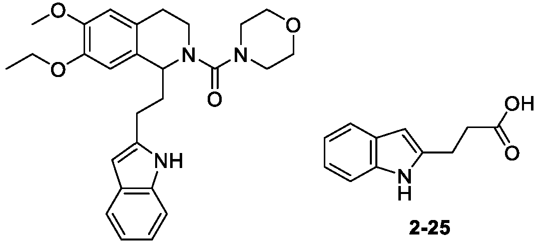

Refering to the synthesis method of compound A1, compound A73 was obtained by replacing 2-5 in Example A1 with compound 2-25. ¹H NMR(500 MHz, DMSO-*d₆*) δ 7.37 - 7.31 (m, 2H), 7.14 - 7.03 (m, 3H), 6.74 (d, *J =* 0.6 Hz, 1H), 6.60 (d, *J =* 1.0 Hz, 1H), 4.99 - 4.92 (m, 1H), 4.15 - 4.00 (m, 2H), 3.83 - 3.73 (m, 3H), 3.57 (t, *J =* 7.1 Hz, 4H), 3.47 (dt, *J* = 12.5, 7.1 Hz, 1H), 3.12 (td, *J* = 7.1, 1.0 Hz, 4H), 2.98 - 2.83 (m, 4H), 2.30 - 2.13 (m, 2H), 1.40 (t, *J =* 8.0 Hz, 3H). ESI-MS *m*/*z* 464.2 [M+H]⁺.

### Example A74: Synthesis of Compound A74

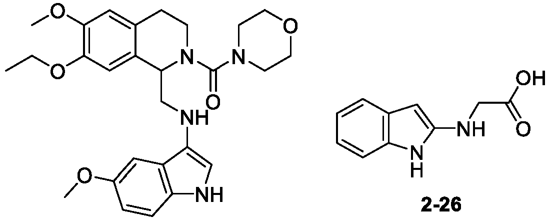

Refering to the synthesis method of compound A1, compound A74 was obtained by replacing 2-5 in Example A1 with compound 2-26. ¹H NMR(500 MHz, DMSO-*d₆*) δ 7.76 (d, *J =* 7.9 Hz, 1H), 7.45 - 7.41 (m, 1H), 7.12 (d, *J* = 7.5 Hz, 1H), 6.82 (dd, *J* = 7.5, 1.6 Hz, 1H), 6.74 (s, 1H), 6.59 (t, *J* = 1.0 Hz, 1H), 6.11 (t, *J =* 6.8 Hz, 1H), 5.09 - 5.02 (m, 1H), 4.06 (qd, *J =* 8.0, 1.3 Hz, 2H), 3.96 (dt, *J =* 12.4, 6.8 Hz, 1H), 3.87 - 3.79 (m, 7H), 3.82 - 3.76 (m, 1H), 3.78 - 3.71 (m, 1H), 3.52 (t, *J =* 7.1 Hz, 4H), 2.95 (d, *J =* 7.0 Hz, 3H), 2.95 - 2.87 (m, 3H), 1.44 - 1.37 (m, 3H). ESI-MS *m*/*z* 495.3 [M+H]⁺.

### Example A75: Synthesis of Compound A75

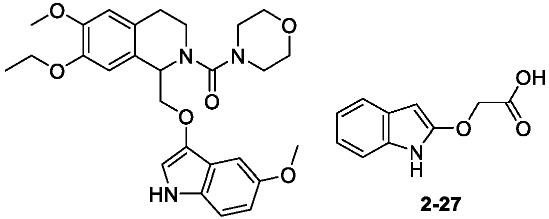

Refering to the synthesis method of compound A1, compound A75 was obtained by replacing 2-5 in Example A1 with compound 2-27. ¹H NMR(500 MHz, DMSO-*d₆*) δ 7.44 - 7.40 (m, 1H), 7.24 (d, *J* = 9.2 Hz, 1H), 7.16 (d, *J =* 7.5 Hz, 1H), 6.84 - 6.79 (m, 2H), 6.59 (t, *J* = 1.1 Hz, 1H), 5.30 - 5.23 (m, 1H), 4.55 (dd, *J =* 12.3, 7.0 Hz, 1H), 4.46 (dd, *J =* 12.4, 7.1 Hz, 1H), 4.06 (qd, *J* = 8.1, 1.4 Hz, 2H), 3.81 (d, *J* = 3.1 Hz, 6H), 3.76 (t, *J* = 7.1 Hz, 2H), 3.52 (t, *J =* 7.1 Hz, 4H), 3.07 - 2.98 (m, 4H), 2.91 (ddd, *J =* 7.2, 6.6, 1.0 Hz, 2H), 1.40 (t, *J =* 8.0 Hz, 3H). ESI-MS *m*/*z* 496.2 [M+H]⁺.

### Example A76: Synthesis of Compound A76

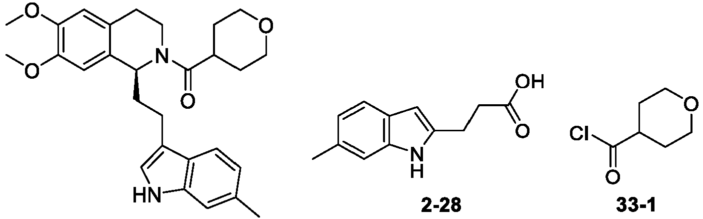

Refering to the synthesis method of compound (S)-A1, compound A76 was obtained by replacing 1-2 in Example (S) - A1 with iodomethane, replacing 2-5 in Example (S) - A1 with compound 2-28, and replacing morpholinyl chloride in Example (S) - A1 with compound 33-1. ¹H NMR (500 MHz, Chloroform-d) δ 9.91 (d, *J =* 8.6 Hz, 1H), 7.55 (d, *J* = 7.5 Hz, 1H), 7.17 (dd, *J =* 1.4, 0.8 Hz, 1H), 7.19 - 7.11 (m, 1H), 6.98 (dq, *J =* 7.8, 1.3 Hz, 1H), 6.83 (d, *J =* 0.6 Hz, 1H), 6.58 (t, *J =* 1.0 Hz, 1H), 4.67 - 4.60 (m, 1H), 3.83 (d, *J* = 6.2 Hz, 6H), 3.81 - 3.74 (m, 2H), 3.74 (d, *J =* 7.0 Hz, 1H), 3.69 (dt, *J =* 12.3, 7.1 Hz, 1H), 3.55 (dt, *J =* 12.4, 7.1 Hz, 2H), 3.18 (dt, *J* = 12.3, 7.1 Hz, 1H), 3.02 (dt, *J =* 12.5, 7.1 Hz, 1H), 2.91 (td, *J* = 7.0, 0.9 Hz, 2H), 2.55 (p, *J =* 7.0 Hz, 1H), 2.47 (d, *J =* 0.9 Hz, 2H), 2.33 (dq, *J =* 12.5, 7.1 Hz, 1H), 2.25 (dq, *J =* 12.5, 7.1 Hz, 1H), 1.70 (qd, *J =* 7.1, 4.3 Hz, 4H). ESI-MS *m*/*z* 462.3 [M+H]⁺.

### Example A77: Synthesis of Compound A77

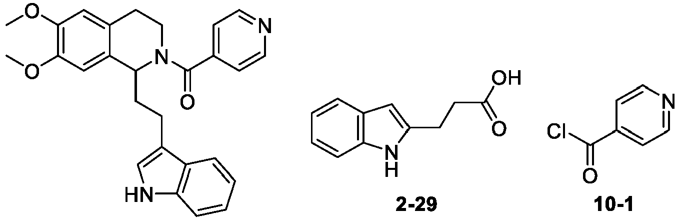

Refering to the synthesis method of compound A1, compound A77 was obtained by replacing 1-2 in Example A1 with iodomethane, replacing 2-5 in Example A1 with Compound 2-29, and replacing morpholinyl chloride in Example A1 with Compound 10-1. ¹H NMR (500 MHz, Chloroform-d) δ 9.94 (d, *J =* 8.4 Hz, 1H), 8.70 - 8.65 (m, 2H), 7.78 - 7.73 (m, 2H), 7.60 (dd, *J =* 7.3, 1.6 Hz, 1H), 7.36 (dd, *J =* 7.4, 1.7 Hz, 1H), 7.18 - 7.12 (m, 2H), 7.09 (td, *J* = 7.4, 1.5 Hz, 1H), 6.82 (s, 1H), 6.58 (t, *J* = 1.0 Hz, 1H), 4.93 - 4.86 (m, 1H), 3.84 (d, *J =* 4.4 Hz, 6H), 3.75 - 3.63 (m, 2H), 3.18 (dt, *J =* 12.2, 7.1 Hz, 1H), 3.02 (dt, *J =* 12.5, 7.1 Hz, 1H), 2.90 (td, *J* = 7.0, 0.9 Hz, 2H), 2.35 - 2.18 (m, 2H). ESI-MS *m*/*z* 442.2 [M+H]⁺.

### Example A80: Synthesis of Compound A80

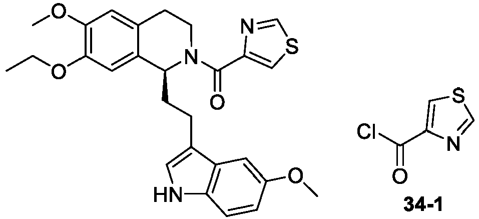

Refering to the synthesis method of compound (S)-A1, compound A80 was obtained by replacing the morpholinyl chloride in Example (S) - A1 with compound 34-1. ¹H NMR (500 MHz, Chloroform-*d*) δ 9.07 (d, *J* = 2.7 Hz, 1H), 7.27 - 7.20 (m, 2H), 7.09 (d, *J =* 8.4 Hz, 1H), 7.05 (d, *J =* 1.5 Hz, 1H), 6.81 (dd, *J =* 7.5, 1.5 Hz, 1H), 6.75 (d, *J =* 0.6 Hz, 1H), 6.60 (t, *J =* 1.0 Hz, 1H), 4.82 - 4.76 (m, 1H), 4.07 (qd, *J* = 7.9, 1.6 Hz, 2H), 3.82 (d, *J =* 12.8 Hz, 5H), 3.72 (t, *J* = 7.1 Hz, 2H), 2.92 (tt, *J* = 7.2, 1.0 Hz, 2H), 2.79 (dt, *J* = 12.2, 7.0 Hz, 1H), 2.66 (dt, *J =* 12.4, 7.1 Hz, 1H), 2.36 - 2.19 (m, 2H), 1.45 - 1.38 (m, 3H). ESI-MS *m*/*z* 492.2 [M+H]⁺.

### Example A83: Synthesis of Compound A83

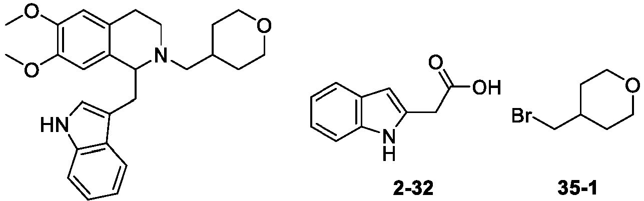

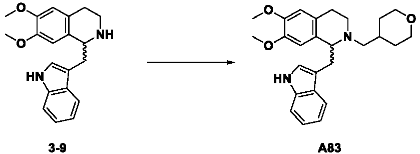

Refering to the synthesis method of intermediate 3-8, intermediate 3-9 was obtained by replacing 1-2 in Example A1 with iodomethane, replacing 2-5 in Example A1 with compound 2-32. To a solution of 3-9 in acetonitrile was added potassium carbonate and 35-1, and refluxed under argon protection for 5 hours. Then cooled down, evaporated the solvent, diluted with dichloromethane, and washed twice with saturated ammonium chloride and sodium chloride. The organic phases were combined, dried with anhydrous sodium sulfate, spundried to remove the solvent, and subjected to column chromatography (petroleum ether: ethyl acetate 1:1) to obtain a off-white solid with a yield of 90% (compound A83). ¹H NMR (500 MHz, Chloroform-d) δ 7.62 (dd, *J =* 7.1, 1.8 Hz, 1H), 7.38 - 7.33 (m, 1H), 7.18 (d, *J* = 8.4 Hz, 1H), 7.12 - 7.02 (m, 2H), 6.78 (d, *J =* 0.6 Hz, 1H), 6.57 (t, *J* = 1.0 Hz, 1H), 3.90 - 3.82 (m, 7H), 3.74 (td, *J =* 7.1, 2.4 Hz, 4H), 3.52 (dd, *J* = 12.4, 7.1 Hz, 1H), 3.06 (dt, *J =* 12.5, 7.2 Hz, 1H), 2.95 (dt, *J =* 12.3, 6.9 Hz, 1H), 2.87 (dd, *J =* 12.4, 7.0 Hz, 1H), 2.85 - 2.76 (m, 1H), 2.74 (dtd, *J* = 12.3, 7.2, 0.9 Hz, 1H), 2.61 (dd, *J =* 12.3, 7.0 Hz, 1H), 2.55 (dd, *J* = 12.4, 6.9 Hz, 1H), 1.95 (hept, *J =* 7.0 Hz, 1H), 1.87 - 1.68 (m, 4H). ESI-MS *m*/*z* 421.2 [M+H]⁺.

### Example A84: Synthesis of Compound A84

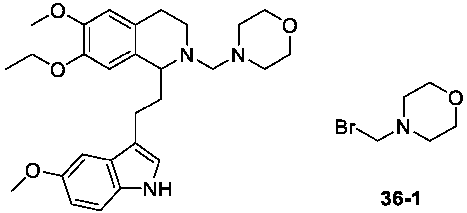

Refering to the synthesis method of compound A83, compound A84 was obtained by replacing the morpholinyl chloride in Example A1 with compound 36-1. ¹H NMR (500 MHz, DMSO-d6) δ 7.25 (d, J = 7.4 Hz, 1H), 7.13 - 7.08 (m, 2H), 6.82 (dd, J = 7.3, 2.0 Hz, 1H), 6.69 (s, 1H), 6.61 (d, J = 0.9 Hz, 1H), 4.11 (q, J = 6.2 Hz, 2H), 3.83 (d, J = 4.0 Hz, 7H), 3.63 - 3.56 (m, 5H), 3.54 (s, 0H), 2.93 - 2.82 (m, 4H), 2.75 - 2.60 (m, 1H), 2.55 (dt, J = 6.1, 3.7 Hz, 4H), 2.11 (m, 2H), 1.41 (t, J = 6.3 Hz, 3H). ESI-MS *m*/*z* 480.3 [M+H]⁺.

### Example A85: Synthesis of Compound A85

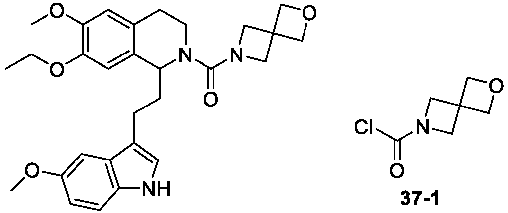

Refering to the synthesis method of compound A1, compound A85 was obtained by replacing the morpholinyl chloride in Example A1 with compound 37-1. ¹H NMR (500 MHz, DMSO-d6) δ 7.25 (d, J = 7.4 Hz, 1H), 7.13 - 7.08 (m, 2H), 6.82 (dd, J = 7.3, 2.0 Hz, 1H), 6.74 (s, 1H), 6.61 (t, J = 1.0 Hz, 1H), 4.92 - 4.86 (m, 1H), 4.11 (q, J = 6.2 Hz, 2H), 3.92 - 3.84 (m, 1H), 3.84 - 3.80 (m, 8H), 3.79 - 3.71 (m, 1H), 3.72 (d, J = 6.2 Hz, 2H), 2.92 (td, J = 5.5, 1.0 Hz, 2H), 2.69 (td, J = 7.8, 2.5 Hz, 2H), 2.14 (m, 2H), 1.41 (t, J = 6.2 Hz, 3H). ESI-MS *mlz* 506.3 [M+H]⁺.

### Example A86: Synthesis of Compound A86

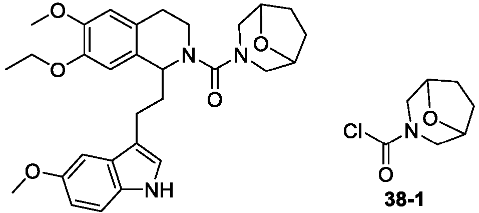

Refering to the synthesis method of compound A1, compound A86 was obtained by replacing the morpholinyl chloride in Example A1 with compound 38-1. ¹H NMR (500 MHz, DMSO-d6) δ 7.25 (d, J = 7.4 Hz, 1H), 7.13 - 7.08 (m, 2H), 6.82 (dd, J = 7.3, 2.0 Hz, 1H), 6.74 (s, 1H), 6.61 (t, J = 1.0 Hz, 1H), 4.92 - 4.86 (m, 1H), 4.24 (m, 2H), 4.11 (q, J = 6.2 Hz, 2H), 3.92 - 3.84 (m, 1H), 3.82 (s, 3H), 3.79 - 3.71 (m, 1H), 3.70 (dd, J = 12.4, 2.7 Hz, 2H), 3.56 (dd, J = 12.5, 2.8 Hz, 2H), 2.92 (td, J = 5.6, 1.0 Hz, 2H), 2.69 (td, J = 7.8, 2.5 Hz, 2H), 2.14 (m, 2H), 1.95 - 1.83 (m, 2H), 1.82 - 1.70 (m, 2H), 1.41 (t, J = 6.2 Hz, 3H). ESI-MS *m*/*z* 520.3 [M+H]⁺.

### Example A87: Synthesis of Compound A87

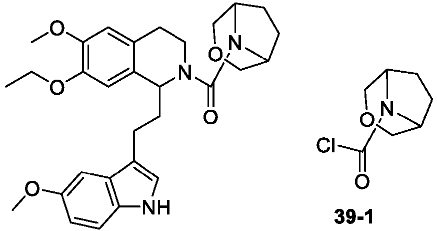

Refering to the synthesis method of compound A1, compound A87 was obtained by replacing the morpholinyl chloride in Example A1 with compound 39-1. ¹H NMR (500 MHz, DMSO-*d₆*) δ 7.25 (d, *J =* 7.4 Hz, 1H), 7.13 - 7.08 (m, 2H), 6.82 (dd, *J =* 7.3, 2.0 Hz, 1H), 6.74 (s, 1H), 6.61 (t, *J =* 1.0 Hz, 1H), 4.96 - 4.90 (m, 1H), 4.16 - 4.07 (m, 4H), 3.89 - 3.81 (m, 7H), 3.76 (s, 2H), 3.77 - 3.68 (m, 3H), 2.92 (td, *J =* 5.5, 1.0 Hz, 2H), 2.69 (td, *J =* 7.8, 2.5 Hz, 2H), 2.14 (m, 2H), 2.02 - 1.90 (m, 2H), 1.88 - 1.76 (m, 2H), 1.41 (t, *J =* 6.3 Hz, 3H). ESI-MS *m*/*z* 520.3 [M+H]⁺.

### Example A88: Synthesis of Compound A88

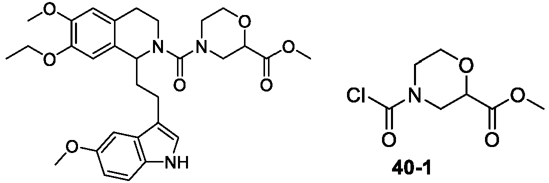

Refering to the synthesis method of compound A1, compound A88 was obtained by replacing the morpholinyl chloride in Example A1 with compound 40-1. ¹H NMR (500 MHz, DMSO-*d₆*) δ 7.25 (d, *J =* 7.4 Hz, 1H), 7.13 - 7.08 (m, 2H), 6.82 (dd, *J =* 7.3, 2.0 Hz, 1H), 6.74 (s, 1H), 6.61 (t, *J =* 1.0 Hz, 1H), 4.92 - 4.86 (m, 1H), 4.62 (t, *J =* 4.2 Hz, 1H), 4.11 (q, *J =* 6.2 Hz, 2H), 3.98 - 3.89 (m, 2H), 3.92 - 3.84 (m, 1H), 3.83 (s, 3H), 3.84 - 3.72 (m, 2H), 3.69 (s, 3H), 3.75 - 3.64 (m, 1H), 3.59 (m, 1H), 3.49 (m, 1H), 2.92 (td, *J= 5.5,* 1.0 Hz, 2H), 2.69 (td, *J =* 7.8, 2.5 Hz, 2H), 2.14 (m, 2H), 1.41 (t, *J =* 6.2 Hz, 3H). ESI-MS *m*/*z* 552.3 [M+H]⁺.

### Example A89: Synthesis of Compound A89

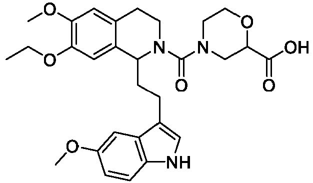

Compound A88 was dissolved in methanol, and 1 M sodium hydroxide aqueous solution was added. The reaction was carried out at room temperature for 2 hours. Then most of the methanol was removed by rotary evaporation, and most of the impurities was washed away with dichloromethane, the aqueous phase was adjust to pH 1-2 with 1M hydrochloric acid, and extracted with ethyl acetate. The ethyl acetate layers were combined, dried with anhydrous sodium sulfate, and spun-dried to remove the solvent to obtain white solid A89 with a yield of 95*%*. ¹H NMR (500 MHz, DMSO-*d₆*) δ 7.25 (d, *J* = 7.4 Hz, 1H), 7.13 - 7.08 (m, 2H), 6.82 (dd, *J* = 7.3, 2.0 Hz, 1H), 6.74 (s, 1H), 6.61 (t, *J* = 1.0 Hz, 1H), 4.92 - 4.86 (m, 1H), 4.44 (t, *J =* 4.3 Hz, 1H), 4.11 (q, *J =* 6.2 Hz, 2H), 3.97 - 3.88 (m, 2H), 3.91 - 3.84 (m, 1H), 3.83 (s, 3H), 3.82 - 3.71 (m, 2H), 3.67 (dd, *J =* 12.3, 4.2 Hz, 1H), 3.59 (m, 1H), 3.49 (m, 1H), 2.92 (td, *J* = 5.6, 1.0 Hz, 2H), 2.69 (td, *J* = 7.8, 2.5 Hz, 2H), 2.14 (m, 2H), 1.41 (t, *J =* 6.2 Hz, 3H). ESI-MS *m*/*z* 538.2 [M+H]⁺.

### Example A90: Synthesis of Compound A90

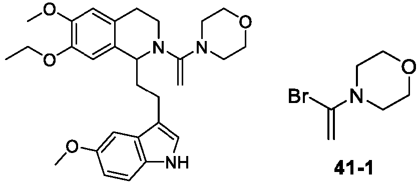

Refering to the synthesis method of compound A83, compound A90 was obtained by replacing the morpholinyl chloride in Example A1 with compound 41-1. ¹H NMR (500 MHz, DMSO-*d₆*) δ 7.25 (d, *J =* 7.4 Hz, 1H), 7.13 - 7.08 (m, 2H), 6.82 (dd, *J =* 7.3, 2.0 Hz, 1H), 6.74 (s, 1H), 6.61 (t, *J =* 1.0 Hz, 1H), 4.66 - 4.59 (m, 1H), 4.56 (d, *J =* 1.0 Hz, 2H), 4.11 (q, *J* = 6.2 Hz, 2H), 3.83 (d, *J =* 4.0 Hz, 6H), 3.73 - 3.67 (m, 2H), 3.69 - 3.62 (m, 4H), 3.41 - 3.31 (m, 4H), 2.87 (m, 2H), 2.73 - 2.62 (m, 2H), 2.23 - 2.05 (m, 2H), 1.41 (t, *J =* 6.3 Hz, 3H). ESI-MS *m*/*z* 492.3 [M+H]⁺.

### Example A96: Synthesis of Compound A96

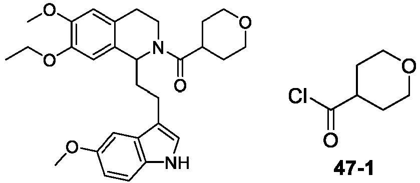

Refering to the synthesis method of compound A1, compound A96 was obtained by replacing the morpholinyl chloride in Example A1 with compound 47-1. ¹H NMR (500 MHz, DMSO-*d₆*) δ 7.25 (d, *J =* 7.4 Hz, 1H), 7.13 - 7.08 (m, 2H), 6.82 (dd, *J =* 7.3, 2.0 Hz, 1H), 6.74 (s, 1H), 6.61 (t, *J* = 1.0 Hz, 1H), 4.92 (t, *J* = 6.4 Hz, 1H), 4.11 (q, *J =* 6.2 Hz, 2H), 3.82 (s, 3H), 3.78 - 3.67 (m, 2H), 3.66 - 3.55 (m, 4H), 2.95 - 2.81 (m, 2H), 2.69 (td, *J =* 7.8, 2.5 Hz, 2H), 2.68 - 2.60 (m, 1H), 2.16 (m, 1H), 2.04 (m, 1H), 1.84 - 1.69 (m, 4H), 1.41 (t, *J* = 6.2 Hz, 3H). ESI-MS *m*/*z* 493.3 [M+H]⁺.

### Example A97: Synthesis of Compound A97

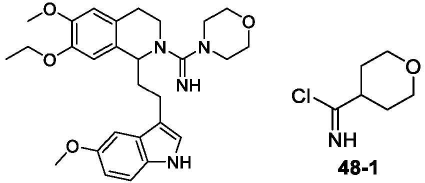

Refering to the synthesis method of compound A1, compound A97 was obtained by replacing the morpholinyl chloride in Example A1 with compound 48-1. ¹H NMR (500 MHz, DMSO-*d₆*) δ 7.60 (s, 1H), 7.25 (d, *J =* 7.4 Hz, 1H), 7.13 - 7.08 (m, 2H), 6.82 (dd, *J* = 7.3, 2.0 Hz, 1H), 6.74 (s, 1H), 6.61 (t, *J =* 1.0 Hz, 1H), 4.82 - 4.76 (m, 1H), 4.11 (q, *J =* 6.2 Hz, 2H), 3.83 (d, *J =* 4.0 Hz, 6H), 3.81 - 3.75 (m, 1H), 3.71 (dt, *J =* 12.3, 5.0 Hz, 1H), 3.70 - 3.64 (m, 4H), 3.67 - 3.57 (m, 2H), 3.44 - 3.36 (m, 2H), 2.86 (td, *J* = 5.1, 1.0 Hz, 2H), 2.73 - 2.63 (m, 2H), 2.22 - 2.07 (m, 2H), 1.41 (t, *J =* 6.2 Hz, 3H). ESI-MS *m*/*z* 493.3 [M+H]⁺.

### Example A98: Synthesis of Compound A98

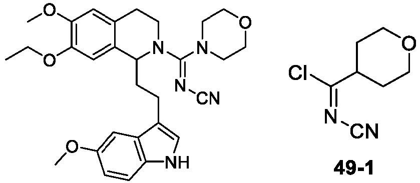

Refering to the synthesis method of compound A1, compound A98 was obtained by replacing the morpholinyl chloride in Example A1 with compound 49-1. ¹H NMR (500 MHz, DMSO-*d₆*) δ 7.25 (d, *J =* 7.4 Hz, 1H), 7.13 - 7.08 (m, 2H), 6.82 (dd, *J =* 7.3, 2.0 Hz, 1H), 6.74 (s, 1H), 6.61 (t, *J* = 1.0 Hz, 1H), 4.83 (t, *J* = 6.1 Hz, 1H), 4.11 (q, *J =* 6.2 Hz, 2H), 3.91 (m, 1H), 3.83 (d, *J =* 4.0 Hz, 6H), 3.76 - 3.66 (m, 5H), 3.60 (dt, *J =* 12.3, 4.6 Hz, 2H), 3.51 - 3.43 (m, 2H), 2.94 - 2.83 (m, 2H), 2.69 (td, *J* = 7.8, 2.5 Hz, 2H), 2.21 - 2.04 (m, 2H), 1.41 (t, *J =* 6.2 Hz, 3H). ESI-MS *m*/*z* 518.3 [M+H]⁺.

### Example A99: Synthesis of Compound A99

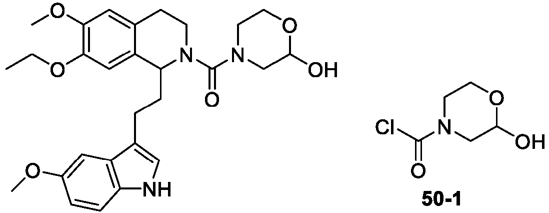

Refering to the synthesis method of compound A1, compound A99 was obtained by replacing the morpholinyl chloride in Example A1 with compound 50-1. ¹H NMR (500 MHz, DMSO-*d₆*) δ 7.25 (d, *J =* 7.4 Hz, 1H), 7.13 - 7.08 (m, 2H), 6.82 (dd, *J =* 7.3, 2.0 Hz, 1H), 6.74 (s, 1H), 6.61 (t, *J =* 1.0 Hz, 1H), 5.92 (d, *J* = 4.0 Hz, 1H), 5.28 (dt, *J* = 4.2, 3.4 Hz, 1H), 4.92 - 4.86 (m, 1H), 4.11 (q, *J =* 6.2 Hz, 2H), 3.83 (s, 3H), 3.92 - 3.70 (m, 5H), 3.67 - 3.54 (m, 2H), 3.47 (m, 1H), 2.92 (td, *J =* 5.5, 1.0 Hz, 2H), 2.69 (td, *J =* 7.8, 2.5 Hz, 2H), 2.14 (m, 2H), 1.41 (t, *J =* 6.2 Hz, 3H). ESI-MS *m*/*z* 510.3 [M+H]⁺.

### Example A100: Synthesis of Compound A100

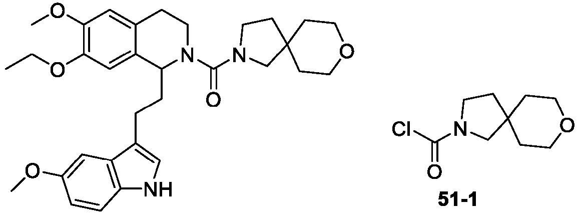

Refering to the synthesis method of compound A1, compound A100 was obtained by replacing the morpholinyl chloride in Example A1 with compound 51-1. ¹H NMR (500 MHz, DMSO-*d₆*) δ 7.25 (d, *J =* 7.4 Hz, 1H), 7.13 - 7.08 (m, 2H), 6.82 (dd, *J =* 7.3, 2.0 Hz, 1H), 6.74 (s, 1H), 6.61 (t, *J =* 1.0 Hz, 1H), 4.92 - 4.86 (m, 1H), 4.11 (q, *J =* 6.2 Hz, 2H), 3.92 - 3.85 (m, 1H), 3.83 (d, *J =* 4.0 Hz, 6H), 3.79 - 3.71 (m, 1H), 3.61 - 3.49 (m, 4H), 3.51 - 3.43 (m, 2H), 3.42 - 3.32 (m, 2H), 2.92 (td, *J =* 5.5, 1.0 Hz, 2H), 2.69 (td, *J =* 7.8, 2.5 Hz, 2H), 2.14 (m, 2H), 1.79 (dd, *J =* 5.0, 3.3 Hz, 1H), 1.71 - 1.65 (m, 1H), 1.66 (td, *J* = 3.8, 2.1 Hz, 2H), 1.57 (m, 2H), 1.41 (t, *J =* 6.3 Hz, 3H). ESI-MS *m*/*z* 548.3 [M+H]⁺.

### Example A101: Synthesis of Compound A101

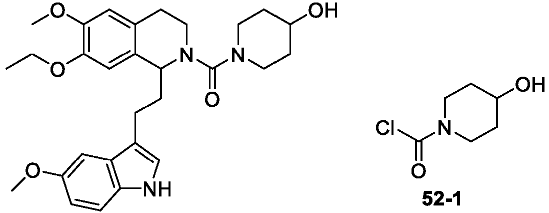

Refering to the synthesis method of compound A83, compound A101 was obtained by replacing the morpholinyl chloride in Example A1 with compound 52-1. ¹H NMR (500 MHz, DMSO-*d₆*) δ 7.25 (d, *J =* 7.4 Hz, 1H), 7.13 - 7.08 (m, 2H), 6.82 (dd, *J =* 7.3, 2.0 Hz, 1H), 6.74 (s, 1H), 6.61 (t, *J =* 1.0 Hz, 1H), 4.92 - 4.86 (m, 1H), 4.24 (d, *J =* 5.0 Hz, 1H), 4.11 (q, *J =* 6.2 Hz, 2H), 3.92 - 3.84 (m, 1H), 3.82 (s, 3H), 3.79 - 3.71 (m, 1H), 3.70 (p, *J* = 4.9 Hz, 1H), 3.53 (m, 2H), 3.35 (m, 2H), 2.92 (td, *J =* 5.6, 1.0 Hz, 2H), 2.69 (td, *J =* 7.8, 2.5 Hz, 2H), 2.14 (m, 2H), 1.87 (m, 2H), 1.69 (m, 2H), 1.41 (t, *J =* 6.2 Hz, 3H). ESI-MS *m*/*z* 508.3 [M+H]⁺.

### Example A102: Synthesis of Compound A102

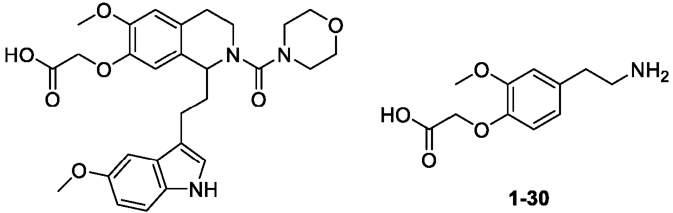

Refering to the synthesis method of compound A1, compound A102 was obtained by replacing 1-5 in Example A1 with compound 1-30. ¹H NMR (500 MHz, DMSO-*d₆*) δ 7.25 (d, *J =* 7.4 Hz, 1H), 7.13 - 7.08 (m, 2H), 6.85 - 6.77 (m, 2H), 6.61 (t, *J =* 1.0 Hz, 1H), 4.92 - 4.86 (m, 1H), 4.69 (s, 2H), 3.92 - 3.85 (m, 1H), 3.82 (d, *J =* 3.0 Hz, 6H), 3.79 - 3.71 (m, 1H), 3.64 - 3.57 (m, 4H), 3.27 - 3.13 (m, 4H), 2.92 (td, *J =* 5.6, 1.0 Hz, 2H), 2.69 (td, *J =* 7.8, 2.5 Hz, 2H), 2.14 (m, 2H). ESI-MS *m*/*z* 524.2 [M+H]⁺.

### Example A103: Synthesis of Compound A103

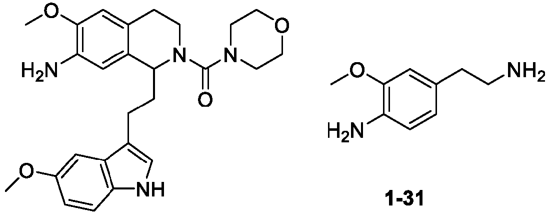

Refering to the synthesis method of compound A1, compound A103 was obtained by replacing 1-5 in Example A1 with compound 1-31. ¹H NMR (500 MHz, DMSO-*d₆*) δ 7.25 (d, *J =* 7.4 Hz, 1H), 7.13 - 7.08 (m, 2H), 6.85 - 6.74 (m, 3H), 4.85 - 4.79 (m, 1H), 4.75 (d, *J =* 6.9 Hz, 1H), 4.69 (d, *J =* 6.9 Hz, 1H), 3.88 (dt, *J =* 12.5, 5.5 Hz, 1H), 3.85 (s, 3H), 3.82 (s, 2H), 3.75 (dt, *J =* 12.5, 5.5 Hz, 1H), 3.65 - 3.57 (m, 4H), 3.27 - 3.13 (m, 4H), 2.90 (td, *J =* 5.4, 1.0 Hz, 2H), 2.69 (td, *J* = 7.8, 2.4 Hz, 2H), 2.25 - 2.08 (m, 2H). ESI-MS *m*/*z* 465.2 [M+H]⁺.

### Example A104: Synthesis of Compound A104

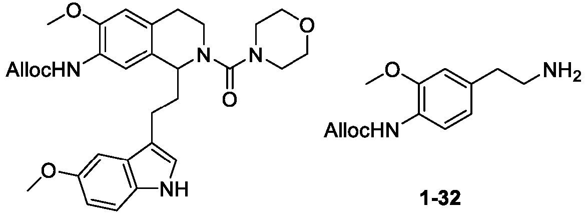

Refering to the synthesis method of compound A1, compound A104 was obtained by replacing 1-5 in Example A1 with compound 1-32. ¹H NMR (500 MHz, DMSO-*d₆*) δ 7.84 (s, 1H), 7.65 (d, *J* = 0.7 Hz, 1H), 7.25 (d, *J =* 7.4 Hz, 1H), 7.13 - 7.08 (m, 2H), 6.85 - 6.80 (m, 2H), 5.97 (tt, *J* = 17.0, 5.8 Hz, 1H), 5.29 (m, 2H), 4.91 (s, 0H), 4.66 (m, 2H), 3.89 (s, 3H), 3.92 - 3.84 (m, 1H), 3.82 (s, 2H), 3.75 (dt, *J =* 12.5, 5.5 Hz, 1H), 3.65 - 3.57 (m, 4H), 3.27 - 3.13 (m, 4H), 2.90 (td, *J =* 5.4, 1.0 Hz, 2H), 2.75 - 2.62 (m, 2H), 2.20 (m, 1H), 2.10 (m, 1H). ESI-MS *m*/*z* 549.3 [M+H]⁺.

### Example A105: Synthesis of Compound A105

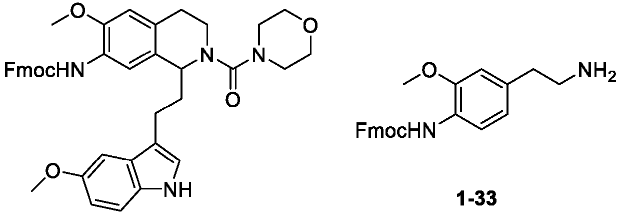

Refering to the synthesis method of compound A1, compound A105 was obtained by replacing 1-5 in Example A1 with compound 1-33. ¹H NMR (500 MHz, DMSO-*d₆*) δ 7.81 (dd, *J =* 7.4, 1.6 Hz, 1H), 7.70 - 7.64 (m, 1H), 7.43 (m, 2H), 7.25 (d, *J =* 7.3 Hz, 0H), 7.13 - 7.08 (m, 1H), 6.85 - 6.80 (m, 1H), 4.52 (d, *J =* 4.8 Hz, 1H), 4.42 (t, *J =* 4.8 Hz, 0H), 3.89 (s, 1H), 3.82 (s, 1H), 3.75 (dt, *J =* 12.5, 5.5 Hz, 0H), 3.64 - 3.57 (m, 2H), 3.27 - 3.13 (m, 2H), 2.90 (td, *J* = 5.4, 1.0 Hz, 1H), 2.69 (td, *J =* 7.8, 2.4 Hz, 1H), 2.24 - 2.06 (m, 1H). ESI-MS *m*/*z* 687.3 [M+H]⁺.

### Example A106: Synthesis of Compound A106

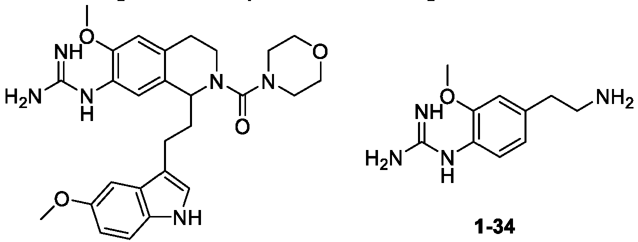

Refering to the synthesis method of compound A1, compound A106 was obtained by replacing 1-5 in Example A1 with compound 1-34. ¹H NMR (500 MHz, DMSO-*d₆*) δ 7.28 - 7.22 (m, 1H), 7.13 - 7.08 (m, 1H), 6.85 - 6.78 (m, 1H), 6.00 (s, 1H), 3.88 (s, 2H), 3.82 (s, 1H), 3.75 (dt, *J =* 12.5, 5.5 Hz, 1H), 3.65 - 3.57 (m, 2H), 3.27 - 3.13 (m, 2H), 2.90 (td, *J =* 5.4, 1.0 Hz, 1H), 2.69 (td, *J* = 7.8, 2.4 Hz, 1H), 2.24 - 2.06 (m, 1H). ESI-MS *m*/*z* 507.3 [M+H]⁺.

### Example A107: Synthesis of Compound A107

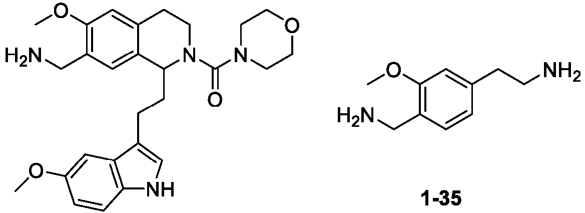

Refering to the synthesis method of compound A1, compound A107 was obtained by replacing 1-5 in Example A1 with compound 1-35. ¹H NMR (500 MHz, DMSO-*d₆*) δ 7.25 (d, *J =* 7.4 Hz, 1H), 7.22 (q, *J =* 0.9 Hz, 1H), 7.13 - 7.08 (m, 2H), 6.82 (dd, *J* = 7.3, 2.0 Hz, 1H), 6.72 (t, *J* = 1.1 Hz, 1H), 5.04 (q, *J =* 6.6 Hz, 1H), 4.92 - 4.86 (m, 1H), 4.68 (q, *J =* 6.6 Hz, 1H), 4.01 - 3.84 (m, 3H), 3.82 (s, 2H), 3.80 - 3.71 (m, 3H), 3.65 - 3.57 (m, 4H), 3.27 - 3.13 (m, 4H), 2.91 (m, 2H), 2.69 (td, *J =* 7.8, 2.5 Hz, 2H), 2.23 - 2.04 (m, 2H). ESI-MS *m*/*z* 479.3 [M+H]⁺.

### Example A108: Synthesis of Compound A108

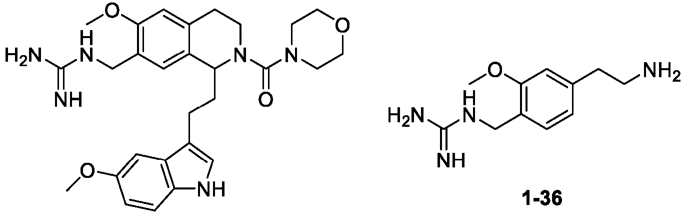

Refering to the synthesis method of compound A1, compound A108 was obtained by replacing 1-5 in Example A1 with compound 1-36. ¹H NMR (500 MHz, DMSO-d6) δ 7.52 (s, 1H), 7.25 (d, J = 7.4 Hz, 1H), 7.23 - 7.18 (m, 2H), 7.13 - 7.08 (m, 2H), 6.82 (dd, J = 7.3, 2.0 Hz, 1H), 6.70 (t, J = 1.0 Hz, 1H), 6.05 (d, J = 6.5 Hz, 1H), 5.99 (d, J = 6.5 Hz, 1H), 4.92 - 4.86 (m, 1H), 4.49 (dd, J = 4.2, 1.0 Hz, 2H), 3.92 - 3.84 (m, 1H), 3.82 (s, 2H), 3.76 (s, 3H), 3.79 - 3.71 (m, 1H), 3.65 - 3.55 (m, 4H), 3.27 - 3.13 (m, 4H), 2.96 - 2.87 (m, 2H), 2.69 (td, J = 7.8, 2.5 Hz, 2H), 2.24 - 2.06 (m, 2H). ESI-MS *m*/*z* 521.3 [M+H]⁺.

### Example A109: Synthesis of Compound A109

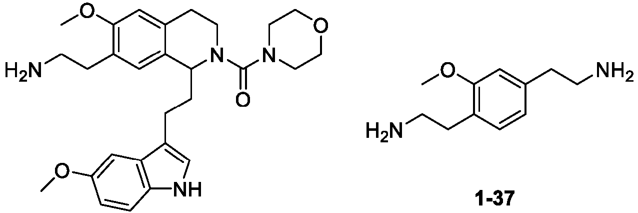

Refering to the synthesis method of compound A1, compound A109 was obtained by replacing 1-5 in Example A1 with compound 1-37. ¹H NMR (500 MHz, DMSO-*d₆*) δ 7.25 (d, *J =* 7.3 Hz, 1H), 7.13 - 7.08 (m, 3H), 6.82 (dd, *J =* 7.3, 2.0 Hz, 1H), 6.73 (t, *J =* 1.0 Hz, 1H), 4.92 - 4.86 (m, 1H), 3.92 - 3.84 (m, 1H), 3.82 (d, *J* = 2.0 Hz, 6H), 3.79 - 3.71 (m, 1H), 3.65 - 3.57 (m, 4H), 3.27 - 3.13 (m, 4H), 3.09 (m, 2H), 2.96 - 2.81 (m, 4H), 2.69 (td, *J* = 7.8, 2.5 Hz, 2H), 2.23 - 2.04 (m, 2H), 1.62 (q, *J* = 6.6 Hz, 1H), 1.49 (q, *J =* 6.6 Hz, 1H). ESI-MS *m*/*z* 493.3 [M+H]⁺.

### Example A110: Synthesis of Compound A110

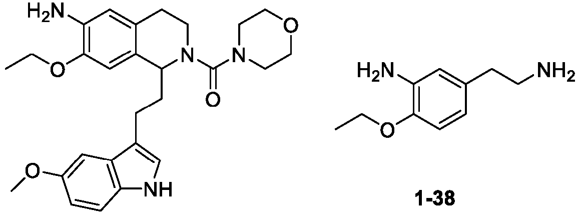

Refering to the synthesis method of compound A1, compound A110 was obtained by replacing 1-5 in Example A1 with compound 1-38. ¹H NMR (500 MHz, DMSO-d6) δ 7.25 (d, J = 7.4 Hz, 1H), 7.13 - 7.08 (m, 2H), 6.82 (dd, J = 7.4, 2.0 Hz, 1H), 6.80 (s, 1H), 6.68 (t, J = 1.1 Hz, 1H), 4.93 - 4.87 (m, 1H), 4.62 (s, 1H), 4.09 (q, J = 6.1 Hz, 2H), 3.88 (m, 1H), 3.82 (s, 2H), 3.75 (m, 1H), 3.65 - 3.57 (m, 4H), 3.27 - 3.13 (m, 4H), 2.93 (m, 2H), 2.69 (td, J = 7.8, 2.5 Hz, 2H), 2.14 (m, 2H), 1.41 (t, J = 6.3 Hz, 3H). ESI-MS *m*/*z* 479.3 [M+H]⁺.

### Example A111: Synthesis of Compound A111

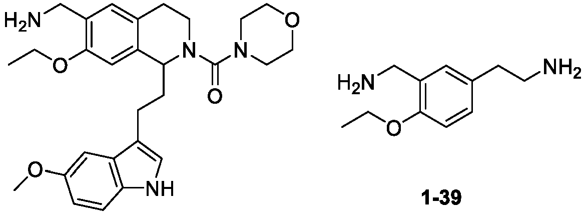

Refering to the synthesis method of compound A1, compound A111 was obtained by replacing 1-5 in Example A1 with compound 1-39. ¹H NMR (500 MHz, DMSO-*d₆*) δ 7.25 (d, *J =* 7.4 Hz, 1H), 7.13 - 7.07 (m, 2H), 7.03 (p, *J =* 1.0 Hz, 1H), 6.83 (s, 1H), 6.85 - 6.80 (m, 1H), 5.00 (q, *J* = 6.5 Hz, 1H), 4.90 (s, 0H), 4.65 (q, *J* = 6.5 Hz, 1H), 4.09 (q, *J* = 6.3 Hz, 2H), 4.04 - 3.92 (m, 2H), 3.92 - 3.84 (m, 1H), 3.82 (s, 2H), 3.79 - 3.71 (m, 1H), 3.65 - 3.57 (m, 4H), 3.27 - 3.13 (m, 4H), 2.92 (td, *J =* 5.4, 1.0 Hz, 2H), 2.69 (td, *J =* 7.8, 2.5 Hz, 2H), 2.24 - 2.05 (m, 2H), 1.42 (t, *J* = 6.2 Hz, 3H). ESI-MS *m*/*z* 493.3 [M+H]⁺.

### Example A112: Synthesis of Compound A112

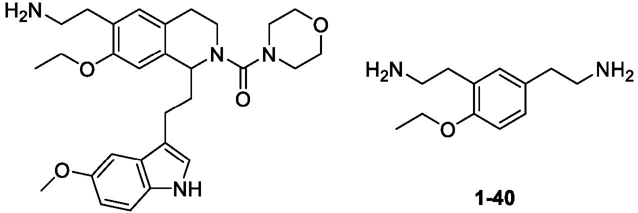

Refering to the synthesis method of compound A1, compound A112 was obtained by replacing 1-5 in Example A1 with compound 1-40. ¹H NMR (500 MHz, DMSO-*d₆*) δ 7.25 (d, *J =* 7.4 Hz, 1H), 7.13 - 7.08 (m, 2H), 6.92 (p, *J =* 1.0 Hz, 1H), 6.82 (dd, *J* = 7.3, 2.0 Hz, 1H), 6.79 (d, *J =* 0.6 Hz, 1H), 4.90 (s, 0H), 4.09 (q, *J =* 6.3 Hz, 2H), 3.88 (m, 1H), 3.82 (s, 2H), 3.75 (m, 1H), 3.65 - 3.57 (m, 4H), 3.27 - 3.13 (m, 4H), 3.09 (m, 2H), 2.96 - 2.81 (m, 4H), 2.73 - 2.65 (m, 1H), 2.24 - 2.05 (m, 2H), 1.62 (q, *J =* 6.6 Hz, 1H), 1.49 (t, *J =* 6.6 Hz, 1H), 1.42 (t, *J =* 6.2 Hz, 3H). ESI-MS *m*/*z* 507.3 [M+H]⁺.

### Example A113: Synthesis of Compound A113

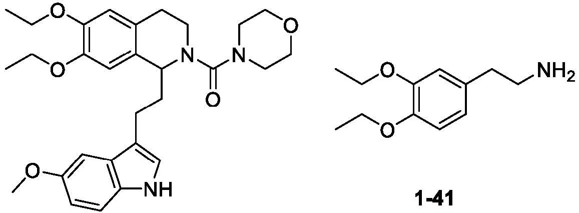

Refering to the synthesis method of compound A1, compound A113 was obtained by replacing 1-5 in Example A1 with compound 1-41. ¹H NMR (500 MHz, DMSO-*d₆*) δ 7.25 (d, *J =* 7.4 Hz, 1H), 7.13 - 7.08 (m, 2H), 6.82 (dd, *J* = 7.3, 2.0 Hz, 1H), 6.76 - 6.70 (m, 2H), 4.92 - 4.86 (m, 1H), 4.11 (q, *J =* 6.2 Hz, 4H), 3.92 - 3.84 (m, 1H), 3.82 (s, 2H), 3.79 - 3.71 (m, 1H), 3.64 - 3.57 (m, 4H), 3.27 - 3.13 (m, 4H), 2.92 (td, *J =* 5.6, 1.0 Hz, 2H), 2.69 (td, *J* = 7.8, 2.5 Hz, 2H), 2.14 (m, 2H), 1.41 (t, *J =* 6.3 Hz, 6H). ESI-MS *m*/*z* 508.3 [M+H]⁺.

### Example A114: Synthesis of Compound A114

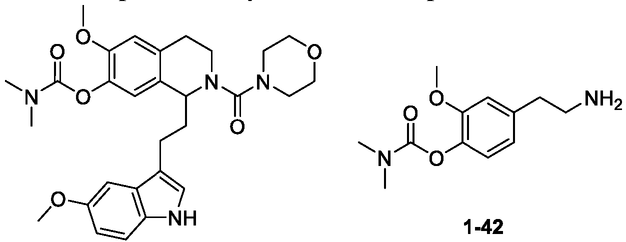

Refering to the synthesis method of compound A1, compound A114 was obtained by replacing 1-5 in Example A1 with compound 1-42. ¹H NMR (500 MHz, DMSO-*d₆*) δ 7.25 (d, *J =* 7.4 Hz, 1H), 7.13 - 7.06 (m, 3H), 6.82 (dd, *J =* 7.3, 2.0 Hz, 1H), 6.65 (t, *J =* 1.0 Hz, 1H), 4.90 (t, *J =* 6.6 Hz, 1H), 3.92 - 3.81 (m, 5H), 3.79 - 3.71 (m, 1H), 3.65 - 3.55 (m, 4H), 3.27 - 3.13 (m, 4H), 2.92 (td, *J =* 5.5, 1.0 Hz, 2H), 2.69 (td, *J =* 7.8, 2.5 Hz, 2H), 2.14 (m, 2H). ESI-MS *m*/*z* 537.3 [M+H]⁺.

### Example A115: Synthesis of Compound A115

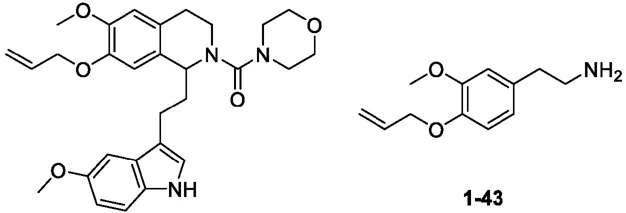

Refering to the synthesis method of compound A1, compound A115 was obtained by replacing 1-5 in Example A1 with compound 1-43. ¹H NMR (500 MHz, DMSO-*d₆*) δ 7.25 (d, *J =* 7.4 Hz, 1H), 7.13 - 7.08 (m, 2H), 6.82 (dd, *J* = 7.3, 2.0 Hz, 1H), 6.76 (d, *J =* 0.7 Hz, 1H), 6.59 (t, *J* = 1.0 Hz, 1H), 6.05 (m, 1H), 5.36 (m, 2H), 4.92 - 4.86 (m, 1H), 4.60 (dt, *J* = 5.5, 1.0 Hz, 2H), 3.92 - 3.85 (m, 1H), 3.82 (d, *J =* 2.6 Hz, 6H), 3.79 - 3.71 (m, 1H), 3.65 - 3.57 (m, 4H), 3.27 - 3.13 (m, 4H), 2.92 (td, *J* = 5.5, 1.0 Hz, 2H), 2.76 - 2.62 (m, 2H), 2.20 (m, 1H), 2.09 (m, 1H). ESI-MS *m*/*z* 506.3 [M+H]⁺.

### Example A116: Synthesis of Compound A116

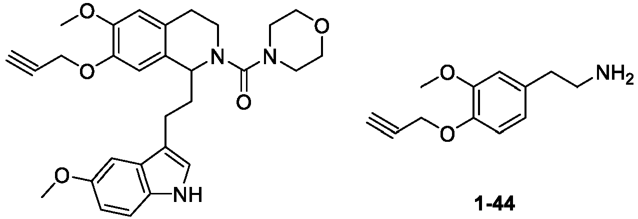

Refering to the synthesis method of compound A1, compound A116 was obtained by replacing 1-5 in Example A1 with compound 1-44. ¹H NMR (500 MHz, DMSO-*d₆*) δ 7.25 (d, *J =* 7.4 Hz, 1H), 7.13 - 7.08 (m, 2H), 6.85 - 6.78 (m, 2H), 6.60 (t, *J =* 1.0 Hz, 1H), 4.92 - 4.86 (m, 1H), 4.86 - 4.78 (m, 2H), 3.92 - 3.84 (m, 1H), 3.82 (d, *J =* 2.0 Hz, 6H), 3.79-3.71 (m, 1H), 3.65 - 3.55 (m, 4H), 3.36 (d, *J* = 6.2 Hz, 0H), 3.27 - 3.13 (m, 4H), 2.92 (td, *J* = 5.6, 1.0 Hz, 2H), 2.69 (td, *J* = 7.8, 2.5 Hz, 2H), 2.14 (m, 2H). ESI-MS *m*/*z* 504.2 [M+H]⁺.

### Example A117: Synthesis of Compound A117

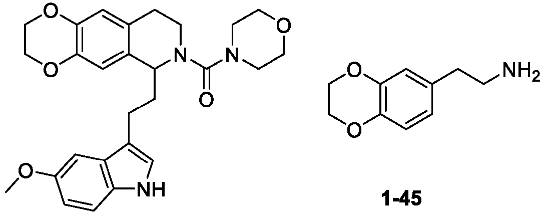

Refering to the synthesis method of compound A1, compound A117 was obtained by replacing 1-5 in Example A1 with compound 1-45. ¹H NMR (500 MHz, DMSO-*d*₆) δ 7.25 (d, *J =* 7.4 Hz, 1H), 7.13 - 7.08 (m, 2H), 6.82 (dd, *J =* 7.3, 2.0 Hz, 1H), 6.75 (s, 1H), 6.64 (t, *J =* 1.1 Hz, 1H), 4.92 - 4.86 (m, 1H), 4.26 (d, *J =* 0.7 Hz, 4H), 3.92 - 3.84 (m, 1H), 3.82 (s, 2H), 3.79 - 3.71 (m, 1H), 3.65 - 3.57 (m, 4H), 3.27 - 3.13 (m, 4H), 2.92 (td, *J =* 5.4, 1.0 Hz, 2H), 2.69 (td, *J =* 7.8, 2.5 Hz, 2H), 2.14 (m, 2H). ESI-MS *m*/*z* 478.2 [M+H]⁺.

### Example A118: Synthesis of Compound A118

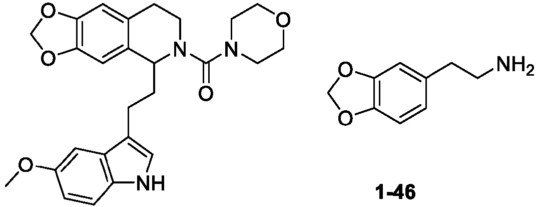

Refering to the synthesis method of compound A1, compound A118 was obtained by replacing 1-5 in Example A1 with compound 1-46. ¹H NMR (500 MHz, DMSO-*d*₆) δ 7.25 (d, *J =* 7.4 Hz, 1H), 7.13 - 7.08 (m, 2H), 6.82 (dd, *J =* 7.3, 2.0 Hz, 1H), 6.67 (s, 1H), 6.57 (t, *J* = 1.0 Hz, 1H), 5.98 (d, *J =* 0.8 Hz, 2H), 4.92 - 4.86 (m, 1H), 3.92 - 3.84 (m, 1H), 3.82 (s, 2H), 3.79 - 3.71 (m, 1H), 3.65 - 3.57 (m, 4H), 3.27 - 3.13 (m, 4H), 2.92 (td, *J =* 5.4, 1.0 Hz, 2H), 2.69 (td, *J =* 7.8, 2.5 Hz, 2H), 2.14 (m, 2H). ESI-MS *m*/*z* 464.2 [M+H]⁺.

### Example A119: Synthesis of Compound A119

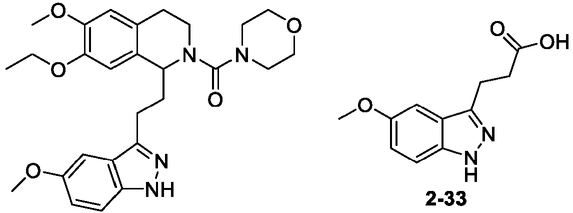

Refering to the synthesis method of compound A1, compound A119 was obtained by replacing 2-5 in Example A1 with compound 2-33. ¹H NMR (500 MHz, DMSO-*d*₆) δ 7.49 (d, *J =* 7.9 Hz, 1H), 7.23 (d, *J =* 2.1 Hz, 1H), 6.85 (dd, *J* = 7.7, 2.0 Hz, 1H), 6.74 (s, 1H), 6.61 (t, *J* = 1.0 Hz, 1H), 4.97 - 4.90 (m, 1H), 4.11 (q, *J =* 6.2 Hz, 2H), 3.92 - 3.84 (m, 1H), 3.83 (d, *J* = 3.6 Hz, 6H), 3.79 - 3.71 (m, 1H), 3.65 - 3.55 (m, 4H), 3.27 - 3.13 (m, 5H), 2.98 - 2.88 (m, 3H), 2.29 (dq, *J =* 14.5, 7.8 Hz, 1H), 2.11 (dq, *J =* 14.5, 7.7 Hz, 1H), 1.41 (t, *J =* 6.3 Hz, 3H). ESI-MS *m*/*z* 495.3 [M+H]⁺.

### Example A120: Synthesis of Compound A120

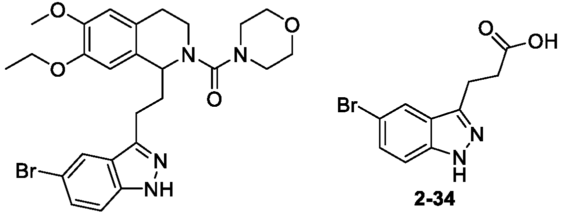

Refering to the synthesis method of compound A1, compound A120 was obtained by replacing 2-5 in Example A1 with compound 2-34. ¹H NMR (500 MHz, DMSO-*d*₆) δ 7.84 (t, *J =* 1.2 Hz, 1H), 7.53 (d, *J =* 1.3 Hz, 2H), 6.74 (s, 1H), 6.61 (t, *J =* 1.0 Hz, 1H), 4.97 - 4.90 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 5H), 2.98 - 2.88 (m, 2H), 2.28 (dq, *J =* 14.5, 7.6 Hz, 1H), 2.13 (dq, *J =* 14.5, 7.8 Hz, 1H), 1.41 (t, *J =* 6.3 Hz, 3H). ESI-MS *m*/*z* 543.2 [M+H]⁺.

### Example A121: Synthesis of Compound A121

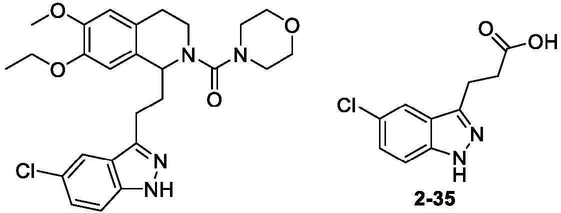

Refering to the synthesis method of compound A1, compound A121 was obtained by replacing 2-5 in Example A1 with compound 2-35. ¹H NMR (500 MHz, DMSO-*d*₆) δ 7.54 (d, *J* = 2.1 Hz, 1H), 7.47 (d, *J* = 7.7 Hz, 1H), 7.35 (dd, *J* = 7.7, 2.0 Hz, 1H), 6.74 (s, 1H), 6.61 (t, *J =* 1.0 Hz, 1H), 4.97 - 4.90 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 5H), 2.98 - 2.88 (m, 2H), 2.28 (dq, *J* = 14.5, 7.6 Hz, 1H), 2.13 (dq, *J* = 14.5, 7.8 Hz, 1H), 1.41 (t, *J* = 6.3 Hz, 3H).ESI-MS *m*/*z* 499.2 [M+H]⁺.

### Example A122: Synthesis of Compound A122

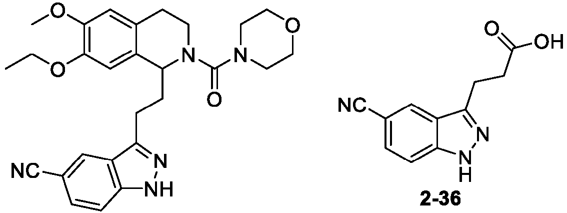

Refering to the synthesis method of compound A1, compound A122 was obtained by replacing 2-5 in Example A1 with compound 2-36. ¹H NMR (500 MHz, DMSO-*d*₆) δ 8.07 (d, *J =* 2.2 Hz, 1H), 7.68 - 7.60 (m, 2H), 6.74 (s, 1H), 6.61 (t, *J =* 1.0 Hz, 1H), 4.97 - 4.90 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 5H), 2.98 - 2.88 (m, 2H), 2.28 (dq, *J =* 14.5, 7.6 Hz, 1H), 2.13 (dq, *J =* 14.5, 7.8 Hz, 1H), 1.41 (t, *J =* 6.3 Hz, 3H). ESI-MS *m*/*z* 490.2 [M+H]⁺.

### Example A123: Synthesis of Compound A123

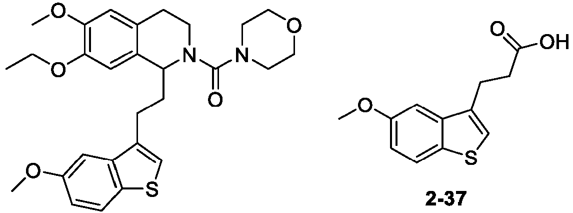

Refering to the synthesis method of compound A1, compound A123 was obtained by replacing 2-5 in Example A1 with compound 2-37. ¹H NMR (500 MHz, DMSO-*d*₆) δ 7.57 (d, *J* = 7.7 Hz, 1H), 7.45 (s, 1H), 7.26 (d, *J =* 1.6 Hz, 1H), 7.05 (dd, *J* = 7.9, 2.0 Hz, 1H), 6.74 (s, 1H), 6.61 (t, *J =* 1.0 Hz, 1H), 4.93 - 4.86 (m, 1H), 4.11 (q, *J =* 6.2 Hz, 2H), 3.92 - 3.85 (m, 1H), 3.83 (d, *J =* 4.2 Hz, 6H), 3.79 - 3.71 (m, 1H), 3.65 - 3.57 (m, 4H), 3.27 - 3.13 (m, 4H), 2.92 (td, *J =* 5.5, 1.0 Hz, 2H), 2.78 (td, *J =* 7.3, 5.0 Hz, 2H), 2.20 - 2.02 (m, 2H), 1.41 (t, *J =* 6.2 Hz, 3H). ESI-MS *m*/*z* 511.2 [M+H]⁺.

### Example A124: Synthesis of compound A124

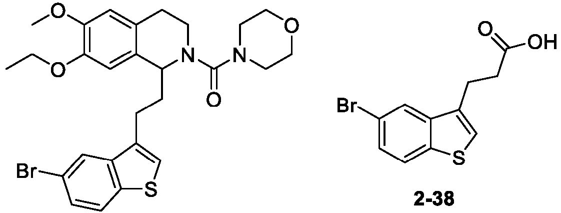

Refering to the synthesis method of compound A1, compound A124 was obtained by replacing 2-5 in Example A1 with compound 2-38. ¹H NMR (500 MHz, DMSO-*d*₆) δ 7.77 - 7.73 (m, 1H), 7.66 (d, *J* = 7.9 Hz, 1H), 7.49 (dd, *J =* 7.9, 2.0 Hz, 1H), 7.43 (s, 1H), 6.74 (s, 1H), 6.61 (t, *J =* 1.0 Hz, 1H), 4.93 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J* = 5.6, 1.0 Hz, 2H), 2.75 (dd, *J =* 14.3, 7.1 Hz, 2H), 2.19 - 2.02 (m, 2H), 1.41 (t, *J =* 6.3 Hz, 3H). ESI-MS *m*/*z* 559.1 [M+H]⁺.

### Example A125: Synthesis of compound A125

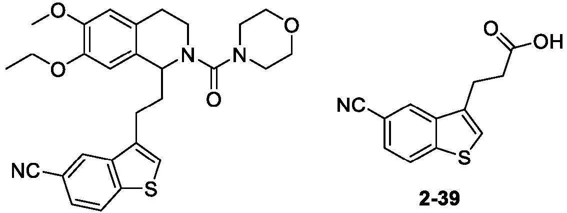

Refering to the synthesis method of compound A1, compound A125 was obtained by replacing 2-5 in Example A1 with compound 2-39. ¹H NMR (500 MHz, DMSO-*d*₆) δ 7.91 (d, *J =* 2.0 Hz, 1H), 7.75 (dd, *J =* 6.4, 1.8 Hz, 1H), 7.71 (d, *J* = 6.3 Hz, 1H), 7.43 (s, 1H), 6.74 (s, 1H), 6.61 (t, *J* = 1.0 Hz, 1H), 4.93 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J =* 5.6, 1.0 Hz, 2H), 2.77 (td, *J =* 7.3, 4.5 Hz, 2H), 2.19 - 2.02 (m, 2H), 1.41 (t, *J* = 6.3 Hz, 3H). ESI-MS *m*/*z* 506.2 [M+H]⁺.

### Example A126: Synthesis of Compound A126

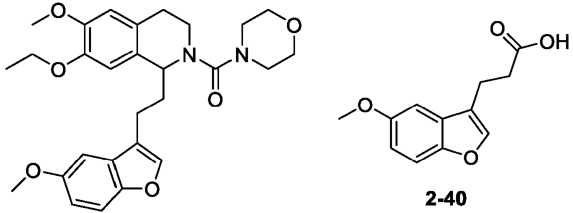

Refering to the synthesis method of compound A1, compound A126 was obtained by replacing 2-5 in Example A1 with compound 2-40. ¹H NMR (500 MHz, DMSO-*d*₆) δ 7.76 (s, 1H), 7.33 (d, *J =* 8.4 Hz, 1H), 7.20 (d, *J =* 2.1 Hz, 1H), 6.96 (dd, *J =* 8.4, 1.9 Hz, 1H), 6.74 (s, 1H), 6.61 (t, *J =* 1.0 Hz, 1H), 4.89 (s, 0H), 4.11 (q, *J =* 6.2 Hz, 2H), 3.92 - 3.85 (m, 1H), 3.83 (d, *J =* 5.4 Hz, 6H), 3.79 - 3.71 (m, 1H), 3.65 - 3.57 (m, 4H), 3.27 - 3.13 (m, 4H), 2.92 (td, *J =* 5.6, 1.0 Hz, 2H), 2.85 - 2.71 (m, 2H), 2.08 (m, 1H), 1.99 (m, 1H), 1.41 (t, *J =* 6.2 Hz, 3H). ESI-MS *m*/*z* 495.2 [M+H]⁺.

### Example A127: Synthesis of compound A127

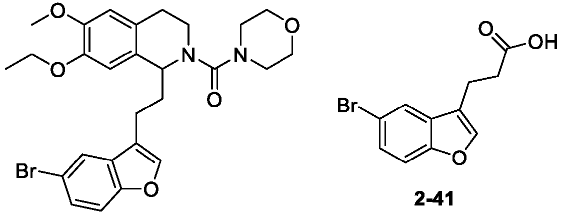

Refering to the synthesis method of compound A1, compound A127 was obtained by replacing 2-5 in Example A1 with compound 2-41. ¹H NMR (500 MHz, DMSO-*d*₆) δ 7.85 (d, *J* = 1.8 Hz, 1H), 7.76 (s, 1H), 7.57 - 7.48 (m, 2H), 6.74 (s, 1H), 6.61 (t, *J =* 1.0 Hz, 1H), 4.89 (s, 0H), 4.11 (q, *J* = 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 4H), 2.95 - 2.89 (m, 2H), 2.85 - 2.71 (m, 1H), 2.08 (m, 1H), 1.99 (m, 1H), 1.41 (t, *J =* 6.3 Hz, 3H). ESI-MS *m*/*z* 543.1 [M+H]⁺.

### Example A128: Synthesis of compound A128

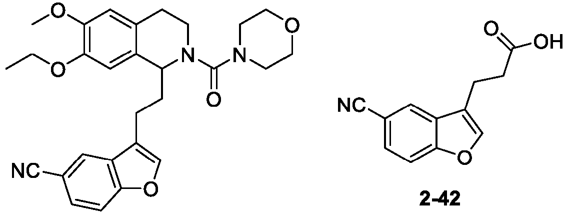

Refering to the synthesis method of compound A1, compound A128 was obtained by replacing 2-5 in Example A1 with compound 2-42. ¹H NMR (500 MHz, DMSO-*d*₆) δ 7.94 (d, *J* = 2.0 Hz, 1H), 7.76 (s, 1H), 7.70 (dd, *J =* 7.0, 1.9 Hz, 1H), 7.53 (d, *J =* 7.0 Hz, 1H), 6.74 (s, 1H), 6.61 (t, *J =* 1.0 Hz, 1H), 4.89 (s, 0H), 4.11 (q, *J =* 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 4H), 2.97 - 2.89 (m, 2H), 2.85 - 2.71 (m, 1H), 2.08 (m, 1H), 1.99 (m, 1H), 1.41 (t, *J =* 6.3 Hz, 3H). ESI-MS *m*/*z* 490.2 [M+H]⁺.

### Example A129: Synthesis of compound A129

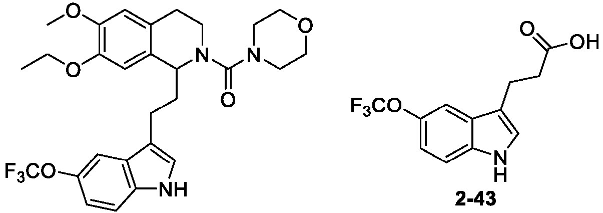

Refering to the synthesis method of compound A1, compound A129 was obtained by replacing 2-5 in Example A1 with compound 2-43. ¹H NMR (500 MHz, DMSO-*d*₆) δ 7.33 - 7.26 (m, 2H), 7.14 - 7.08 (m, 2H), 6.74 (s, 1H), 6.61 (t, *J =* 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J =* 5.6, 1.0 Hz, 2H), 2.69 (td, *J* = 7.8, 2.5 Hz, 2H), 2.14 (m, 2H), 1.41 (t, *J =* 6.3 Hz, 3H). ESI-MS *m*/*z* 548.2 [M+H]⁺.

### Example A130: Synthesis of Compound A130

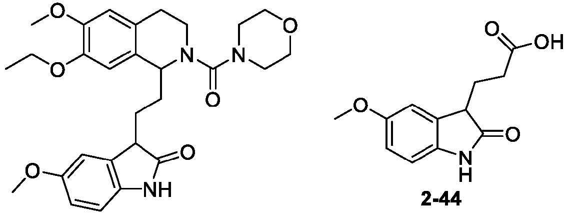

Refering to the synthesis method of compound A1, compound A130 was obtained by replacing 2-5 in Example A1 with compound 2-44. ¹H NMR (500 MHz, DMSO-*d*₆) δ 9.91 (s, 1H), 7.38 - 7.32 (m, 1H), 7.13 (d, *J =* 2.0 Hz, 1H), 6.84 (dd, *J =* 8.8, 1.9 Hz, 1H), 6.74 (s, 1H), 6.61 (t, *J =* 1.0 Hz, 1H), 4.90 - 4.84 (m, 1H), 4.11 (q, *J =* 6.2 Hz, 2H), 3.92 - 3.71 (m, 6H), 3.67 (t, *J =* 6.0 Hz, 1H), 3.64 - 3.57 (m, 4H), 3.27 - 3.13 (m, 4H), 2.92 (td, *J =* 5.6, 1.0 Hz, 2H), 2.08 - 1.92 (m, 4H), 1.41 (t, *J =* 6.2 Hz, 3H). ESI-MS *m*/*z* 510.3 [M+H]⁺.

### Example A131: Synthesis of Compound A131

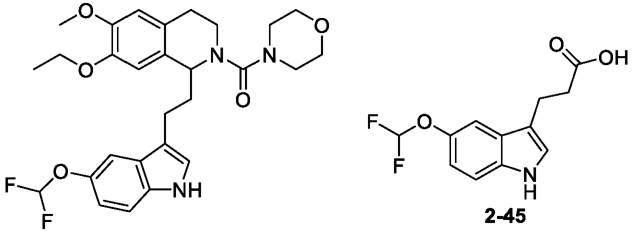

Refering to the synthesis method of compound A1, compound A131 was obtained by replacing 2-5 in Example A1 with compound 2-45. ¹H NMR (500 MHz, DMSO-*d*₆) δ 7.31 - 7.24 (m, 2H), 7.14 - 7.06 (m, 2H), 6.74 (s, 1H), 6.61 (t, *J =* 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 4H), 2.95 - 2.89 (m, 2H), 2.69 (td, *J* = 7.8, 2.5 Hz, 2H), 2.14 (m, 2H), 1.41 (t, *J =* 6.3 Hz, 3H). ESI-MS *m*/*z* 530.2 [M+H]⁺.

### Example A132: Synthesis of Compound A132

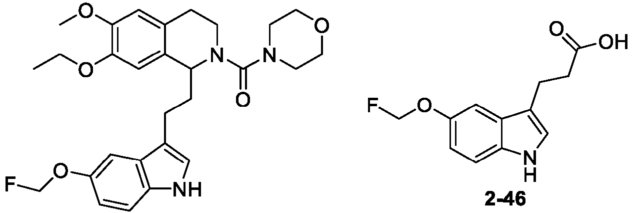

Refering to the synthesis method of compound A1, compound A132 was obtained by replacing 2-5 in Example A1 with compound 2-46. ¹H NMR (500 MHz, DMSO-*d*₆) δ 7.30 (d, *J =* 7.3 Hz, 1H), 7.16 (d, *J =* 1.5 Hz, 1H), 7.11 (d, *J =* 6.9 Hz, 1H), 6.94 (dd, *J =* 7.3, 2.0 Hz, 1H), 6.74 (s, 1H), 6.61 (t, *J =* 1.0 Hz, 1H), 5.89 (d, *J =* 0.8 Hz, 1H), 5.80 (d, *J =* 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J* = 5.6, 1.0 Hz, 2H), 2.69 (td, *J* = 7.8, 2.5 Hz, 2H), 2.14 (m, 2H), 1.41 (t, *J* = 6.3 Hz, 3H). ESI-MS *m*/*z* 512.2 [M+H]⁺.

### Example A133: Synthesis of Compound A133

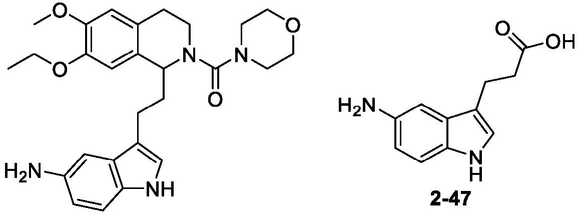

Refering to the synthesis method of compound A1, compound A133 was obtained by replacing 2-5 in Example A1 with compound 2-47. ¹H NMR (500 MHz, DMSO-*d*₆) δ 7.25 (d, *J =* 7.1 Hz, 1H), 7.08 (d, *J =* 7.1 Hz, 1H), 6.95 (d, *J =* 2.0 Hz, 1H), 6.74 (s, 1H), 6.63 - 6.55 (m, 2H), 4.92 - 4.86 (m, 1H), 4.89 (s, 2H), 4.11 (q, *J =* 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J =* 5.5, 1.0 Hz, 2H), 2.69 (td, *J =* 7.8, 2.4 Hz, 2H), 2.14 (m, 2H), 1.41 (t, *J =* 6.3 Hz, 3H). ESI-MS *m*/*z* 479.3 [M+H]⁺.

### Example A134: Synthesis of Compound A134

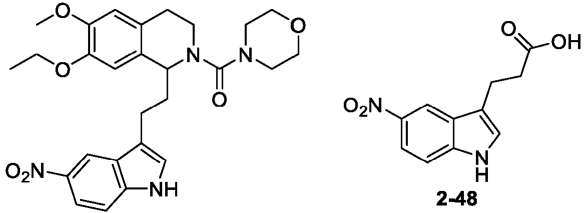

Refering to the synthesis method of compound A1, compound A134 was obtained by replacing 2-5 in Example A1 with compound 2-48. ¹H NMR (500 MHz, DMSO-*d*₆) δ 8.63 (d, *J =* 2.0 Hz, 1H), 8.16 (dd, *J =* 8.1, 2.0 Hz, 1H), 7.46 (d, *J =* 8.0 Hz, 1H), 7.15 (d, *J = 7.0* Hz, 1H), 6.74 (s, 1H), 6.61 (t, *J* = 1.0 Hz, 1H), 4.92 - 4.86 (m, 1H), 4.11 (q, *J* = 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J =* 5.6, 1.0 Hz, 2H), 2.76 - 2.68 (m, 2H), 2.14 (m, 2H), 1.41 (t, *J =* 6.3 Hz, 3H). ESI-MS *m*/*z* 509.2 [M+H]⁺.

### Example A135: Synthesis of Compound A135

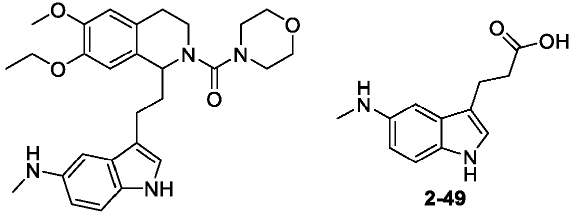

Refering to the synthesis method of compound A1, compound A135 was obtained by replacing 2-5 in Example A1 with compound 2-49. ¹H NMR (500 MHz, DMSO-*d*₆) δ 7.26 (d, *J =* 7.4 Hz, 1H), 7.14 (d, *J =* 7.0 Hz, 1H), 7.09 (q, *J =* 4.8 Hz, 1H), 6.85 (d, *J =* 2.1 Hz, 1H), 6.74 (s, 1H), 6.65 - 6.59 (m, 2H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 4H), 2.97 - 2.89 (m, 5H), 2.69 (td, *J =* 7.8, 2.4 Hz, 2H), 2.14 (m, 2H), 1.41 (t, *J =* 6.2 Hz, 3H). ESI-MS *m*/*z* 493.3 [M+H]⁺.

### Example A136: Synthesis of Compound A136

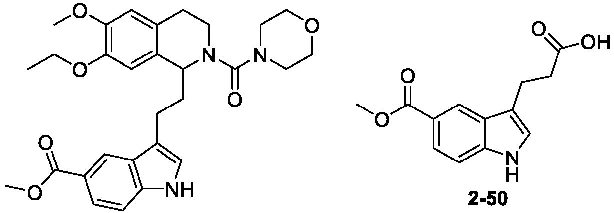

Refering to the synthesis method of compound A1, compound A136 was obtained by replacing 2-5 in Example A1 with compound 2-50. ¹H NMR (500 MHz, DMSO-*d*₆) δ 8.38 (d, *J =* 2.0 Hz, 1H), 7.91 (dd, *J =* 7.2, 1.9 Hz, 1H), 7.37 (d, *J* = 7.2 Hz, 1H), 7.13 (d, *J =* 7.0 Hz, 1H), 6.74 (s, 1H), 6.61 (t, *J* = 1.0 Hz, 1H), 4.92 - 4.86 (m, 1H), 4.11 (q, *J* = 6.2 Hz, 2H), 3.89 (s, 3H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J =* 5.6, 1.0 Hz, 2H), 2.69 (td, *J =* 7.8, 2.6 Hz, 2H), 2.14 (m, 2H), 1.41 (t, *J =* 6.2 Hz, 3H). ESI-MS *m*/*z* 522.3 [M+H]⁺.

### Example A137: Synthesis of Compound A137

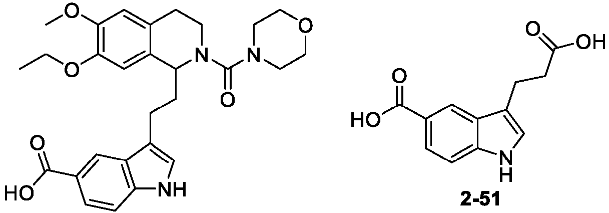

Refering to the synthesis method of compound A1, compound A137 was obtained by replacing 2-5 in Example A1 with compound 2-51. ¹H NMR (500 MHz, DMSO-*d*₆) δ 8.41 (d, *J* = 1.7 Hz, 1H), 7.88 (dd, *J* = 7.3, 2.0 Hz, 1H), 7.42 (d, *J =* 7.4 Hz, 1H), 7.14 (d, *J =* 7.1 Hz, 1H), 6.74 (s, 1H), 6.61 (t, *J* = 1.0 Hz, 1H), 4.92 - 4.86 (m, 1H), 4.11 (q, *J* = 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J* = 5.6, 1.0 Hz, 2H), 2.69 (td, *J* = 7.8, 2.6 Hz, 2H), 2.14 (m, 2H), 1.41 (t, *J =* 6.3 Hz, 3H). ESI-MS *m*/*z* 508.2 [M+H]⁺.

### Example A138: Synthesis of Compound A138

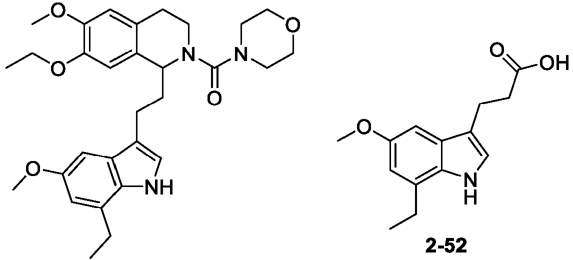

Refering to the synthesis method of compound A1, compound A138 was obtained by replacing 2-5 in Example A1 with compound 2-52. ¹H NMR (500 MHz, DMSO-*d*₆) δ 9.45 (d, *J =* 7.1 Hz, 1H), 7.09 (d, *J =* 6.9 Hz, 1H), 7.03 (d, *J =* 2.3 Hz, 1H), 6.76 - 6.69 (m, 2H), 6.61 (t, *J =* 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.72 (s, 2H), 3.64 - 3.57 (m, 4H), 3.27 - 3.13 (m, 4H), 2.92 (td, *J =* 5.6, 1.0 Hz, 2H), 2.72 - 2.60 (m, 4H), 2.14 (m, 2H), 1.41 (t, *J =* 6.2 Hz, 3H), 1.26 (t, *J* = 7.2 Hz, 3H). ESI-MS *m*/*z* 522.3 [M+H]⁺.

### Example A139: Synthesis of Compound A139

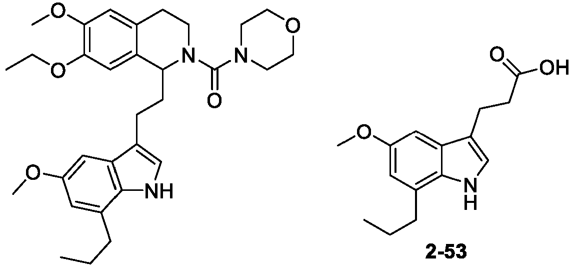

Refering to the synthesis method of compound A1, compound A139 was obtained by replacing 2-5 in Example A1 with compound 2-53. ¹H NMR (500 MHz, DMSO-*d*₆) δ 9.72 (d, *J =* 7.1 Hz, 1H), 7.09 (d, *J =* 6.9 Hz, 1H), 7.03 (d, *J =* 2.3 Hz, 1H), 6.74 (s, 1H), 6.63 - 6.56 (m, 2H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.72 (s, 2H), 3.64 - 3.57 (m, 4H), 3.27 - 3.13 (m, 4H), 2.92 (td, *J =* 5.6, 1.0 Hz, 2H), 2.80 (t, *J =* 6.3 Hz, 2H), 2.64 (d, *J =* 7.8 Hz, 1H), 2.14 (m, 2H), 1.73 (m, 2H), 1.41 (t, *J =* 6.2 Hz, 3H), 0.96 (t, *J =* 7.5 Hz, 3H). ESI-MS *m*/*z* 536.3 [M+H]⁺.

### Example A140: Synthesis of Compound A140

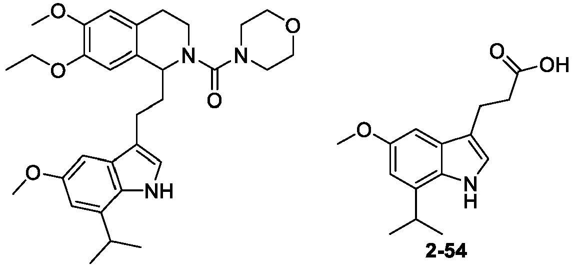

Refering to the synthesis method of compound A1, compound A140 was obtained by replacing 2-5 in Example A1 with compound 2-54. ¹H NMR (500 MHz, DMSO-*d*₆) δ 9.48 (d, *J =* 7.5 Hz, 1H), 7.09 (d, *J =* 7.4 Hz, 1H), 7.03 (d, *J =* 2.3 Hz, 1H), 6.74 (d, *J =* 2.6 Hz, 2H), 6.61 (t, *J =* 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.72 (m, 1H), 3.71 (s, 2H), 3.64 - 3.56 (m, 4H), 3.56 - 3.45 (m, 1H), 3.27 - 3.13 (m, 4H), 2.92 (td, *J* = 5.6, 1.0 Hz, 2H), 2.64 (t, *J* = 7.8 Hz, 2H), 2.24 - 2.05 (m, 2H), 1.44 (d, *J =* 6.6 Hz, 3H), 1.44 - 1.37 (m, 6H). ESI-MS *m*/*z* 536.3 [M+H]⁺.

### Example A141: Synthesis of Compound A141

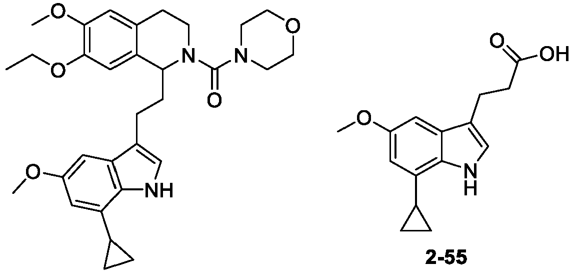

Refering to the synthesis method of compound A1, compound A141 was obtained by replacing 2-5 in Example A1 with compound 2-55. ¹H NMR (500 MHz, DMSO-*d*₆) δ 9.98 (d, *J =* 7.5 Hz, 1H), 7.09 (d, *J =* 7.4 Hz, 1H), 7.03 (d, *J =* 2.3 Hz, 1H), 6.79 - 6.72 (m, 2H), 6.61 (t, *J =* 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.72 (m, 1H), 3.71 (s, 2H), 3.65 - 3.57 (m, 4H), 3.49 - 3.41 (m, 1H), 3.27 - 3.13 (m, 4H), 2.92 (td, *J =* 5.6, 1.0 Hz, 2H), 2.64 (t, *J =* 7.8 Hz, 2H), 2.14 (m, 2H), 1.41 (t, *J =* 6.2 Hz, 3H), 1.11 - 0.97 (m, 4H). ESI-MS *m*/*z* 534.3 [M+H]⁺.

### Example A142: Synthesis of Compound A142

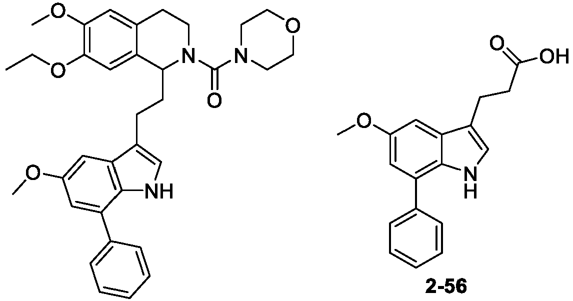

Refering to the synthesis method of compound A1, compound A142 was obtained by replacing 2-5 in Example A1 with compound 2-56. ¹H NMR (500 MHz, DMSO-*d*₆) δ 7.54 - 7.46 (m, 1H), 7.43 - 7.33 (m, 2H), 7.11 (dd, *J =* 4.6, 2.4 Hz, 1H), 6.74 (s, 0H), 6.61 (t, *J* = 1.0 Hz, 0H), 4.92 - 4.86 (m, 0H), 4.11 (q, *J =* 6.2 Hz, 1H), 3.92 - 3.71 (m, 3H), 3.65 - 3.57 (m, 2H), 3.27 - 3.13 (m, 2H), 2.92 (td, *J =* 5.6, 1.0 Hz, 1H), 2.63 (t, *J =* 7.9 Hz, 1H), 2.24 - 2.05 (m, 1H), 1.41 (t, *J =* 6.2 Hz, 1H). ESI-MS *m*/*z* 570.3 [M+H]⁺.

### Example A143: Synthesis of Compound A143

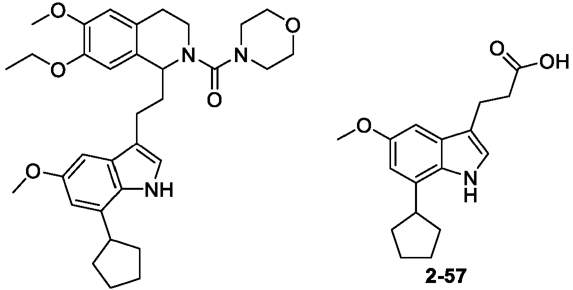

Refering to the synthesis method of compound A1, compound A143 was obtained by replacing 2-5 in Example A1 with compound 2-57. ¹H NMR (500 MHz, DMSO-*d*₆) δ 9.98 (d, *J* = 7.5 Hz, 1H), 7.09 (d, *J =* 7.4 Hz, 1H), 7.03 (d, *J =* 2.3 Hz, 1H), 6.74 (s, 1H), 6.70 (d, *J* = 2.4 Hz, 1H), 6.61 (t, *J* = 1.0 Hz, 1H), 4.92 - 4.86 (m, 1H), 4.11 (q, *J* = 6.2 Hz, 2H), 3.92 - 3.84 (m, 1H), 3.83 (s, 2H), 3.79 - 3.72 (m, 1H), 3.71 (s, 2H), 3.65 - 3.57 (m, 4H), 3.27 - 3.13 (m, 4H), 2.92 (td, *J* = 5.5, 1.0 Hz, 2H), 2.92 - 2.84 (m, 1H), 2.64 (t, *J =* 7.8 Hz, 2H), 2.24 - 2.05 (m, 2H), 1.85 (m, 2H), 1.83 - 1.73 (m, 2H), 1.76 - 1.59 (m, 4H), 1.41 (t, *J =* 6.2 Hz, 3H). ESI-MS *m*/*z* 562.3 [M+H]⁺.

### Example A144: Synthesis of Compound A144

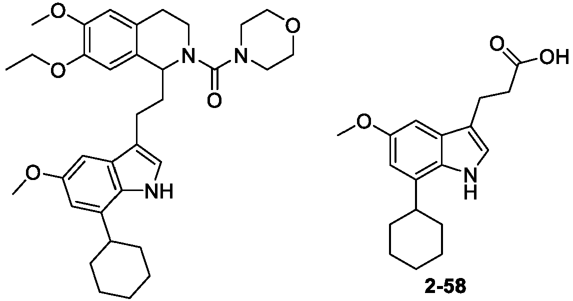

Refering to the synthesis method of compound A1, compound A144 was obtained by replacing 2-5 in Example A1 with compound 2-58. ¹H NMR (500 MHz, DMSO-*d*₆) δ 9.98 (d, *J* = 7.5 Hz, 1H), 7.09 (d, *J =* 7.4 Hz, 1H), 7.03 (d, *J =* 2.3 Hz, 1H), 6.74 (s, 1H), 6.70 (d, *J* = 2.4 Hz, 1H), 6.61 (t, *J* = 1.0 Hz, 1H), 4.92 - 4.86 (m, 1H), 4.11 (q, *J* = 6.2 Hz, 2H), 3.92 - 3.84 (m, 1H), 3.83 (s, 2H), 3.79 - 3.72 (m, 1H), 3.71 (s, 2H), 3.65 - 3.55 (m, 4H), 3.38 - 3.31 (m, 1H), 3.27 - 3.13 (m, 4H), 2.92 (td, *J =* 5.5, 1.0 Hz, 2H), 2.64 (d, *J =* 15.7 Hz, 1H), 2.14 (m, 2H), 1.82 - 1.69 (m, 4H), 1.69 - 1.56 (m, 2H), 1.56 - 1.39 (m, 6H), 1.40 (s, 1H). ESI-MS *m*/*z* 576.3 [M+H]⁺.

### Example A145: Synthesis of Compound A145

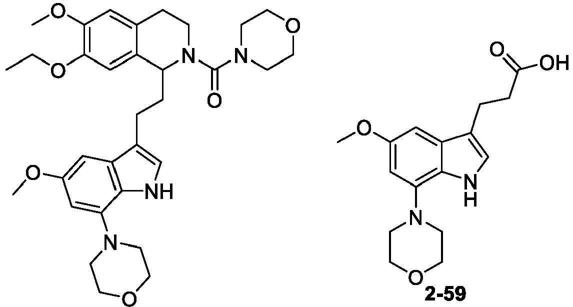

Refering to the synthesis method of compound A1, compound A145 was obtained by replacing 2-5 in Example A1 with compound 2-59. ¹H NMR (500 MHz, DMSO-*d*₆) δ 7.15 (d, *J =* 2.2 Hz, 1H), 7.08 (d, *J =* 7.5 Hz, 1H), 6.74 (s, 1H), 6.61 (t, *J =* 1.0 Hz, 1H), 6.29 (d, *J =* 2.2 Hz, 1H), 4.92 - 4.86 (m, 1H), 4.11 (q, *J =* 6.2 Hz, 2H), 3.92 - 3.84 (m, 1H), 3.84 - 3.71 (m, 10H), 3.64 - 3.55 (m, 4H), 3.31 (td, *J =* 5.8, 3.3 Hz, 4H), 3.27 - 3.13 (m, 4H), 2.92 (td, *J* = 5.5, 1.0 Hz, 2H), 2.67 (td, *J =* 7.9, 1.0 Hz, 2H), 2.24 - 2.05 (m, 2H), 1.41 (t, *J* = 6.3 Hz, 3H). ESI-MS *m*/*z* 579.3 [M+H]⁺.

### Example A146: Synthesis of Compound A146

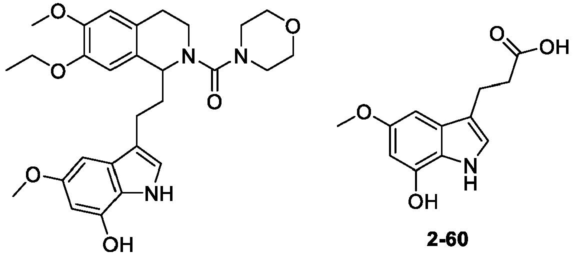

Refering to the synthesis method of compound A1, compound A146 was obtained by replacing 2-5 in Example A1 with compound 2-60. ¹H NMR (500 MHz, DMSO-*d*₆) δ 9.15 (s, 1H), 7.01 (d, *J =* 7.3 Hz, 1H), 6.95 (d, *J =* 2.3 Hz, 1H), 6.74 (s, 1H), 6.61 (t, *J =* 1.0 Hz, 1H), 6.31 (d, *J =* 2.2 Hz, 1H), 4.92 - 4.86 (m, 1H), 4.11 (q, *J =* 6.2 Hz, 2H), 3.92 - 3.85 (m, 1H), 3.83 (d, *J* = 5.0 Hz, 6H), 3.79 - 3.71 (m, 1H), 3.64 - 3.57 (m, 4H), 3.27 - 3.13 (m, 4H), 2.92 (td, *J =* 5.6, 1.0 Hz, 2H), 2.67 (t, *J =* 7.8 Hz, 2H), 2.24 - 2.05 (m, 2H), 1.41 (t, *J* = 6.2 Hz, 3H). ESI-MS *m*/*z* 510.3 [M+H]⁺.

### Example A147: Synthesis of Compound A147

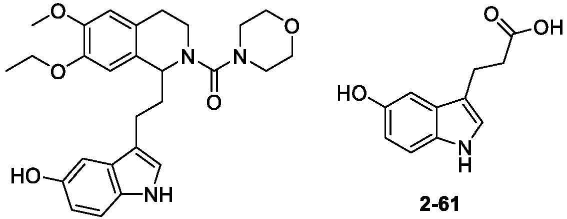

Refering to the synthesis method of compound A1, compound A147 was obtained by replacing 2-5 in Example A1 with compound 2-61. ¹H NMR (500 MHz, DMSO-*d*₆) δ 8.65 (s, 1H), 7.21 (d, *J* = 7.6 Hz, 1H), 7.11 (d, *J =* 7.0 Hz, 1H), 7.07 - 7.03 (m, 1H), 6.76 - 6.71 (m, 2H), 6.61 (t, *J =* 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 4H), 2.95 - 2.89 (m, 2H), 2.69 (td, *J =* 7.8, 2.5 Hz, 2H), 2.14 (m, 2H), 1.41 (t, *J =* 6.3 Hz, 3H). ESI-MS *m*/*z* 480.2 [M+H]⁺.

### Example A148: Synthesis of Compound A148

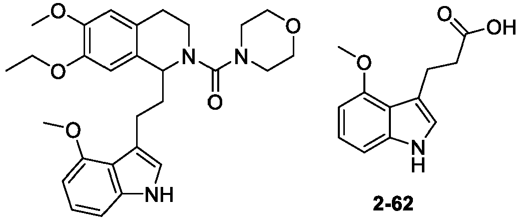

Refering to the synthesis method of compound A1, compound A148 was obtained by replacing 2-5 in Example A1 with compound 2-62. ¹H NMR (500 MHz, DMSO-*d*₆) δ 7.08 (dd, *J =* 4.4, 0.8 Hz, 2H), 7.05 (d, *J =* 6.9 Hz, 1H), 6.80 - 6.72 (m, 2H), 6.61 (t, *J =* 1.0 Hz, 1H), 4.92 - 4.86 (m, 1H), 4.11 (q, *J* = 6.2 Hz, 2H), 3.92 - 3.81 (m, 5H), 3.79 - 3.71 (m, 1H), 3.64 - 3.55 (m, 4H), 3.26 - 3.13 (m, 4H), 2.92 (td, *J* = 5.6, 1.0 Hz, 2H), 2.64 (t, *J =* 7.8 Hz, 2H), 2.18 (m, 1H), 2.05 (m, 1H), 1.41 (t, *J =* 6.2 Hz, 3H). ESI-MS *m*/*z* 494.3 [M+H]⁺.

### Example A149: Synthesis of Compound A149

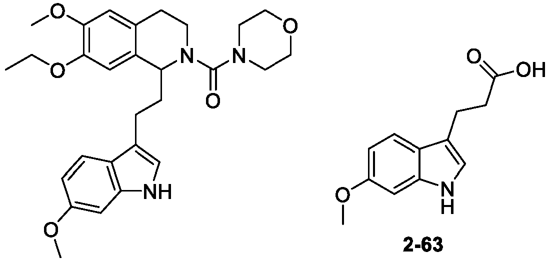

Refering to the synthesis method of compound A1, compound A149 was obtained by replacing 2-5 in Example A1 with compound 2-63. ¹H NMR (500 MHz, DMSO-*d*₆) δ 7.50 (dd, *J =* 8.6, 0.9 Hz, 1H), 7.11 (d, *J =* 7.0 Hz, 1H), 6.90 (d, *J =* 2.2 Hz, 1H), 6.81 (dd, *J =* 8.5, 2.4 Hz, 1H), 6.74 (s, 1H), 6.61 (t, *J =* 1.0 Hz, 1H), 4.92 - 4.86 (m, 1H), 4.11 (q, *J =* 6.2 Hz, 2H), 3.92 - 3.85 (m, 1H), 3.83 (d, *J =* 6.1 Hz, 6H), 3.79 - 3.71 (m, 1H), 3.65 - 3.55 (m, 4H), 3.26 - 3.13 (m, 4H), 2.92 (td, *J =* 5.5, 1.0 Hz, 2H), 2.76 - 2.64 (m, 2H), 2.14 (m, 2H), 1.41 (t, *J =* 6.2 Hz, 3H). ESI-MS *m*/*z* 494.3 [M+H]⁺.

### Example A150: Synthesis of Compound A150

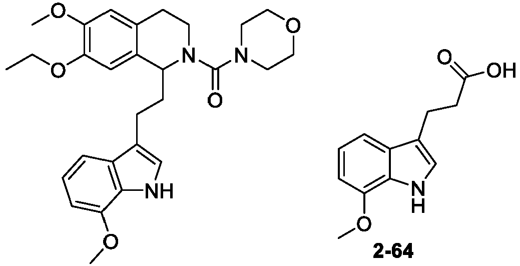

Refering to the synthesis method of compound A1, compound A150 was obtained by replacing 2-5 in Example A1 with compound 2-64. ¹H NMR (500 MHz, DMSO-*d*₆) δ 9.73 (d, *J =* 7.1 Hz, 1H), 7.51 - 7.45 (m, 1H), 7.21 (t, *J =* 7.7 Hz, 1H), 7.04 (d, *J =* 7.1 Hz, 1H), 6.82 (dd, *J =* 7.8, 1.1 Hz, 1H), 6.74 (s, 1H), 6.61 (t, *J =* 1.0 Hz, 1H), 4.92 - 4.86 (m, 1H), 4.11 (q, *J* = 6.2 Hz, 2H), 3.94 (s, 2H), 3.92 - 3.84 (m, 1H), 3.83 (s, 2H), 3.79 - 3.71 (m, 1H), 3.65 - 3.55 (m, 4H), 3.26 - 3.13 (m, 4H), 2.92 (td, *J =* 5.6, 1.0 Hz, 2H), 2.68 (td, *J =* 7.8, 2.2 Hz, 2H), 2.14 (m, 2H), 1.41 (t, *J =* 6.2 Hz, 3H). ESI-MS *m*/*z* 494.3 [M+H]⁺.

### Example A151: Synthesis of Compound A151

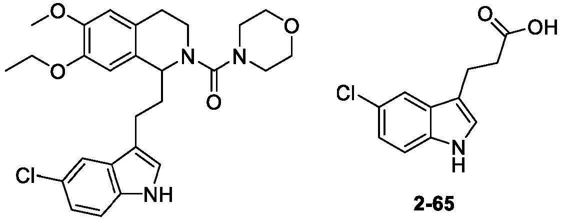

Refering to the synthesis method of compound A1, compound A151 was obtained by replacing 2-5 in Example A1 with compound 2-65. ¹H NMR (500 MHz, DMSO-*d*₆) δ 7.47 - 7.43 (m, 1H), 7.30 (d, *J =* 7.2 Hz, 1H), 7.25 (dd, *J =* 7.3, 1.8 Hz, 1H), 7.11 (d, *J =* 7.0 Hz, 1H), 6.74 (s, 1H), 6.61 (t, *J* = 1.0 Hz, 1H), 4.92 - 4.86 (m, 1H), 4.11 (q, *J* = 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 4H), 2.97 - 2.89 (m, 2H), 2.69 (td, *J =* 7.8, 2.4 Hz, 2H), 2.14 (m, 2H), 1.41 (t, *J* = 6.3 Hz, 3H). ESI-MS *m*/*z* 498.2 [M+H]⁺.

### Example A152: Synthesis of Compound A152

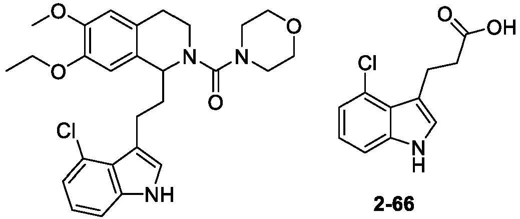

Refering to the synthesis method of compound A1, compound A152 was obtained by replacing 2-5 in Example A1 with compound 2-66. ¹H NMR (500 MHz, DMSO-*d*₆) δ 7.30 (m, 2H), 7.28 - 7.22 (m, 1H), 7.13 (d, *J* = 6.9 Hz, 1H), 6.74 (s, 1H), 6.61 (t, *J* = 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J* = 5.6, 1.0 Hz, 2H), 2.76 (t, *J* = 7.7 Hz, 2H), 2.18 (m, 1H), 2.08 (m, 1H), 1.41 (t, *J =* 6.2 Hz, 3H). ESI-MS *m*/*z* 498.2 [M+H]⁺.

### Example A153: Synthesis of Compound A153

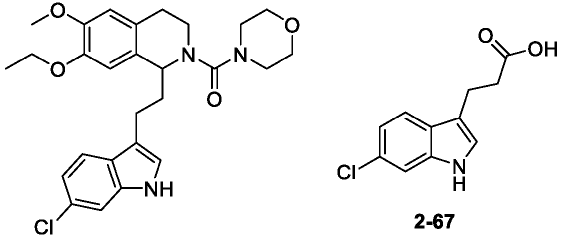

Refering to the synthesis method of compound A1, compound A153 was obtained by replacing 2-5 in Example A1 with compound 2-67. ¹H NMR (500 MHz, DMSO-*d*₆) δ 7.63 (d, *J =* 8.2 Hz, 1H), 7.29 (d, *J* = 1.9 Hz, 1H), 7.26 (dd, *J* = 8.4, 2.3 Hz, 1H), 7.11 (d, *J =* 7.0 Hz, 1H), 6.74 (s, 1H), 6.61 (t, *J* = 1.0 Hz, 1H), 4.92 - 4.86 (m, 1H), 4.11 (q, *J* = 6.2 Hz, 2H), 3.92 - 3.84 (m, 1H), 3.83 (s, 2H), 3.79 - 3.71 (m, 1H), 3.65 - 3.55 (m, 4H), 3.27 - 3.13 (m, 4H), 2.97 - 2.88 (m, 2H), 2.76 - 2.64 (m, 2H), 2.14 (m, 2H), 1.41 (t, *J =* 6.3 Hz, 3H). ESI-MS *m*/*z* 498.2 [M+H]⁺.

### Example A154: Synthesis of Compound A154

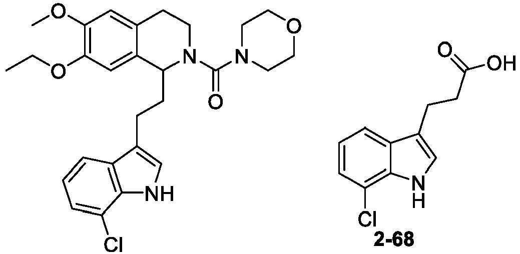

Refering to the synthesis method of compound A1, compound A154 was obtained by replacing 2-5 in Example A1 with compound 2-68. ¹H NMR (500 MHz, DMSO-*d*₆) δ 9.95 (d, *J =* 6.9 Hz, 1H), 7.62 (dd, *J =* 7.7, 1.2 Hz, 1H), 7.33 (dd, *J =* 7.7, 1.2 Hz, 1H), 7.22 (t, *J* = 7.7 Hz, 1H), 7.11 (d, *J* = 7.1 Hz, 1H), 6.74 (s, 1H), 6.61 (t, *J =* 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J =* 5.5, 1.0 Hz, 2H), 2.69 (td, *J =* 7.8, 2.4 Hz, 2H), 2.14 (m, 2H), 1.41 (t, *J =* 6.3 Hz, 3H). ESI-MS *m*/*z* 498.2 [M+H]⁺.

### Example A155: Synthesis of Compound A155

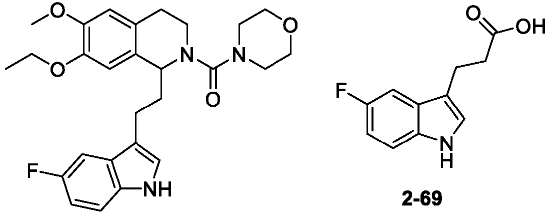

Refering to the synthesis method of compound A1, compound A155 was obtained by replacing 2-5 in Example A1 with compound 2-69. ¹H NMR (500 MHz, DMSO-*d*₆) δ 7.37 (m, 2H), 7.12 (d, *J =* 7.0 Hz, 1H), 7.07 (m, 1H), 6.74 (s, 1H), 6.61 (t, *J =* 1.0 Hz, 1H), 4.92 - 4.86 (m, 1H), 4.11 (q, *J =* 6.2 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J =* 5.6, 1.0 Hz, 2H), 2.69 (td, *J =* 7.8, 2.4 Hz, 2H), 2.14 (m, 2H), 1.41 (t, *J =* 6.3 Hz, 3H). ESI-MS *m*/*z* 482.2 [M+H]⁺.

### Example A156: Synthesis of Compound A156

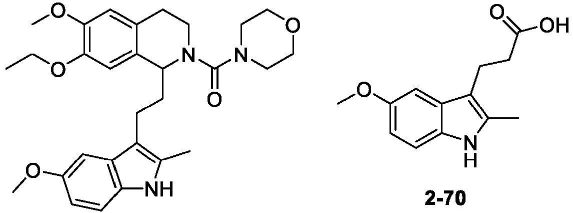

Refering to the synthesis method of compound A1, compound A156 was obtained by replacing 2-5 in Example A1 with compound 2-70. ¹H NMR (500 MHz, DMSO-*d*₆) δ 7.17 (d, *J =* 7.4 Hz, 1H), 7.02 (dd, *J =* 7.2, 1.9 Hz, 1H), 6.78 (d, *J =* 2.2 Hz, 1H), 6.74 (s, 1H), 6.61 (t, *J =* 1.0 Hz, 1H), 4.89 (s, 0H), 4.89 (d, *J =* 13.3 Hz, 0H), 4.11 (q, *J =* 6.2 Hz, 2H), 3.92 - 3.84 (m, 1H), 3.83 (d, *J =* 4.0 Hz, 6H), 3.79 - 3.71 (m, 1H), 3.65 - 3.57 (m, 4H), 3.27 - 3.13 (m, 4H), 2.92 (td, *J =* 5.5, 1.0 Hz, 2H), 2.65 (dt, *J =* 15.3, 7.6 Hz, 1H), 2.57 (dt, *J* = 15.4, 7.7 Hz, 1H), 2.24 (m, 1H), 2.06 (m, 1H), 1.41 (t, *J* = 6.2 Hz, 3H). ESI-MS *m*/*z* 508.3 [M+H]⁺.

### Example A157: Synthesis of Compound A157

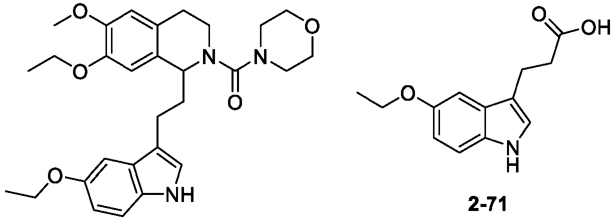

Refering to the synthesis method of compound A1, compound A157 was obtained by replacing 2-5 in Example A1 with compound 2-71. ¹H NMR (500 MHz, DMSO-*d*₆) δ 7.30 (d, *J =* 7.4 Hz, 1H), 7.11 (d, *J =* 7.0 Hz, 1H), 7.05 (d, *J =* 2.0 Hz, 1H), 6.85 (dd, *J =* 7.3, 1.8 Hz, 1H), 6.74 (s, 1H), 6.61 (t, *J* = 1.0 Hz, 1H), 4.92 - 4.86 (m, 1H), 4.11 (q, *J* = 6.2 Hz, 2H), 4.03 (q, *J =* 6.6 Hz, 2H), 3.92 - 3.84 (m, 1H), 3.83 (s, 2H), 3.79 - 3.71 (m, 1H), 3.65 - 3.57 (m, 4H), 3.27 - 3.13 (m, 4H), 2.92 (td, *J =* 5.5, 1.0 Hz, 2H), 2.69 (td, *J =* 7.8, 2.5 Hz, 2H), 2.14 (m, 2H), 1.40 (dt, *J =* 10.3, 6.4 Hz, 6H). ESI-MS *m*/*z* 508.3 [M+H]⁺.

### Example A158: Synthesis of Compound A158

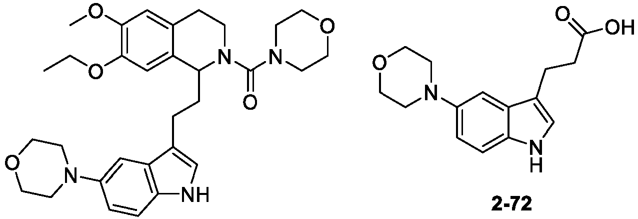

Refering to the synthesis method of compound A1, compound A158 was obtained by replacing 2-5 in Example A1 with compound 2-72. ¹H NMR (500 MHz, DMSO-*d*₆) δ 7.29 (d, *J=* 6.6 Hz, 1H), 7.09 (d, *J =* 7.0 Hz, 1H), 6.98 (d, *J =* 1.7 Hz, 1H), 6.84 (dd, *J =* 6.7, 2.0 Hz, 1H), 6.74 (s, 1H), 6.61 (t, *J* = 1.0 Hz, 1H), 4.92 - 4.86 (m, 1H), 4.11 (q, *J* = 6.2 Hz, 2H), 3.92 - 3.84 (m, 1H), 3.83 (s, 2H), 3.79 - 3.71 (m, 5H), 3.64 - 3.57 (m, 4H), 3.33 (m, 4H), 3.27 - 3.13 (m, 4H), 2.92 (td, *J* = 5.5, 1.0 Hz, 2H), 2.69 (td, *J =* 7.8, 2.4 Hz, 2H), 2.14 (m 2H), 1.41 (t, *J =* 6.3 Hz, 3H). ESI-MS *m*/*z* 549.3 [M+H]⁺.

### Example A159: Synthesis of Compound A159

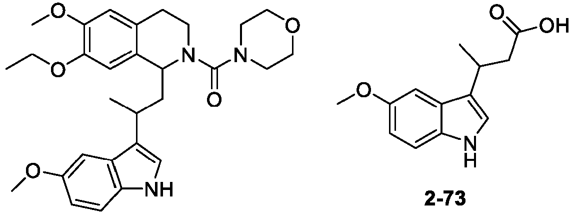

Refering to the synthesis method of compound A1, compound A159 was obtained by replacing 2-5 in Example A1 with compound 2-73. ¹H NMR (500 MHz, DMSO-*d*₆) δ 8.82 (d, *J* = 6.9 Hz, 1H), 7.25 (dd, *J* = 17.7, 7.2 Hz, 2H), 7.18 (d, *J* = 2.1 Hz, 1H), 6.82 (dd, *J* = 7.3, 2.0 Hz, 1H), 6.74 (d, *J =* 0.6 Hz, 1H), 6.61 (t, *J =* 1.0 Hz, 1H), 4.94 (s, 0H), 4.11 (q, *J* = 6.2 Hz, 2H), 3.92 - 3.85 (m, 1H), 3.83 (d, *J =* 4.0 Hz, 6H), 3.79 - 3.71 (m, 1H), 3.64 - 3.55 (m, 4H), 3.27 - 3.13 (m, 4H), 2.92 (td, *J =* 5.6, 1.0 Hz, 2H), 2.87 (p, *J =* 7.1 Hz, 1H), 2.16 (dt, *J* = 13.1, 7.5 Hz, 1H), 1.96 (dt, *J =* 12.9, 7.3 Hz, 1H), 1.41 (t, *J =* 6.2 Hz, 3H), 1.33 (d, *J* = 7.1 Hz, 3H). ESI-MS *m*/*z* 508.3 [M+H]⁺.

### Example A160: Synthesis of Compound A160

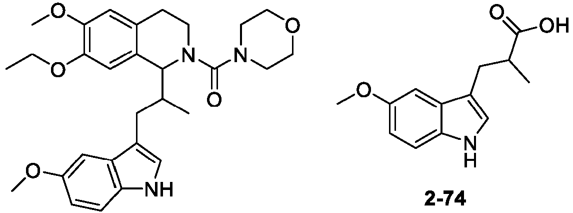

Refering to the synthesis method of compound A1, compound A160 was obtained by replacing 2-5 in Example A1 with compound 2-74. ¹H NMR (500 MHz, DMSO-*d*₆) δ 7.25 (d, *J =* 7.3 Hz, 1H), 7.15 - 7.10 (m, 2H), 6.82 (dd, *J* = 7.3, 2.0 Hz, 1H), 6.69 (d, *J =* 0.7 Hz, 1H), 6.59 (t, *J =* 1.0 Hz, 1H), 4.91 - 4.85 (m, 1H), 4.11 (q, *J =* 6.2 Hz, 2H), 3.89 - 3.81 (m, 7H), 3.72 (m, 1H), 3.65 - 3.57 (m, 4H), 3.27 - 3.13 (m, 4H), 2.99 - 2.84 (m, 2H), 2.73 - 2.54 (m, 3H), 1.41 (t, *J* = 6.3 Hz, 3H), 1.01 (dd, *J* = 7.4, 1.5 Hz, 3H). ESI-MS *m*/*z* 508.3 [M+H]⁺.

### Example A161: Synthesis of Compound A161

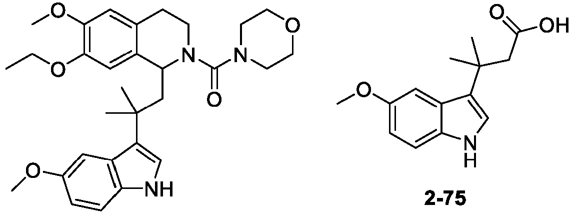

Refering to the synthesis method of compound A1, compound A161 was obtained by replacing 2-5 in Example A1 with compound 2-75. ¹H NMR (500 MHz, DMSO-*d*₆) δ 9.18 (d, *J =* 6.7 Hz, 1H), 7.27 (dd, *J =* 13.6, 6.9 Hz, 2H), 7.09 (d, *J =* 1.7 Hz, 1H), 6.83 (dd, *J =* 7.3, 2.0 Hz, 1H), 6.75 (s, 1H), 6.61 (t, *J =* 1.0 Hz, 1H), 4.96 (t, *J =* 6.8 Hz, 1H), 4.11 (q, *J* = 6.2 Hz, 2H), 3.94 - 3.86 (m, 1H), 3.83 (d, *J =* 4.0 Hz, 6H), 3.80 - 3.72 (m, 1H), 3.64 - 3.57 (m, 4H), 3.27 - 3.13 (m, 4H), 2.92 (td, *J =* 5.5, 1.0 Hz, 2H), 2.28 (dd, *J =* 12.9, 6.7 Hz, 1H), 2.17 (dd, *J =* 12.9, 6.7 Hz, 1H), 1.46 - 1.38 (m, 5H), 1.39 (s, 3H). ESI-MS *m*/*z* 522.3 [M+H]⁺.

### Example A162: Synthesis of Compound A162

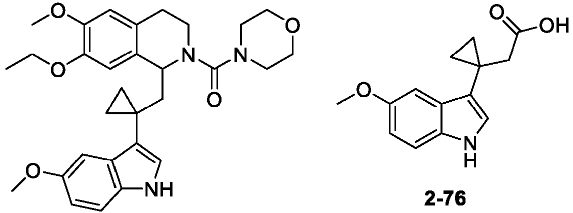

Refering to the synthesis method of compound A1, compound A162 was obtained by replacing 2-5 in Example A1 with compound 2-76. ¹H NMR (500 MHz, DMSO-*d*₆) δ 9.16 (d, *J =* 7.3 Hz, 1H), 7.29 (dd, *J* = 7.4, 1.8 Hz, 2H), 7.03 (d, *J =* 1.6 Hz, 1H), 6.83 (dd, *J =* 7.3, 2.0 Hz, 1H), 6.75 (s, 1H), 6.61 (t, *J =* 1.0 Hz, 1H), 4.97 (t, *J =* 6.1 Hz, 1H), 4.11 (q, *J* = 6.2 Hz, 2H), 3.94 - 3.86 (m, 1H), 3.83 (d, *J =* 4.0 Hz, 6H), 3.80 - 3.72 (m, 1H), 3.64 - 3.57 (m, 4H), 3.27 - 3.13 (m, 4H), 2.92 (td, *J =* 5.6, 1.0 Hz, 2H), 2.30 (dd, *J =* 12.7, 6.1 Hz, 1H), 2.07 (dd, *J =* 12.9, 6.1 Hz, 1H), 1.69 - 1.58 (m, 2H), 1.41 (t, *J =* 6.2 Hz, 3H), 1.24 - 1.17 (m, 1H), 1.19 - 1.12 (m, 1H). ESI-MS *m*/*z* 520.3 [M+H]⁺.

### Example A163: Synthesis of Compound A163

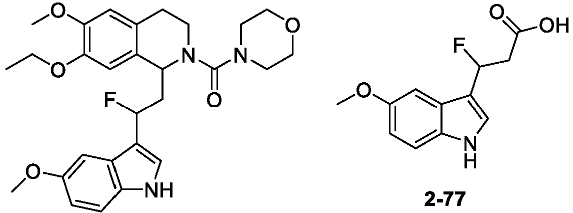

Refering to the synthesis method of compound A1, compound A163 was obtained by replacing 2-5 in Example A1 with compound 2-77. ¹H NMR (500 MHz, DMSO-*d*₆) δ 9.28 (d, *J =* 6.9 Hz, 1H), 7.46 (d, *J =* 6.9 Hz, 1H), 7.29 - 7.24 (m, 2H), 6.83 (dd, *J* = 7.3, 2.0 Hz, 1H), 6.74 (s, 1H), 6.61 (t, *J =* 1.0 Hz, 1H), 5.95 (t, *J =* 6.7 Hz, 0H), 5.86 (t, *J =* 6.6 Hz, 0H), 5.04 (s, 0H), 4.11 (q, *J =* 6.2 Hz, 2H), 3.90 - 3.81 (m, 7H), 3.78 - 3.70 (m, 1H), 3.65 - 3.57 (m, 4H), 3.27 - 3.13 (m, 4H), 2.92 (td, *J =* 5.5, 1.0 Hz, 2H), 2.58 - 2.38 (m, 2H), 1.41 (t, *J* = 6.2 Hz, 3H). ESI-MS *m*/*z* 512.2 [M+H]⁺.

### Example A164: Synthesis of Compound A164

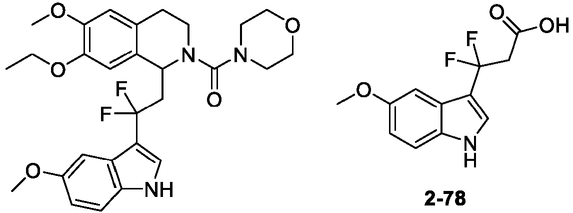

Refering to the synthesis method of compound A1, compound A164 was obtained by replacing 2-5 in Example A1 with compound 2-78. ¹H NMR (500 MHz, DMSO-*d*₆) δ 9.44 (d, *J* = 6.3 Hz, 1H), 7.58 (d, *J =* 6.5 Hz, 1H), 7.31 - 7.26 (m, 2H), 6.83 (dd, *J =* 7.3, 2.0 Hz, 1H), 6.75 (s, 1H), 6.61 (t, *J* = 1.0 Hz, 1H), 5.01 (dd, *J* = 7.4, 6.6 Hz, 1H), 4.11 (q, *J* = 6.2 Hz, 2H), 3.91 - 3.83 (m, 1H), 3.83 (d, *J =* 4.0 Hz, 6H), 3.78 - 3.70 (m, 1H), 3.65 - 3.55 (m, 4H), 3.27 - 3.04 (m, 5H), 2.95 - 2.77 (m, 3H), 1.41 (t, *J =* 6.2 Hz, 3H). ESI-MS *m*/*z* 530.2 [M+H]⁺.

### Example A165: Synthesis of Compound A165

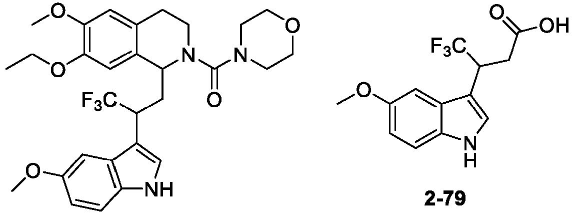

Refering to the synthesis method of compound A1, compound A165 was obtained by replacing 2-5 in Example A1 with compound 2-79. ¹H NMR (500 MHz, DMSO-*d*₆) δ 9.01 (d, *J =* 6.7 Hz, 1H), 7.37 (d, *J* = 6.7 Hz, 1H), 7.27 (d, *J* = 7.3 Hz, 1H), 7.14 (d, *J* = 2.1 Hz, 1H), 6.83 (dd, *J =* 7.3, 2.0 Hz, 1H), 6.74 (d, *J =* 0.7 Hz, 1H), 6.61 (t, *J =* 1.0 Hz, 1H), 5.00 (s, 0H), 4.52 - 4.41 (m, 1H), 4.11 (q, *J =* 6.2 Hz, 2H), 3.92 - 3.85 (m, 1H), 3.83 (d, *J* = 4.0 Hz, 6H), 3.79 - 3.71 (m, 1H), 3.65 - 3.57 (m, 4H), 3.27 - 3.13 (m, 4H), 2.92 (td, *J =* 5.6, 1.0 Hz, 2H), 2.35 (m, 1H), 2.22 - 2.12 (m, 1H), 1.41 (t, *J =* 6.2 Hz, 3H). ESI-MS *m*/*z* 562.2 [M+H]⁺.

### Example A166: Synthesis of Compound A166

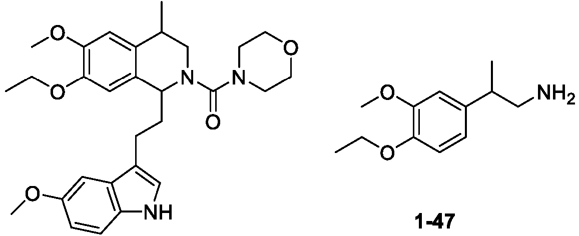

Refering to the synthesis method of compound A1, compound A166 was obtained by replacing 1-5 in Example A1 with compound 1-47. ¹H NMR (500 MHz, DMSO-*d*₆) δ 7.25 (d, *J =* 7.4 Hz, 1H), 7.13 - 7.08 (m, 2H), 6.82 (dd, *J =* 7.3, 2.0 Hz, 1H), 6.75 (s, 1H), 6.71 (s, 1H), 4.94 (t, *J* = 6.4 Hz, 1H), 4.11 (q, *J =* 6.2 Hz, 2H), 3.83 (d, *J* = 4.0 Hz, 6H), 3.76 (d, *J =* 5.1 Hz, 2H), 3.64 - 3.55 (m, 4H), 3.45 - 3.35 (m, 1H), 3.27 - 3.13 (m, 4H), 2.69 (td, *J* = 7.8, 2.4 Hz, 2H), 2.20 (m, 1H), 2.10 (m, 1H), 1.41 (t, *J =* 6.2 Hz, 3H), 1.31 (d, *J* = 6.7 Hz, 3H). ESI-MS *m*/*z* 508.3 [M+H]⁺.

### Example A167: Synthesis of Compound A167

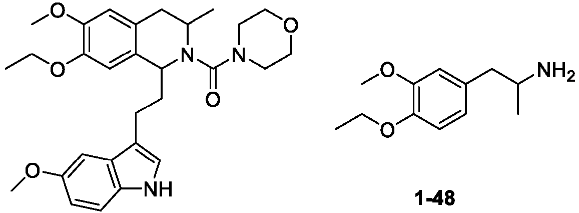

Refering to the synthesis method of compound A1, compound A167 was obtained by replacing 1-5 in Example A1 with compound 1-48. ¹H NMR (500 MHz, DMSO-*d*₆) δ 7.25 (d, *J =* 7.4 Hz, 1H), 7.13 - 7.08 (m, 2H), 6.82 (dd, *J* = 7.3, 2.0 Hz, 1H), 6.74 (d, *J =* 0.8 Hz, 1H), 6.69 (t, *J =* 1.0 Hz, 1H), 5.04 (t, *J =* 6.5 Hz, 1H), 4.28 (h, *J =* 7.0 Hz, 1H), 4.11 (q, *J =* 6.2 Hz, 2H), 3.83 (d, *J =* 4.0 Hz, 6H), 3.64 - 3.57 (m, 4H), 3.27 - 3.20 (m, 1H), 3.23 - 3.15 (m, 3H), 3.12 (m, 1H), 2.79 - 2.65 (m, 3H), 2.22 (m, 1H), 2.06 (m, 1H), 1.41 (t, *J =* 6.2 Hz, 3H), 1.20 (d, *J =* 6.9 Hz, 3H). ESI-MS *m*/*z* 508.3 [M+H]⁺.

### Example A168: Synthesis of Compound A168

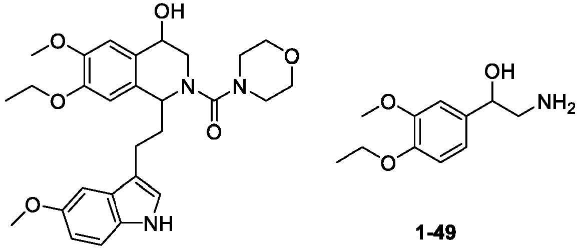

Refering to the synthesis method of compound A1, compound A168 was obtained by replacing 1-5 in Example A1 with compound 1-49. ¹H NMR (500 MHz, DMSO-*d*₆) δ 7.25 (d, *J* = 7.4 Hz, 1H), 7.13 - 7.08 (m, 2H), 6.85 - 6.80 (m, 2H), 6.72 (d, *J =* 0.7 Hz, 1H), 5.19 (dt, *J* = 5.2, 4.6 Hz, 1H), 4.94 (dd, *J* = 7.5, 5.7 Hz, 2H), 4.10 (q, *J* = 6.1 Hz, 2H), 3.85 - 3.78 (m, 7H), 3.77 (dd, *J* = 12.3, 4.8 Hz, 1H), 3.64 - 3.57 (m, 4H), 3.27 - 3.13 (m, 4H), 2.69 (td, *J =* 7.8, 2.5 Hz, 2H), 2.19 (m, 1H), 2.08 (m, 1H), 1.41 (t, *J =* 6.3 Hz, 3H). ESI-MS *m*/*z* 510.3 [M+H]⁺.

### Example A169: Synthesis of Compound A169

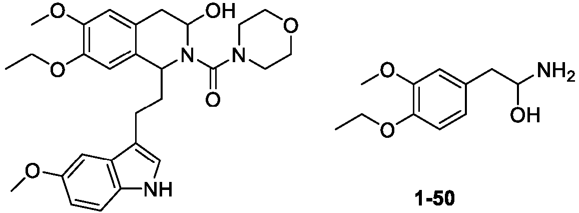

Refering to the synthesis method of compound A1, compound A169 was obtained by replacing 1-5 in Example A1 with compound 1-50. ¹H NMR (500 MHz, DMSO-*d*₆) δ 7.25 (d, *J =* 7.4 Hz, 1H), 7.13 - 7.08 (m, 2H), 6.82 (dd, *J* = 7.3, 2.0 Hz, 1H), 6.74 (s, 1H), 6.66 (t, *J =* 1.0 Hz, 1H), 6.02 (d, *J* = 5.4 Hz, 1H), 5.86 (td, *J =* 6.6, 5.4 Hz, 1H), 5.04 (t, *J =* 6.6 Hz, 1H), 4.11 (q, *J =* 6.2 Hz, 2H), 3.83 (d, *J =* 4.0 Hz, 6H), 3.60 (m, 4H), 3.27 - 3.20 (m, 2H), 3.22 - 3.15 (m, 2H), 3.07 - 2.99 (m, 2H), 2.85 - 2.72 (m, 2H), 2.23 (m, 1H), 2.13 (m, 1H), 1.41 (t, *J =* 6.2 Hz, 3H). ESI-MS *m*/*z* 510.3 [M+H]⁺.

### Example A170: Synthesis of Compound A170

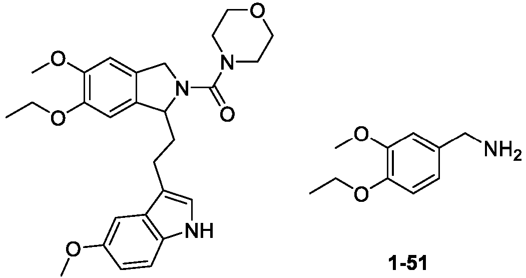

Refering to the synthesis method of compound A1, compound A170 was obtained by replacing 1-5 in Example A1 with compound 1-51. ¹H NMR (500 MHz, DMSO-*d*₆) δ 7.25 (d, *J* = 7.4 Hz, 1H), 7.13 - 7.08 (m, 2H), 6.94 (t, *J =* 1.1 Hz, 1H), 6.82 (dd, *J* = 7.3, 2.0 Hz, 1H), 6.62 (s, 1H), 4.94 (t, *J =* 5.9 Hz, 1H), 4.69 - 4.59 (m, 2H), 4.11 (q, *J =* 6.2 Hz, 2H), 3.83 (d, *J* = 2.8 Hz, 6H), 3.64 - 3.57 (m, 4H), 3.27 - 3.13 (m, 4H), 2.69 (td, *J =* 7.8, 2.5 Hz, 2H), 2.24 - 2.14 (m, 1H), 2.14 - 2.04 (m, 1H), 1.41 (t, *J =* 6.3 Hz, 3H). ESI-MS *m*/*z* 480.2 [M+H]⁺.

### Example A171: Synthesis of Compound A171

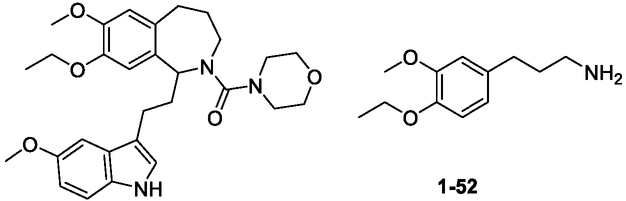

Refering to the synthesis method of compound A1, compound A171 was obtained by replacing 1-5 in Example A1 with compound 1-52. ¹H NMR (500 MHz, DMSO-*d*₆) δ 7.25 (d, *J =* 7.4 Hz, 1H), 7.13 - 7.08 (m, 2H), 6.82 (dd, *J* = 7.3, 2.0 Hz, 1H), 6.74 (s, 1H), 6.61 (t, *J* = 1.1 Hz, 1H), 4.86 - 4.80 (m, 1H), 4.11 (q, *J =* 6.2 Hz, 2H), 3.83 (d, *J =* 4.0 Hz, 6H), 3.65 - 3.58 (m, 2H), 3.62 - 3.55 (m, 3H), 3.27 - 3.13 (m, 6H), 2.82 (m, 2H), 2.69 (td, *J =* 7.8, 2.5 Hz, 2H), 2.19 (dq, *J* = 12.9, 7.6 Hz, 1H), 2.08 (dq, *J* = 12.9, 7.6 Hz, 1H), 1.95 - 1.78 (m, 2H), 1.41 (t, *J =* 6.3 Hz, 3H). ESI-MS *m*/*z* 508.3 [M+H]⁺.

### Biological activity testing experiment

### 1. Detection method: Measurement of intracellular cAMP concentration

### 1.1 Experimental Materials and Instruments

CAMP detection kit (PerkinElmer), cell culture box (Thermo Fisher Scientific), EnVision multifunctional enzyme-linked immunosorbent assay (PerkinElmer), Forskolin (Sigma Aldrich), IBMX (Sigma Aldrich), DSMO (Sigma Aldrich), hINSL5 (Genova, human insulin-like peptide 5), hRXFP4-CHO stably transfected cell line for stable expression of human RXFP4, and test compound.

### 1.2 Test Method

Stably transfected hRXFP4-CHO cells were seeded at 2.5 × 10⁵cells/mL the day before the experiment to maintain a growth density of 70% -80% in the culture dish the next day. On the day of the experiment, cells were digested with trypsin free digestion solution or 0.02% EDTA PBS solution, centrifugated, the supernatant was discarded, and cAMP experimental buffer (0.5 mM IBMX+5 mM HEPES+0.1% BSA) was prepared using 1 × HBSS (calcium and magnesium free) solution. The cells were resuspended and inoculated to a 384 well plate at 4000 cells per well. The compound which was diluted with DMSO to different concentrations were added to the above 384 well plate. At the same time, 500 nM Forskolin was added and incubated at room temperature for 40 minutes. Then Eu cAMP and ULight anti cAMP working solutions were added according to the cAMP detection kit instructions. After incubation at room temperature for 40 minutes, an EnVision multifunctional enzyme-linked immunosorbent assay reader was used to read the results (excitation wavelength of 320 or 340 nm, detection emission wavelength of 665 nm).

### 1.3 Experimental results

The in vitro RXFP4 agonist activity test results of the compound are shown in Table 1.

**Table 1. In vitro RXFP4 agonist activity test results of compounds**

| Compound number | pEC₅₀ ± S.E.M. | Eₘₐₓ ± S.E.M. (%INSL5) |
|---|---|---|
| **A1** | 7.06 ± 0.11 | 87.64 ± 4.58 |
| **(S)-A1** | 7.24 ± 0.12 | 98.76 ± 5.71 |
| **(R)-A1** | 6.03 ± 0.33 | 60.14 ± 10.64 |
| **A3** | 6.69 ± 0.18 | 101.82 ± 9.08 |
| **A4** | 7.24 ± 0.18 | 62.00 ± 5.42 |
| **A5** | 4.99 ± 0.17 | 56.43 ± 4.70 |
| **A6** | 5.03 ± 0.27 | 51.74 ± 6.72 |
| **A9** | 4.83 ± 0.24 | 44.09 ± 5.25 |
| **A30** | 6.37 ± 0.20 | 82.33 ± 8.47 |
| **A57** | 7.56 ± 0.13 | 82.15 ± 4.95 |
| **A58** | 6.35 ± 0.15 | 92.58 ± 7.46 |
| **A60** | 6.38 ± 0.21 | 72.49 ± 8.04 |
| **A69** | 5.00 ± 0.28 | 58.19 ± 7.80 |
| **A76** | 5.50 ± 0.37 | 43.69 ± 7.43 |
| **A77** | 6.36 ± 0.44 | 25.96 ± 5.50 |
| **A80** | N.D.^{a} | N.D. |
| **A83** | N.D. | N.D. |

| | | |
|---|---|---|
| ^{a}N.D. = Not determined | | |

### 2. Detection method: Ligand receptor affinity detection

### 2.1 Experimental Materials and Instruments

Stably transfected HRXFP4 CHO cells and Eu-R₃/I5 (lanthanide element europium labeled chimeric peptide R₃/I5), BMG POLARstar reader (BMG Labtech), and test compound.

### 2.2 Test Methods

HRXFP4 CHO was introduced into a 96 well plate at 80000 cells per well. After 24 hours of cell culture in the incubator, discarded the supernatant, and washed once with PBS, then a binding buffer containing 1% BSA was added and incubated at room temperature for 1 hour. The cell plate was then washed once with PBS, followed by the addition of different concentrations of positive controls and test compounds, and incubated at room temperature with 5 nM Eu-R₃/I5 for 1 hour. After washing the cell plate once with PBS, an enhancement solution was added. After 45 minutes, the fluorescence value was measured using a BMG POLARstar reader (excitation wavelength of 340 nm, detection emission wavelength of 614 nm).

### 2.3 Experimental results

The in vitro ligand receptor affinity test results of the compound are shown in Table 2

**Table 2: In vitro ligand receptor affinity test results of compounds**

| **Compound number** | **pKᵢ ±S.E.M.** | **Span ± S.E.M. (% R₃/I5)** |
|---|---|---|
| **A1** | **6.83 ± 0.10** | 88.98 ± 6.07 |
| **(S)-A1** | 7.23 ± 0.15 | 81.87 ± 4.47 |
| **(R)-A1** | <4 | N.D.a |
| **A3** | 6.68 ± 0.28 | 86.26 ± 6.71 |
| **A4** | 6.20 ± 0.16 | 66.34 ± 7.29 |
| **A5** | <4 | N.D. |
| **A6** | <4 | N.D. |
| **A9** | <4 | N.D. |
| **A30** | 6.07 ± 0.16 | 81.61 ± 7.65 |
| **A57** | 6.89 ± 0.20 | 92.06 ± 3.95 |
| **A58** | <4 | N.D. |
| **A60** | 5.77 ± 0.30 | 75.02 ± 7.56 |
| **A69** | <4 | N.D. |
| **A76** | <4 | N.D. |
| **A77** | <4 | N.D. |
| **A80** | <4 | N.D. |
| **A83** | <4 | N.D. |

| | | |
|---|---|---|
| ^{a} N.D. = Not determined | | |

### Pharmacodynamic evaluation of compounds in vivo

**The promoting effect of compounds on intestinal peristalsis in constipation model mice**

### 1. Experimental reagents, instruments, and animals

### 1.1 Experimental Materials

Mosapride Citrate Tablets (Chengdu Kanghong Pharmaceutical); HE staining kit (KeyGEN Biotech); Loperamide Hydrochloride Capsules (Xi'an Janssen Pharmaceutical Co. LTD); Nitric oxide test kit/microplate method (mlbio); Anti-TRPV1 antibody, Anti-CGRP Antibody(Thermo Fisher Scientifi); Anti-AQP3 antibody, Goat Anti-Rabbit IgG H&L(Alexa Fluor ^{®} 488), Goal Anti use IgG H&L (Alexa Fluor) ^{®} 488) (Abcam), Mouse VIP ELISA Kit (LSBio); Mouse 5-HT ELISA Kit(mlbio).

### 1.2 Experimental Instruments

FORMA 700 ultra-low temperature refrigerator (Thermo Fisher Scientific); YC-300L type drug storage cabinet (Zhongke Meiling Low Temperature Technology Co., Ltd.); Direct-Q with pump ultrapure water analyzer (Millipore); SW-CJ-2FD Ultra Purification Workbench (Suzhou Purification Equipment Co., Ltd.); 3K1 low-temperature high-speed centrifuge (Sigma Aldrich); BS224 electronic balance (Beijing Sedolis Instrument System Co., Ltd.); ZS-MV-IV Small Animal Anesthesia Machine (Beijing Zhongshidi Chuang Technology Development Co., Ltd.); Y-3102 Mouse Metabolic Cage (Shanghai Yuyan Scientific Instrument Co., Ltd.); Paraffin embedding machine, slicer (LEICA); Olympus inverted phase contrast microscope, (Olympus); Zeiss LSM laser confocal microscope (Zeiss, Germany); Berthold LB941 microplate multifunctional enzyme-linked immunosorbent assay (ELISA) reader.

### 1.3 Experimental animals

SPF grade ICR mice, male, 6-8 weeks old, weighing 20 ± 2 g, provided by Changzhou Kavins Experimental Animal Co., Ltd., animal certificate number: SCXK (Su) 2022-0010, raised in SPF grade environment, indoor temperature controlled at 22±2 °C, free feeding and drinking.

### 2. Animal models and dosing regimens

### 2.1 Mouse Model Construction

ICR mice are housed in SPF grade animal rooms, with environmental conditions controlled at 20 °C-26 °C, relative humidity of 40%-70%, 12 hours of alternating light and dark, and free diet; After one week of adaptive feeding, the model group mice were orally administered with 3 mg/kg loperamide at a volume of 0.1 mL/10 g once daily for 5 consecutive days; Normal control group mice were orally administered an equal volume of physiological saline solution daily.

### 2.2 Experimental Grouping

Control group: Mice were orally administered an equal volume of physiological saline once a day for 7 consecutive days (n=8);

Model group: After the establishment of the constipation model in mice, an equal volume of physiological saline was administered orally once a day for 7 consecutive days (n=8);

Positive control group: After establishing a constipation model in mice, 10 mg/kg Mosapride Citrate was orally administered at a volume of 0.1 mL/10 g once daily for 7 consecutive days (n=8);

Experimental group: After establishing a constipation model in mice, the experimental group mice were divided into three groups and orally administered with compound (S) - A1, 1 mg/kg, 5 mg/kg, and 25 mg/kg, respectively. The gavage volume was 0.1 ml/10 g once a day for 7 consecutive days (n=8 per group);

### 3. Testing indicators and methods

After the completion of modeling and the last administration, each group of mice was placed in a metabolic cage and allowed to move freely for 2 hours. Mouse feces were collected, and the weight, quantity, hardness, and water content thereof were calculated. The effect of the compound on mouse colon function was evaluated through bead expulsion experiments; after the bead expulsion experiment, the mice were euthanized by CO₂ method, and colon tissue (approximately 2 cm long colon tissue near the anus) was taken. The morphological changes of the mouse colon was observed by HE staining , and the thickness of the colon muscle layer, mucosal thickness, goblet cells, and number of Libeikun's glands were counted; blood was collected and the levels of nitric oxide (NO), serotonin (5-HT), and vasoactive intestinal peptide (VIP) in mouse serum were tested by reagent kits; changes in the expression levels of aquaporin 3 (AQP3), transient receptor potential channel V1 (TRPV1), and calcitonin gene-related peptide (CGRP) in mouse colon tissue were detected by immunohistochemical experiments.

### 3.1 Fecal moisture content detection

Each group of mice were placed in a metabolic cage and allowed to move freely for 2 hours. Mouse feces were collected and weighed in a dry EP tube,the wet weight of the feces were recorded. Then the feces were placed in a drying oven and baked at a temperature of 50 °C for 4 hours, then weighed outside and baked in the oven again, weighed once every 2 hours until there is no change in the weight of the feces, and the dry weight of the feces were obtained. Equation for calculating fecal moisture content: fecal moisture content (%)=[(wet weight of feces - dry weight of feces)/wet weight of feces] × 100%.

### 3.2 Bead Discharge Experiment

After using a small animal anesthesia machine to anesthetize mice, glass microspheres with a diameter of 1.5 mm were inserted into the distal colon about 3 cm away from the anal opening. When the glass microspheres were inserted into the distal colon of the mice, they were placed in a separate cage and the time between waking up and the expulsion of the glass microspheres was recorded as the time for the beaddischarged.

### 3.3 HE staining

1) Baking: the prepared tissue paraffin sections were placed in an electric constant temperature drying oven and baked at 60 °C for 3 hours;
2) The dried paraffin sections were dewaxed with conventional xylene, hydrated with downward gradient ethanol and washed with distilled water;
3) The nucleus were stained with hematoxylin for 2 minutes, differentiated with hydrochloric acid and alcohol for a few seconds, and washed with water to return to blue;
4) The sections were stained with Eosin Stain for 1 minute, and rinsed off the remaining Stain solution with water;
5) The sectionswere dehydrated and dried using gradient alcohol, vitrification with xylene, and sealed with neutral gum;
6) a randomly selected field of view was taken photos using a phase contrast microscope under a 400x magnification field of view.

### 3.4 Immune tissue fluorescence

1) paraffin sections of mouse colon tissue was washed with PBS for 5 minutes x 3 times;
2) The liquid around the tissue sections was wiped off, a circle 2 mm away from the tissue was drew with an immunohistochemical pento prevent dilution of various liquid components added later;
3) Then incubated and soaked at room temperature for 10 minutes with 0.4% Triton X-100, and washed with PBS for 5 minutes x 3 times;
4) Epitope retrieval (1.5 minutes after the pressure limiting valve of the pressure cooker emits gas, the pressure was reduced with cold water, natural cooled and washed with PBS for 5 minutes × 3 times;
5) Blocked with 5% sheep serum at 37 °C for 10 minutes;
6) Discarded the serum, added primary antibody dropwise (specific primary antibody and dilution ratio see Table 1), placed in a wet box, and reacted overnight at 4 °C;
7) Sucked off the antibody reaction solution and washed with PBS for 5 minutes three times;
8) Added fluorescently labeled secondary antibody dropwise (dilution ratio see Table 1) and incubated in a 37 °C wet box in the dark for 1-2 hours;
9) Sucked off the antibody reaction solution and washed with PBS for 5 minutes three times;
10) sealed with 90% glycerol and placed in a dark box;
11) Observated and photographed using a confocal laser microscope under a 630 x field of view.

**Table 3: Antibodies and their dilution ratios**

| **Antibodies** | **Dilution (Application)** |
|---|---|
| AQP3 | 2 µg/ml (IF) |
| TRPV1 | 1:100 (IF) |
| CGRP | 1:100 (IF) |
| Goat Anti-Rabbit IgG H&L (Alexa Fluor ^{®} 488) | 1:1000 (IF) |
| Goat Anti-Mouse IgG H&L (Alexa Fluor ^{®} 488) | 1:1000 (IF) |

### 3.4 NO content detection

Serum: to a 100 µ L of serum original solution was added 200 µ L of reagent I and mixed well, and 100 µ L of reagent II was added, vortexed thoroughly, stood for 10 minutes, 3500-4000 rpm/min, centrifuged for 15 minutes, and 160 µ L of supernatant was taken.

### Operation table

| Reagent name | Blank well | Standard well | Measurement well |
|---|---|---|---|
| Double steamed water | 0.16 | - | - |
| 20 µ M Sodium Nitrite Standard Solution (mL) | - | 0.16 | - |
| Supernatant (mL) | - | - | 0.16 |
| Color developing reagent (mL) | 0.08 | 0.08 | 0.08 |
| Mixed well, stood for 15 minutes, wavelength 550 nm, and the absorbance OD values of each well was measured using an enzyme-linked immunosorbent assay (ELISA) reader. | | | |

### Preparation of standard curve

2 mmol/L sodium nitrite standard solution was diluted with double distilled water at 1:39, 1:79, 1:99, 1:159, 1:319, and 1:39. The concentrations of sodium nitrite standard solution were 0.05 mM, 0.025 mM, 0.02 mM, 0.0125 mM, 0.00625 mM, and 0.003125 mM, respectively.

### Operation table

| Reagent name | Blank well | Standard well |
|---|---|---|
| Double steamed water | 0.16 | - |
| Different concentrations of sodium nitrite standard solution (mL) | - | 0.16 |
| Color developing reagent (mL) | 0.08 | 0.08 |
| Mixed well, stood for 15 minutes, wavelength 550 nm, and the absorbance OD values of each well was measured using an enzyme-linked immunosorbent assay (ELISA) reader. | | |

### 3.5 ELISA detection of serum VIP content

1) All reagents and samples were equilibrated at room temperature, thoroughly mixed (to avoid foaming), and all reagents, diluted standard solutions, controls, and samples were prepared;
2) 50 µL of standard, blank, or sample were added to each well, with the concentrations of the standard was 6.173, 18.52, 55.57, 166.7, and 500 pg/mL, respectively;
3) 50 µ L of detection reagent A working solution was added to each well immediately, coverd with a sealing plate, stirred gently to ensure thoroughly mixed, and incubated at 37 °C for 1 hour;
4) the liquid was aspirated from each well and wash three times by adding approximately 350 µ L of washing buffer via a spray bottle, multi-channel pipette, or automatic cleaner. After each cleaning, let it stand for 1-2 minutes. After the last washing, the remaining washing buffer was aspirated, then the washing plate was inverted and pated to dry the excess liquid with absorbent paper;
5) 100 µ L of detection reagent B working solution was added to each well, gently stirred to ensure thoroughly mixed, covered with new plate sealer, and incubated at 37 °C for 30 minutes;
6) the liquid was aspirated from each well and wash 5 times as described in step 4;
7) 90 µ L of TMB substrate solution was added to each well, gently stirred to ensure thoroughly mixed, coverd with new plate sealer, and incubated at 37 °C for 10-20 minutes. Avoiding light exposure and monitored regularly until the optimal color was achieved;
8) 50 µ L of termination solution was added to each well in the same order and time as the TMB substrate solution, mixed well, and the solution turns from blue to yellow;
9) the optical density (OD value) of each well were measured in sequence using an enzyme-linked immunosorbent assay (ELISA) reader at a wavelength of 450 nm.

### 3.7 ELISA detection of serum 5-HT content

1) Before use, all reagents were mixed thoroughly and equilibrated at room temperature;
2) Addition of standard samples: standard wells and sample wells were set up, 50 µ L of standard samples of different concentrations was added to each standard well, with standard sample concentrations of 240, 120, 60, 30, and 15 ng/mL, respectively;
3) Sample addition: blank wells (blank control wells without sample and enzyme-linked immunosorbent assay, the rest steps are the same) and test sample wells were set up, respectively. 40 µ L of sample diluent was added to the test sample wellon the enzyme-linked immunosorbent assay (ELISA) coated plate, and then 10 µ L of the test sample (with a final dilution of 5 times) was added. the sample was added to the bottom of the enzyme-linked immunosorbent assay plate well to avoid touching the well wall as much as possible, and gently shook to mix well;
4) Incubation: the plate was covered with a sealing film and incubated at 37 °C for 30 minutes;
5) Preparation: a 30 times concentrated washing solution was diluted with distilled water for later use;
6) Washing: Carefully removed the sealing film, discarded the liquid, shakedried, filled each well with washing solution, stood for 30 seconds, discarded, repeated 5 times, pated-dried;
7) Enzyme addition: 50 µ L of enzyme labeled reagent was added to each well, except for blank wells;
8) Incubation: the operation was the same as 4;
9) Washing: the operation was the same as 6;
10) Color development: 50 µ L of color developer A was add to each well, then 50 µ L of color developer B was added, shook gently to mix well, and developed color at 37 °C in the dark for 15 minutes;
11) Termination: 50 µ L of termination solution was add to each well, and the solution turn from blue to yellow;
12) Measurement: calibrated to zero the blank well and measured the absorbance (OD value) of each well in sequence at a wavelength of 450 nm.

### 4. Experimental results

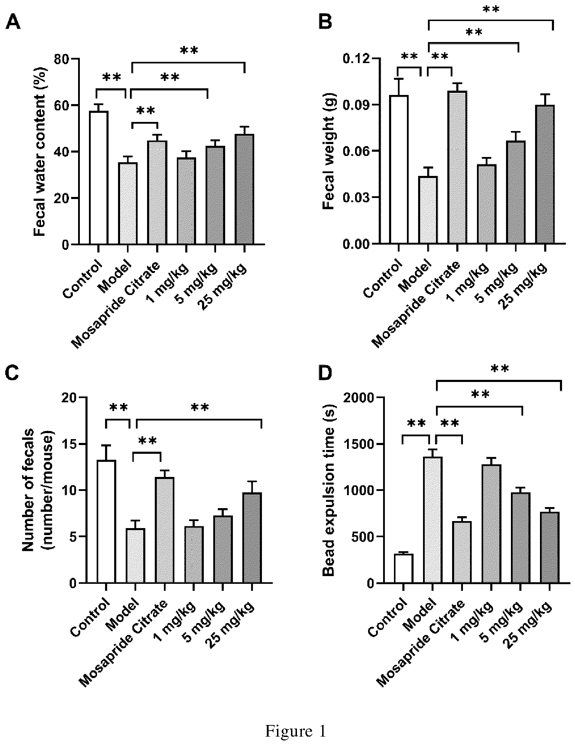

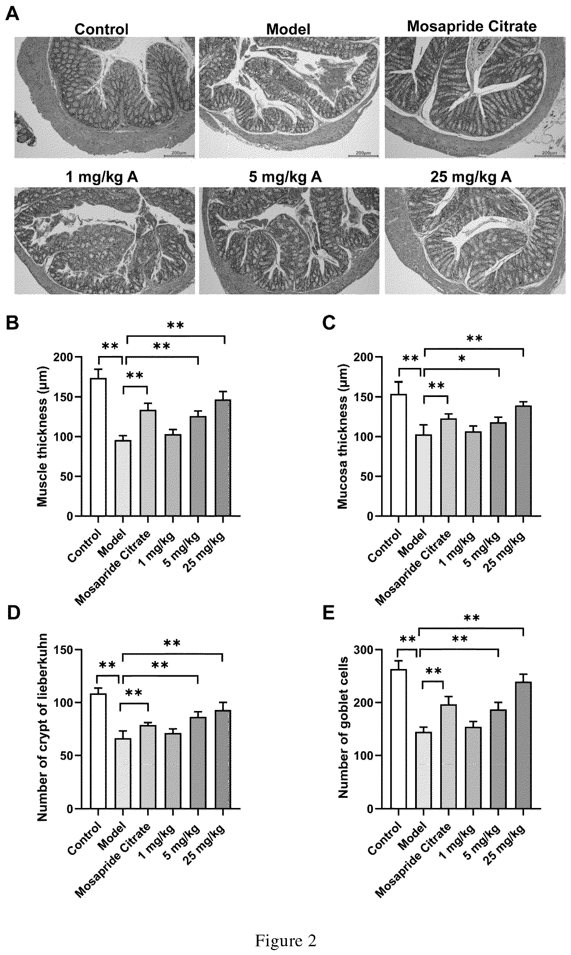

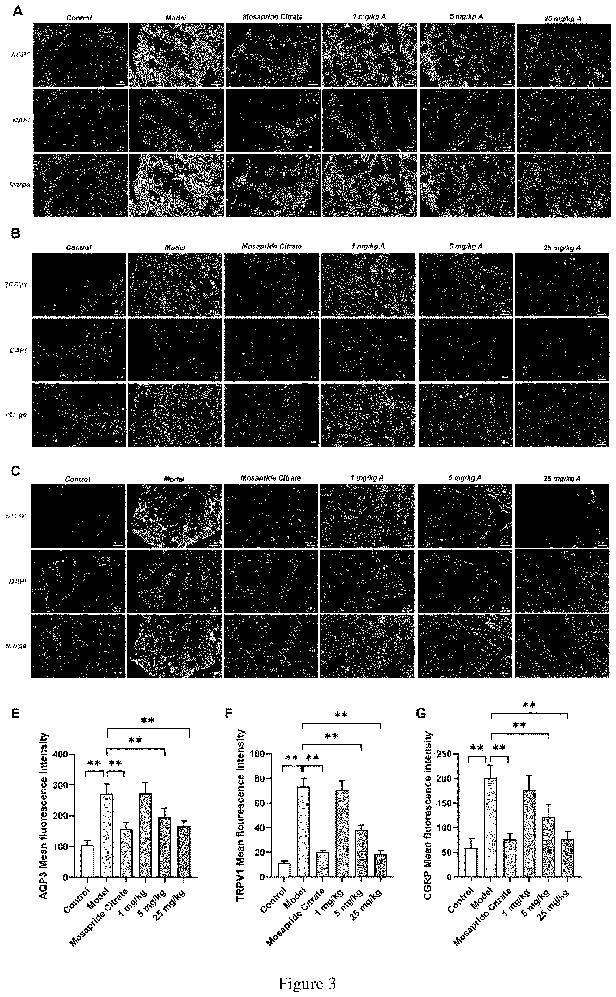

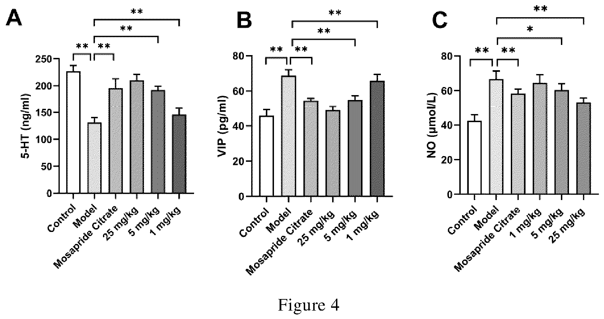

Compared with the Control group, mice in the Model group had decreased fecal volume, decreased fecal water content, decreased fecal pellet number, increased hardness, increased bead expulsion time, disorganized arrangement of intestinal villi in mouse colon tissues, massive inflammatory infiltration, thinning of the muscular layer of the colon and the mucous membrane, decreased number of cup cells and Liebequist's glands, and significant increased expression levels of AQP3 protein, TRPV1 protein and CGRP in the colon tissues, significantly reduced serum 5-HT level, and significantly increased VIP level and NO level. Compared with the Model group, mice treated with the compounds orally showed increased fecal volume, increased fecal water content, increased number of fecal pellets, decreased hardness, decreased time of bead expulsion, and enhanced intestinal peristalsis (Figure 1); the thinning of the muscularis propria of the colon and mucous membranes was significantly improved, and the numbers of cup cells and Liebequen's glands were all increased to different degrees (Figure 2); the expression levels of AQP3 protein, TRPV1 protein and CGRP in colon tissues were significantly reduced (Figure 3); the serum 5-HT level was increased, and VIP and NO levels were decreased (Figure 4).

All references mentioned in the present invention are cited as references in this application, as if each reference were cited separately. In addition, it should be understood that after reading the above teaching content of the present invention, those skilled in the art can make various changes or modifications to the present invention, and these equivalent forms also fall within the scope of the claims attached to this application.

## Claims

1. A nitrogen-containing heterocyclic compound of formula (I), or a pharmaceutically acceptable salt, a enantiomer, a diastereomer, or a racemate thereof: 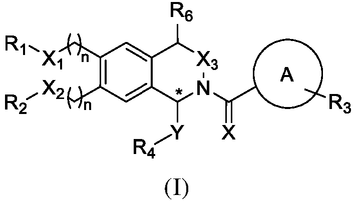 wherein,
the chiral carbon atom C* is independently a S-configuration, a R-configuration, a racemate, or a combination thereof;
n=0, 1, or 2;
X₁ and X₂ are independently selected from the group consisting of: O and NH;
X₃ is a chemical bond, CHR₆ or (CHR₆)₂;
R₁ and R₂ are independently selected from the group consisting of: hydrogen, deuterium, tritium, halogen, hydroxyl, carboxyl, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted C6-C10 aryl, substituted or unsubstituted 5-7 membered heterocyclyl, substituted or unsubstituted C1-C6 alkylphenyl, substituted or unsubstituted C1-C6 alkyl 5-7 membered heteroaryl, substituted or unsubstituted C3-C12 cycloalkyl, substituted or unsubstituted C2-C10 acyl, substituted or unsubstituted C2-C10 ester group, amino, substituted or unsubstituted C1-C6 alkylamine, substituted or unsubstituted C1-C6 amido, -SOR₅, -SOR₅, -OCOR₅, C(=NH)NH₂, Fmoc and Alloc;
and when n=0, R₁ and R₂ are not methyl at the same time; when n=1, R₁ and R₂ are not hydrogen at the same time;
or R₁ and R₂ together with the adjacent (CH₂) ₙO and C=C form a substituted or unsubstituted 5-7 membered heterocycle, wherein the heterocycle is a fully saturated heterocycle, a partially unsaturated heterocycle, or an aromatic heterocycle;
X is selected from the group consisting of: O, S, CHR₆, C₂H₄ (i.e., forming a cyclopropyl group at the X substitution position) and NR₆; wherein R₆ is selected from the group consisting of: H, CN, C1-C6 alkyl and C1-C6 alkoxy;
Y is a linking group selected from the group consisting of: chemical bonds, C1-C6 straight or branched alkyl, - CH₂NH -, C2-C6 straight or branched alkenyl, -CH₂O-, -CH₂S-, -CONH-, -NHCO-, -COO-, 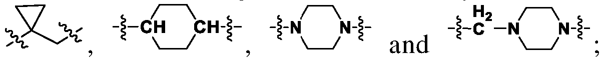
ring A is a substituted or unsubstituted group selected from the group consisting of: a 5-12 membered nitrogen-containing heterocyclyl (including monocyclic, fused polycyclic, bridged or spirocyclic groups, wherein the connecting site preferably located on the nitrogen atom), a C6-C12 aryl (preferably a C6-C10 aryl group), and a 5-12 membered heteroaryl (preferably a 5-7 membered heteroaryl); wherein the heterocyclyl or heteroaryl includes heteroatoms selected from the group consisting of N, NH, S, O and S(O)₂ as the ring skeleton;
R₄ is selected from the following groups that are unsubstituted or substituted with 1-3 substituents: C3-C7 alkyl, 5-12 membered heterocyclyl, C6-C12 aryl, 5-12 membered heteroaryl (preferably 5-7 membered heteroaryl, or benzo 5-7 membered heteroaryl); wherein each of the heterocyclyl or heteroaryl contains 1-3 heteroatoms selected from oxygen, sulfur, and nitrogen; the substituents are independently selected from halogen, C1-C6 straight or branched alkyl, C2-C6 straight or branched alkenyl, C2-C6 straight or branched alkynyl, C1-C6 straight or branched alkoxy,C1-C6 straight or branched alkylcarbonyloxy, cyano, nitro, hydroxyl, amino, hydroxymethyl, trifluoromethyl, trifluoromethoxy, carboxyl, thiol, C1-C4 acyl, amido, sulfonyl, aminosulfonyl, C1-C4 alkyl substituted sulfonyl, or two substituents located on adjacent ring atoms together with the attached carbon atom form a 5-7 membered ring;
R₃ and R₅ are each independently selected from the group consisting of: hydrogen, deuterium, tritium, halogen, unsubstituted or 1-3 halogens substituted C1-C6 alkyl, unsubstituted or 1-3 halogens substituted C3-C6 cycloalkyl, unsubstituted or 1-3 halogens substituted C6-C10 aryl, unsubstituted or 1-3 halogens substituted C1-C3 alkyl-C6-C10 aryl, unsubstituted or 1-3 halogens substituted 5-7 membered heteroaryl;
unless otherwise specified, the term "substituted" refers to one or more hydrogen atoms on the group are substituted by substituent selected from the group consisting of: C1-C10 alkyl, C1-C10 alkoxy, C3-C10 cycloalkyl, C1-C10 alkoxy, 5-12 membered heterocyclyl, halogen, hydroxyl, carboxyl (-COOH), C1-C10 aldehyde, C2-C10 acyl, C2-C10 ester group, cyano, amino, and phenyl; wherein the phenyl includes an unsubstituted phenyl or a substituted phenyl with 1-3 substituents, wherein the substituents are selected from the group consisting of halogen, C1-C10 alkyl, cyano, hydroxyl, nitro, C3-C10 alkyl, C1-C10 alkoxy, and amino;
and the compound is not a structure selected from the group consisting of: 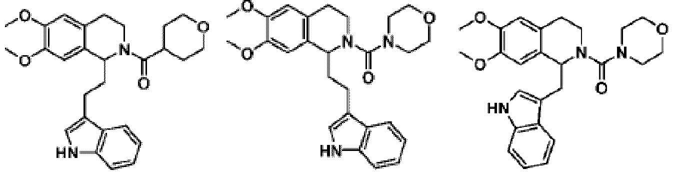 and

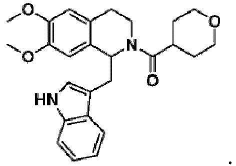

2. A nitrogen-containing heterocyclic compound represented by formula (I), or a pharmaceutically acceptable salt, a enantiomer, a diastereomer, or a racemate thereof: 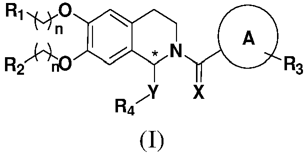 wherein,
the chiral carbon atom C* is independently a S-configuration, a R-configuration, racemate, or a combination thereof;
n=0, 1, or 2;
R₁ and R₂ are independently selected from the group consisting of: hydrogen, deuterium, tritium, halogen, hydroxyl, carboxyl, substituted or unsubstituted C1-C6 alkyl, substituted or unsubstituted C1-C6 alkoxy, substituted or unsubstituted C6-C10 aryl, substituted or unsubstituted 5-7 membered heterocyclyl, substituted or unsubstituted C1-C6 alkylphenyl, substituted or unsubstituted C1-C6 alkyl 5-7 membered heteroaryl, substituted or unsubstituted C3-C12 cycloalkyl, substituted or unsubstituted C2-C10 acyl, substituted or unsubstituted C2-C10 ester group, amino, substituted or unsubstituted C1-C6 alkylamine, substituted or unsubstituted C1-C6 amido, - OR₅, - OSOR₅ and - OCOR₅;
and when n=0, R₁ and R₂ are not methyl at the same time; when n=1, R₁ and R₂ are not hydrogen at the same time;
or R₁ and R₂ together with the adjacent (CH₂) ₙO and C=C form a substituted or unsubstituted 5-7 membered heterocycle, wherein the heterocycle is a fully saturated heterocycle, a partially unsaturated heterocycle, or an aromatic heterocycle;
X is O or S;
Y is a linking group selected from the group consisting of chemical bond, C1-C6 straight or branched alkyl, - CH₂NH -, C2-C6 straight or branched alkenyl, -CH₂O-, -CH₂S-, -CONH-, -NHCO-, -COO-, 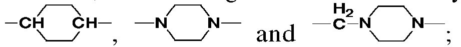
ring A is a substituted or unsubstituted group selected from the group consisting of: 5-12 membered nitrogen-containing heterocycle (with the connecting site preferably located on the nitrogen atom), C6-C12 aryl (preferably C6-C10 aryl), 5-12 membered heteroaryl (preferably 5-7 membered heteroaryl); wherein the heterocyclyl or heteroaryl includes heteroatoms selected from the group consisting of N, NH, S, O, and S(O)₂ as the ring skeleton;
R₄ is selected from the following groups that are unsubstituted or substituted with 1-3 substituents: C3-C7 cycloalkyl, 5-12 heterocyclyl, C6-C12 aryl, 5-12 heteroaryl (preferably 5-7 heteroaryl, or benzo 5-7 heteroaryl); wherein each of the heterocyclyl or heteroaryl contains 1-3 heteroatoms selected from oxygen, sulfur and nitrogen; the substituents are independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, trifluoromethoxy, carboxyl, thiol, C1-C4 acyl, amido, sulfonyl, aminosulfonyl, C1-C4 alkyl substituted sulfonyl, or two substituents located on adjacent ring atoms together with the attached carbon atoms form a 5-7 membered ring;
R₃ and R₅ are independently selected from the group consisting of: hydrogen, deuterium, tritium, halogen, unsubstituted or 1-3 halogens substituted C1-C6 alkyl, or unsubstituted or 1-3 halogens substituted C3-C6 cycloalkyl, unsubstituted or 1-3 halogens substituted C6-C10 aryl, unsubstituted or 1-3 halogens substituted C1-C3 alkyl-C6-C10 aryl, unsubstituted or 1-3 halogens substituted 5-7 membered heteroaryl.

3. The nitrogen-containing heterocyclic compound according to claim 1, or a pharmaceutically acceptable salt, a enantiomer, a diastereomer, or a racemate thereof, wherein ring A is selected from the group consisting of: aziridinyl, azetidinyl, pyrrolidinyl, piperidinyl, azepanyl, morpholinyl, piperazinyl, homopiperazinyl, thiomorpholinyl, thiomorpholinyl in which the cyclic sulfur is substituted by sulfoxide or sulfone, imidazolidinyl, pyrazinyl, hexahydropyrimidinyland 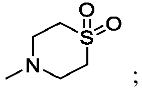 and ring A can be optionally substituted by 1-2 groups selected from hydrogen, C1-C3 straight or branched alkyl, halogen, hydroxyl, and C1-C4 alkoxycarbonyl.

4. The nitrogen-containing heterocyclic compound according to claim 1, or a pharmaceutically acceptable salt, a enantiomer, a diastereomer, or a racemate thereof, wherein R₁ and R₂ are each independently selected from the group consisting of hydrogen, deuterium, tritium, halogen, hydroxyl, carboxyl, phenyl, substituted or unsubstituted C1-C6 alkyl, substituted or unsubstituted C1-C6 alkoxy, substituted or unsubstituted 5-7 membered heterocyclyl, substituted or unsubstituted C1-C6 alkyl 5-7 membered heteroaryl, substituted or unsubstituted C3-C8 cycloalkyl, substituted or unsubstituted C2-C10 acyl, substituted or unsubstituted C2-C10 ester group, amino, substituted or unsubstituted C1-C6 alkylamine, substituted or unsubstituted C1-C6 amido, - SOR₅, - SOR₅ and - OCOR₅.

5. The nitrogen-containing heterocyclic compound according to claim 1, or a pharmaceutically acceptable salt, a enantiomer, a diastereomer, or a racemate thereof,, wherein
X is O;
Y is selected from the group consisting of: -CH₂-, -CH₂-CH₂-, -CH₂-CH₂-CH₂-, - CH₂NH -, -CH₂O- and -CH₂S-.

6. The nitrogen-containing heterocyclic compound according to claim 1, or a pharmaceutically acceptable salt, a enantiomer, a diastereomer, or a racemate thereof,,wherein R₄ is selected from the following groups that are unsubstituted or substituted with 1-3 substituents: C6-C10 aryl, 5-7 membered heteroaryl or benzo 5-7 membered heteroaryl; preferably, the heterocycle and heteroaromatic ring moiety in the group is selected from the groups formed from indole, benzodioxole isoxazole, pyridine, pyrazole, dihydroimidazopyridine, imidazopyridine, benzothiophene, dihydrobenzodioxolane, quinolone, pyrrole, benzofuran, indazole, benzimidazole, quinoline and 1,3-dioxoindoleline.

7. The nitrogen-containing heterocyclic compound according to claim 1, or a pharmaceutically acceptable salt, a enantiomer, a diastereomer, or a racemate thereof, wherein hydrogen, deuterium, tritium, halogen, unsubstituted or 1-3 halogens substituted C1-C6 alkyl, or unsubstituted or 1-3 halogens substituted C3-C6 cycloalkyl.

8. The nitrogen-containing heterocyclic compound according to claim 1, or a pharmaceutically acceptable salt, a enantiomer, a diastereomer, or a racemate thereof,, wherein the tetrahydroisoquinoline compound is selected from the following compounds:
| number | structure | name |
|---|---|---|
| **A1** | 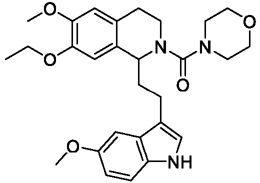 | (7-ethoxy-6-methoxy-1-(2-(5-methoxy-1H-indol-3-yl)ethyl)-3,4-dihydroisoquinolin-2(1H)-yl) (morpholino)methanone |
| **(S)-A1** | 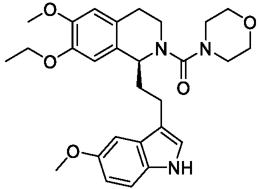 | (S)-(7-ethoxy-6-methoxy-1-(2-(5-methoxy-1H-indol-3-yl)ethyl)-3,4-dihydroisoquinolin-2(1H)-yl) (morpholino)methanone |
| **(R)-A1** | 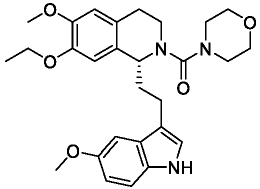 | (R)-(7-ethoxy-6-methoxy-1-(2-(5-methoxy-1H-indol-3-yl)ethyl)-3,4-dihydroisoquinolin-2(1H)-yl) (morpholino)methanone |
| **A2** | 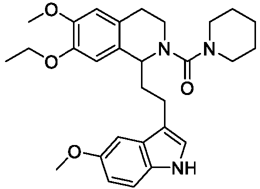 | (7-ethoxy-6-methoxy-1-(2-(5-methoxy-1H-indol-3-yl)ethyl)-3,4-dihydroisoquinolin-2(1H)-yl) (piperidin-1-yl)methanone |
| **A3** | 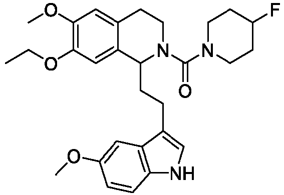 | (4-fluoropiperidin-1-yl)(7-ethoxy-6-methoxy-1-(2-(5-methoxy-1H-indol-3-yl)ethyl)-3,4-dihydroisoquinolin-2(1H)-yl)methanone |
| **A4** | 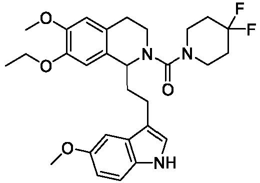 | (4,4-difluoropiperidin-1-yl)(7-ethoxy-6-methoxy-1-(2-(5-methoxy-1H-indol-3-yl)ethyl)-3,4-dihydroisoquinolin-2(1H)-yl)methanone |
| **A5** | 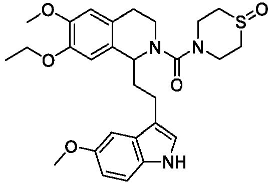 | (7-ethoxy-6-methoxy-1-(2-(5-methoxy-1H-indol-3-yl)ethyl)-3,4-dihydroisoquinolin-2(1H)-yl)(1-oxothiomorpholinyl)methanone |
| **A6** | 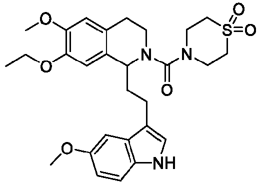 | (1,1-dioxothiomorpholino)(7-ethoxy-6-methoxy-1-(2-(5-methoxy-1H-indol-3-yl)ethyl)-3,4-dihydroisoquinolin-2(1H)-yl) methylmethanone |
| **A7** | 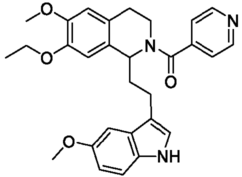 | (7-ethoxy-6-methoxy-1-(2-(5-methoxy-1H-indol-3-yl)ethyl)-3,4-dihydroisoquinolin-2(1H)-yl) (pyridin-4-yl)methanone |
| **A8** | 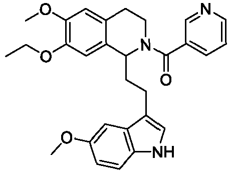 | (7-ethoxy-6-methoxy-1-(2-(5-methoxy-1H-indol-3-yl)ethyl)-3,4-dihydroisoquinolin-2(1H)-yl) (pyridin-3-yl)methanone |
| **A9** | 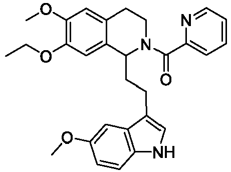 | (7-ethoxy-6-methoxy-1-(2-(5-methoxy-1H-indol-3-yl)ethyl)-3,4-dihydroisoquinolin-2(1H)-yl) (pyridin-2-yl)methanone |
| **A10** | 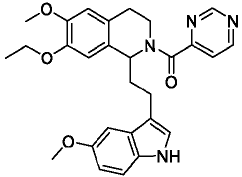 | (7-ethoxy-6-methoxy-1-(2-(5-methoxy-1H-indol-3-yl)ethyl)-3,4-dihydroisoquinolin-2(1H)-yl) (pyrimidin-4-yl)methanone |
| **A11** | 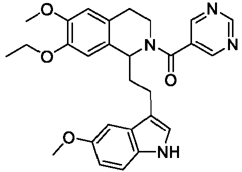 | (7-ethoxy-6-methoxy-1-(2-(5-methoxy-1H-indol-3-yl)ethyl)-3,4-dihydroisoquinolin-2(1H)-yl) (pyrimidin-5-yl)methanone |
| **A12** | 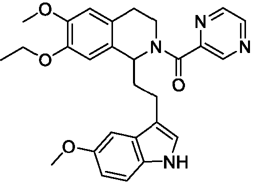 | (7-ethoxy-6-methoxy-1-(2-(5-methoxy-1H-indol-3-yl)ethyl)-3,4-dihydroisoquinolin-2(1H)-yl) (pyrazin-2-yl)methanone |
| **A13** | 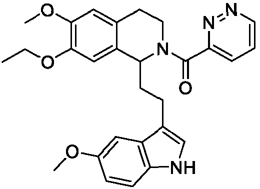 | (7-ethoxy-6-methoxy-1-(2-(5-methoxy-1H-indol-3-yl)ethyl)-3,4-dihydroisoquinolin-2(1H)-yl) (pyridazin-3-yl)methanone |
| **A14** | 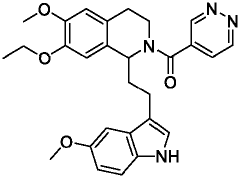 | (7-ethoxy-6-methoxy-1-(2-(5-methoxy-1H-indol-3-yl)ethyl)-3,4-dihydroisoquinolin-2(1H)-yl) (pyridazin-4-yl)methanone |
| **A15** | 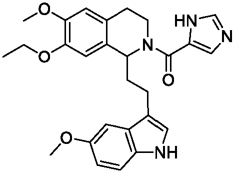 | (7-ethoxy-6-methoxy-1-(2-(5-methoxy-1H-indol-3-yl)ethyl)-3,4-dihydroisoquinolin-2(1H)-yl)(1H-imidazol-5-yl)methanone |
| **A16** | 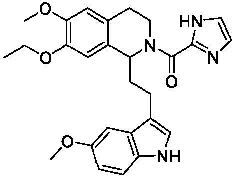 | (7-ethoxy-6-methoxy-1-(2-(5-methoxy-1H-indol-3-yl)ethyl)-3,4-dihydroisoquinolin-2(1H)-yl)(1H-imidazol-2-yl)methanone |
| **A17** | 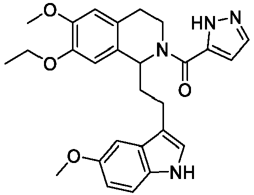 | (7-ethoxy-6-methoxy-1-(2-(5-methoxy-1H-indol-3-yl)ethyl)-3,4-dihydroisoquinolin-2(1H)-yl)(1H-pyrazol-5-yl)methanone |
| **A18** | 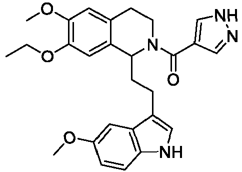 | (7-ethoxy-6-methoxy-1-(2-(5-methoxy-1H-indol-3-yl)ethyl)-3,4-dihydroisoquinolin-2(1H)-yl)(1H-pyrazol-4-yl)methanone |
| **A19** | 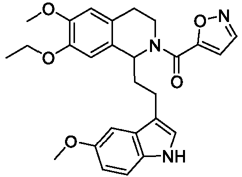 | (7-ethoxy-6-methoxy-1-(2-(5-methoxy-1H-indol-3-yl)ethyl)-3,4-dihydroisoquinolin-2(1H)-yl) (isoxazole-5-yl)methanone |
| **A20** | 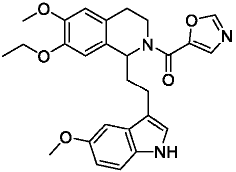 | (7-ethoxy-6-methoxy-1-(2-(5-methoxy-1H-indol-3-yl)ethyl)-3,4-dihydroisoquinolin-2(1H)-yl) (oxazol-2-yl)methanone |
| **A21** | 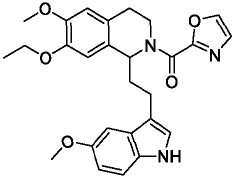 | (7-ethoxy-6-methoxy-1-(2-(5-methoxy-1H-indol-3-yl)ethyl)-3,4-dihydroisoquinolin-2(1H)-yl) (oxazol-2-yl)methanone |
| **A22** | 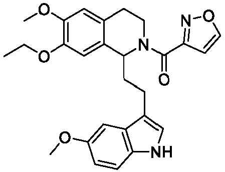 | (7-ethoxy-6-methoxy-1-(2-(5-methoxy-1H-indol-3-yl)ethyl)-3,4-dihydroisoquinolin-2(1H)-yl) (isoxazol-3-yl)methanone |
| **A23** | 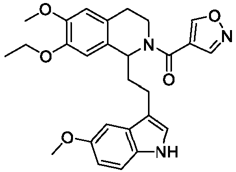 | (7-ethoxy-6-methoxy-1-(2-(5-methoxy-1H-indol-3-yl)ethyl)-3,4-dihydroisoquinolin-2(1H)-yl) (isoxazol-4-yl)methanone |
| **A24** | 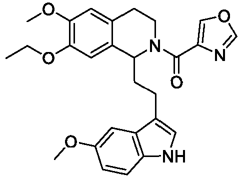 | (7-ethoxy-6-methoxy-1-(2-(5-methoxy-1H-indol-3-yl)ethyl)-3,4-dihydroisoquinolin-2(1H)-yl) (oxazol-4-yl)methanone |
| **A25** | 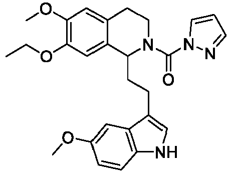 | (7-ethoxy-6-methoxy-1-(2-(5-methoxy-1H-indol-3-yl)ethyl)-3,4-dihydroisoquinolin-2(1H)-yl)(1H-pyrazol-1-yl)methanone |
| **A26** | 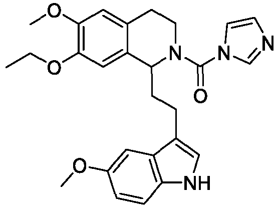 | (7-ethoxy-6-methoxy-1-(2-(5-methoxy-1H-indol-3-yl)ethyl)-3,4-dihydroisoquinolin-2(1H)-yl)(1H-imidazol-1-yl)methanone |
| **A27** | 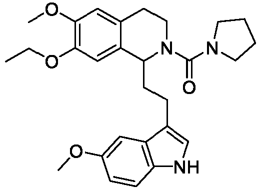 | (7-ethoxy-6-methoxy-1-(2-(5-methoxy-1H-indol-3-yl)ethyl)-3,4-dihydroisoquinolin-2(1H)-yl) (pyrrolidin-1-yl)methanone |
| **A28** | 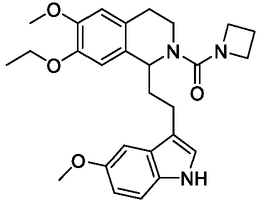 | Nitrogen heterocyclic but-1-yl(7-ethoxy-6-methoxy-1-(2-(5-methoxy-1H-indol-3-yl) ethyl)-3,4-dihydroisoquinolin-2(1H)-yl)methanone |
| **A29** | 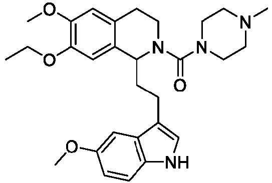 | (7-ethoxy-6-methoxy-1-(2-(5-methoxy-1H-indol-3-yl)ethyl)-3,4-dihydroisoquinolin-2(1H)-yl)(4-methylpiperazin-1-yl)methanone |
| **A30** | 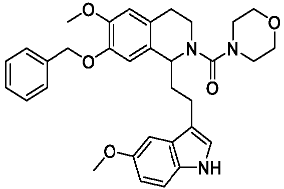 | (7-(benzyloxy)-6-methoxy-1-(2-(5-methoxy-1H-indol-3-yl)ethyl)-3,4-dihydroisoquinolin-2(1H)-yl) (morpholino)methanone |
| **A31** | 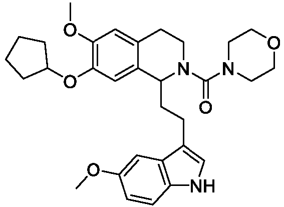 | (7-(Cyclopentaxy)-6-methoxy-1-(2-(5-methoxy-1H-indol-3-yl) ethyl)-3,4-dihydroisoquinolin-2 (1H)-yl)(morpholino)methanone |
| **A32** | 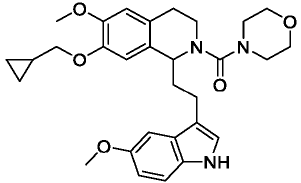 | (7-(Cyclopropylmethoxy)-6-methoxy-1-(2-(5-methoxy-1H-indol-3-yl)ethyl)-3,4-dihydroisoquinolin-2(1H)-yl) (morpholino)methanone |
| **A33** | 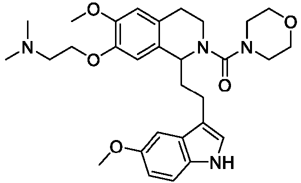 | (7-(2-(dimethylamino)ethoxy)-6-methoxy-1-(2-(5-methoxy-1H-indol-3-yl)ethyl)-3,4-dihydroisoquinolin-2(1H)-yl) (morpholino)methanone |
| **A34** | 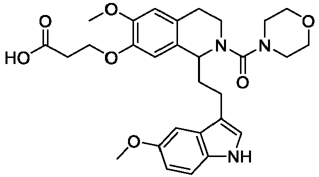 | 3-((6-methoxy-1-(2-(5-methoxy-1H-indol-3-yl)ethyl)-2-(morpholin-4-carbonyl)-1,2,3,4-tetrahydroisoquinolin-7-yl)oxy) propionic acid |
| **A35** | 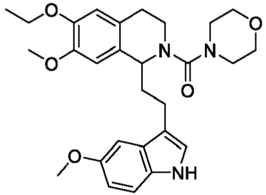 | (6-Ethoxy-7-methoxy-1-(2-(5-methoxy-1H-indol-3-yl)ethyl)-3,4-dihydroisoquinolin-2(1H)-yl) (morpholino)methanone |
| **A36** | 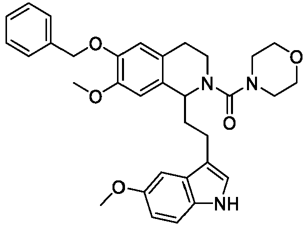 | (6-(benzyloxy)-7-methoxy-1-(2-(5-methoxy-1H-indol-3-yl)ethyl)-3,4-dihydroisoquinolin-2(1H)-yl) (morpholino)methanone |
| **A37** | 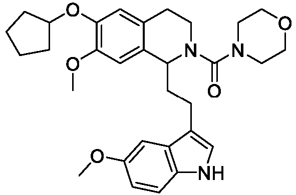 | (6-(Cyclopentaxy)-7-Methoxy-1-(2-(5-methoxy-1H-indol-3-yl) ethyl)-3,4-Dihydroisoquinolin-2 (1H)-yl)(morpholino)methanone |
| **A38** | 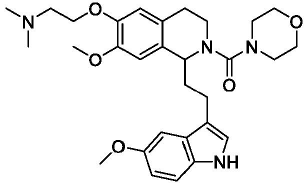 | (6-(2-(dimethylamino)ethoxy)-7-methoxy-1-(2-(5-methoxy-1H-indol-3-yl)ethyl)-3,4-dihydroisoquinolin-2(1H)-yl) (morpholino)methanone |
| **A39** | 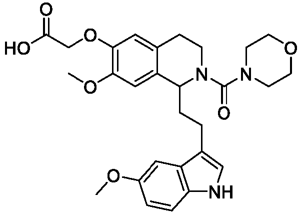 | 2-((7-Methoxy-1-(2-(5-methoxy-1H-indol-3-yl)ethyl)-2-(morpholin-4-carbonyl)-1,2,3,4-tetrahydroisoquinolin-6-yl)oxy) acetic acid |
| **A40** | 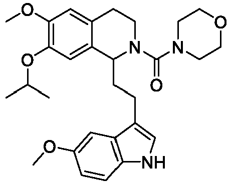 | (7-isopropoxy-6-methoxy-1-(2-(5-methoxy-1H-indol-3-yl)ethyl)-3,4-dihydroisoquinolin-2(1H)-yl) (morpholino)methanone |
| **A41** | 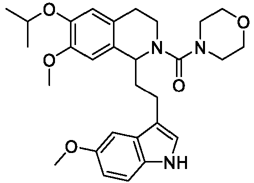 | (6-isopropoxy-7-methoxy-1-(2-(5-methoxy-1H-indol-3-yl)ethyl)-3,4-dihydroisoquinolin-2(1H)-yl) (morpholino)methanone |
| **A42** | 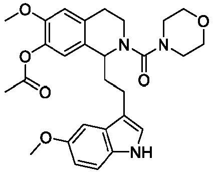 | 6-methoxy-1-(2-(5-methoxy-1H-indol-3-yl)ethyl)-2-(morpholine-4-carbonyl)-1,2,3,4-tetrahydroisoquinoline-7-ylacetate |
| **A43** | 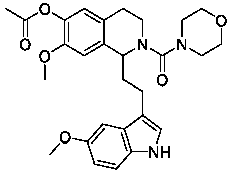 | 7-Methoxy-1-(2-(5-methoxy-1H-indol-3-yl)ethyl)-2-(morpholine-4-carbonyl)-1,2,3,4-tetrahydroisoquinoline-6-ylacetate |
| **A44** | 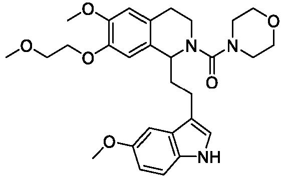 | (6-methoxy-1-(2-(5-methoxy-1H-indol-3-yl)ethyl)-7-(2-methoxyethoxy)-3,4-dihydroisoquinolin-2(1H)-yl) (morpholino)methanone |
| **A45** | 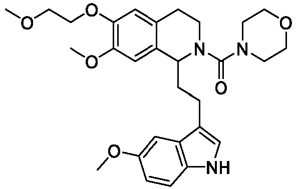 | (7-Methoxy-1-(2-(5-methoxy-1H-indol-3-yl)ethyl)-6-(2-methoxyethoxy)-3,4-dihydroisoquinolin-2(1H)-yl) (morpholino)methanone |
| **A46** | 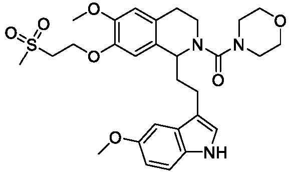 | (6-methoxy-1-(2-(5-methoxy-1H-indol-3-yl)ethyl)-7-(2-(methylsulfonyl)ethoxy)-3,4-dihydroisoquinolin-2(1H)-yl) (morpholino)methanone |
| **A47** | 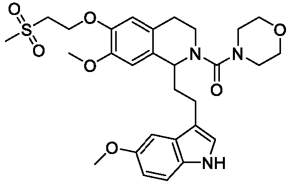 | (7-Methoxy-1-(2-(5-methoxy-1H-indol-3-yl)ethyl)-6-(2-(methylsulfonyl)ethoxy)-3,4-dihydroisoquinolin-2(1H)-yl) (morpholino)methanone |
| **A48** | 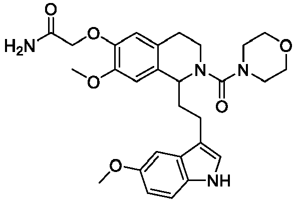 | 2-((7-Methoxy-1-(2-(5-methoxy-1H-indol-3-yl)ethyl)-2-(morpholin-4-carbonyl)-1,2,3,4-tetrahydroisoquinolin-6-yl)oxy) acetamide |
| **A49** | 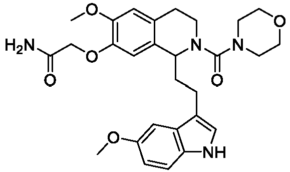 | 2-((6-methoxy-1-(2-(5-methoxy-1H-indol-3-yl)ethyl)-2-(morpholin-4-carbonyl)-1,2,3,4-tetrahydroisoquinolin-7-yl)oxy) acetamide |
| **A50** | 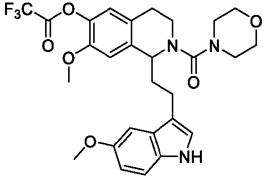 | 7-Methoxy-1-(2-(5-methoxy-1H-indol-3-yl)ethyl)-2-(morpholin-4-carbonyl)-1,2,3,4-tetrahydroisoquinolin-6-yl 2,2,2-trifluoroacetate |
| **A51** | 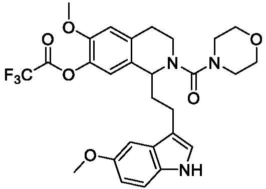 | 6-methoxy-1-(2-(5-methoxy-1H-indol-3-yl)ethyl)-2-(morpholin-4-carbonyl)-1,2,3,4-tetrahydroisoquinolin-7-yl 2,2,2-trifluoroacetate |
| **A52** | 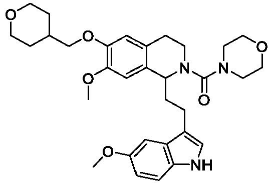 | (7-Methoxy-1-(2-(5-methoxy-1H-indol-3-yl)ethyl)-6-(tetrahydro-2H-pyran-4-yl)methoxy)-3,4-dihydroisoquinolin-2(1H)-yl) (morpholino)methanone |
| **A53** | 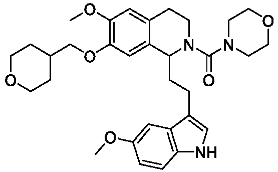 | (6-methoxy-1-(2-(5-methoxy-1H-indol-3-yl)ethyl)-7-(tetrahydro-2H-pyran-4-yl)methoxy)-3,4-dihydroisoquinolin-2(1H)-yl) (morpholino)methanone |
| **A54** | 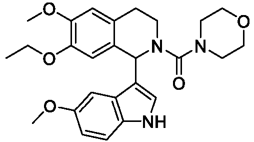 | (7-ethoxy-6-methoxy-1-(5-methoxy-1H-indol-3-yl)-3,4-dihydroisoquinolin-2(1H)-yl) (morpholino)methanone |
| **A55** | 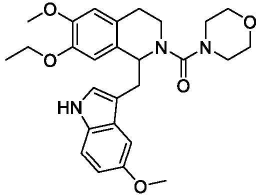 | (7-ethoxy-6-methoxy-1-((5-methoxy-1H-indol-3-yl)methyl)-3,4-dihydroisoquinolin-2(1H)-yl) (morpholino)methanone |
| **A56** | 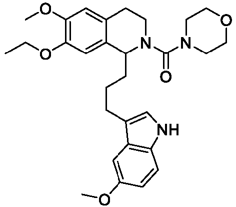 | (7-ethoxy-6-methoxy-1-(3-(5-methoxy-1H-indol-3-yl)propyl)-3,4-dihydroisoquinolin-2(1H)-yl) (morpholino)methanone |
| **A57** | 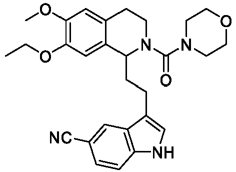 | 3-(2-(7-ethoxy-6-methoxy-2-(morpholine-4-carbonyl)-1,2,3,4-tetrahydroisoquinolin-1-yl)ethyl)-1H-indole-5-carbonitrile |
| **A58** | 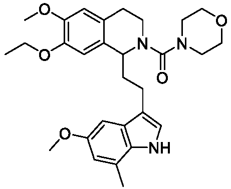 | (7-ethoxy-6-methoxy-1-(2-(5-methoxy-7-methyl-1H-indol-3-yl) ethyl)-3,4-dihydroisoquinolin-2 (1H)-yl)(morpholino)methanone |
| **A59** | 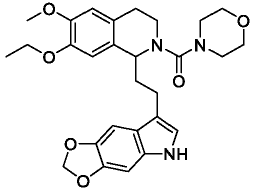 | (1-(2-(5H-[1,3]dioxolane[4,5-f] indole-7-yl)ethyl)-7-ethoxy-6-methoxy-3,4-dihydroisoquinolin-2 (1H)-yl)(morpholino)methanone |
| **A60** | 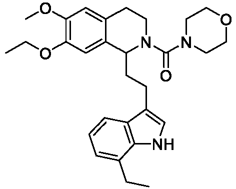 | (7-Ethoxy-1-(2-(7-ethyl-1H-indol-3-yl)ethyl)-6-methoxy-3,4-dihydroisoquinolin-2(1H)-yl) (morpholino)methanone |
| **A61** | 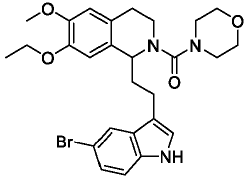 | (1-(2-(5-bromo-1H-indol-3-yl) ethyl)-7-ethoxy-6-methoxy-3,4-dihydroisoquinolin-2(1H)-yl) (morpholino)methanone |
| **A62** | 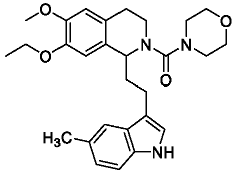 | (7-ethoxy-6-methoxy-1-(2-(5-methyl-1H-indol-3-yl)ethyl)-3,4-dihydroisoquinolin-2(1H)-yl) (morpholino)methanone |
| **A63** | 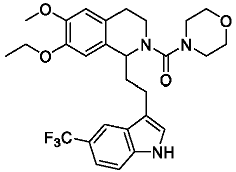 | (7-ethoxy-6-methoxy-1-(2-(5-(trifluoromethyl)-1H-indol-3-yl) ethyl)-3,4-dihydroisoquinolin-2 (1H)-yl)(morpholino)methanone |
| **A64** | 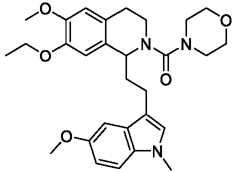 | (7-ethoxy-6-methoxy-1-(2-(5-methoxy-1-methyl-1H-indol-3-yl) ethyl)-3,4-dihydroisoquinolin-2 (1H)-yl)(morpholino)methanone |
| **A65** | 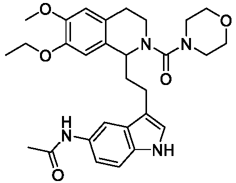 | N-(3-(2-(7-ethoxy-6-methoxy-2-(morpholine-4-carbonyl)-1,2,3,4-tetrahydroisoquinolin-1-yl)ethyl)-1H-indole-5-yl)acetamide |
| **A66** | 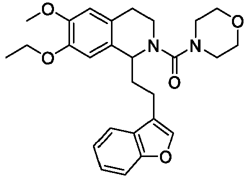 | (1-(2-(benzofuran-3-yl)ethyl)-7-ethoxy-6-methoxy-3,4-dihydroisoquinolin-2(1H)-yl) (morpholino)methanone |
| **A67** | 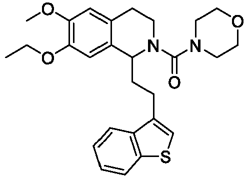 | (1-(2-(benzo[b]thiophen-3-yl) ethyl)-7-ethoxy-6-methoxy-3,4-dihydroisoquinolin-2(1H)-yl) (morpholino)methanone |
| **A68** | 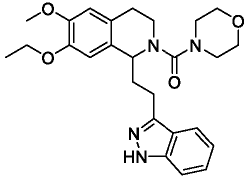 | (1-(2-(1H-indazol-3-yl)ethyl)-7-ethoxy-6-methoxy-3,4-dihydroisoquinolin-2(1H)-yl) (morpholino)methanone |
| **A69** | 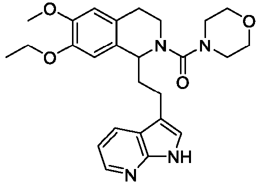 | (1-(2-(1H-pyrrolo[2,3-b]pyridin-3-yl)ethyl)-7-ethoxy-6-methoxy-3,4-dihydroisoquinolin-2(1H)-yl) (morpholino)methanone |
| **A70** | 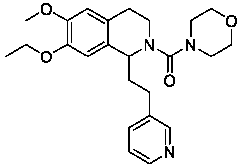 | (7-ethoxy-6-methoxy-1-(2-(pyridin-3-yl)ethyl)-3,4-dihydroisoquinolin-2(1H)-yl) (morpholino)methanone |
| **A71** | 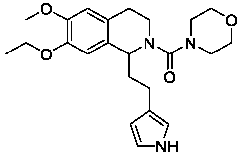 | (1-(2-(1H-pyrrole-3-yl)ethyl)-7-ethoxy-6-methoxy-3,4-dihydroisoquinolin-2(1H)-yl) (morpholino)methanone |
| **A72** | 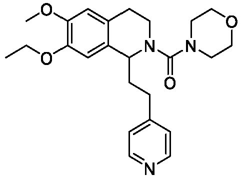 | (7-ethoxy-6-methoxy-1-(2-(pyridin-4-yl)ethyl)-3,4-dihydroisoquinolin-2(1H)-yl) (morpholino)methanone |
| **A73** | 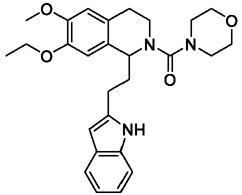 | (1-(2-(1H-indol-2-yl)ethyl)-7-ethoxy-6-methoxy-3,4-dihydroisoquinolin-2(1H)-yl) (morpholino)methanone |
| **A74** | 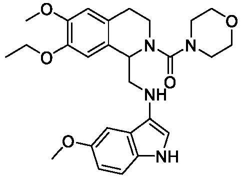 | (7-ethoxy-6-methoxy-1-((5-methoxy-1H-indol-3-yl)amino) methyl)-3,4-dihydroisoquinolin-2 (1H)-yl)(morpholino)methanone |
| **A75** | 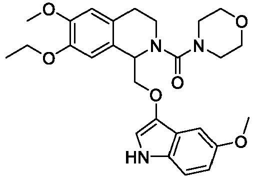 | (7-ethoxy-6-methoxy-1-((5-methoxy-1H-indol-3-yl)oxy) methyl)-3,4-dihydroisoquinolin-2 (1H)-yl)(morpholino)methanone |
| **A76** | 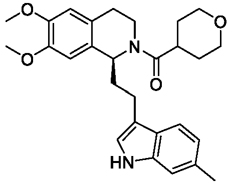 | (S)-(6,7-dimethoxy-1-(2-(6-methyl-1H-indol-3-yl)ethyl)-3,4-dihydroisoquinolin-2(1H)-yl) (tetrahydro-2H-pyran-4-yl)methanone |
| **A77** | 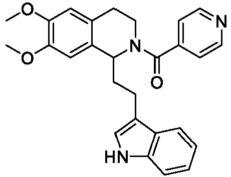 | (1-(2-(1H-indol-3-yl)ethyl)-6,7-dimethoxy-3,4-dihydroisoquinolin-2 (1H)-yl)(pyridin-4-yl)methanone |
| **A80** | 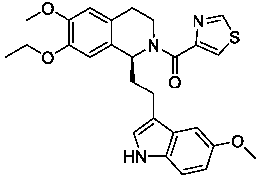 | (S)-(7-ethoxy-6-methoxy-1-(2-(5-methoxy-1H-indol-3-yl)ethyl)-3,4-dihydroisoquinolin-2(1H)-yl) (thiazol-4-yl)methanone |
| **A83** | 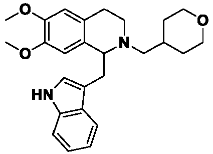 | 1-(1H-indol-3-yl)methyl)-6,7-dimethoxy-2-((tetrahydro-2H-pyran-4-yl)methyl)-1,2,3,4-tetrahydroisoquinoline |
| **A84** | 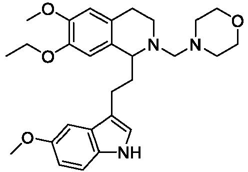 | 4-((7-ethoxy-6-methoxy-1-(2-(5-methoxy-1H-indol-3-yl)ethyl)-3,4-dihydroisoquinolin-2(1H)-yl) methyl)morpholine |
| **A85** | 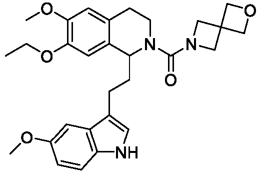 | (7-ethoxy-6-methoxy-1-(2-(5-methoxy-1H-indol-3-yl)ethyl)-3,4-dihydroisoquinolin-2(1H)-yl)(2-oxo-6-azaspiro[3.3]heptan-6-yl)methanone |
| **A86** | 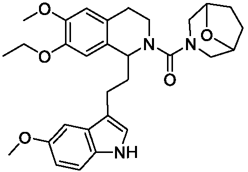 | (8-oxo-3-azabicyclo[3.2.1]oct-3-yl) (7-ethoxy-6-methoxy-1-(2-(5-methoxy-1H-indol-3-yl)ethyl)-3,4-dihydroisoquinolin-2(1H)-yl)methanone |
| **A87** | 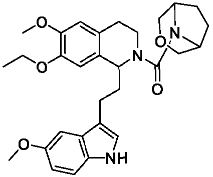 | (3-Oxo-8-azabicyclo[3.2.1]octan-8-yl)(7-ethoxy-6-methoxy-1-(2-(5-methoxy-1H-indol-3-yl)ethyl)-3,4-dihydroisoquinolin-2(1H)-yl)methanone |
| **A88** | 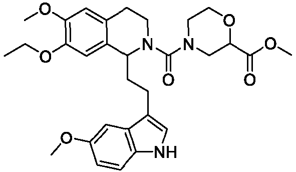 | Methyl 4-(7-ethoxy-6-methoxy-1-(2-(5-methoxy-1H-indol-3-yl)ethyl)-1,2,3,4-tetrahydroisoquinoline-2-carbonyl)morpholine-2-carboxylate |
| **A89** | 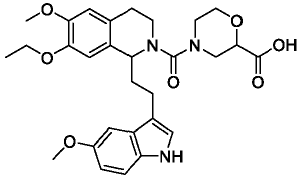 | 4-(7-ethoxy-6-methoxy-1-(2-(5-methoxy-1H-indol-3-yl)ethyl)-1,2,3,4-tetrahydroisoquinoline-2-carbonyl)morpholine-2-carboxylic acid |
| **A90** | 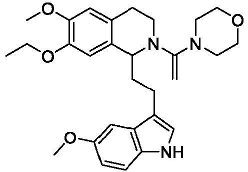 | 4-(1-(7-ethoxy-6-methoxy-1-(2-(5-methoxy-1H-indol-3-yl)ethyl)-3,4-dihydroisoquinolin-2(1H)-yl) ethenyl)morpholine |
| **A96** | 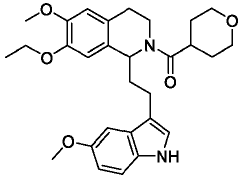 | (7-ethoxy-6-methoxy-1-(2-(5-methoxy-1H-indol-3-yl)ethyl)-3,4-dihydroisoquinolin-2(1H)-yl) (tetrahydro-2H-pyran-4-yl)methanone |
| **A97** | 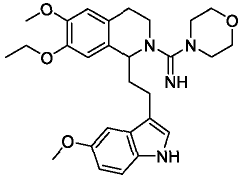 | (7-ethoxy-6-methoxy-1-(2-(5-methoxy-1H-indol-3-yl)ethyl)-3,4-dihydroisoquinolin-2(1H)-yl) (morpholinyl)carboxamide |
| **A98** | 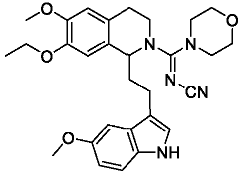 | (E)N-((7-ethoxy-6-methoxy-1-(2-(5-methoxy-1H-indol-3-yl)ethyl)-3,4-dihydroisoquinolin-2(1H)-yl) (morpholinyl)methylene) cyanamide |
| **A99** | 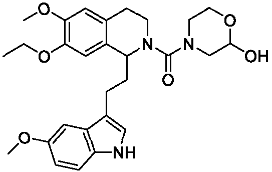 | (7-ethoxy-6-methoxy-1-(2-(5-methoxy-1H-indol-3-yl)ethyl)-3,4-dihydroisoquinolin-2(1H)-yl)(2-hydroxymorpholino)methanone |
| **A100** | 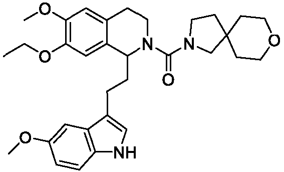 | (7-ethoxy-6-methoxy-1-(2-(5-methoxy-1H-indol-3-yl)ethyl)-3,4-dihydroisoquinolin-2(1H)-yl)(8-oxa-2-azaspiro[4.5]dec-2-yl)methanone |
| **A101** | 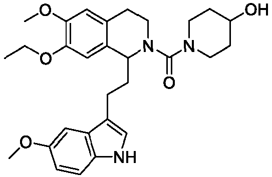 | (7-ethoxy-6-methoxy-1-(2-(5-methoxy-1H-indol-3-yl)ethyl)-3,4-dihydroisoquinolin-2(1H)-yl)(4-hydroxypiperidin-1-yl)methanone |
| **A102** | 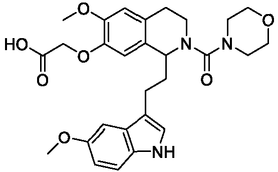 | 2-((6-methoxy-1-(2-(5-methoxy-1H-indol-3-yl)ethyl)-2-(morpholin-4-carbonyl)-1,2,3,4-tetrahydroisoquinolin-7-yl)oxy) aceticacid |
| **A103** | 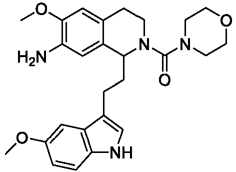 | (7-Amino-6-methoxy-1-(2-(5-methoxy-1H-indol-3-yl)ethyl)-3,4-dihydroisoquinolin-2(1H)-yl) (morpholinyl)methanone |
| **A104** | 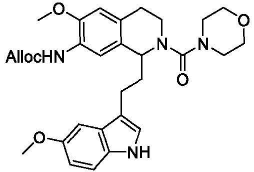 | Allyl(6-methoxy-1-(2-(5-methoxy-1H-indol-3-yl)ethyl)-2-(morpholin-4-carbonyl)-1,2,3,4-tetrahydroisoquinolin-7-yl) carbamate |
| **A105** | 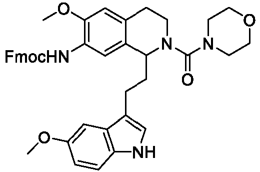 | (9H-fluoren-9-yl)methyl(6-methoxy-1-(2-(5-methoxy-1H-indol-3-yl)ethyl)-2-(morpholine-4-carbonyl)-1,2,3,4-tetrahydroisoquinolin-7-yl) carbamate |
| **A106** | 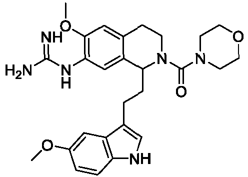 | 1-(6-methoxy-1-(2-(5-methoxy-1H-indol-3-yl)ethyl)-2-(morpholin-4-carbonyl)-1,2,3,4-tetrahydroisoquinolin-7-yl) guanidine |
| **A107** | 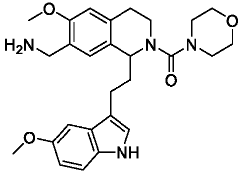 | (7-(Aminomethyl)-6-methoxy-1-(2-(5-methoxy-1H-indol-3-yl) ethyl)-3,4-dihydroisoquinolin-2 (1H)-yl)(morpholinyl)methanone |
| **A108** | 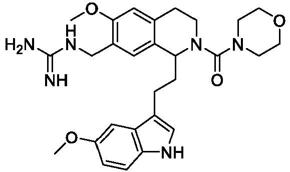 | 1-((6-methoxy-1-(2-(5-methoxy-1H-indol-3-yl)ethyl)-2-(morpholin-4-carbonyl)-1,2,3,4-tetrahydroisoquinolin-7-yl)methyl) guanidine |
| **A109** | 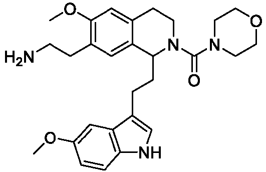 | (7-(2-aminoethyl)-6-methoxy-1-(2-(5-methoxy-1H-indol-3-yl) ethyl)-3,4-dihydroisoquinolin-2 (1H)-yl)(morpholinyl)methanone |
| **A110** | 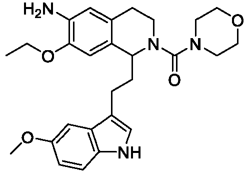 | (6-Amino-7-ethoxy-1-(2-(5-methoxy-1H-indol-3-yl)ethyl)-3,4-dihydroisoquinolin-2(1H)-yl) (morpholinyl)methanone |
| **A111** | 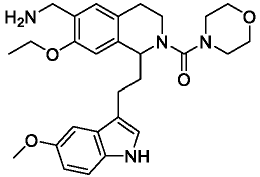 | (6-(Aminomethyl)-7-ethoxy-1-(2-(5-methoxy-1H-indol-3-yl)ethyl)-3,4-dihydroisoquinolin-2(1H)-yl) (morpholinyl)methanone |
| **A112** | 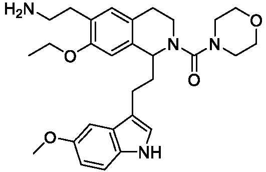 | (6-(2-aminoethyl)-7-ethoxy-1-(2-(5-methoxy-1H-indol-3-yl)ethyl)-3,4-dihydroisoquinolin-2(1H)-yl) (morpholinyl)methanone |
| **A113** | 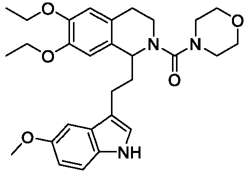 | (6,7-diethoxy-1-(2-(5-methoxy-1H-indol-3-yl)ethyl)-3,4-dihydroisoquinolin-2(1H)-yl) (morpholinyl)methanone |
| **A114** | 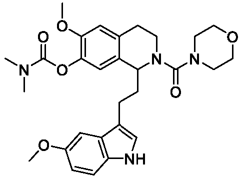 | 6-methoxy-1-(2-(5-methoxy-1H-indol-3-yl)ethyl)-2-(morpholin-4-carbonyl)-1,2,3,4-tetrahydroisoquinoline-7-yldimethylaminoformate |
| **A115** | 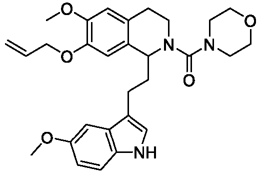 | (7-(Allyloxy)-6-methoxy-1-(2-(5-methoxy-1H-indol-3-yl)ethyl)-3,4-dihydroisoquinolin-2(1H)-yl) (morpholinyl)methanone |
| **A116** | 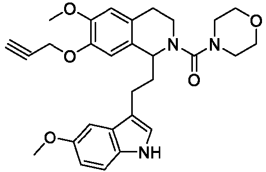 | (6-methoxy-1-(2-(5-methoxy-1H-indol-3-yl)ethyl)-7-(prop-2-yn-1-yloxy)-3,4-dihydroisoquinolin-2 (1H)-yl)(morpholinyl)methanone |
| **A117** | 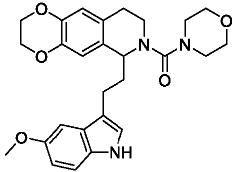 | (6-(2-(5-methoxy-1H-indol-3-yl) ethyl)-2,3,8,9-tetrahydro-[1,4] dioxy[2,3-g]isoquinolin-7(6H)- yl)(morpholinyl)methanone |
| **A118** | 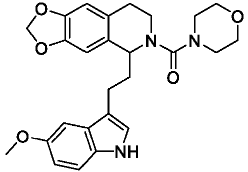 | (5-(2-(5-methoxy-1H-indol-3-yl) ethyl)-7,8-dihydro-[1,3]dioxolane [4,5-g]isoquinolin-6(5H)-yl) (morpholinyl)methanone |
| **A119** | 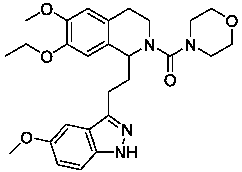 | (7-ethoxy-6-methoxy-1-(2-(5-methoxy-1H-indazol-3-yl)ethyl)-3,4-dihydroisoquinolin-2(1H)-yl) (morpholinyl)methanone |
| **A120** | 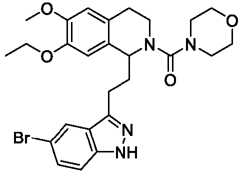 | (1-(2-(5-bromo-1H-indazol-3-yl) ethyl)-7-ethoxy-6-methoxy-3,4-dihydroisoquinolin-2(1H)-yl) (morpholinyl)methanone |
| **A121** | 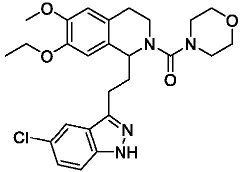 | (1-(2-(5-Chloro-1H-indazol-3-yl) ethyl)-7-ethoxy-6-methoxy-3,4-dihydroisoquinolin-2(1H)-yl) (morpholinyl)methanone |
| **A122** | 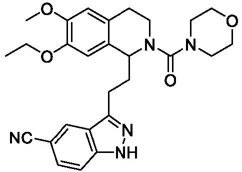 | 3-(2-(7-ethoxy-6-methoxy-2-(morpholine-4-carbonyl)-1,2,3,4-tetrahydroisoquinolin-1-yl)ethyl)-1H-indazole-5-nitrile |
| **A123** | 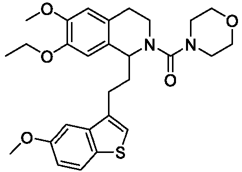 | (7-ethoxy-6-methoxy-1-(2-(5-methoxybenzo[b]thiophen-3-yl) ethyl)-3,4-dihydroisoquinolin-2 (1H)-yl)(morpholinyl)methanone |
| **A124** | 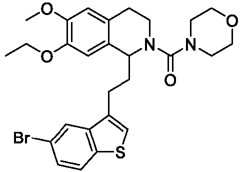 | (1-(2-(5-bromobenzo[b]thiophen-3-yl)ethyl)-7-ethoxy-6-methoxy-3,4-dihydroisoquinolin-2(1H)-yl) (morpholinyl)methanone |
| **A125** | 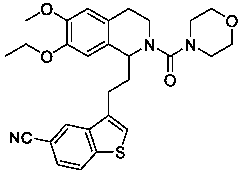 | 3-(2-(7-ethoxy-6-methoxy-2-(morpholin-4-carbonyl)-1,2,3,4-tetrahydroisoquinolin-1-yl)ethyl) benzo[b]thiophene-5-nitrile |
| **A126** | 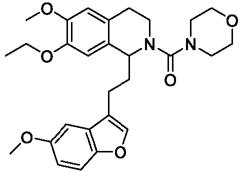 | (7-ethoxy-6-methoxy-1-(2-(5-methoxybenzofuran-3-yl)ethyl)-3,4-dihydroisoquinolin-2(1H)-yl) (morpholinyl)methanone |
| **A127** | 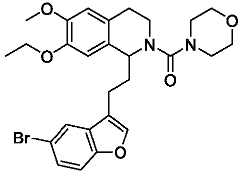 | (1-(2-(5-bromobenzofuran-3-yl) ethyl)-7-ethoxy-6-methoxy-3,4-dihydroisoquinolin-2(1H)-yl) (morpholinyl)methanone |
| **A128** | 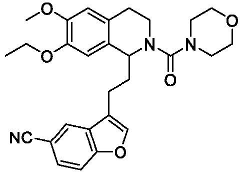 | 3-(2-(7-ethoxy-6-methoxy-2-(morpholin-4-carbonyl)-1,2,3,4-tetrahydroisoquinolin-1-yl)ethyl) benzofuran-5-carbonitrile |
| **A129** | 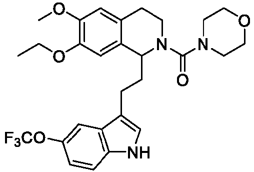 | (7-ethoxy-6-methoxy-1-(2-(5-(trifluoromethoxy)-1H-indol-3-yl) ethyl)-3,4-dihydroisoquinolin-2 (1H)-yl)(morpholinyl)methanone |
| **A130** | 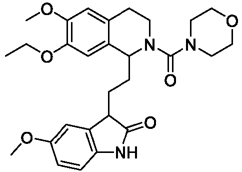 | 3-(2-(7-ethoxy-6-methoxy-2-(morpholin-4-carbonyl)-1,2,3,4-tetrahydroisoquinolin-1-yl)ethyl)-5-methoxyindol-2-one |
| **A131** | 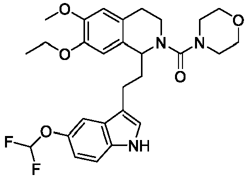 | (1-(2-(5-(difluoromethoxy)-1H-indol-3-yl)ethyl)-7-ethoxy-6-methoxy-3,4-dihydroisoquinolin-2 (1H)-yl)(morpholinyl)methanone |
| **A132** | 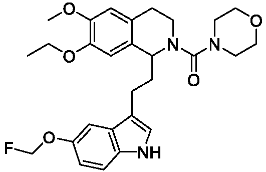 | (7-Ethoxy-1-(2-(5-(fluoromethoxy)-1H-indol-3-yl) ethyl)-6-methoxy-3,4-dihydroisoquinolin-2(1H)-yl) (morpholinyl)methanone |
| **A133** | 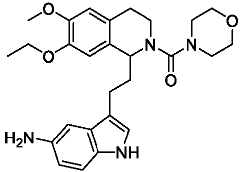 | (1-(2-(5-amino-1H-indol-3-yl) ethyl)-7-ethoxy-6-methoxy-3,4-dihydroisoquinolin-2(1H)-yl) (morpholinyl)methanone |
| **A134** | 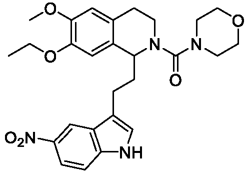 | (7-ethoxy-6-methoxy-1-(2-(5-nitro-1H-indol-3-yl)ethyl)-3,4-dihydroisoquinolin-2(1H)-yl) (morpholinyl)methanone |
| **A135** | 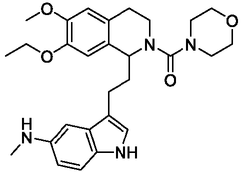 | (7-ethoxy-6-methoxy-1-(2-(5-(methylamino)-1H-indol-3-yl) ethyl)-3,4-dihydroisoquinolin-2 (1H)-yl)(morpholinyl)methanone |
| **A136** | 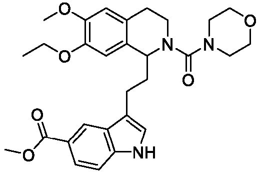 | Methyl3-(2-(7-ethoxy-6-methoxy-2-(morpholine-4-carbonyl)-1,2,3,4-tetrahydroisoquinolin-1-yl)ethyl)-1H-indole-5-carboxylate |
| **A137** | 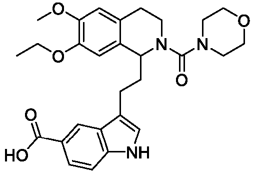 | 3-(2-(7-ethoxy-6-methoxy-2-(morpholin-4-carbonyl)-1,2,3,4-tetrahydroisoquinolin-1-yl)ethyl)-1H-indole-5-carboxylicacid |
| **A138** | 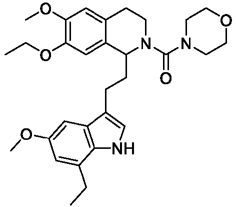 | (7-Ethoxy-1-(2-(7-ethyl-5-methoxy-1H-indol-3-yl)ethyl)-6-methoxy-3,4-dihydroisoquinolin-2 (1H)-yl)(morpholinyl)methanone |
| **A139** | 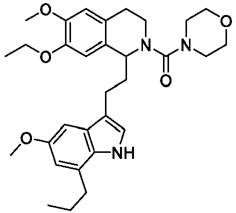 | (7-ethoxy-6-methoxy-1-(2-(5-methoxy-7-propy-1H-indol-3-yl) ethyl)-3,4-dihydroisoquinolin-2 (1H)-yl)(morpholinyl)methanone |
| **A140** | 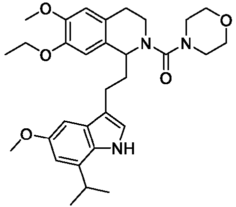 | (7-Ethoxy-1-(2-(7-isopropyl-5-methoxy-1H-indol-3-yl)ethyl)-6-methoxy-3,4-dihydroisoquinolin-2 (1H)-yl)(morpholinyl)methanone |
| **A141** | 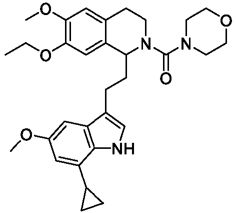 | (1-(2-(7-cyclopropyl-5-methoxy-1H-indol-3-yl)ethyl)-7-ethoxy-6-methoxy-3,4-dihydroisoquinolin-2 (1H)-yl)(morpholinyl)methanone |
| **A142** | 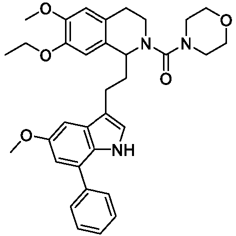 | (7-ethoxy-6-methoxy-1-(2-(5-methoxy-7-phenyl-1H-indol-3-yl) ethyl)-3,4-dihydroisoquinolin-2 (1H)-yl)(morpholinyl)methanone |
| **A143** | 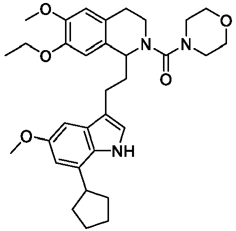 | (1-(2-(7-Cyclopentyl-5-methoxy-1H-indol-3-yl)ethyl)-7-ethoxy-6-methoxy-3,4-dihydroisoquinolin-2 (1H)-yl)(morpholinyl)methanone |
| **A144** | 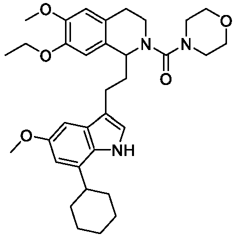 | (1-(2-(7-cyclohexyl-5-methoxy-1H-indol-3-yl)ethyl)-7-ethoxy-6-methoxy-3,4-dihydroisoquinolin-2 (1H)-yl)(morpholinyl)methanone |
| **A145** | 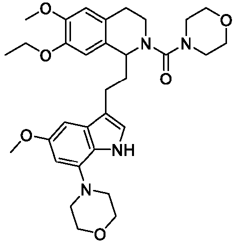 | (7-ethoxy-6-methoxy-1-(2-(5-methoxy-7morpholino-1H-indol-3-yl)ethyl)-3,4-dihydroisoquinolin-2 (1H)-yl)(morpholinyl)methanone |
| **A146** | 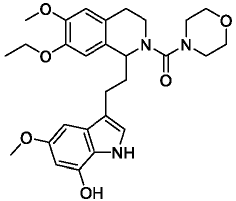 | (7-ethoxy-1-(2-(7-hydroxy-5-methoxy-1H-indol-3-yl)ethyl)-6-methoxy-3,4-dihydroisoquinolin-2 (1H)-yl)(morpholinyl)methanone |
| **A147** | 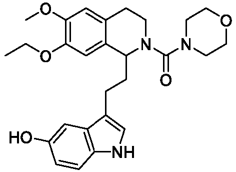 | (7-Ethoxy-1-(2-(5-hydroxy-1H-indol-3-yl)ethyl)-6-methoxy-3,4-dihydroisoquinolin-2(1H)-yl) (morpholinyl)methanone |
| **A148** | 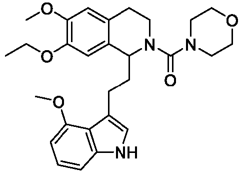 | (7-ethoxy-6-methoxy-1-(2-(4-methoxy-1H-indol-3-yl)ethyl)-3,4-dihydroisoquinolin-2(1H)-yl) (morpholinyl)methanone |
| **A149** | 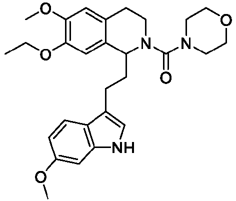 | (7-ethoxy-6-methoxy-1-(2-(6-methoxy-1H-indol-3-yl)ethyl)-3,4-dihydroisoquinolin-2(1H)-yl) (morpholinyl)methanone |
| **A150** | 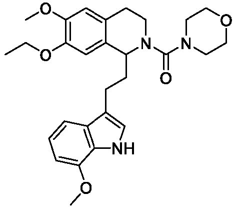 | (7-ethoxy-6-methoxy-1-(2-(7-methoxy-1H-indol-3-yl)ethyl)-3,4-dihydroisoquinolin-2(1H)-yl) (morpholinyl)methanone |
| **A151** | 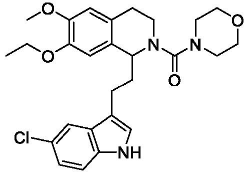 | (1-(2-(5-Chloro-1H-indol-3-yl) ethyl)-7-ethoxy-6-methoxy-3,4-dihydroisoquinolin-2(1H)-yl) (morpholinyl)methanone |
| **A152** | 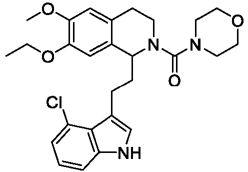 | (1-(2-(4-chloro-1H-indol-3-yl) ethyl)-7-ethoxy-6-methoxy-3,4-dihydroisoquinolin-2(1H)-yl) (morpholinyl)methanone |
| **A153** | 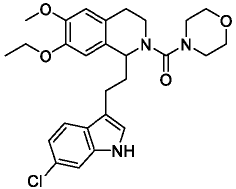 | (1-(2-(6-Chloro-1H-indol-3-yl) ethyl)-7-ethoxy-6-methoxy-3,4-dihydroisoquinolin-2(1H)-yl) (morpholinyl)methanone |
| **A154** | 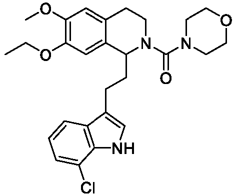 | (1-(2-(7-chloro-1H-indol-3-yl) ethyl)-7-ethoxy-6-methoxy-3,4-dihydroisoquinolin-2(1H)-yl) (morpholinyl)methanone |
| **A155** | 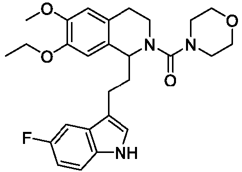 | (7-ethoxy-1-(2-(5-fluoro-1H-indol-3-yl)ethyl)-6-methoxy-3,4-dihydroisoquinolin-2(1H)-yl) (morpholinyl)methanone |
| **A156** | 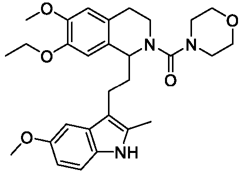 | (7-ethoxy-6-methoxy-1-(2-(5-methoxy-2-methyl-1H-indol-3-yl) ethyl)-3,4-dihydroisoquinolin-2 (1H)-yl)(morpholinyl)methanone |
| **A157** | 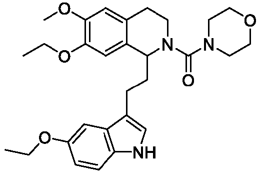 | (7-ethoxy-1-(2-(5-ethoxy-1H-indol-3-yl)ethyl)-6-methoxy-3,4-dihydroisoquinolin-2(1H)-yl) (morpholinyl)methanone |
| **A158** | 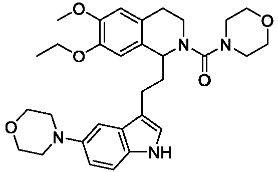 | (7-ethoxy-6-methoxy-1-(2-(5-morpholino-1H-indol-3-yl)ethyl)-3,4-dihydroisoquinolin-2(1H)-yl) (morpholinyl)methanone |
| **A159** | 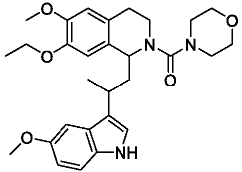 | (7-ethoxy-6-methoxy-1-(2-(5-methoxy-1H-indol-3-yl)propyl)-3,4-dihydroisoquinolin-2(1H)-yl) (morpholinyl)methanone |
| **A160** | 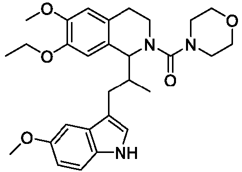 | (7-ethoxy-6-methoxy-1-(1-(5-methoxy-1H-indol-3-yl)propan-2-yl)-3,4-dihydroisoquinolin-2(1H)-yl)(morpholinyl)methanone |
| **A161** | 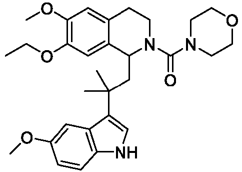 | (7-ethoxy-6-methoxy-1-(2-(5-methoxy-1H-indol-3-yl)-2-methylpropyl)-3,4-dihydroisoquinolin-2(1H)-yl) (morpholinyl)methanone |
| **A162** | 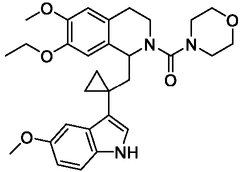 | (7-ethoxy-6-methoxy-1-((1-(5-methoxy-1H-indol-3-yl) cyclopropyl)methyl)-3,4-dihydroisoquinolin-2(1H)-yl) (morpholinyl)methanone |
| **A163** | 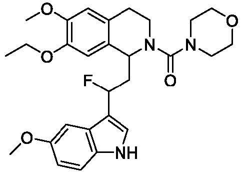 | (7-ethoxy-1-(2-fluoro-2-(5-methoxy-1H-indol-3-yl)ethyl)-6-methoxy-3,4-dihydroisoquinolin-2 (1H)-yl)(morpholinyl)methanone |
| **A164** | 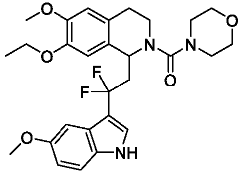 | (1-(2,2-difluoro-2-(5-methoxy-1H-indol-3-yl)ethyl)-7-ethoxy-6-methoxy-3,4-dihydroisoquinolin-2 (1H)-yl)(morpholinyl)methanone |
| **A165** | 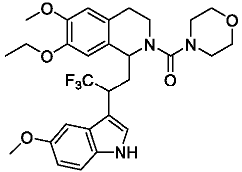 | (7-ethoxy-6-methoxy-1-(3,3,3-trifluoro-2-(5-methoxy-1H-indol-3-yl)propyl)-3,4-dihydroisoquinolin-2(1H)-yl) (morpholinyl)methanone |
| **A166** | 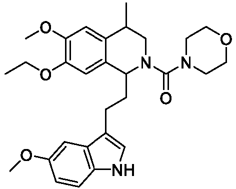 | (7-ethoxy-6-methoxy-1-(2-(5-methoxy-1H-indol-3-yl)ethyl)-4-methyl-3,4-dihydroisoquinolin-2 (1H)-yl)(morpholinyl)methanone |
| **A167** | 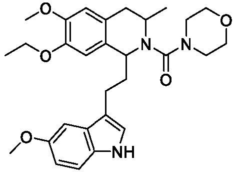 | (7-ethoxy-6-methoxy-1-(2-(5-methoxy-1H-indol-3-yl)ethyl)-3-methyl-3,4-dihydroisoquinolin-2 (1H)-yl)(morpholinyl)methanone |
| **A168** | 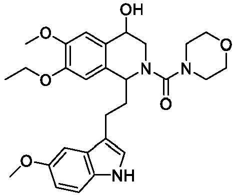 | (7-ethoxy-4-hydroxy-6-methoxy-1-(2-(5-methoxy-1H-indol-3-yl) ethyl)-3,4-dihydroisoquinolin-2 (1H)-yl)(morpholinyl)methanone |
| **A169** | 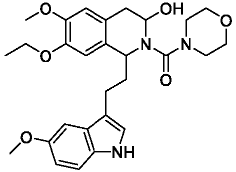 | (7-ethoxy-3-hydroxy-6-methoxy-1-(2-(5-methoxy-1H-indol-3-yl) ethyl)-3,4-dihydroisoquinolin-2 (1H)-yl)(morpholinyl)methanone |
| **A170** | 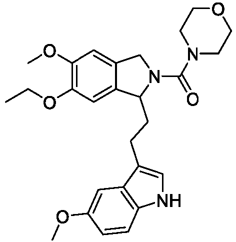 | (6-Ethoxy-5-methoxy-1-(2-(5-methoxy-1H-indol-3-yl)ethyl) isoindol-2-yl) (morpholinyl)methanone |
| **A171** | 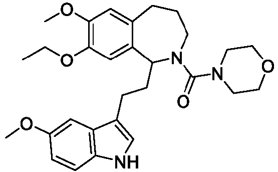 | (8-ethoxy-7-methoxy-1-(2-(5-methoxy-1H-indol-3-yl)ethyl)-1,3,4,5-tetrahydro-2H-benzo[c] aza-2-yl)(morpholinyl)methanone |

9. A method for preparing the compound of formula (I) according to claim 1, wherein the method comprises the steps of:
(1) Reacting the compound of formula II and the compound of formula I_{c} in an inert solvent in the presence of a condensing agent to obtain the compound of formula I_{d}; Preferably, the condensing agent is EDCI (1-ethyl - (3-dimethylaminopropyl) carbodiimide hydrochloride); 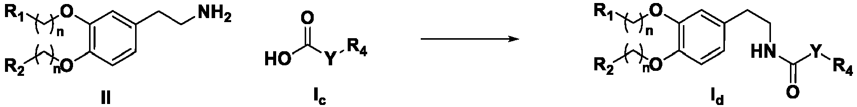
(2) In an inert solvent, the compound of formula I_{d} udgergoes a Bischler Napieralski cyclization reaction to obtain the compound of formula Iₑ; Preferably, the cyclization reaction uses phosphorus oxychloride as the Lewis acid; 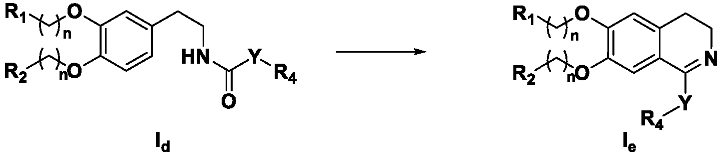
(3) In an inert solvent, the compound of formula Iₑ udgergoes a reduction reaction to obtain the compound of formula I_{f}; Preferably, the reduction reaction uses borohydride as a reducing agent or Noyori catalyst as an asymmetric reduction catalyst; 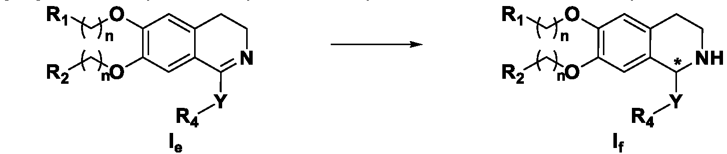
(4) In an inert solvent, the compound of formula I_{f} and 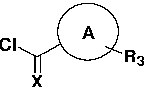 udgergoes a condensation reaction to obtain the compound of formula (I); 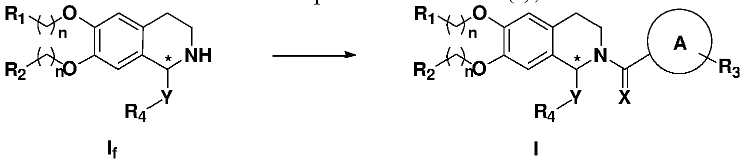
wherein the definitions of the group in each of the above formula are as described in any one of claims 1-8.

10. A pharmaceutical composition, wherein the pharmaceutical composition comprises: a therapeutic effective amount of a compound of formula (I) according to claim 1, or a pharmaceutically acceptable salt thereof, and a pharmaceutically acceptable carrier.

11. A use of a compound of formula (I) according to claim 1in the preparation of a pharmaceutical composition for the treatment of diseases or disorders associated with the activity or expression level of relaxin family peptide receptor 4.

12. The use according to claim 11, wherein the compound is used in the preparation of a pharmaceutical composition for treating diseases or disorders selected from the group consisting of constipation, anorexia, or glucolipid metabolic diseases .
